(19) Europäisches Patentamt
European Patent Office
Office européen des brevets

(11) EP 4 265 621 A1

(12) EUROPEAN PATENT APPLICATION
published in accordance with Art. 153(4) EPC

(43) Date of publication:
25.10.2023 Bulletin 2023/43

(21) Application number: 21908838.2

(22) Date of filing: 27.10.2021

(51) International Patent Classification (IPC):
*C07D 498/04* (2006.01) *C07D 487/04* (2006.01)
*A61K 31/5383* (2006.01) *A61K 31/4985* (2006.01)
*A61K 31/506* (2006.01) *A61P 31/10* (2006.01)

(52) Cooperative Patent Classification (CPC):
**A61K 31/4985; A61K 31/506; A61K 31/5383; A61K 31/5386; A61K 31/542; A61K 31/551; A61K 31/553; A61P 31/10; C07D 471/04; C07D 487/04; C07D 498/04; C07D 498/10; C07D 498/14; C07D 498/16; C07D 513/04;** (Cont.)

(86) International application number:
PCT/CN2021/126634

(87) International publication number:
WO 2022/134837 (30.06.2022 Gazette 2022/26)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 21.12.2020 CN 202011519683
25.05.2021 CN 202110573594

(71) Applicants:
- **Shenzhen Sungening Bio-Medical Co., Ltd.**
  **Shenzhen, Guangdong 518000 (CN)**
- **Chengdu Hyperway Pharmaceuticals Co., Ltd.**
  **Chengdu, Sichuan 610041 (CN)**
- **Shenzhen Hyperway Pharmaceuticals Co., Ltd.**
  **Shenzhen, Guangdong 518116 (CN)**

(72) Inventors:
- **HUANG, Shenghong**
  **Shenzhen, Guangdong 518000 (CN)**
- **YAO, Renchong**
  **Shenzhen, Guangdong 518000 (CN)**
- **SONG, Zhiquan**
  **Shenzhen, Guangdong 518000 (CN)**
- **YI, Shoubing**
  **Chengdu, Sichuan 610000 (CN)**
- **YUAN, Chenguang**
  **Shenzhen, Guangdong 518000 (CN)**
- **LI, Yingfu**
  **Chengdu, Sichuan 610000 (CN)**

(74) Representative: **Ipey**
**Apex House**
**Thomas Street**
**Trethomas**
**Caerphilly CF83 8DP (GB)**

(54) **PYRROLE DERIVATIVE, AND PREPARATION METHOD AND USE THEREFOR**

(57) The invention discloses a series of pyrrole derivatives and their preparation method and therapeutic use. This type of compounds can be used as DHODH-specific inhibitors that have significant inhibitory and blocking effects on fungal DHODH. The compounds can be used for treatment of diseases caused by fungal infections. They can also be used to prepare agricultural fungicides. The anti-fungal activity of the compounds thus hold great promise in the future.

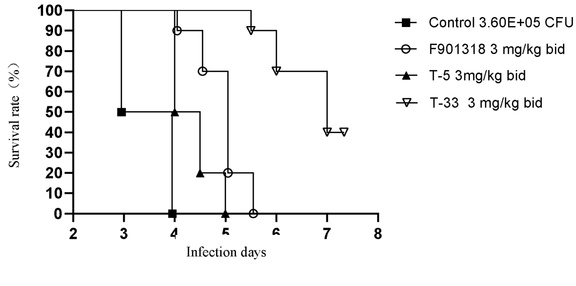


Figure 1

EP 4 265 621 A1

(52) Cooperative Patent Classification (CPC): (Cont.)
**C07D 519/00;** Y02P 20/55

## Description

### Technical Field

The present invention relates to the field of pharmaceutical technology, in particular to a compound used as an inhibitor of fungal dihydroorotate dehydrogenase (DHODH) and to the method of preparation and the use thereof.

### Background Technology

DHODH catalyzes the step 4 reaction in de novo synthesis route of pyrimidine nucleotides, which is a flavin-dependent mitochondrial enzyme. Pyrimidines are the key components of RNA biosynthesis and DNA biosynthesis, and DHODH-inhibitors are designed to inhibit cell growth by inhibiting DHODH activity and thereby depleting intracellular pyrimidine nucleotides.

Many clinical evidence to confirm that inhibitors targeting human dihydroorotic dehydrogenase (hDHODH) can be used to treat diseases including cancer, autoimmune diseases, and inflammation, etc. Inhibitors of dihydroorotic dehydrogenase targeting viruses or fungi can be used to treat diseases associated with viral or fungal infections, respectively.

Invasive fungal infections, known as hidden killers, are a major cause of eventual death in patients with tumors, AIDS, organ transplants, etc. Currently, the use of many broad-spectrum antibiotics and other powerful drugs worldwide has led to the increasing patients with fungal infections. Strains of fungi that cause fungal infections include Aspergillus, Sporozoites, etc. Therapies for drug-resistant infections and infections caused by refractory pathogens are still lacking. The number of patients with pulmonary aspergillosis is about 3 million worldwide and about 240,000 of the patients are in Europe. For patients without timely diagnosis and long-term antifungal therapy, the 5-year mortality rate is as high as nearly 80%.

Because it is difficult to find a drug target compatible with the human body, there are currently only four broad classes of antifungal drugs available for use in humans: 1) polyenes (e.g., amphotericin B): binding to ergosterol (a fungal membrane component) and the target is similar to cholesterol in mammalian cells; 2) azoles (e.g., itraconazole): inhibiting ergosterol synthesis via cytochrome p450, which also presents in mammalian cells; 3) allylamines (e.g. terbinafine): inhibiting ergosterol synthesis via the fungus-specific enzyme squalene epoxidase; 4) echinocandins (e.g. micafungin): targeting β-glucan synthesis, specific to the fungal cell wall, with fewer side effects.

Which further complicating the treatment of fungal infections is the resistance to all four classes of antifungal drugs in different fungal pathogens, and has prompted researchers to design new anti-infective strategies and drugs. Therefore, further development of a more efficient and safer class of DHODH-inhibitors with fewer side effects is necessary and of great importance.

### Content of the Invention

The main technical problem addressed by the present invention is to provide a compound that can effectively inhibit fungi.

To solve the above technical problem, one of the technical solutions used in the present invention is:

The present invention provides a compound having the structure shown in formula I or its tautomer, mesomer, racemate, enantiomer, diastereoisomer or its mixture, and pharmaceutically acceptable hydrate, solvate or salt:

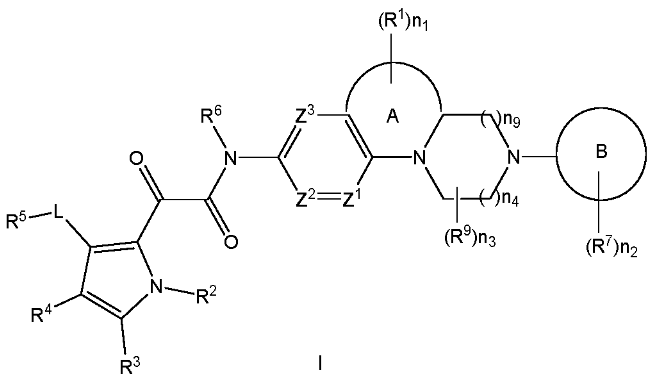

I

Ring A is a 5- to 10-membered ring, and the 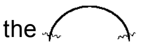 in ring A is selected from alkyl, heteroalkyl, acyl, ester, acylamino, sulfonyl, sulfonamido, sulfinyl, and sulfinamido group;

Ring B is selected from substituted or non-substituted aryl, substituted or non-substituted heteroaryl, substituted or non-substituted cycloalkyl, substituted or non-substituted heterocycloalkyl;

Each occurrence of $R^1$ is independently selected from hydrogen, halogen, cyano group, =O, hydroxyl, $-NH_2$, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted aryl, substituted or non-substituted heteroaryl, ester, acyl, acylamino, sulfinyl, sulfonyl, sulfonamido, sulfinamido group, or any two $R^1$s and their connected atoms form substituted or non-substituted 3- to 12-membered cycloalkyl, substituted or non-substituted 3- to 12-membered heteroalkyl;

The stated any two $R^1$s include two $R^1$s at two adjacent substitution sites, or two $R^1$s at non-adjacent substitution sites, or two $R^1$s in the same substitution site; the rest of the analogous cases are the same.

$R^2$, $R^3$, and $R^4$ are independently selected from hydrogen, halogen, nitro, cyano group, hydroxyl, $-NH_2$, substituted or non-substituted C1 to C10 alkyl, substituted or non-substituted 2 to 10-membered heteroalkyl, substituted or non-substituted 3 to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester, acylamino, sulfonyl, sulfonamido, boronic acid group, boronic acid ester group, phosphoryl, or $R^2$ and $R^3$ together form substituted or non-substituted substituted C1 to C6 alkyl, substituted or non-substituted 3- to 10-membered heteroalkyl, or $R^2$, $R^3$ and their connected atoms form substituted or non-substituted heteroaromatic ring;

$R^5$ is selected from substituted or non-substituted C1 to C10 alkyl, substituted or non-substituted 2 to 10-membered heteroalkyl, substituted or non-substituted 3- to 12-membered cycloalkyl, substituted or non-substituted 3- to 12-membered heterocycloalkyl, substituted or non-substituted aryl, and substituted or non-substituted heteroaryl;

$R^6$ is selected from hydrogen, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, and substituted or non-substituted alkenyl;

Each occurrence of $R^7$ is independently selected from hydrogen, halogen, cyano group, =O, hydroxyl, $-NH_2$, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted aryl, substituted or non-substituted heteroaryl, acyl, ester, acylamino, sulfonyl, sulfonamido, boronic acid group, and boronic acid ester group, or any two $R^7$s and their connected atoms form substituted or non-substituted 3- to 12-membered hydrocarbon rings, substituted or non-substituted 3- to 12-membered heterohydrocarbon rings;

Each occurrence of $R^9$ is independently selected from hydrogen, halogen, nitro, cyano group, hydroxy, $-NH_2$, =O, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester, acylamino, sulfonyl, sulfonamido, boronic acid group, boronic acid ester group, and phosphoryl, or any two $R^9$s and their connected atoms form substituted or non-substituted hydrocarbon ring, and substituted or non-substituted heterohydrocarbon ring;

$Z^1$, $Z^2$, and $Z^3$ are independently selected from N or $CR^8$;

Each occurrence of $R^8$ is independently selected from hydrogen, halogen, nitro, cyano group, hydroxyl, $-NH_2$, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester, acylamino, sulfonyl, sulfonamido, boronic, boronic ester, phosphoryl, or any two $R^8$ and their connected atoms forming a substituted or non-substituted 5- to 9-membered alkenyl rings, substituted or non-substituted 5- to 9-membered heteroalkenyl rings, substituted or non-substituted benzene ring, substituted or non-substituted 5- to 9-membered heteroaromatic rings;

Or adjacent $R^8$ and $R^9$ together form substituted or non-substituted C1 to C6 alkyl group, substituted or non-substituted 2- to 6-membered heteroalkyl, substituted or non-substituted acyl, substituted or non-substituted ester, substituted or non-substituted acylamino, substituted or non-substituted sulfonyl, substituted or non-substituted sulfonamido, substituted or non-substituted sulfinyl, substituted or non-substituted sulfinamido group.

$n_1$, $n_2$, and $n_3$ are independently selected from 0, 1, 2, 3, 4, 5, and 6; $n_4$ and $n_9$ are independently selected from 1, 2, and 3;

L is selected from one bond, O, S, S(=O), $S(=O)_2$, C(=O), $NR^{10}$, $-(CR^{11}R^{12})n_5-$, $-S(=O)n_6NR^{13}-$, $-NR^{14}S(=O)n7NR^{15}-$, $-C(=O)NR^{16}-$, $-NR^{17}C(=O)NR^{18}-$, C2 to C4 alkenylidene, and C2 to C4 alkynylidene;

$n_5$ is selected from 1, 2, 3, and 4; $n_6$ and $n_7$ are independently selected from 0, 1, and 2;

$R^{10}$, $R^{13}$, $R^{14}$, $R^{15}$, $R^{16}$, $R^{17}$, and $R^{18}$ are independently selected from hydrogen, acyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, and substituted or non-substituted alkenyl;

Or each occurrence of $R^{11}$ and $R^{12}$ are independently selected from hydrogen, halogen, hydroxyl, and C1 to C10 alkyl;

Wherein, the substituents of $R^1$, $R^2$, $R^3$, $R^4$, $R^5$, $R^6$, $R^7$, $R^8$, $R^9$, $R^{10}$, $R^{13}$, $R^{14}$, $R^{15}$, $R^{16}$, $R^{17}$, and $R^{18}$ are independently selected from halogen, alkyl, cycloalkyl, heteroalkyl, heterocycloalkyl, alkenyl, alkynyl, hydroxyl, cyano group, $-NH_2$, nitro, carboxyl, acyl, ester, acylamino, aryl, heteroaryl, sulfinyl, sulfonyl, sulfonamido, sulfinamido group.

The phrase "each occurrence of $R^1$ is independently selected from" in the present invention means that different $R^1$s can be selected from the same or different groups when $n_1$, which limits the number of $R^1$, is greater than 1. For example, when $n_1$ is 2, one $R_5$ may be selected from substituted or non-substituted alkyl group and the other $R^1$ may be selected from halogen; or, when $n_1$ is 2, the two $R^1$s may be selected from substituted or non-substituted alkyl group; the rest of the analogous cases are the same.

The unfixed position of $R^9$ in

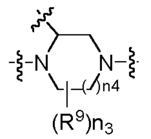

means that $R^9$ can be at any substitutable positions in the ring structure in which it is located; the same goes for the rest of the substituents whose positions are not fixed.

The sentence that $R^2$ and $R^3$ together form substituted or non-substituted C1 to C6 alkyl means that, the two substituents, $R^2$ and $R^3$, are connected and together form alkyl having two connected ends, such as $-CH_2CH_2CH_2-$ and $-CH_2CH_2CH_2CH_2-$,etc, and the rest similar cases are the same.

The phrase "adjacent $R^8$ and $R^9$" refers to the closest spatially located $R^8$ and $R^9$ in the compounds of the present invention, i.e.,

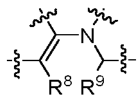

.

Further, $Z^2$ is $CR^8$, and $Z^1$ and $Z^3$ are independently selected from N or $CR^8$.

Further, $Z^2$ and $Z^3$ are $CR^8$, and $Z^1$ is selected from N or $CR^8$.

Further, ring A is substituted or non-substituted 5- to 7-membered rings, and the heteroatoms in ring A are selected from O, N, and S.

Further, each occurrence of $R^1$ is independently selected from hydrogen, halogen, cyano group, =O, hydroxyl, $-NH_2$, carboxyl, substituted or non-substituted C1 to C6 alkyl, substituted or non-substituted 2- to 7-membered heteroalkyl, substituted or non-substituted 3-to 9-membered cycloalkyl, substituted or non-substituted 3- to 9-membered heterocycloalkyl, substituted or non-substituted C2 to C6 alkenyl, substituted or non-substituted phenyl, substituted or non-substituted 5- to 10-membered heteroaryl, ester, acyl, acylamino, sulfinyl, sulfonyl, sulfonamido, sulfinamido group, or any two $R^1$s and their connected atoms form substituted or non-substituted 3- to 6-membered hydrocarbon rings, wherein the substituents of $R^1$ are selected from halogen, alkyl, cycloalkyl , heteroalkyl, heterocycloalkyl, alkenyl, alkynyl, hydroxy, cyano group, amino, carboxyl, acyl, ester, acylamino, aryl, and heteroaryl;

Further, each occurrence of $R^1$ is independently selected from hydrogen, halogen, cyano group, =O, substituted or non-substituted C1 to C6 alkyl, substituted or non-substituted 2- to 7-membered heteroalkyl, substituted or non-substituted 3- to 9-membered cycloalkyl, substituted or non-substituted 3- to 9-membered heterocycloalkyl, or two $R^1$s in the same substitution site and their connected atoms form substituted or non-substituted 3- to 6-membered cyclic hydrocarbon rings, wherein the substituents of $R^1$ are selected from halogen, alkyl, cycloalkyl, heteroalkyl, heterocycloalkyl, hydroxyl, cyano group, and amino.

Further, each occurrence of $R^1$ is independently selected from hydrogen, halogen, =O, substituted or non-substituted C1 to C4 alkyl, substituted or non-substituted 2- to 5-membered alkoxies, substituted or non-substituted 3- to 6-membered cycloalkyl, substituted or non-substituted 3- to 6-membered heterocycloalkyl, or two $R^1$s in the same substitution site and their connected atoms form substituted or non-substituted 3- to 6-membered cycloalkyl, wherein the substituents of $R^1$ are selected from halogen, alkyl, cycloalkyl, heteroalkyl, heterocycloalkyl, hydroxyl, cyano group, and amino.

Further, each occurrence of $R^1$ is independently selected from hydrogen, halogen, =O, C1 to C4 alkyl substituted or non-substituted by halogen, and 3- to 6-membered cycloalkyl substituted or non-substituted by halogen, or two $R^1$s at the same substitution site and their connected atoms form substituted or non-substituted 3- to 6-membered cycloalkyl.

Further, each occurrence of $R^1$ is independently selected from hydrogen, F, Cl, =O, C1 to C4 alkyl substituted or non-substituted by F, cyclopropyl substituted or non-substituted by halogen, or two $R^1$ at the same substitution site and their connected atoms form substituted or non-substituted cyclopropyl.

The sentence that two $R^1$s at the same substitution site and their connected atoms form substituted or non-substituted cyclopropyl refers to

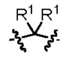

forms substituted or non-substituted

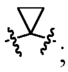

the same goes for the rest.

Further, $n_1$ is selected from 0, 1, 2, 3, and 4; further selected from 0, 1, and 2.

Further, the compounds stated in the present invention have the structure shown in Formula II or its tautomer, mesomer, racemate, enantiomer, diastereoisomer or its mixture forms thereof, pharmaceutically acceptable hydrate, solvate or salt:

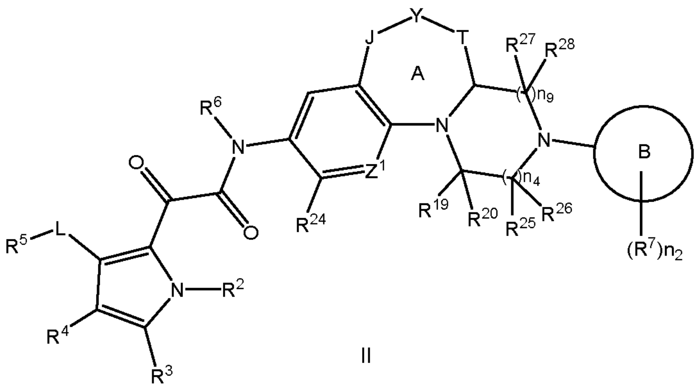

II

J, Y, and T are independently selected from one bond, O, S, S(=O), $S(=O)_2$, $CR^{21}R^{22}$, C=O, and $NR^{23}$;

$R^{21}$, $R^{22}$, $R^{23}$, and $R^{24}$ are independently selected from hydrogen, halogen, hydroxyl, substituted or non-substituted C1 to C4 alkyl, substituted or non-substituted C1 to C4 alkenyl, or $R^{21}$, $R^{22}$ and their connected atoms form substituted or non-substituted 3- to 6-membered cycloalkyl; the substituents in $R^{21}$, $R^{22}$, $R^{23}$, and $R^{24}$ are independently selected from halogen, hydroxyl, $-NH_2$, ester, acyl, C1- to C4 alkyl, and 3- to 6-membered cycloalkyl;

$Z^1$ is selected from N or $CR^8$;

$R^8$ is selected from hydrogen, halogen, nitro, cyano group, hydroxyl, $-NH_2$, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted phenyl, substituted or non-substituted 5- to 9-membered heteroaryl, acyl, ester, acylamino, sulfonyl, sulfonamido, boronic acid group, boronic acid ester group, and phosphoryl, or $R^8$, $R^{24}$ and their connected atoms form substituted or non-substituted 5- to 7-membered alkenyl rings, substituted or non-substituted 5- to 7-membered heteroarene rings, substituted or non-substituted benzene ring, substituted or non-substituted 5- to 7-membered heteroaromatic rings;

$R^{19}$ and $R^{20}$ are independently selected from hydrogen, halogen, cyano group, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, acyl, ester, acylamino, sulfonyl, sulfonamido, or $R^{19}$ and $R^{20}$ together form = O, or $R^{19}$, $R^{20}$ and their the connected atoms form substituted or non-substituted 3- to 6-membered cycloalkyl;

Or $R^8$ and $R^{19}$ together form substituted or non-substituted C1 to C6 alkyl, substituted or non-substituted 2- to 6-membered heteroalkyl, substituted or non-substituted acyl, substituted or non-substituted ester, substituted or non-substituted acylamino, substituted or non-substituted sulfonyl, substituted or non-substituted sulfonamido, substituted or non-substituted sulfinyl, substituted or non-substituted sulfinamido group;

Each occurrence of $R^{25}$, $R^{26}$, $R^{27}$, and $R^{28}$ are independently selected from hydrogen, halogen, cyano group, hydroxyl, $-NH_2$, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, acyl, ester, acylamino, sulfonyl, and sulfonamido, or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;

The substituents in $R^8$, $R^{19}$, $R^{20}$, $R^{25}$, $R^{26}$, $R^{27}$, and $R^{28}$ are independently selected from halogen, C1 to C10 alkyl, 3- to 12-membered cycloalkyl, 2- to 10-membered heteroalkyl, 3-to 6-membered heterocycloalkyl, C2 to C10 alkenyl, C2 to C10 alkynyl, hydroxyl, cyano group, $-NH_2$, carboxyl, acyl, ester, acylamino, phenyl, 5- to 9-membered heteroaryl, sulfinyl, sulfonyl, sulfonamido, and sulfinamido group;

Further, $R^{21}$ and $R^{22}$ are independently selected from hydrogen, halogen, hydroxyl, and C1 to C4 alkyl substituted or non-substituted by halogen, or $R^{21}$, $R^{22}$ and their connected atoms form substituted or non-substituted cyclopropyl;

$R^{23}$ is selected from hydrogen and C1 to C4 alkyl.

Further, J is selected from O, S, S(=O), $S(=O)_2$, $CH_2$, CHF, $CF_2$, CHOH, C=O, $NR^{23}$, and

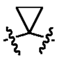;

Y is selected from one bond, O, S, S(=O), $S(=O)_2$, $CH_2$, CHF, $CF_2$, C=O, and $NR^{23}$; T is selected from one bond, S(=O), $S(=O)_2$, $CH_2$, CHF, $CF_2$, C=O, and

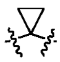;

Preferably, J is selected from O, $CH_2$, $CF_2$, CHF, CHOH, C=O, NH, and

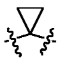;

Y is selected from one bond, O, $CH_2$, C=O, and NH; and T is selected from one bond, $CH_2$, C=O, and

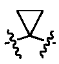.

Further, ring A is selected from substituted or non-substituted groups as follows.

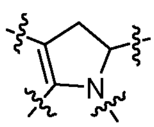, 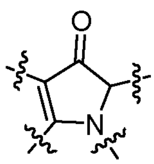, , 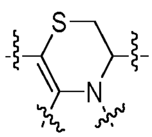, 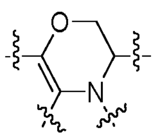, 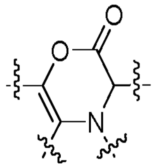, 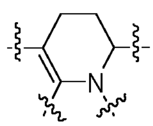,

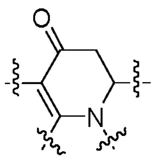 、 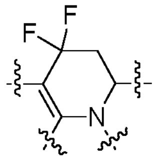 、 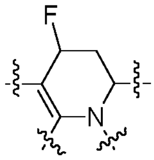 、 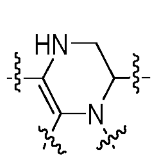 、 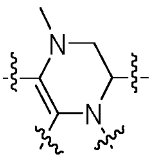 、 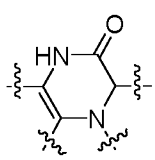 、

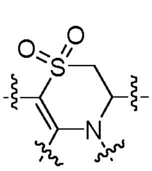 、 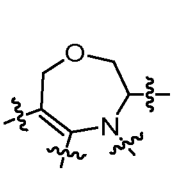 、 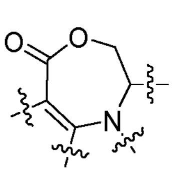 、 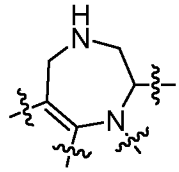 、 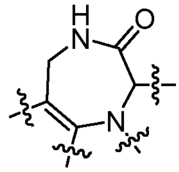 、 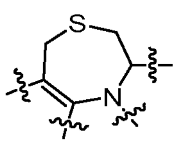

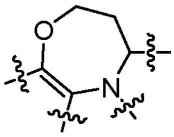 、 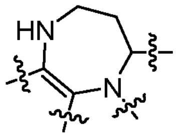 、 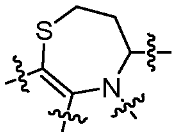 、 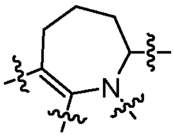 ;

Further, ring A is selected from substituted or non-substituted groups as follows:

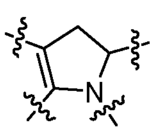 、 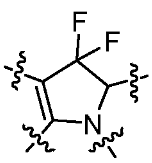 、 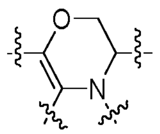 、 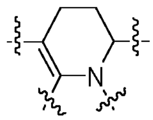 、 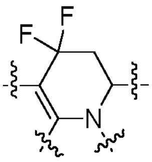 、 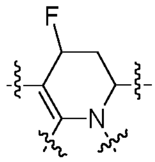 、

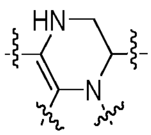 、 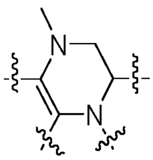 、 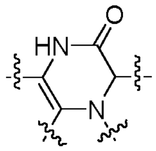 、 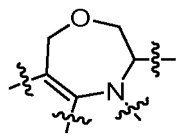 、 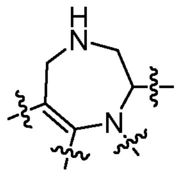 、 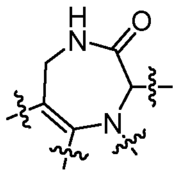 、

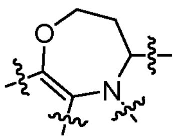 、 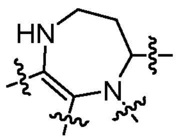 、 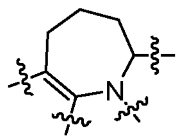 ;

In the specific embodiments of the present invention, ring A is selected from the following substituted or non-substituted groups:

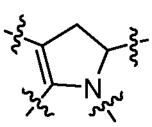 , 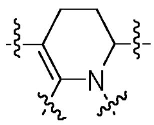 , 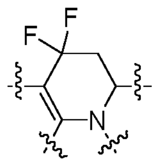 ,

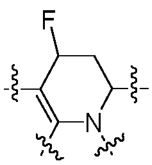 , 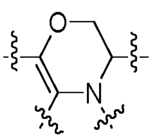 , 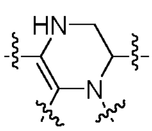 , 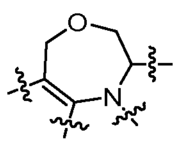 , 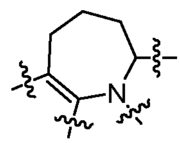 .

Further, ring A is selected from substituted or non-substituted groups as follows:

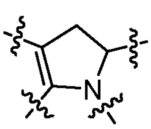 , 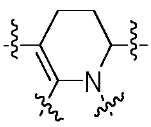 , 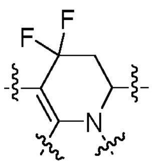 , 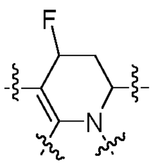 , 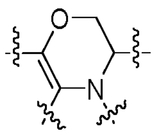 , 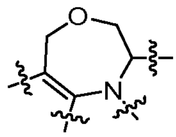 .

Further, L is selected from one-bond, S, S(=O), $S(=O)_2$, C(=O), $-(CR^{11}R^{12})$ ns-, -C(=O) $NR^{16}$-, C2 to C4 alkynylenes; Further, L is selected from the one-bond and C2 to C4 alkynylenes.

In the specific embodiments of the present invention, L is selected from one bond.

Further, the compounds stated in the present invention have the structure shown in formula III or formula IV or their tautomers, mesomers, racemates, enantiomers, diastereoisomers or mixture forms thereof, and pharmaceutically acceptable hydrates, solvates or salts:

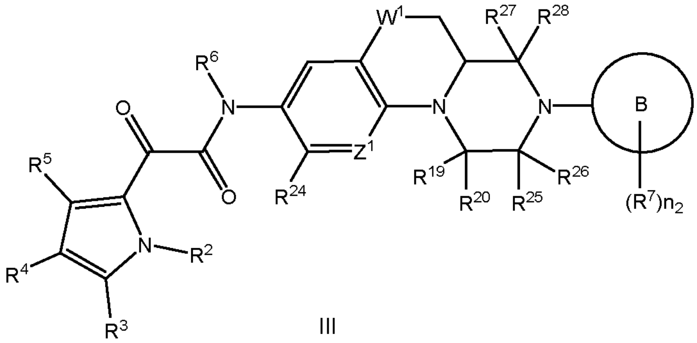

III

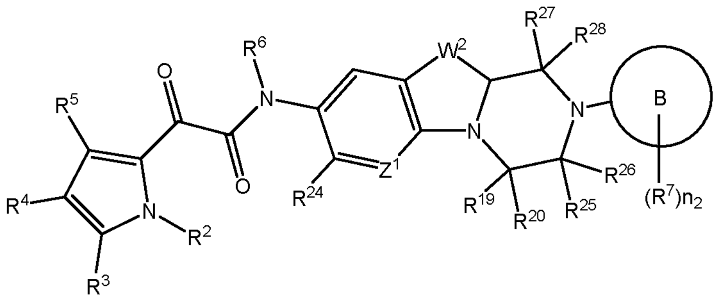

IV

$W^1$ and $W^2$ are independently selected from O, $CH_2$, CHF, $CF_2$, NH, and $NCH_3$, preferably O, $CF_2$, and $CH_2$;
$Z^1$ is selected from N or $CR^8$;
$R^8$ is selected from hydrogen, halogen, cyano group, hydroxyl, $-NH_2$, substituted or non-substituted C1 to C4 alkyl, substituted or non-substituted 2- to 5-membered alkoxies, substituted or non-substituted 2- to 5-membered azacycloalkyl, substituted or non-substituted 3- to 6-membered cycloalkyl, substituted or non-substituted C2 to C4 alkenyl, acyl, ester, acylamino, boronic acid group, or boronic acid ester group, or $R^8$and $R^{19}$ together form substituted or non-substituted C1 to C2 alkyl, substituted or non-substituted 2-membered heteroalkyl, and substituted or non-substituted ester;
Further, $R^8$ is selected from H, F, Cl, cyano group, -OH, $-N(CH_3)_2$, $-N(CH_2CH_3)_2$, - $C(=O)CH_3$, $-C(=O)CH_2CH_3$, $-C(=O)OCH_3$, $-C(=O)OCH_2CH_3$, $-B(OH)_2$,

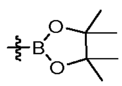 ,

$-CH_3$ , - $CH_2CH_3$, $-CH(CH_3)_2$,

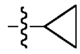,

$-CH_2OH$, $-OCH_3$, $-OCH_2CH_3$, $-CF_3$, $-CHF_2$, and $-CF_2CF_3$, or $R^8$ and $R^{19}$ together form substituted or non-substituted groups as follows:

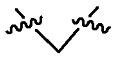, 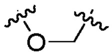,

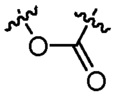.

Further, $R^8$ is selected from H, F, -OH, and $-B(OH)_2$, or $R^8$ and $R^{19}$ and their connected atoms form the following groups:

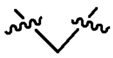, 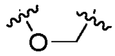, 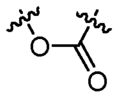.

In specific embodiments of the present invention, $R^8$ is selected from Hand F.

In the specific embodiments of the present invention, $R^{24}$ is selected from hydrogen and halogen, preferably H, F, and Cl; more preferably H.

Further, each occurrence of $R^{25}$, $R^{26}$, $R^{27}$, and $R^{28}$ are independently selected from hydrogen, halogen, cyano group, hydroxyl, $-NH_2$, carboxyl, substituted or non-substituted C1 to C6 alkyl, substituted or non-substituted 2- to 6-membered heteroalkyl, substituted or non-substituted 3- to 6-membered cycloalkyl, substituted or non-substituted 3- to 6-membered heterocycloalkyl, substituted or non-substituted C2 to C6 alkenyl, acyl, ester, acylamino, sulfonyl, sulfonamido, or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl.

Further, each occurrence of $R^{25}$, $R^{26}$, $R^{27}$, and $R^{28}$ are independently selected from hydrogen, halogen, substituted or non-substituted C1 to C6 alkyl, substituted or non-substituted 2- to 6-membered heteroalkyl, substituted or non-substituted 3- to 6-membered cycloalkyl, 3- to 6-membered heterocycloalkyl, acyl, and ester, or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;

The substituents in $R^{25}$, $R^{26}$, $R^{27}$, and $R^{28}$ are independently selected from halogen, C1 to C6 alkyl, 3- to 6-membered cycloalkyl, 2- to 6-membered heteroalkyl, 3- to 6-membered heterocycloalkyl, hydroxyl, cyano group, $-NH_2$, carboxyl, acyl, ester group, acylamino group, sulfinyl group, sulfonyl group, sulfonamido, and sulfinamido group.

Further, each occurrence of $R^{25}$ and $R^{27}$ are independently selected from hydrogen, halogen, substituted or non-substituted C1 to C6 alkyl, substituted or non-substituted 2- to 6-membered heteroalkyl, substituted or non-substituted 3- to 6-membered cycloalkyl, acyl, and ester group; $R^{26}$ and $R^{28}$ are H;

Or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;

Further, $R^{25}$, $R^{26}$, $R^{27}$, and $R^{28}$ are H.

Further, $R^{19}$ is selected from hydrogen, halogen, cyano group, substituted or non-substituted C1 to C6 alkyl, substituted or non-substituted 2- to 7-membered heteroalkyl, acyl, and ester; $R^{20}$ is H, or $R^{19}$ and $R^{20}$ together form = O, or $R^{19}$, $R^{20}$ and their connected atoms form substituted or non-substituted 3- to 6-membered cycloalkyl; or $R^{19}$ and $R^8$ together form any of the groups which $R^8$ and $R^{19}$ described in the preceding paragraph together form.

Further, $R^{19}$ and $R^{20}$ are H, or $R^{19}$ and $R^{20}$ together form =O, or $R^{19}$, $R^{20}$ and their connected atoms form substituted or non-substituted cyclopropyl; or $R^{19}$ and $R^8$ together form any of the groups which $R^8$ and $R^{19}$ described in the preceding paragraph together form.

Further, $R^{19}$ and $R^{20}$ are both H.

Further, ring B is selected from phenyl, naphthyl, substituted or non-substituted 5- to 10-membered heteroaryl, substituted or non-substituted 3- to 9-membered cycloalkyl, and substituted or non-substituted 3- to 9-membered heterocycloalkyl.

Further, ring B is selected from substituted or non-substituted 5- to 6-membered heteroaryl.

Further, ring B is selected from substituted or non-substituted groups as follows: phenyl, pyrimidinyl, pyridinyl, pyrazinyl, pyridazinyl, thienyl, pyrazolyl, imidazolyl, oxazolyl, thiazolyl, triazolyl, tetrazolyl, thiadiazolyl, and oxadiazolyl.

In the specific embodiments of the present invention, ring B is selected from pyrimidinyl, pyridyl, pyridazinyl, thiazolyl, triazolyl, oxadiazolyl; preferably pyrimidinyl, pyridyl, pyridazinyl, thiazolyl; more preferably pyrimidinyl.

Further, each occurrence of $R^7$ is selected from hydrogen, halogen, cyano group, hydroxyl, substituted or non-substituted C1 to C6 alkyl, substituted or non-substituted 2- to 7-membered heteroalkyl, substituted or non-substituted 3- to 6-membered cycloalkyl, substituted or non-substituted 3- to 6-membered heterocycloalkyl, acyl, ester, acylamino, sulfonyl, and sulfonamido, or two $R^7$s adjacent and their connected atoms form substituted or non-substituted 3- to 6-membered hydrocarbon rings, substituted or non-substituted 3- to 6-membered heterocyclic rings; the heteroatoms of heteroalkyl, heterocycloalkyl, heteroaryl and heterocyclic ring which are stated in $R^7$ are selected from one or more of N, O, and S; further, each occurrence of $R^7$ is independently selected from hydrogen, halogen, cyano group, substituted or non-substituted C1 to C6 alkyl, substituted or non-substituted 2 to 7-membered oxyalkyl, substituted or non-substituted 2 to 7-membered azacycloalkyl, substituted or non-substituted 3 to 6-membered cycloalkyl, substituted or non-substituted 3 to 6-membered heterocycloalkyl, and sulfonamido group, or two $R^7$s adjacent and their connected atoms form substituted or non-substituted 3 to 6-membered heteroalkyl rings, The substituents in $R^7$ are selected from halogen, C1 to C6 alkyl, 3 to 6-membered cycloalkyl, 2 to 7-membered heteroalkyl, 3 to 6-membered heterocycloalkyl, hydroxyl, cyano group, $-NH_2$, acyl, ester, acylamino, sulfinyl, sulfonyl, sulfonamido, and sulfinamido; $n_2$ is selected from 0, 1, 2, 3, 4, and 5.

Further, each occurrence of $R^7$ is independently selected from hydrogen, halogen, cyano group, -OH, $-N(CH_3)_2$, $-N(CH_2CH_3)_2$, $-S(=O)_2NH_2$, $-CH_3$, $-CH_2CH_3$, $-CH(CH_3)_2$,

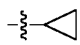,

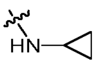 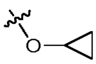,

morpholinyl, piperidinyl, piperazinyl, azetidinyl, pyrrolidinyl, $-(CH_2)n_8-OH$, - $CH(OH)-CH_2OH$, $-C(CH_3)_2OH$, $-OCH_2CH(OH)-CH_2OH$, $-OCH_2CH_2OCH_3$, - $OCH_2CH_2OCH_2CH_3$, $-OCH_3$, $-OCH_2CH_3$, $-OCH(CH_3)_2$, $-CF_3$, $-CHF_2$, and $-CF_2CF_3$, or two $R^7$s adjacent and their connected atoms substituted or non-substituted 3- to 6-membered azahydrocarbon rings, 3- to 6-membered oxyhydrocarbon rings, $n_8$ is selected from 1, 2, 3, and 4; $n_2$ is selected from 0, 1, 2, and 3.

Further, each occurrence of $R^7$ is independently selected from F, Cl, cyano group, -OH, - $N(CH_3)_2$, $-S(=O)_2NH_2$, $-CH_3$,

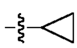, 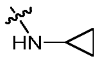, 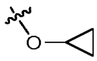,

morpholinyl, - $CH(OH)-CH_2OH$, - $C(CH_3)2OH$, $-OCH_3$, and $-CF_3$, or two $R^7$s adjacent and their connected atoms form substituted or non-substituted

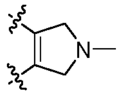;

the substituents in $R^7$ are selected from halogen, C1 to C6 alkyl, 3- to 6-membered cycloalkyl, 2- to 6-membered heteroalkyl, 3- to 6-membered heterocycloalkyl, hydroxyl, cyano group, $-NH_2$, acyl, and ester.

Further, each occurrence of $R^7$ is independently selected from F, $-OCH_3$, $-CF_3$, $-CH_3$, - OH, cyano; further, $n_2$ is 1.

Further, $R^5$ is selected from substituted or non-substituted C1 to C4 alkyl, substituted or non-substituted 2- to 6-membered heteroalkyl, substituted or non-substituted 3- to 6-membered cycloalkyl, substituted or non-substituted 3- to 6-membered heterocycloalkyl, substituted or non-substituted phenyl, and substituted or non-substituted 5- to 6-membered heteroaryl; the substituents in $R^5$ are selected from halogen, C1~ C6 alkyl, 3- to 6-membered cycloalkyl, 2- to 6-membered heteroalkyl, 3- to 6-membered heterocycloalkyl, hydroxyl, cyano group, amino, acyl, ester, acylamino, sulfinyl,

sulfonyl, sulfonamido, and sulfinamido group.

Further, $R^5$ is selected from substituted or non-substituted tetrahydropyranyl, substituted or non-substituted C1 to C4 alkyl, substituted or non-substituted phenyl, substituted or non-substituted thienyl, substituted or non-substituted pyridinyl, substituted or non-substituted pyrimidinyl, preferably substituted or non-substituted phenyl, the substituents in $R^5$ are selected from halogen or C1~C6 alkyl; further, the substituents in $R^5$ are selected from F, Cl, and C1 to C4 alkyl.

Further, $R^2$ and $R^3$are independently selected from hydrogen, halogen, cyano group, hydroxyl, $-NH_2$, substituted or non-substituted C1 to C6 alkyl, substituted or non-substituted 2- to 6-membered heteroalkyl, substituted or non-substituted 3- to 6-membered cycloalkyl, substituted or non-substituted 3- to 6-membered heterocycloalkyl, substituted or non-substituted C2 to C6 alkenyl, acyl, ester, acylamino, sulfonyl, and sulfonamido group, or $R^2$ and $R^3$ together form substituted or non-substituted C3 to C4 alkyl, substituted or non-substituted 3- to 4-membered heteroalkyl; the substituents in $R^2$ and $R^3$ are independently selected from halogen, C1 to C6 alkyl, 3- to 6-membered cycloalkyl, 2- to 6-membered heteroalkyl, 3- to 6-membered heterocycloalkyl, C2 to C4 alkenyl, C2 to C4 alkynyl, hydroxyl, cyano group, $-NH_2$, nitro, carboxyl, acyl, ester, acylamino, sulfinyl, sulfonyl, sulfonamido, and sulfinamido group;

$R^4$ is selected from hydrogen, C1 to C4 alkyl, C1 to C4 haloalkyl, C1 to C4 alkoxy, and C2 to C4 alkenyl.

Further, $R^2$ and $R^3$ are independently selected from hydrogen, cyano group, substituted or non-substituted C1 to C4 alkyl, and substituted or non-substituted 2- and 4- heteroalkyl, or $R^2$ and $R^3$ together form substituted or non-substituted C3 to C4 alkyl, substituted or non-substituted 3- to 4-membered heteroalkyl; the substituents in $R^2$ and $R^3$ are independently selected from halogen, hydroxyl, C1 to C4 alkyl, 3- to 6-membered cycloalkyl, 2- to 4-membered heteroalkyl, 3- to 6-membered heterocycloalkyl, and C2 to C4 alkenyl;

$R^4$ is selected from hydrogen and methyl.

Further, $R^2$ and $R^3$ are independently selected from hydrogen, cyano group, substituted or non-substituted methyl, substituted or non-substituted ethyl, substituted or non-substituted

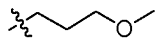,

and substituted or non-substituted

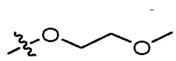,

or $R^2$ and $R^3$ together form substituted or non-substituted

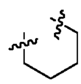,

or substituted or non-substituted

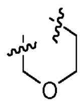;

preferably, $R^2$ and $R^3$ are independently selected from hydrogen, cyano group, substituted or non-substituted methyl, substituted or non-substituted

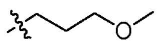,

or $R^2$ and $R^3$ together form substituted or non-substituted

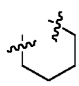,

or substituted or non-substituted

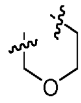;

more preferably, $R^2$ and $R^3$ are independently selected from hydrogen, cyano group, and substituted or non-substituted methyl; $R^4$ is selected from hydrogen.

Even further, the substituents in $R^2$ and $R^3$are independently selected from F, Cl, and hydroxyl.

Further, $R^6$ is selected from hydrogen, halogen-substituted or non-substituted C1 to C6 alkyl, and substituted or non-substituted 3- to 6-membered cycloalkyl.

Further, $R^6$ is selected from hydrogen, fluorine-substituted or non-substituted C1 to C4 alkyl, preferably H.

The stated compounds are selected from the following structures, or their R or S configurations:

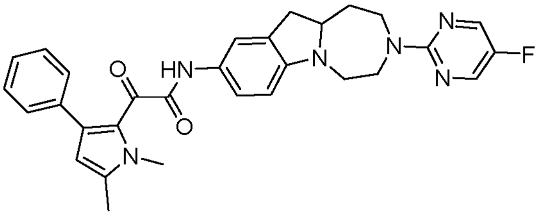,
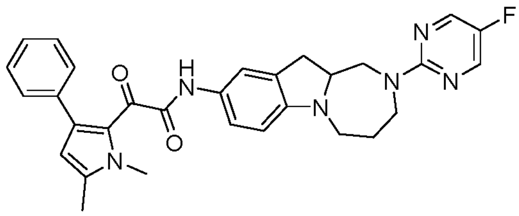,

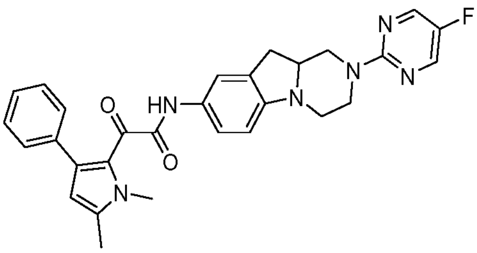,
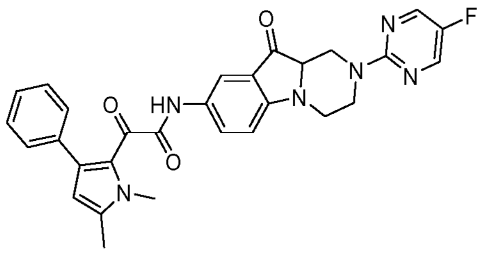,

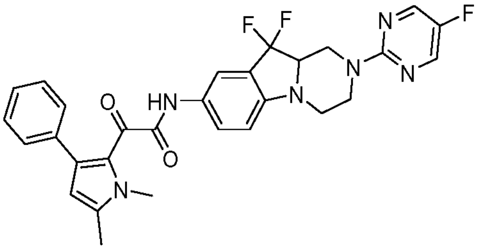,
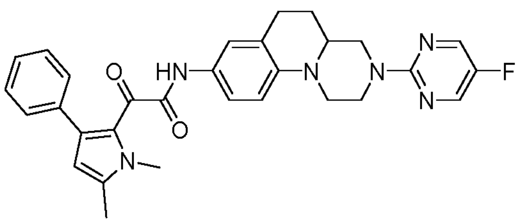,

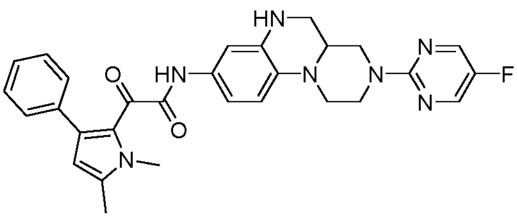,
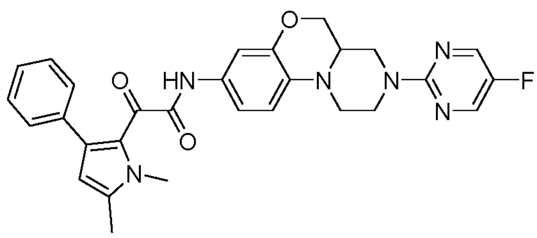,

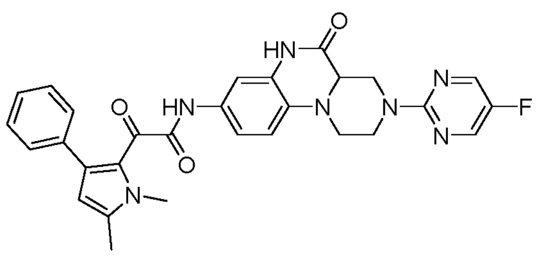
,
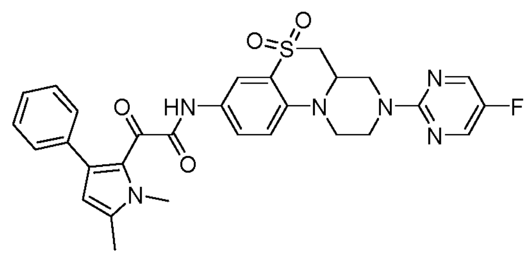
,
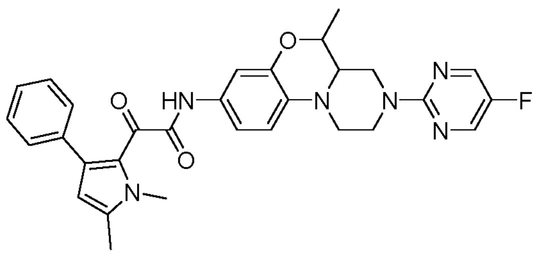
,
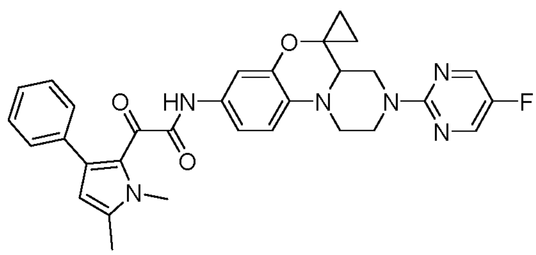
,
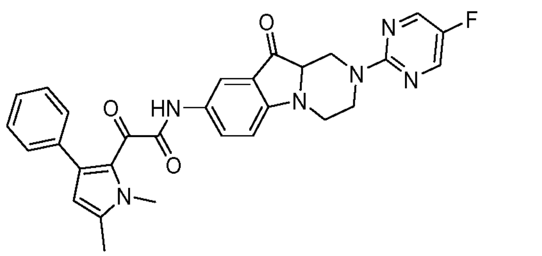
,
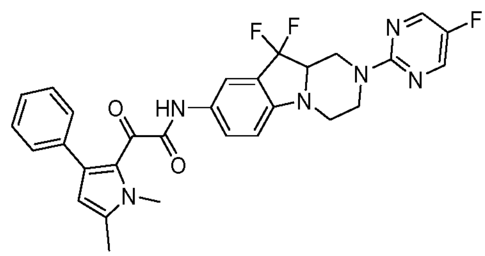
,
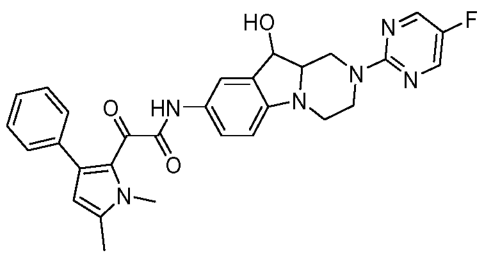
,
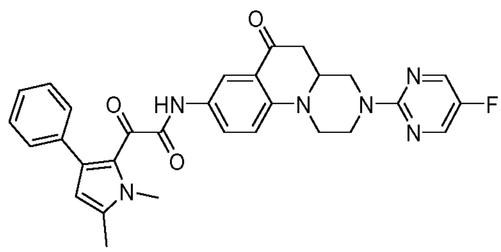
,
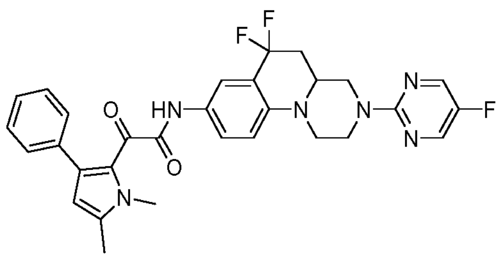
,
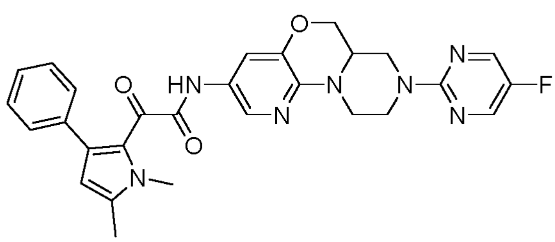
,
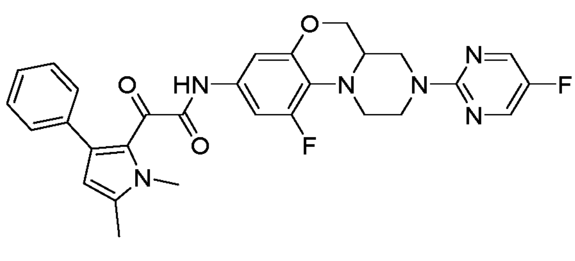
,
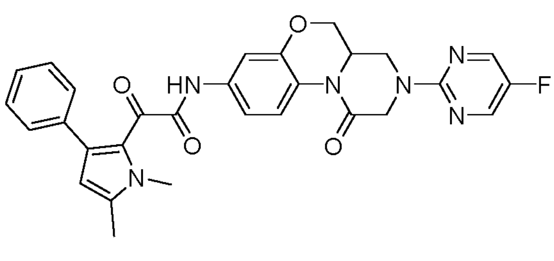
,

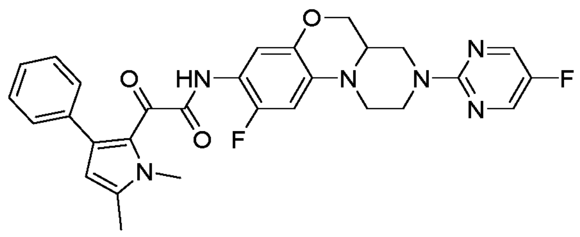

,

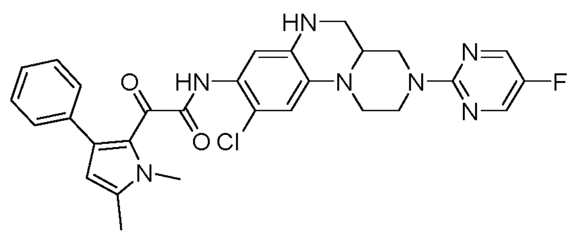

,

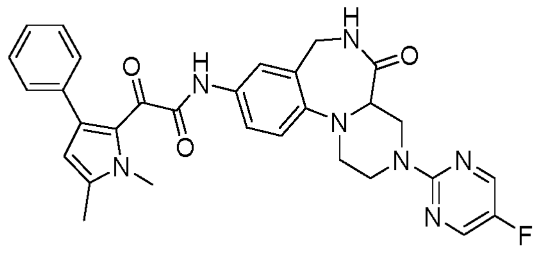

,

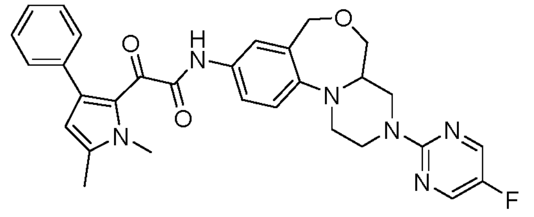

,

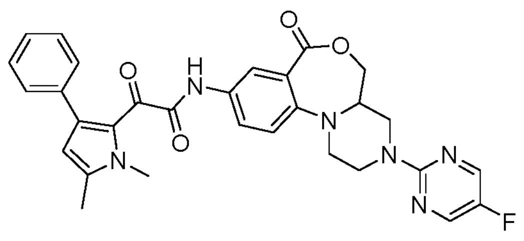

,

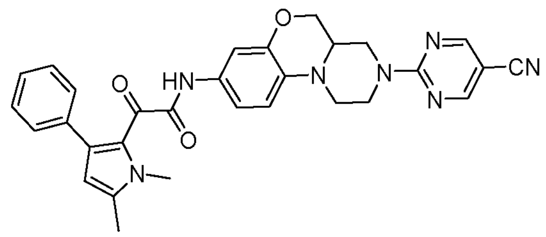

,

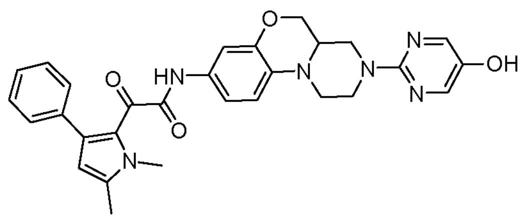

,

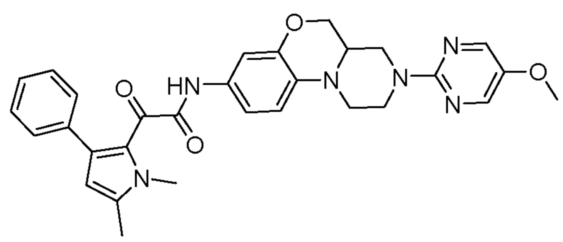

,

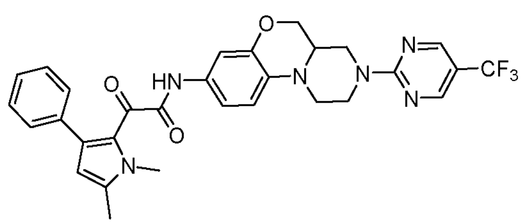

,

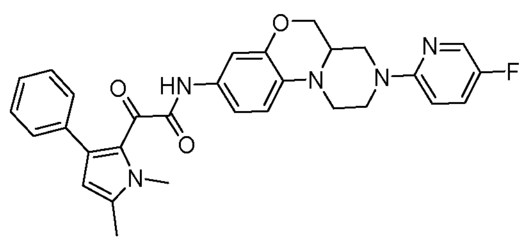

,

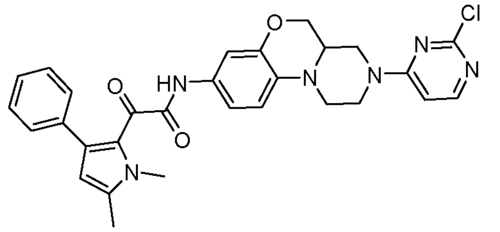

,

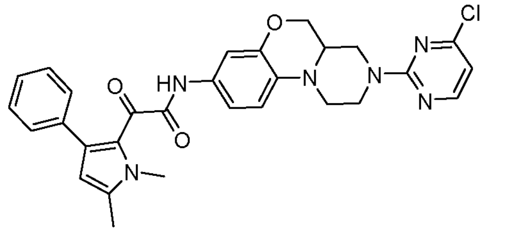

,

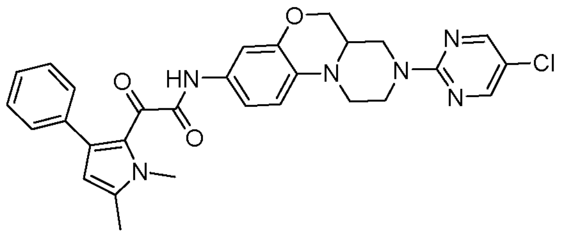
,
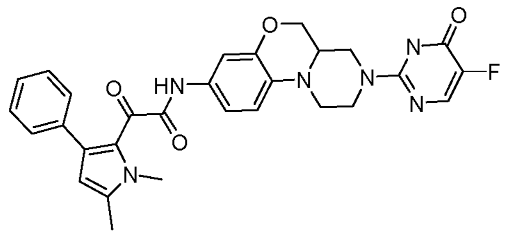
,
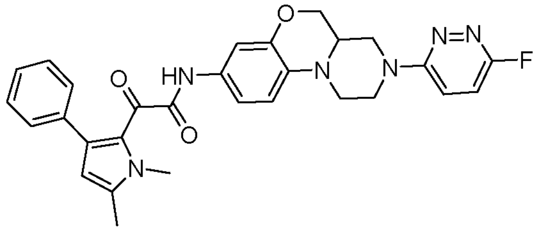
,
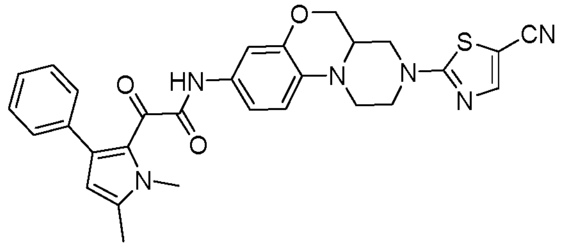
,
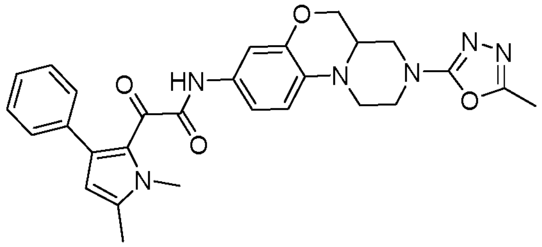
,
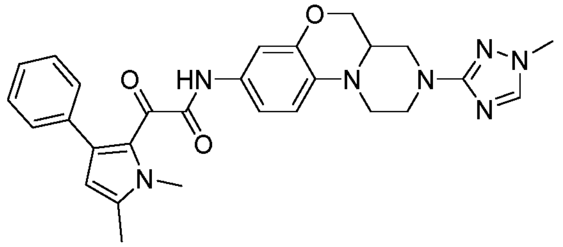
,
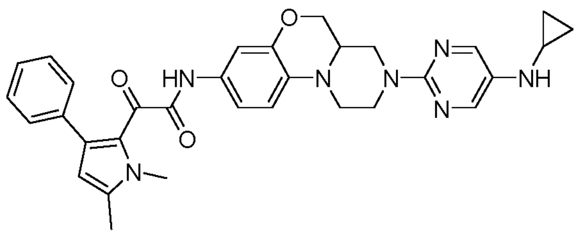
,
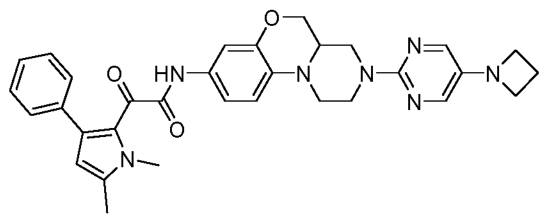
,
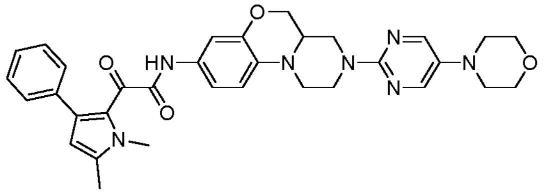
,
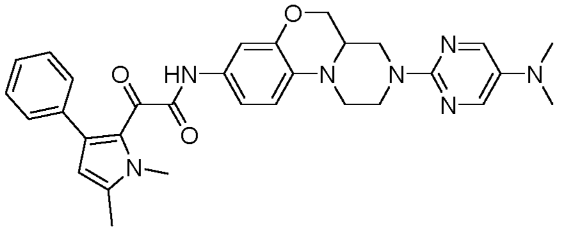
,
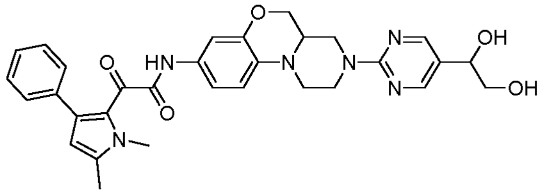
,
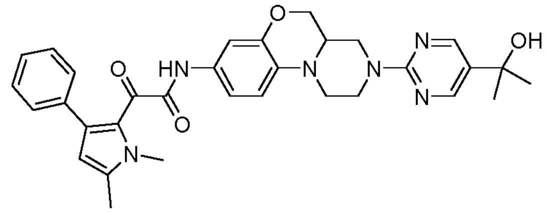
,

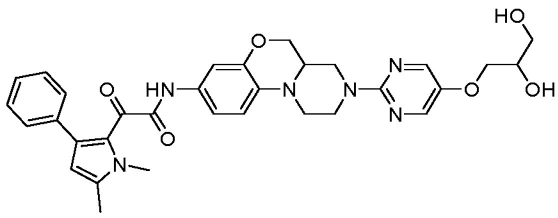
,
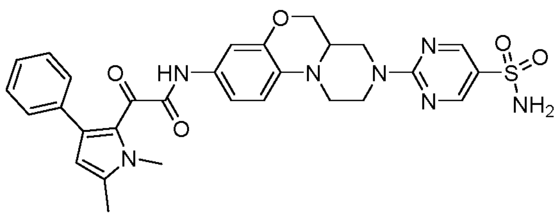
,
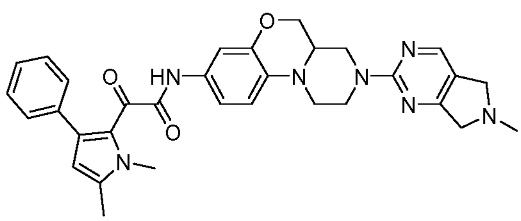
,
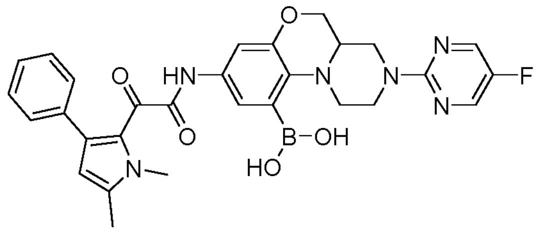
,
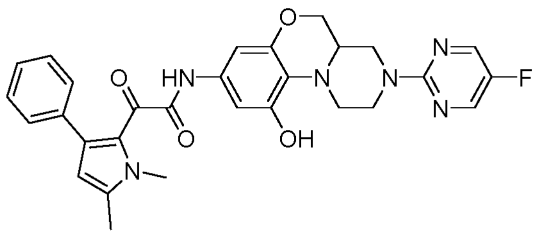
,
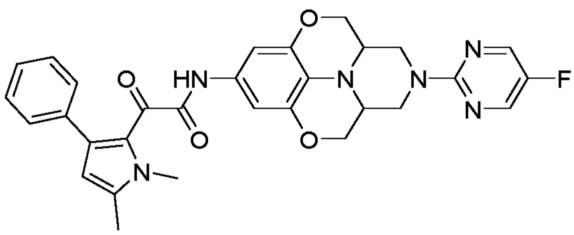
,
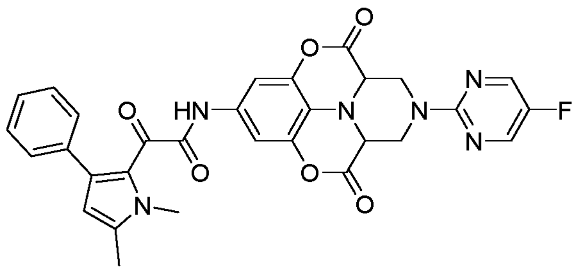
,
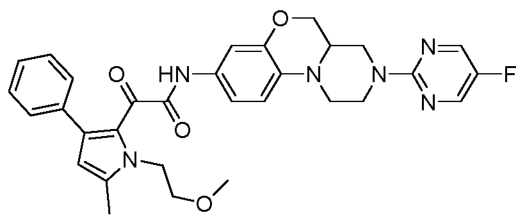
,
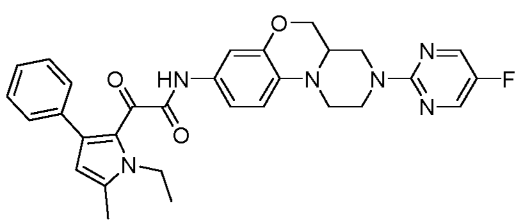
,
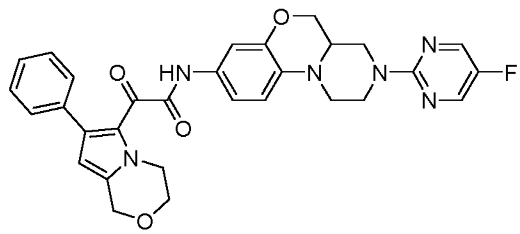
,
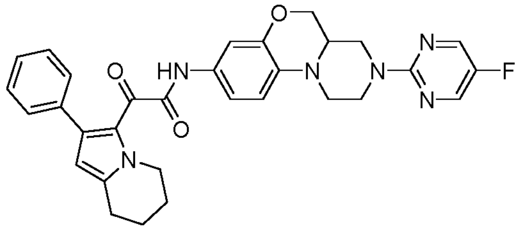
,
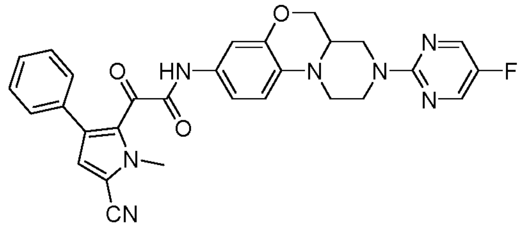
,

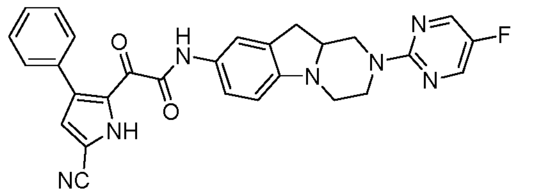

,

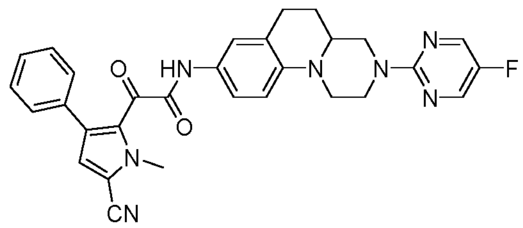

,

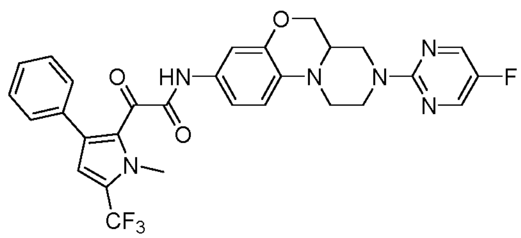

,

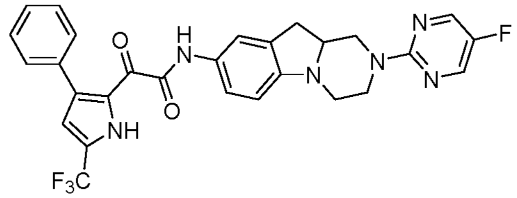

,

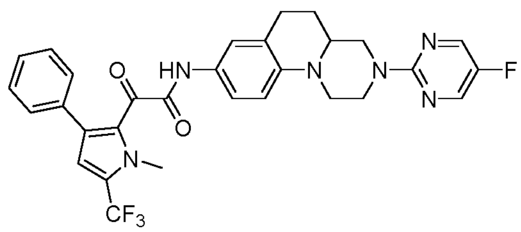

,

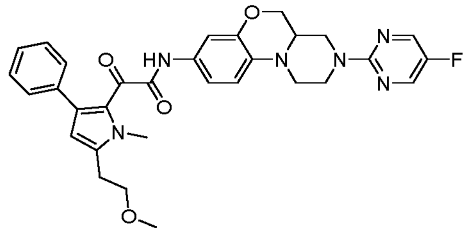

,

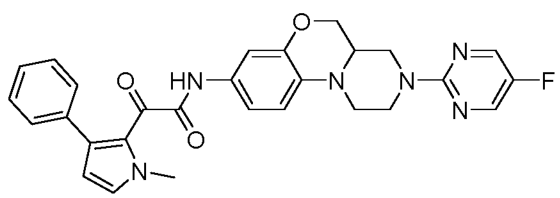

,

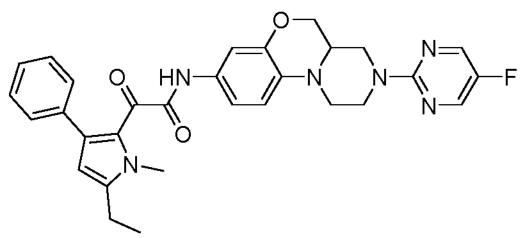

,

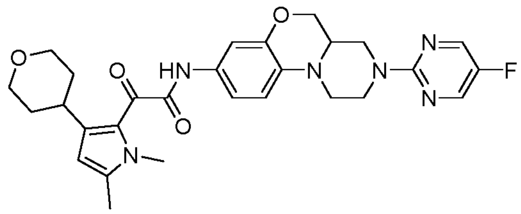

,

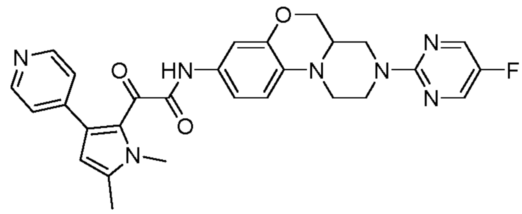

,

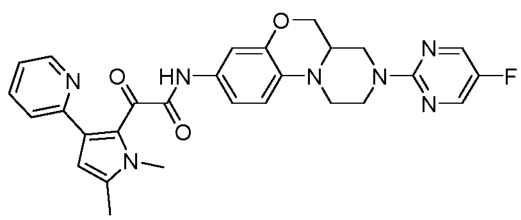

,

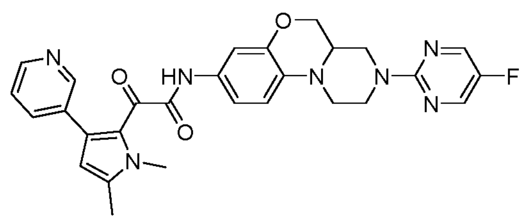

,

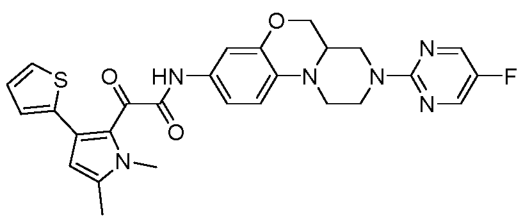

,

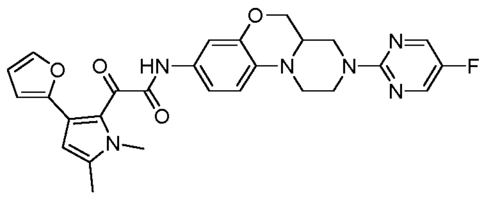

,

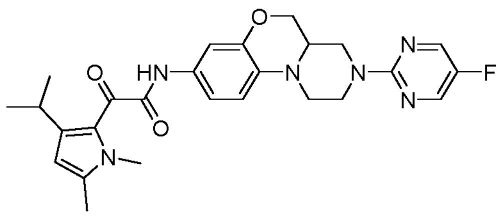

,

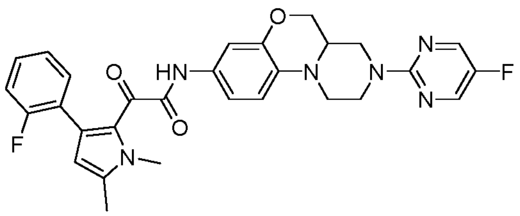

,

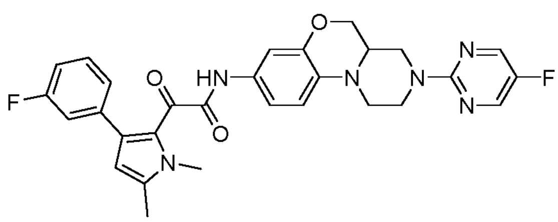

,

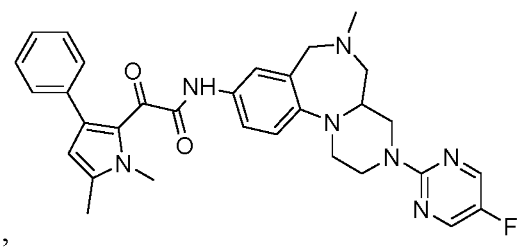

,

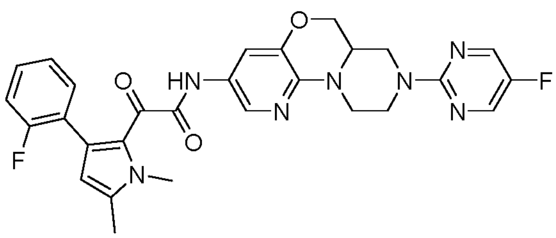

,

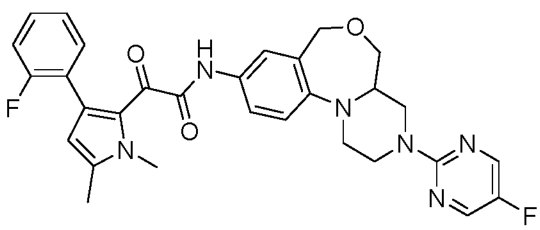

.

The present invention also provides a pharmaceutical composition of which the active ingredients are selected from one or more combination from the followings: the compounds of stated in the claims or their stereoisomers, solvates, hydrates, pharmaceutically acceptable salts, esters, co-crystals, N-oxides, isotopically labeled compounds, metabolites, and pre-drugs.

The present invention also provides the uses of the followings in the preparation of DHODH-inhibitors: the compounds stated in the present invention or their stereoisomers, solvates, hydrates, pharmaceutically acceptable salts, or co-crystals.

The present invention also provides the uses of the followings in the preparation of drugs for the prevention of fungal infections or the treatment for the diseases caused by fungal infections: the compounds stated in the present invention or their stereoisomers, solvates, hydrates, pharmaceutically acceptable salts, or co-crystals.

The present invention also provides the uses of the followings in the preparation of fungicide: the compounds stated in the present invention or their stereoisomers, solvates, hydrates, pharmaceutically acceptable salts, or co-crystals.

Further, the stated fungi are selected from one or more organisms of the following genera: Absidia, Acremonium, Alternaria, Aspergillus, Bipolaris, Blastomyces, Blumeria, Cladosporium, Coccidioides Colletotrichium, Curvularia, Encephalitozoon, Epicoccum, Epidermophyton, Exophiala, Exserohilum, Fusarium, Histoplasma, Leptosphaeria Microsporum, Mycosphaerella, Neurospora, Paecilomyces, Penicillium, Phytophthora, Plasmopara, Pneumocystis, Pyricularia, Pythium, Puccinia, Rhizoctonia, Rhizomucor, Scedosporium, Scopula riopsis, Trichophyton, Trichosporon, and Ustilago;

Further, the stated fungi are selected from one or more organisms of the following genera: Aspergillus, Scedosporium, Fusarium, preferably Aspergillus and/or Scedosporium, more preferably Aspergillus.

Further, the stated fungi are selected from one or more of the followings: A. fumigatus, A. flavus, A. terreus, A. niger, A. lentulus, S. apiospermum, S. prolificans, and S. species.

Further, the stated fungi are selected from one or more of the followings: A. fumigatus, A. flavus, A. terreus, A. niger, and S. prolificans.

Further, the fungal-induced diseases stated are selected from diseases of systemic infection with fungi, diseases of superficial infection with fungi.

Further, the stated diseases of systemic infection with fungi are selected from pulmonary aspergillosis, allergic bronchopulmonary aspergillosis, systemic aspergillosis, asthma, coccidioidomycosis, paracoccidioidomycosis, sporotrichosis, chromoblastomycosis, rhinocerebral mucormycosis, blastomycosis, histoplasmosis, lobomycosis, phaeohyphomycosis, disseminated sporotrichosis, fungal colonization of cystic fibrosis, and sinusitis;

The stated diseases of superficial infection with fungi are selected from ringworm, onychomycosis, and tinea pedis.

The following intermediates for preparing the aforementioned compounds, such as M-9, IM2, and IM3, are also provided in the present invention. The aforementioned intermediates allow further synthesis to obtain the aforementioned compounds or their analogues.

The present invention also provides the methods for the preparation of the compounds stated in the present invention, including the preparation of intermediates M-9:

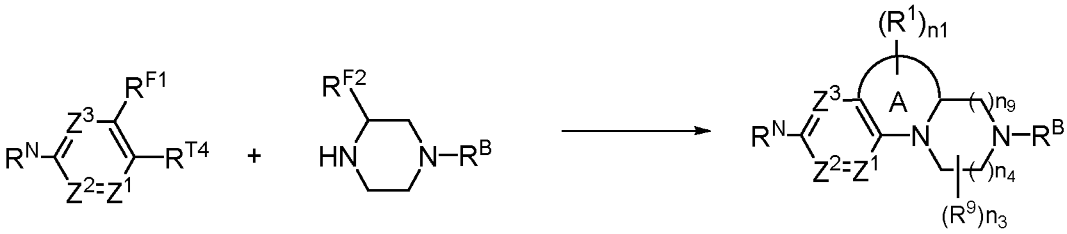

M-7 M-8 M-9

M-7 reacts with M-8 to produce compound M-9, the types of the reaction are selected from one or more of the followings: substitution reaction, condensation reaction, reduction reaction, free radical reaction, and metal-catalyzed coupling reaction.

$R^{T4}$ is selected from halogen, aldehyde group, and C1 to C6 alkyl sulfonate group substituted by halogen (e.g., trifluoromethanesulfonyl group)

$R^{F1}$ and $R^{F2}$ are expressed as functional groups capable of forming a ring structure by themselves or by transformation with the atoms to which the groups are connected, each independently is selected from hydrogen, halogen, nitro, cyano group, aldehyde, hydroxyl, carboxyl, substituted C1 to C6 alkyl, substituted C1 to C6 alkoxy, or the following substituted or non-substituted groups: mercapto, sulfone, sulfoxide group, keto-group, amino, ester, acylamino, hydroxymethyl, and aminomethyl, wherein the substituents are selected from halogen, hydroxyl, carboxyl, and ester;

$R^N$ is selected from amino or groups capable of being transformed to amino, preferably amino, nitro, carboxyl, ester, halogen, more preferably amino and nitro;

$R^B$ is selected from protecting groups of hydrogen and amino group, further selected from hydrogen, tert-butoxycarbonyl (Boc), benzyloxycarbonyl (Cbz), 9-fluorenylmethoxycarbonyl (Fmoc), benzyl (Bn), p-methoxybenzyl (PMB) and 2, 4-and dimethoxybenzyl (DMB)

$R^1$, $R^9$, $Z^1$, $Z^2$, $Z^3$, $n_1$, $n_3$, $n_4$, are $n_9$ as defined in the compounds of the present invention.

All the existing reaction conditions which can make the following substituents connected each other to give the ring structures can be applied to the present invention: hydrogen, halogen, nitro, cyano group, formyl group, hydroxyl, carboxyl, substituted C1 to C6 alkyl, substituted C1 to C6 alkoxy, sulfydryl, sulfuryl, sulfoxide, keto-group, amino, ester, acylamino, hydroxymethyl, and aminomethyl.

Further, $R^{T4}$ is selected from halogen; $R^{F1}$ is selected from halogen, formyl group, ester, sulfonyl chloride group, and hydroxymethyl; $R^{F2}$ is selected from hydrogen and hydroxymethyl.

In some specific embodiments of the present invention, M-7 is coupled to NH in M-8 through $R^{T4}$ first, and then form a ring to give M-9.

Further, $R^{T4}$ is selected from F, Cl, $R^{F1}$ is selected from aldehyde, ester, sulfonyl chloride group, hydroxymethyl; $R^{F2}$ is selected from hydrogen and hydroxymethyl;

Further, the reaction conditions for coupling $R^{T4}$ with NH in M-8 are: Mix M-7, M-8, base and solvent for reaction.

Further, the stated base is selected from inorganic bases, preferably sodium carbonate and/or potassium carbonate, more preferably potassium carbonate;

Further, the stated solvents are selected from one or more of DMSO, DMF, and toluene;

Further, the reaction temperature is 80 to 120°C, preferably 100°C.

In some specific embodiments of the present invention, $R^{F1}$ is an aldehyde group, $R^{F2}$ is hydrogen, and the stated ring formation reaction process is:

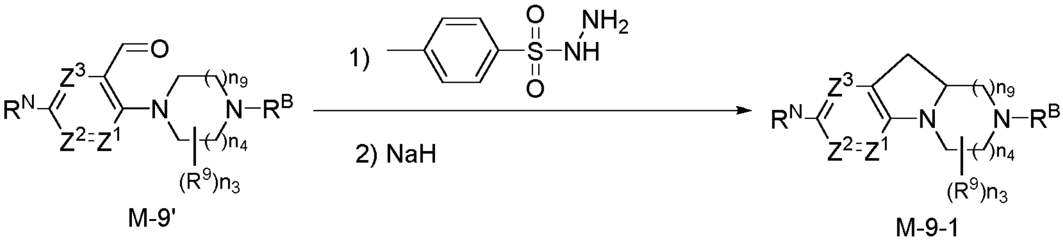


M-9'    M-9-1

The aldehyde group in M-9' first reacts with

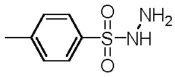

to produce an imine structure, and then the ring is closed in the presence of NaH to produce M-9-1.

In some specific embodiments of the present invention, $R^{F1}$ is hydroxymethyl and $R^{F2}$ is hydroxymethyl, and the stated ring formation reaction process is:

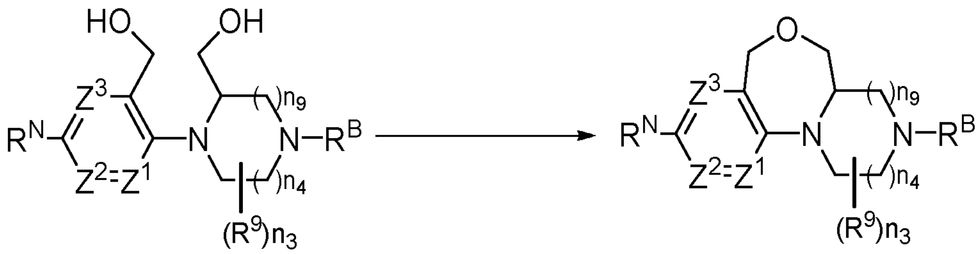

M-9"    M-9-2

M-9" produces M-9 by dehydration condensation reaction; further, M-9" produces M-9-2 via dehydration condensation by concentrated sulfuric acid.

In some specific embodiments of the present invention, M-7 is coupled and cyclized with M-8 simultaneously to give M-9 in a one-step reaction.

Further, $R^{F1}$ is F, $R^{F2}$ is hydroxymethyl, and $R^{T4}$ is selected from F. The reaction process is as follows:

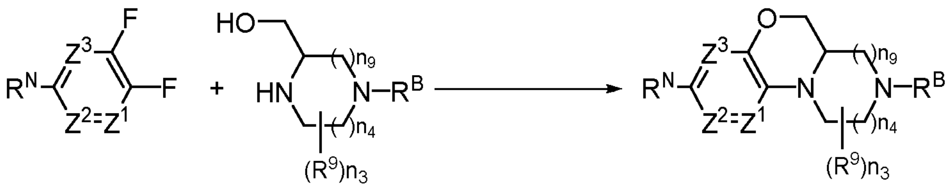

M-7'    M-8'    M-9-3

M-7' reacts with M-8' in the presence of a base to produce M-9-3; further, the stated base is selected from KOH and/or NaOH.

Further, the preparation of the compounds of the present invention further comprises the preparation of intermediate IM-2 by the following synthetic route:

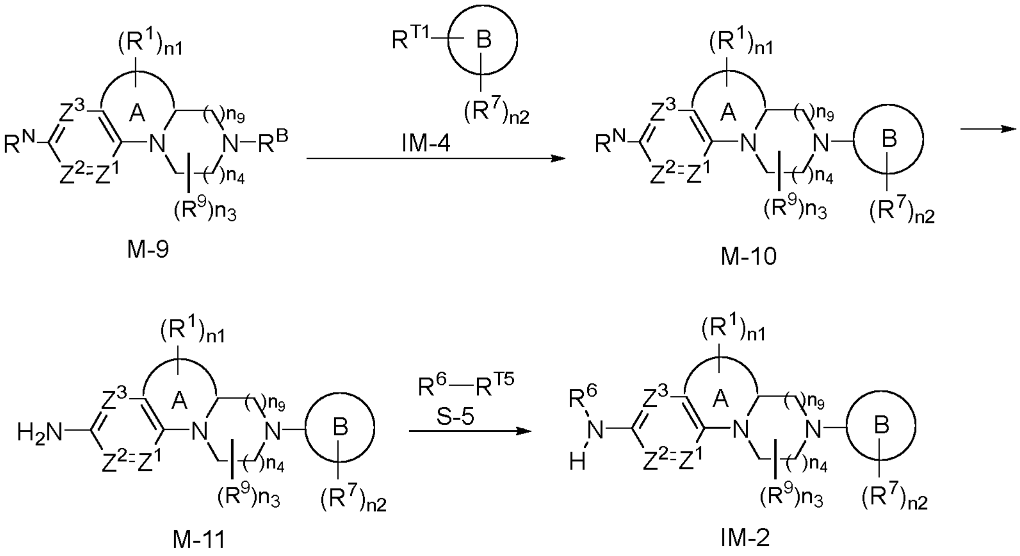


M-9, M-10, M-11, IM-2

(1) M-9 reacts with IM-4 to produce M-10 by catalytic action at the presence of a base or transition metal catalyst;
(2) $R^N$ in compound M-10 is transformed to $-NH_2$;
(3) M-11 reacts with S-5 in the presence of a base (e.g., NaH) or a reducing agent (e.g., sodium borohydride) to produce IM-2 (this step should be understood to be omitted when $R^6$ in the compound is H).

$R^{T1}$ and $R^{T5}$ are independently selected from halogen, aldehyde group, and C1 to C6 alkyl sulfonate group substituted by halogen
$R^6$, $R^7$, and $n_2$ are as defined in the compounds of the present invention.

The existing reaction conditions that can transform nitro, carboxyl, ester, halogen and other substituents to amino groups can be applied to the present invention. For example, nitro is transformed to amino by a reduction reaction, the conditions of the reduction reaction can be a palladium-carbon-catalyzed hydrogenation reaction in the presence of hydrogen, or in the presence of iron or zinc powder.

Further, the base in step (1) is an inorganic base, preferably potassium carbonate and/or sodium carbonate; the transition metal catalyst in step (1) is palladium catalyst, further selected from one or more of the followings: tetrakis (triphenylphosphine) palladium, palladium acetate, tris (dibenzylideneacetone) dipalladium, [1, 1'-bis (diphenylphosphino) ferrocene] palladium dichloride, bis (triphenylphosphine) palladium dichloride, and t-BuXPhos-Pd-G3.

Further, compound IM-2 reacts with IM-1, in the presence of a base, to produce the compounds stated in any one of claims 1 to 26, compound IM-1 having the following structure:

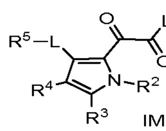

IM-1 ,

Wherein $Lg^3$ is selected from leaving groups, preferably halogen, C1 to C6 alkoxy, C1 to C6 alkyl sulfonate group substituted by halogen; $R^2$, $R^3$, $R^4$, $R^5$, and L are defined in the compounds of the present invention.

Further, the preparation of the compounds of the present invention also includes the preparation of intermediate IM-3, and the synthetic route is as follows:

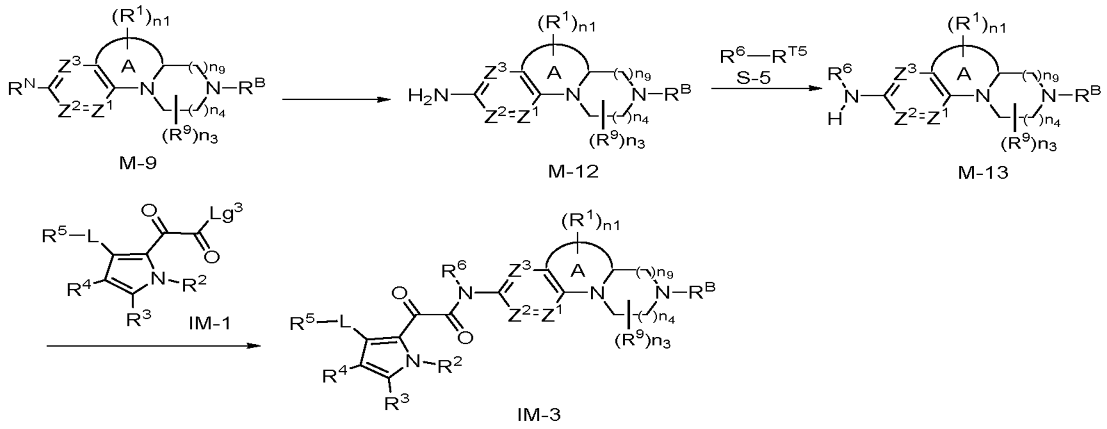


M-9 M-12 M-13 IM-1 IM-3

(1) $R^N$ in M-9 is transformed to $-NH_2$;

(2) M-12 reacts with S-5 in the presence of a base (e.g., NaH) or a reducing agent (e.g., sodium borohydride) to produce IM-13 (this step should be understood to be omitted when $R^6$ in the compound is H);

(3) M-13 reacts with IM-1, in the presence of a base or condensation agent, to produce IM-3;

$Lg^3$ is selected from leaving groups, preferably halogen, C1 to C6 alkoxy, C1 to C6 alkyl sulfonate group substituted by halogen;

$R^2$, $R^3$, $R^4$, $R^5$, and L are defined in the compound of the present invention;

Further, the base in step (3) is N, N-diisopropylethylamine (DIEA); the condensation agent in step (3) is selected from one or both of 1-(3-dimethylaminopropyl)-3-ethylcarbodiimide (EDCI) and O-(7-azabenzotriazol-1-yl)-N, N, N', N'-tetramethyluronium hexafluorophosphate (HATU).

Further, compound IM-3 reacts with IM-4, in the presence of a base, to produce the compounds stated in any one of claims 1 to 26, compound IM-4 having the following structure:

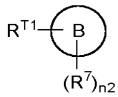

IM-4 ,

Wherein $R^{T1}$ is selected from halogen, C1 to C6 alkoxy, C1 to C6 alkyl sulfonate group substituted with halogen.

In the present invention, where a substituent has two connecting ends, the manner of connection is not limited, e.g., if adjacent $R^8$ and $R^9$ together form substituted or non-substituted ester, adjacent $R^8$ and $R^9$ together with the atoms connected to them can form

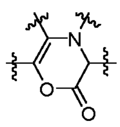 or 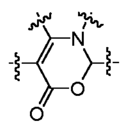 .

The stated "sinusitis" includes but is not limited to nasosinusitis, maxillary sinusitis, frontal sinusitis, antral gastritis, and sphenoiditis.

Pharmaceutical compositions containing the compounds of the present invention or their stereoisomers, solvates, hydrates, pharmaceutically acceptable salts or eutectics thereof may contain pharmaceutically acceptable excipients.

The term "pharmaceutically acceptable" in the present invention refers to any material that does not interfere with effectiveness of biological activity of active ingredients and is not toxic to the host to which it is administered.

Pharmacologically acceptable excipients mentioned in the present invention is a general term for all additional materials in the drug other than the active pharmaceutical ingredient, the excipients should have the following properties: (1) It has no toxicity to the human body, and almost no side effects; (2) It's chemically stable, not easily affected by temperature, pH, and storage time, etc.; (3) It has no incompatibility with the active pharmaceutical ingredient, and does not affect efficacy and quality analysis thereof; (4) It shows no interaction with packaging materials.

The excipients in this invention include but not limited to fillers (diluents), lubricants (flow aids or anti-adhesive agents), dispersants, wetting agents, adhesives, conditioning agents, solubilizers, antioxidants, antibacterial agents, emulsifiers, and disintegrants, etc. Adhesives include sugar syrup, Arabic gum, gelatin, sorbitol, astragalus gum, cellulose and its derivatives (such as microcrystalline cellulose, sodium carboxymethyl cellulose, ethyl cellulose or hydroxypropyl methyl cellulose, etc.), gelatin syrup, sugar syrup, starch syrup or polyvinylpyrrolidone, etc.;fillers contain lactose, powdered sugar, dextrin, starch and its derivatives, cellulose and its derivatives, inorganic calcium salts (such as calcium sulfate, calcium phosphate, calcium hydrogen phosphate, precipitated calcium carbonate, etc.), sorbitol or glycine, etc.; lubricants include micronized silica gel, magnesium stearate, talc, aluminum hydroxide, boric acid, hydrogenated vegetable oil, polyethylene glycol, etc.; disintegrants include starch and its derivatives (such as sodium carboxymethyl starch, sodium starch glycolate, pre-gelatinized starch, modified starch, hydroxypropyl starch, corn starch, etc.), polyvinylpyrrolidone or microcrystalline cellulose, etc. Wetting agents include sodium dodecyl sulfate, water or alcohol, etc.; antioxidants include sodium sulfite, sodium bisulfite, sodium metabisulfite, dibutylbenzene acid, etc.; bacterial inhibitors include 0.5% phenol, 0.3% cresol, 0.5% trichloro-tert-butanol, etc.; conditioning agents include hydrochloric acid, citric acid, potassium (sodium) hydroxide, sodium citrate and buffers (including sodium dihydrogen phosphate and disodium hydrogen phosphate), etc.; emulsifiers include polysorbate 80, sorbitan oleate (span 80), Pluronic™ F-68, lecithin, soy phospholipids, etc.; solubilizers include tween-80, bile, glycerol, etc. The term "pharmaceutically acceptable salt" refers to salt formed by the compound of the present invention with acid or alkali suitable for use as a drug. The acid mentioned above and alkali are broadly defined as lewis acid and lewis alkali. Acids suitable for salt formation include but not limited to inorganic acids such as hydrochloric, hydrobromic, hydrofluoric, sulfuric, nitric, and phosphoric acids; organic acids such as formic, acetic, propionic, oxalic, malonic, succinic, fumaric, maleic, lactic, malic, tartaric, citric, picric, methanesulfonic, benzenemethanesulfonic, and benzenesulfonic acids; and acidic amino acids such as aspartic and glutamic acids.

There are no particular limitations on the manner of administration of the compounds or pharmaceutical compositions of the present invention, representative routes of administration include (but are not limited to): oral, parenteral (intravenous, intramuscular or subcutaneous), and topical administration.

Solid dosage forms used for oral administration include capsules, tablets, pills, suspensions, and granules. In these solid dosage forms, the active compound is mixed with at least one conventional inert excipient (or carrier), such as sodium citrate or dicalcium phosphate, or with(a) (a) fillers or bulking agents, e.g., starch, lactose, sucrose, glucose, mannitol, and silicic acid; (b) binders, e.g., hydroxymethyl cellulose, alginate, gelatin, polyvinylpyrrolidone, sucrose, and arabic gum; (c) humectants, e.g., glycerin; (d) disintegrants, e.g., agar, calcium carbonate, potato starch or tapioca starch, alginic acid, certain composite silicates, and sodium carbonate; (e) retarders, e.g., paraffin; (f) absorption accelerator agents, e.g., quaternary amine compounds; (g) wetting agents, e.g., cetearyl alcohol and glycerol monostearate; (h) adsorbents, e.g., kaolin; and (i) lubricants, e.g., talc, calcium stearate, talc, calcium stearate, magnesium stearate, solid polyethylene glycol, sodium dodecyl sulfate, or their mixtures. As for capsules, tablets, and pills, the dosage form may also contain a buffering agent.

Solid dosage such as tablets, sugar pills, capsules, pills and granules are allowed to be prepared using coating and shell materials such as enteric coatings and other materials well known in the field. They are allowed to contain opacifiers, and the active compound or compounds in such compositions may be released in a delayed manner in some part of digestive tract. Examples of embedded components used are polymeric substances and waxy substances. If necessary, the active compound combined with one or more of the above excipients may also be made in microcapsule form.

Liquid dosage for oral administration includes pharmaceutically acceptable emulsions, solutions, suspensions, syrups, or tinctures. In addition to the active compound, the liquid dosage form may include inert diluents routinely adopted in the area, such as water or other solvents, and solubilizers and emulsifiers, e.g., ethanol, isopropyl alcohol, ethyl carbonate, ethyl acetate, propylene glycol, 1,3-butanediol, dimethylformacylamino, and oils, particularly cottonseed oil, peanut oil, maize germ, olive oil, castor oil and sesame oil or mixtures of these substances.

In addition to these inert diluents, the compositions may also comprise accessory ingredients such as wetting agents, emulsifiers and suspensions, sweeteners, flavor correctors, and fragrances.

In addition to the active compounds, the suspension liquid may comprise suspensions such as ethoxylated isooctadecanol, polyoxyethylene sorbitol and dehydrated sorbitol esters, microcrystalline cellulose, and methanolic aluminum and agar or mixtures of these substances.

Compositions for parenteral injection may comprise aqueous or anhydrous solutions, dispersions, suspension liquid or emulsions which are sterile and physiologically acceptable, as well as sterile powder which is to be redissolved into sterile injectable solution and dispersion liquid. Suitable aqueous and non-aqueous carriers, diluent, and solvent or excipients include water, ethanol, polyols, and their suitable mixtures.

Dosage forms of the compounds of the present invention for topical administration include ointment, pulvis, patch, spray and inhalation. The active ingredient is mixed under sterile conditions with carrier and any preservative and buffering agent which are physiologically acceptable, or with propellants that may be required if necessary.

The compounds of the present invention can likewise be used in injectable preparations. Wherein, the stated injection is selected from liquid injection (injection), sterile powder for injection (powde) or tablet for injection (refer to the molded or machine-pressed tablet made by aseptic operation, dissolved with water for injection for subcutaneous or intramuscular injection when ready for use).

Wherein, the stated powder for injection contains at least excipient in addition to the above compounds. The excipient, in the invention, is a composition purposively add to drug, and should not have pharmacological properties in the amount used. However, the excipient may contribute to the processing, dissolving or dissolution of the drug, and may contribute to stability through targeted administration route.

The term "substitution" refers to the replacement of a hydrogen atom in a molecule by a different atom or molecule.

The term "member" refers to the number of backbone atoms that make up ring or heteroalkyl group.

The term "one bond" in the present invention refers to only one linkage bond therein, which can also be understood as "none".

The substitution group "=O" refers to that several substituents of the same atom together form one or more =O, e.g.,

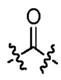, 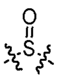, or 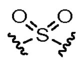.

The term "Alkyl", an aliphatic hydrocarbon group, refers to saturated hydrocarbyl. The alkyl portion may be straight-chain alkyl or branched alkyl. Typical alkyl includes but are not limited to methyl, ethyl, propyl, isopropyl, butyl, isobutyl, tert-butyl, pentyl, and hexyl.

C1-Cn in the present invention includes C1-C2, C1-C3 ......C1-Cn, with n being an integer greater than one; the prefixes as substituents indicate the minimum and maximum number of carbon atoms in substituent, for example, "C1-C6 alkyl" refers to alkyl group straight or branched containing one to six carbon atoms.

The term "hydrocarbon ring" refers to a ring structure where atoms in the ring skeleton are all carbon atoms, and the carbon atoms can be connected to each other by single bonds or double bonds. Hydrocarbon ring includes saturated hydrocarbon ring, alkene ring and aromatic ring structure.

The term "alkenyl ring" refers to groups of which the ring skeleton has carbon-carbon double bond.

The term "heteroalkyl" refers to an alkyl group having heteroatoms, wherein, the heteroatoms include but are not limited to O, S, N, and P; alkoxy, alkyl sulfide, and alkyl ammonia, etc. are all heteroalkyl.

The term "alkenyl" refers to aliphatic hydrocarbon group having at least one carbon-carbon double bond. The stated alkenyl may be straight-chain or branched.

The term "alkenylene" refers to a divalent hydrocarbon group being of straight or branched chain and having one or more carbon-carbon double bonds, such as -CH=CH- and - $CH_2CH=CH$-. When a compound of the present invention contains alkenylene, the stated compound may exist in the pure E form (entgegen), in the pure Z form (zusammen), or in their any mixture forms.

The term "alkynyl" refers to aliphatic hydrocarbon group having at least one carbon-carbon triple bond. The stated alkynyl may be straight-chain or branched.

The term "alkynylene" refers to a straight or branched divalent hydrocarbon group having one or more carbon-carbon triple bonds, such as

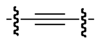 and 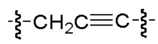.

The term "acylamino" is a chemical structure having the formula -C(O)NHR or - NHC(O)R. When there're two connected ends, the structure is -C(O)NH($CH_2$) a- or - NHC(O)($CH_2$) a-, wherein R can be selected from alkyl, heteroalkyl, cycloalkyl, heterocycloalkyl, aryl, heteroaryl, etc., and a is a natural number.

The term "sulfonyl" is a chemical structure having the formula -S(=O)$_2$R. When there're two connected ends, the structure is -S(=O)$_2$($CH_2$) a-, wherein R can be selected from alkyl, heteroalkyl, cycloalkyl, heterocycloalkyl, aryl, heteroaryl, amino, etc., and a is a natural number.

The term "sulfinyl" refers to a chemical structure having the formula -S (=O) R and, when there're two connected ends, the structure is -S(=O) ($CH_2$) a-, wherein, R can be selected from alkyl, heteroalkyl, cycloalkyl, heterocycloalkyl, aryl, heteroaryl, amino, etc., and a is a natural number.

The term "sulfonamido" refers to a chemical structure having the formula -S(=O)$_2$NHR or -NHC(O)R, when there're two connected ends, the structure is - S(=O)$_2$NH($CH_2$) a- or - NHS (=O)$_2$ ($CH_2$) a-, wherein, R can be selected from alkyl, heteroalkyl, cycloalkyl, heterocycloalkyl, aryl, heteroaryl, etc., and a is a natural number.

The term "sulfinamido group" refers to a chemical structure having the formula -S (=O) NHR or -NHS(O)R, when there're two connected ends, the structure is -S(=O) NH $(CH_2)$a- or -NHS (=O)$(CH_2)$a-, wherein, R can be selected from alkyl, heteroalkyl, cycloalkyl, heterocycloalkyl, aryl, heteroaryl, etc., and a is a natural number.

The term "phosphoryl" is a chemical structure having the formula -P(=O) RR', when there're two connected ends, the structure is -P(=O)R$(CH_2)$a-, wherein R and R' are independently selected from alkyl, heteroalkyl, cycloalkyl, heterocycloalkyl, aryl, heteroaryl, hydroxy, amino, etc., and a is a natural number.

The term "Ester" refers to a chemical structure having the formula -C(O)OR or - OC(O)R, when there're two connected ends, the structure is -C(O)O$(CH_2)$a- or - OC(O)$(CH_2)$a-, wherein R can be selected from alkyl, heteroalkyl, cycloalkyl, heterocycloalkyl, aryl, and heteroaryl, etc., and a is a natural number.

The term "acyl" is a chemical structure having the formula -C(O)R, when there're two connected ends, the structure is -C(O) $(CH_2)$a-, wherein R is selected from alkyl, heteroalkyl, cycloalkyl, heterocycloalkyl, aryl, and heteroaryl, etc., and a is a natural number.

The term "cycloalkyl" refers to a saturated or unsaturated cyclic hydrocarbon substituent, e.g., "C3 to C6 cycloalkyl" refers to a cycloalkyl group with a carbon atom number of 3 to 6; in the present invention, cycloalkyl may also include a non-aromatic structure with unsaturated bonds in the ring skeleton.

The term "heterocycloalkyl" refers to a cycloalkyl group with at least one heteroatom in the ring skeleton.

Heteroatoms include but are not limited to O, S, N, P, and Si.

The "cyclo" refers to any covalent closed structure, including carbocycle (e.g., aryl or cycloalkyl), heterocycle (e.g., heteroaryl or heterocycloalkyl), aromatic group (e.g., aryl or heteroaryl), or non-aromatic group (e.g., cycloalkyl or heterocycloalkyl). The "cyclo" referred to in the present invention may be monocyclic or polycyclic, and to be parallel, spiro or bridge ring.

Typical heterocyclic alkyl groups include but are not limited to:

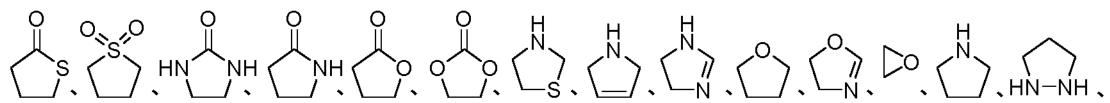

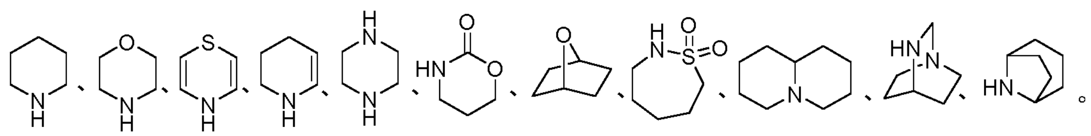

The term "aryl" refers to that a planar ring having a delocalized π-electron system and 4n+2π electrons, where n is an integer. Aryl ring may comprise five, six, seven, eight, nine or more than nine atoms. Aryl group include but are not limited to phenyl, naphthyl, phenanthryl, anthryl, fluorenyl, and indenyl, etc.

Typical heteroaryl groups include but are not limited to:

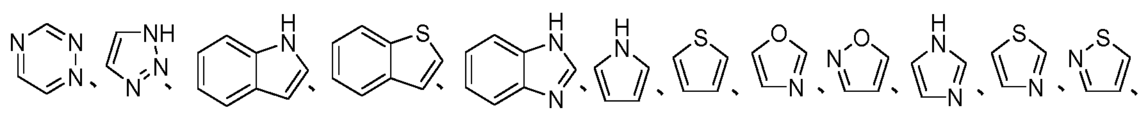

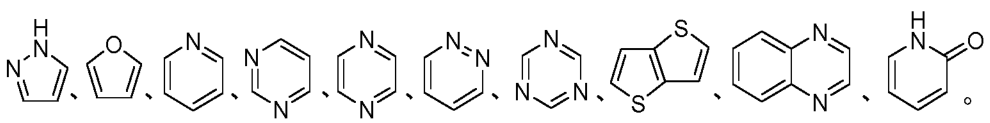

The term "halogen" refers to fluorine, chlorine, bromine or iodine.

In the description, the stated alkyl, heteroalkyl, cyclic, heterocyclic, amino, ester, carbonyl, acylamino, sulfonyl, phosphoryl, boronic acid group, boronic acid ester group, guanidino, acylguanidine, aryl, heteroaryl, imino, etc. may be non-substituted alkyl, heteroalkyl, cyclic group, heterocyclic, amino, ester, carbonyl, acylamino, sulfonyl, phosphoryl, boronic, boronic ester, guanidino, acylguanidine, aryl, heteroaryl, imino, or be substituted alkyl, heteroalkyl, cyclic group, heterocyclic group, amino, ester, carbonyl, acylamino, sulfonyl, phosphoryl, boronic acid group, boronic ester, guanidino, acylguanidino, aryl, heteroaryl, and imino.

In the foregoing, except as already indicated, the stated "substituted-" means that the mentioned group could be substituted by one or more additional groups, the additional groups are independently selected from alkyl, cycloalkyl,

aryl, carboxyl, heteroaryl, heterocycloalkyl, hydroxy, alkoxy, alkylthio, aryloxy, O=, guanidinyl, cyano group, nitro, acyl, halogen, haloalkyl, amino, etc.

The beneficial effect of the present invention is that the present invention provides a series of compounds with significant inhibitory effect on DHODH activity, which provide a new treatment targeting DHODH for the diseases such as fungal infections, and can be used for the preparation of drugs for related diseases and also for the preparation of agricultural fungicides, which have broad application prospects.

**Illustrations of the accompanying figures**

Figure 1. Survival rate experiment I of mouse bloodstream infection model with Aspergillus fumigatus
Figure 2. Survival rate experiment II of mouse bloodstream infection model with Aspergillus fumigatus

**Description of the preferred embodiments**

The technical solutions of the present invention are clearly and completely described below, and it is clear that the described embodiments are only a part of the examples of the invention. Based on the examples in the invention, all other examples obtained by general technician this field without making creative labor fall within the scope of protection of the invention.

The reaction is monitored with thin-layer chromatography (TLC) or liquid-phase mass spectrometry (LC-MS). The developing solvent systems used for purification with silica columns or thin-layer preparative plates include but are not limited to: dichloromethane/methanol system, hexane/ethyl acetate system and petroleum ether/ethyl acetate system, with the volume ratio of the solvent adjusted according to polarity of compound, or adjusted by adding ammonia water, triethylamine, etc. The developing solvent systems used for purification with reverse-phase preparation include but are not limited to: (a) Phase A: water, Phase B: acetonitrile; (b) Phase A: water, Phase B: methanol.

If not otherwise specified in the examples, the temperature of the reaction is room temperature (20 °C to 30 °C).

Unless otherwise specified, the reagents used in the examples are commercially available.

In the invention, the structures of the compounds are determined by mass spectrum (MS) and/or nuclear magnetic resonance ($^1$HNMR). The chemical abbreviations have the following meanings:

DMF: N, N-dimethylformamide
THF: Tetrahydrofuran
DIEA: N, N-diisopropylethylamine
PE: Petroleum ether
EA: Ethyl acetate
DCM: Dichloromethane
HATU: O-(7-azabenzotriazol-1-yl)-N, N, N', N'-tetramethylurea hexafluorphosphate
DMSO: Dimethyl sulfoxide
Tol.: Toluene
$K_2CO_3$: Potassium carbonate
AcOH: Acetic acid
t-BuXPhos-Pd-G3: methanesulfonato(2-di-tert-butyl phosphino-2',4',6'-triisopropyl-1, 1'-biphenyl) (2'-amino-1,1'-biphenyl-2-yl) palladium (II)
t-BuONa: Sodium tert-butoxide
2-Ethoxyethanol: Ethylene glycol monoethyl ether,
TBAB: Tetrabutylammonium bromide

**Example 1**

Preparation of 2-(1,5-dimethyl-3-phenyl-1H-pyrrol-2-yl)-*N*-(2-(5-fluoropyrimidin-2-yl)-1,2,3,4,10,10a-hexahydropyrazino[1,2-*a*]indol-8-yl)-2-oxoacetamide **(T-1)**

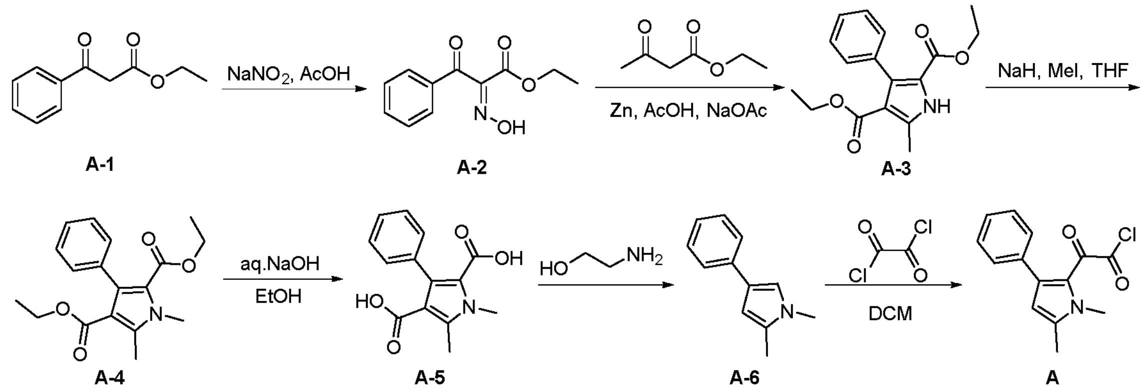


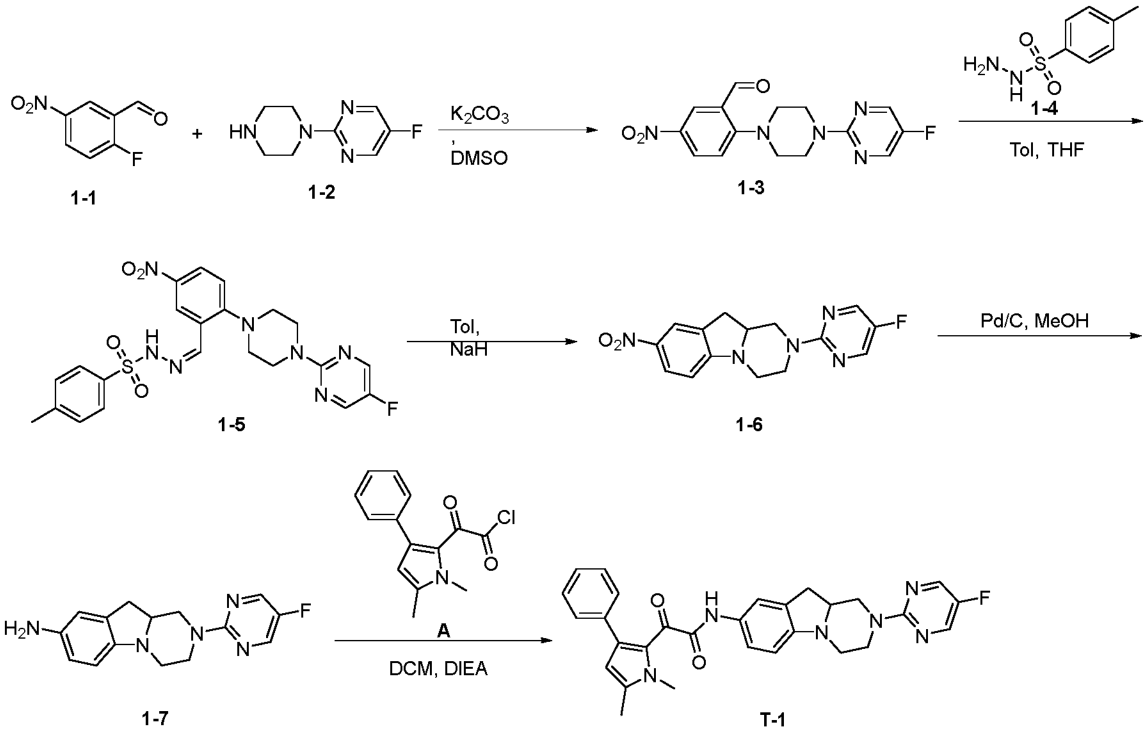


Step 1: Preparation of (Z)-2-(hydroxyimino)-3-oxo-3-phenylpropanoic acid ethyl ester **(A-2)**

Add compound A-1 (10.0 g, 52.1 mmol) and glacial acetic acid (20 mL) to a reaction flask, and drop the solution containing sodium nitrite (15.8 g, 229.2 mmol) and water (30 mL) slowly to the reaction system during which maintain the temperature between 0 °C and 10 °C, and the addition is completed after 2 h. Then the reaction is carried out at room temperature for 1 h, tested by LC-MS to confirm that the reaction is complete. Add 20 mL of water into the flask, stir for 1 h, and filtrate. Wash the cake with 15 mL of water, then dissolve cake with dichloromethane (20 mL), wash with water (2 × 30 mL) and saturated saline (2 × 30 mL) successively, dry with anhydrous $Na_2SO_4$, and evaporate under vacuum to give 8.1 g of white solid product A-2. The yield is 70%.

Step 2: Preparation of diethyl 5-methyl-3-phenyl-1*H*-pyrrole-2,4-dicarboxylate **(A-3)**

Add zinc powder (7.4 g, 113.2 mmol), anhydrous sodium acetate (7.7 g, 93.9 mmol), ethyl acetoacetate (5.5 g, 42.3 mmol) and glacial acetic acid (2 mL) to a reaction flask, then heat in oil bath to 60 °C. Then a solution of compound A-2 (8.1 g, 36.7 mmol) and glacial acetic acid (3 mL) is added in three portions in 1h. Heat to 70 °C, and react for 3 h, add zinc powder (3.7 g, 56.6 mmol) and react for 1 h, using TLC to confirm that the reaction of the raw materials is complete. Cool to room temperature and filter, remove the solvent by evaporation under vacuum and coevaporatetwice with toluene (1 mL), pour the residual liquid into water and extract with ethyl acetate, wash the organic phase with saturated salt water and dry with anhydrous $Na_2SO_4$, evaporate under vacuum, the crude product is purified through silica gel column to give 7.0 g of light pink solid product A-3, with the yield of 62%.

Step 3: Preparation of diethyl 1,5-dimethyl-3-phenyl-1*H*-pyrrole-2,4-dicarboxylate **(A-4)**

Add sodium hydride (1.9 g, 47.5 mmol, 60%) and THF (28 mL) to a reaction flask, drop solution of compound A-3 (7.0 g, 23.2 mmol) in THF (28 mL) at 0 °C and complete in 1 h. The reaction is carried out at room temperature for 1 h and then placed in ice water bath, add iodomethane (8.3 g, 58.5 mmol) slowly. React at room temperature for 18 h, using LC-MS to confirm that the reaction is complete. Add water to stop the reaction, then add 1 N hydrochloric acid solution (14 mL), extract twice with ethyl acetate. Combine the organic phase, wash with water and then saturated salt water, dry with anhydrous $Na_2SO_4$, evaporate under vacuum, and 6.5 g of yellow solid crude product A-4 is obtained, with the yield of 89%.

Step 4: Preparation of 1,5-dimethyl-3-phenyl-1*H*-pyrrole-2,4-dicarboxylic acid **(A-5)**

Add compound A-4 (6.5 g, 22.60 mmol) and ethanol (33 mL) to a reaction flask, then add solution of sodium hydroxide (9.000 g, 226.00 mmol) in water (26 mL), and the reaction system is heated and reflux for 18 h, using TLC to confirm that the reaction is completed. Remove ethanol by evaporation under vacuum, and dissolve the residue with water (10 mL), cool to 0 °C, adjust pH to 2 with concentrated hydrochloric acid, stir below 10 °C for 1 h, filter. The filter cake is washed successively with water (10 mL) and petroleum ether (10 mL), combine the filtrate, evaporate under vacuum below 60 °C to obtain 4.8 g of gray solid product A-5, with the yield of 90%.

Step 5: Preparation of 1,2-dimethyl-4-phenyl-1*H*-pyrrole **(A-6)**

Add compound A-5 (4.8 g, 18.5 mmol) and ethanolamine (13 mL) to a reaction flask, and the reaction solution is purged with nitrogen, and react for 1 h at 175 °C, using LC-MS to assure complete reaction. Add water to the reaction system, extract twice with ethyl acetate, and combine the organic phase, wash with saturated salt water, dry with anhydrous $Na_2SO_4$, evaporate under vacuum, and the crude product is purified through silica gel column after to give 2.9 g of white solid product A-6, with the yield of 91%.

Step 6: Preparation of 2-(1,5-dimethyl-3-phenyl-1*H*-pyrrol-2-yl)-2-oxoacetyl chloride **(A)**

Add compound A-6 (100 mg, 0.58 mmol) and DCM (2 mL) to a reaction flask, then add oxalyl chloride (81 mg, 0.64 mmol) slowly at 0 °C. After addition, react at room temperature for 1 h, using LC-MS to confirm a complete reaction. The resulting reaction solution is used directly in the next step.

Step 7: Preparation of 2-(4-(5-fluoropyrimidin-2-yl) piperazin-1-yl)-5-nitrobenzaldehyde **(1-3)**

Add compound 1-1 (500 mg, 2.96 mmol), 1-2 (539 mg, 2.96 mmol), DMSO (3 mL) and potassium carbonate (613 mg, 4.44 mmol) to a reaction flask. The reaction mixture is heated to 100 °C, and stirred overnight, using LC-MS to confirm a complete reaction. Cool, dilute with water, and filter. Dry the filter cake to give 950 mg of products 1-3, with the yield of 97%.

Step 8: Preparation of (Z)-*N'*-(2- (4-(5-fluoropyrimidin-2-yl) piperazin-1-yl)-5-nitrobenzylidene)-4-methylbenzenesulfono hydrazide **(1-5)**

Add compounds 1-3 (1.8 g, 5.43 mmol), 1-4 (1.1 g, 5.91 mmol), toluene (30 ml) and THF (20 mL) to a reaction flask and the reaction solution is stirred at room temperature overnight, using TLC to confirm a complete reaction of the raw materials. Evaporate under vacuum to give 2.8 g of crude product 1-5, which is directly used in the next reaction step.

Step 9: Preparation of 2-(5-fluoropyrimidin-2-yl)-8-nitro-1,2,3,4,10,10a-hexahydropyrazino[1,2-*a*] indole **(1-6)**

Add compounds 1-5 (2.8 g, 5.6 mmol), toluene (50 ml), NaH (60% in mineral oil, 1.3 g, 32.5 mmol) to a reaction flask, heat to 130 °C and stir for 1 h, using LC-MS to confirm a complete reaction. Cool to room temperature, and place in an ice bath, dilute with water, and extract twice with ethyl acetate. Combine the organic phase, wash with saturated salt water, and dry with anhydrous $Na_2SO_4$, evaporate under vacuum, purify the crude product through silica gel column to give 1.2 g of product 1-6, with the two-step yield of 70%.

Step 10: Preparation of 2-(5-fluoropyrimidin-2-yl)-1,2,3,4,10,10a-hexahydropyrazino[1,2-*a*] indol-8-amine **(1-7)**

Add compounds 1-6 (80 mg, 0.25 mmol), methanol (20 mL), and Pd/C (50 mg) to a reaction flask, purged with

hydrogen. The reaction solution is stirred at room temperature under hydrogen atmosphere for 3 h, using TLC to confirm complete reaction of the raw materials. Filter, evaporate under vacuum to give 70 mg of crude product 1-7, which is directly used in the next reaction step.

Step 11: Preparation of 2-(1,5-dimethyl-3-phenyl-1H-pyrrol-2-yl)-*N*-(2-(5-fluoropyrimidin-2-yl)-1,2,3,4,10,10a-hexahydropyrazino[1,2-*a*]indol-8-yl)-2-oxoacetamide (T-1)

Add compounds 1-7 (70 mg, 0.25 mmol), DCM (5 ml), and DIEA (95 mg, 0.74 mmol) to a reaction flask, cool in ice bath, and drop in a solution of compound A (77 mg, 0.29 mmol) in DCM (1 ml), react overnight at room temperature, and using LC-MS to confirm a complete reaction. Add water to the reaction mixture, extract twice with DCM, and combine the organic phase, wash with saturated saline, dry with anhydrous $Na_2SO_4$, evaporate under vacuum. The crude product is purified through silica gel column to obtain 62 mg of product T-1, with the yield of 50%.
LC-MS (ESI) m/z $(M+H)^+$: 511.2

$^1$H NMR (400 MHz, DMSO-$d_6$) $\delta$ 10.09 (s, 1H), 8.47 (d, $J$ = 0.8 Hz, 2H), 7.26-7.21 (m, 2H), 7.16-7.09 (m, 3H), 6.86- 6.83 (m, 1H), 6.83-6.79 (m, 1H), 6.39 (d, $J$ = 8.4 Hz, 1H), 6.09 (s, 1H), 4.66-4.61 (m, 1H), 4.58-4.52 (m, 1H), 3.81 (s, 3H), 3.66-3.60 (m, 1H), 3.00 (td, $J$ = 12.8, 3.2 Hz, 1H), 2.94-2.83 (m, 2H), 2.78 (td, $J$ = 12.0, 3.2 Hz, 1H), 2.53-2.50 (m, 2H), 2.32 (s, 3H).

**Example 2**

Preparation of 2-(1,5-dimethyl-3-phenyl-1H-pyrrol-2-yl)-*N*-(2-(5-fluoropyrimidin-2-yl)-2,3,4,5,11,11a-hexahydro-1*H*-[1,4]diaza[1,2-*a*]indol-9-yl)-2-oxoacetamide **(T-2-a)** and 2-(1,5-dimethyl-3-phenyl-1*H*-pyrrol-2-yl)-N-(3-(5-fluoropyrimidin-2-yl)-2,3,4,5,11,11a-hexahydro-1*H*-[1,4]diaza[1,7-*a*]indol-9-yl)-2-oxoacetamide **(T-2-b)**

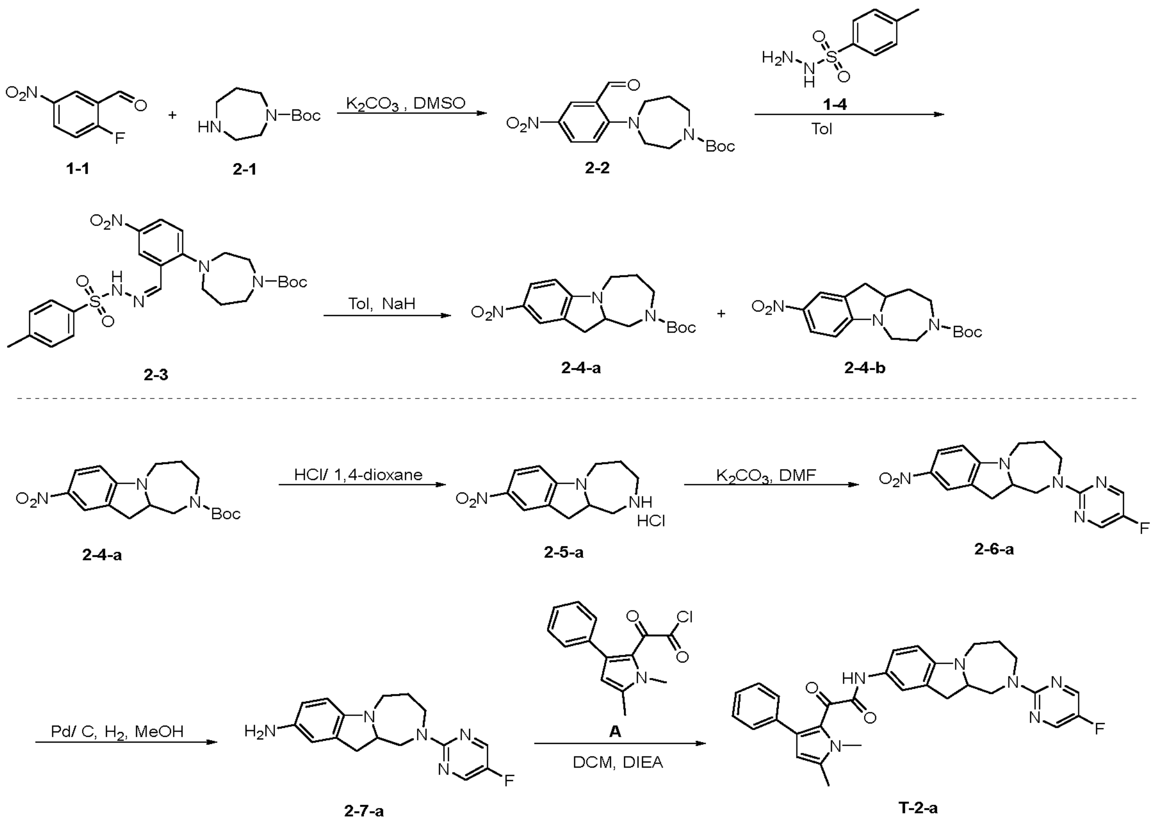


Step 1: Preparation of tert-butyl 4-(2-formyl-4-nitrophenyl)-1,4-diazole-1-carboxylate (2-2)

Add compound 1-1 (2.0 g, 11.8 mmol), 2-1 (2.4 g, 12.0 mmol), DMSO (50 mL) and potassium ycarbonate (2.5 g, 18.1 mmol) sequentially to a reaction flask, heat the reaction mixture to 100 °C and stir overnight, using LC-MS to confirm a complete reaction. Cool, dilute with water, extract with EA, and combine the organic phase, evaporate under vacuum, and purify through silica gel column to give 4.0 g of product 2-2, with the yield of 97%.

Step 2: Preparation of tert-butyl (Z)-4-(4-nitro-2-((2-tosylhydrazineylidene)methyl)phenyl) -1,4-diazepane-1-carboxylate **(2-3)**

Add compound 2-2 (700 mg, 2.0 mmol), 1-4 (410 mg, 2.2 mmol) and toluene (10 ml) sequentially to a reaction flask, stir at room temperature overnight, using TLC to confirm complete reaction of the raw materials. The reaction solution is evaporated under vacuum to give 1.1 g of crude product 2-3, which is directly used in the next reaction step.

Step 3: Preparation of 9-nitro-4,5,11,11a-tetrahydro-1*H*-[1,4] diazepino[1,2-*a*] indole-2(3*H*)-carboxylic acid tert-butyl ester **(2-4-a)** and 9-nitro-1,2,4,5,11,11a-hexahydro-3*H*-[1,4] diazepino [1,7-*a*] indole-3-carboxylic acid tert-butyl ester **(2-4-b)**

Add compound 2-3 (1.11 g, 2.14 mmol), toluene (50 ml), and NaH (515 mg, 12.88 mmol) to a reaction flask, heat to 130 °C and stir for 1 h, using LC-MS to confirm a complete reaction. Cool the mixture to room temperature, and place in the ice bath, dilute with water. Extract twice with ethyl acetate, and combine the organic phase, wash with saturated salt water, dry with anhydrous $Na_2SO_4$, evaporate under vacuum, the crude product is purified through silica gel column to give 280 mg of product 2-4-a and 230 mg of product 2-4-b, with the two-step yields of 42% and 34%, respectively.

Step 4: Preparation of 9-nitro-2,3,4,5,11,11a-hexahydro-1*H*-[1,4]**diazepino**[1,2-*a*]indole hydrochloride **(2-5-a)**

Add compound 2-4-a (280 mg, 0.84 mmol) and 1,4-dioxane solution containing hydrogen chloride (2 ml) to a reaction flask, stir at room temperature for 1 h using TLC to confirm complete reaction of the raw materials. Dilute the reaction mixture with 1,4-dioxane (20 ml), and then evaporate under vacuum to give 260 mg of crude product 2-5-a, which is directly used in the next reaction step.

Step 5: Preparation of 2-(5-fluoropyrimidin-2-yl)-9-nitro-2,3,4,5,11,11a-hexahydro-1H-[1,4]**diazepino**[1,2-a]indole **(2-6-a)**

Add compound 2-5-a (260 mg, 0.85 mmol), DMF (3 ml), 2-chloro-5-fluoropyrmidine (140 mg, 1.06 mmol) and $K_2CO_3$(760 mg, 5.50 mmol) successively to a reaction flask, heat to 100 °C and stir overnight, using LC-MS to confirm a complete reaction. Cool the mixture to room temperature, add water, and extract twice with ethyl acetate. Combine the organic phase, wash with saturated salt water, and dry with anhydrous $Na_2SO_4$, evaporate under vacuum, the crude product is purified through silica gel column to give 140 mg of product 2-6-a, with the two-step yield of 50%.

Step 6: Preparation of 2-(5-fluoropyrimidin-2-yl)-2,3,4,5,11,11a-hexahydro-1*H*-[1,4]**diazepino**[1,2-*a*]indol-9-amine **(2-7-a)**

Add compound 2-6-a (140 mg, 0.42 mmol), methanol (10 mL), and Pd/C (50 mg) sequentially to a reaction flask, then purge with hydrogen, and the reaction solution is stirred at room temperature in hydrogen for 3 h , using TLC to cinfirm complete reaction of the raw materials. Filter, evaporate under vacuum to give 120 mg of crude product 2-7-a, which is directly used in the next reaction step.

Step 7: Preparation of 2-(1,5-dimethyl-3-phenyl-1H-pyrrol-2-yl)-*N*-(2-(5-fluoropyrimidin-2-yl)-2,3,4,5,11,11a-hexahydro-1*H*-[1,4]**diazepino**[1,2-*a*]indol-9-yl)-2-oxoacetamide **(T-2-a)**

Add compound 2-7-a (60 mg, 0.20 mmol), DCM (5 ml) and DIEA (130 mg, 1.00 mmol) to a reaction flask, cool in an ice bath, drop in the solution of compound A (75 mg, 0.29 mmol) in DCM (1 ml), and react overnight at room temperature, using LC-MS to confirm a complete reaction. Add water, and extract twice with DCM. Combine the organic phase, wash with saturated saline, and dry with anhydrous $Na_2SO_4$, evaporate under vacuum. The crude product is purified by thin-layer preparation plates to give 22 mg of product T-2-a, with the yield of 21%.

LC-MS (ESI) m/z $(M+H)^+$: 525.2
$^1$H NMR (400 MHz, DMSO-$d_6$) $\delta$ 10.06 (s, 1H), 8.45 (d, *J*= 0.8 Hz, 2H), 7.25-7.20 (m, 2H), 7.16-7.09 (m, 3H), 6.81-6.76 (m, 2H), 6.31 (d,*J*= 8.8 Hz, 1H), 6.09 (s, 1H), 4.40 (dd, *J*= 14.0 Hz, 3.2 Hz, 1H), 4.14-4.05 (m, 1H), 3.80 (s, 3H), 3.67-3.56 (m, 2H), 3.52-3.42 (m, 1H), 3.21 (dd, *J*= 14.0, 10.0 Hz, 1H), 3.04 (dd, *J*= 16.0, 9.2 Hz, 1H), 2.64-2.56 (m, 1H), 2.56-2.50 (m, 1H), 2.31 (s, 3H), 1.98-1.84 (m, 2H).

Compound T-2-b is synthesized using methods similar to that described in steps of four to seven in Example 2.

LC-MS (ESI) m/z $(M+H)^+$: 525.2

$^1H$ NMR (400 MHz, DMSO-$d_6$) δ 10.04 (s, 1H), 8.43 (d, *J*= 0.8 Hz, 2H), 7.24-7.19 (m, 2H), 7.15-7.08 (m, 3H), 6.78-6.72 (m, 2H), 6.24 (d, *J*= 8.4 Hz, 1H), 6.08 (s, 1H), 4.13-3.95 (m, 2H), 3.86-3.81 (m, 1H), 3.80 (s, 3H), 3.76-3.70 (m, 1H), 3.70-3.50 (m, 3H), 3.07 (dd, *J*= 16.0, 8.8 Hz, 1H), 2.85-2.76 (m, 1H), 2.31 (s, 3H), 2.06-1.99 (m, 1H), 1.85-1.75 (m, 1H).

**Example 3**

Preparation of 2-(1,5-dimethyl-3-phenyl-1*H*-pyrrol-2-yl)-*N*-(3-(5-fluoropyrimidin-2-yl)-1,2,3,4,4a,5-hexahydrobenzo[*b*]pyrazino[1,2-*d*][1,4]oxazin-8-yl)-2-oxoacetamide **(T-3)**

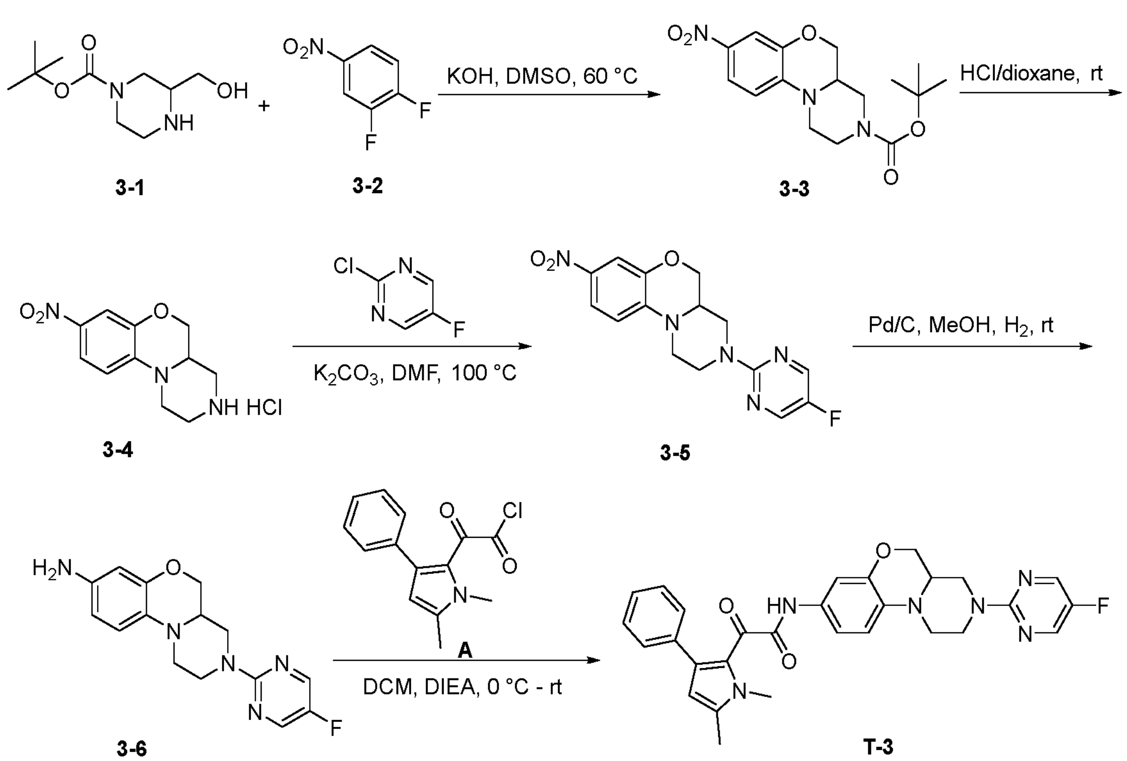


Step 1: Preparation of **tert-butyl** 8-nitro-1,2,4a,5-tetrahydrobenzo[*b*]pyrazino[1,2-d][1,4]oxazine-3(4*H*)-**carboxylate (3-3)**

Add compound 3-1 (680 mg, 3.15 mmol) and DMSO (10 mL) to a reaction flask, then add 3-2 (500 mg, 3.15 mmol) and potassium hydroxide (530 mg, 9.45 mmol), and the reaction solution is purged with nitrogen. Heat to 60 °C and stir overnight, using LC-MS to confirm a complete reaction. Cool, add water, extract twice with dichloromethane. Combine the organic phase, and wash with water and saturated salt water respectively, dry with anhydrous $Na_2SO_4$, and evaporate under vacuum. The crude product is purified through silica gel column to give 650 mg of product 3-3, with the yield of 61%.

Step 2: Preparation of 8-nitro-1,2,3,4,4a,5-hexahydrobenzo[b]pyrazino[1,2-d][1,4]oxazine hydrochloride (3-4)

Add compound 3-3 (650 mg, 1.94 mmol) and 1,4-dioxane (3 mL) to a reaction flask, then add 1, 4-dioxane solution of hydrogen chloride (4 M in 1,4-dioxane, 10 mL). The reaction solution is stirred at room temperature for 2 h, using TLC to confirm complete reaction of the raw materials. Evaporate under vacuum to give 550 mg of crude product 3-4, which is directly used in the next reaction step without purification.

Step 3: Preparation of 3-(5-fluoropyrimidin-2-yl)-8-nitro-1,2,3,4,4a,5-hexahydrobenzo[*b*]pyrazino[1,2-*d*] [1,4] oxazine **(3-5)**

Add compound 3-4 (550 mg, crude) and DMF (10 mL) to a reaction flask, followed by 2-chloro-5-fluoropyrimidine (324 mg, 2.43 mmol) and potassium carbonate (840 mg, 6.08 mmol), and the reaction solution is purged with nitrogen. Heat the reaction mixture to 100 °C and stir overnight, using LC-MS to confirm a complete reaction. Cool, add water, and extract twice with ethyl acetate. Combine the organic phase, wash with water and then saturated salt water, dry with anhydrous $Na_2SO_4$, and evaporate under vacuum. The crude product is purified through silica gel column to give 330 mg of product 3-5, with the two-step yield of 51%.

Step 4: Preparation of 3-(5-fluoropyrimidin-2-yl)-1,2,3,4,4a,5-hexahydrobenzo[*b*]pyrazino[1,2-*d*] [1,4] oxazin-8-amine **(3-6)**

Add compound 3-5 (330 mg, 0.99 mmol) and methanol (10 mL) to a reaction flask, then add Pd/C (66 mg). The reaction solution is purged with nitrogen and then with hydrogen. Stir overnight at room temperature in hydrogen atmosphere, using TLC to confirm complete reaction of the raw materials. Filter, and the filtrate is evaporate under vacuum and the crude product is purified through silica gel column to give 150 mg of product 3-6, with the yield of 50%.

Step 5: Preparation of 2-(1,5-dimethyl-3-phenyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-3)**

Add compound 3-6 (50 mg, 0.17 mmol), DIEA (66 mg, 0.51 mmol) and DCM (3 mL) to a reaction flask, and the reaction solution is purged with nitrogen and cool to 0 °C in ice bath. Drop DCM (2 mL) solution of compound A (52 mg, 0.20 mmol) to the reaction solution, and stir overnight while the temperature of reaction solution is slowly recovered to room temperature, using LC-MS to confirm a complete reaction. Combine the organic phase, wash with saturated salt water, dry with anhydrous $Na_2SO_4$, evaporate under vacuum, and the crude product is purified through thin-layer preparation plates to give 80 mg of product T-3, with the yield of 91%.

LC-MS (ESI) m/z $(M+H)^+$: 527.2
$^1H$ NMR (400 MHz, DMSO-$d_6$) δ 10.17 (s, 1H), 8.50 (s, 2H), 7.25-7.20 (m, 2H), 7.16-7.09 (m, 3H), 6.77-6.72 (m, 1H), 6.68 (dd J*J*= 8.8, 2.4 Hz, 1H), 6.64 (d, *J*= 2.4 Hz, 1H), 6.10 (s, 1H), 4.65-4.51 (m, 2H), 4.34 (dd, *J*= 10.8 Hz, 2.4 Hz, 1H), 3.92 (dd, *J*= 10.8 Hz, 8.8 Hz, 1H), 3.84-3.76 (m, 4H), 3.12-2.96 (m, 2H), 2.72-2.55 (m, 2H), 2.32 (s, 3H).

**Examples 4~6: Preparation of compounds T-4~T-6**

Compounds **of T-4 to T-6** are prepared by the methods used to prepare compound T-3 and using different raw materials (including replacing compound 3-2 in the first step in example 3 with 1, 2, 3-trifluoro-5-nitrobenzene (4-2), and replacing compound 3-1 in the first step in Example 3 with (R)-3-(hydroxymethyl) piperazine-1-carboxylic acid tert-butyl ester (5-1) and (S)-3-(hydroxymethyl) piperazine-1-carboxylic acid tert-butyl ester (6-1), respectively). The structural formulas of **T-4 to T-6,** LC-MS and $^1H$-NMR data are shown in Table 1.

**Table 1: Structures, LC-MS and $^1H$-NMR data of Examples 4 to 6**

| Examples | Compound structure | LC-MS: (ESI) m/z | $^1H$ NMR |
|---|---|---|---|
| 4 | 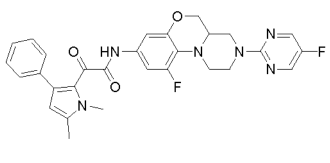 | 545.2 | $^1H$ NMR (400 MHz, DMSO-$d_6$) δ 10.38 (s, 1H), 8.49 (d, *J* = 0.4 Hz, 2H), 7.22-7.17 (m, 2H), 7.15-7.09 (m, 3H), 6.61 (dd, *J* = 15.2, 2.4 Hz, 1H), 6.55-6.51 (m, 1H), 6.11 (d, *J* = 0.4 Hz, 1H), 4.26 (dd, *J* = 10.8 Hz, 2.8 Hz, 1H), 4.15 (dd, *J* = 13.2 Hz, 3.6 Hz, 1H), 4.08-4.01 (m, 1H), 3.95 (dd, *J* = 10.8 Hz, 9.2 Hz, 1H), 3.82 (s, 3H), 3.68-3.60 (m, 1H), 3.59-3.51 (m, 1H), 3.39 (dd, *J* = 13.2 Hz, 7.6 Hz, 1H), 3.22-3.13 (m, 1H), 2.97 (ddd, J = 11.6, 8.4, 2.8 Hz, 1H), 2.32 (s, 3H). |

(continued)

| Examples | Compound structure | LC-MS: (ESI) m/z | $^1$H NMR |
|---|---|---|---|
| 5 | 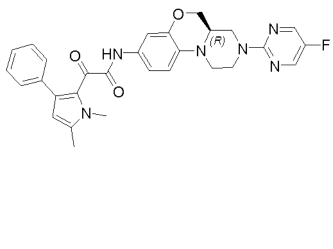 | 527.2 | $^1$H NMR (400 MHz, DMSO-$d_6$) δ 10.17 (s, 1H), 8.49 (s, 2H), 7.22-7.17 (m, 2H), 7.15-7.07 (m, 3H), 6.61 (dd, $J$ = 14.8 Hz, 2.4 Hz, 1H), 6.55-6.52 (m, 1H), 6.11 (d, $J$ = 0.4 Hz, 1H), 4.26 (dd, $J$ = 10.8, 2.4 Hz, 1H), 4.15 (dd, $J$ = 13.2, 3.6 Hz, 1H), 4.08-4.00 (m, 1H), 3.95 (dd, $J$ = 10.8, 9.2 Hz, 1H),3.80 (s, 3H), 3.68-3.60 (m, 1H), 3.60-3.51 (m, 1H), 3.39 (dd, $J$ = 13.2, 7.6 Hz, 1H), 3.21-3.14 (m, 1H), 2.97 (ddd, $J$ = 11.6, 8.4, 2.8 Hz, 1H), 2.32 (s, 3H). |
| 6 | 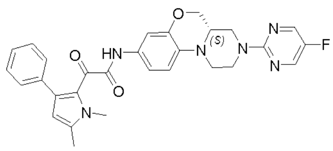 | 527.2 | $^1$H NMR (400 MHz, DMSO-$d_6$) δ 10.17 (s, 1H), 8.51 (s, 2H), 7.25-7.20 (m, 2H), 7.16-7.09 (m, 3H), 6.78-6.73 (m, 1H), 6.68 (dd, $J$ = 8.8, 2.4 Hz, 1H), 6.64 (d, $J$ = 2.4 Hz, 1H), 6.11 (s, 1H), 4.66-4.51 (m, 2H), 4.34 (dd, $J$ = 10.8 Hz, 2.4 Hz, 1H), 3.97-3.89 (m, 1H), 3.84-3.78 (m, 4H), 3.12-2.96 (m, 2H), 2.72-2.55 (m, 2H), 2.32 (s, 3H). |

**Example** 7

Preparation of N-(3-(5-cyanopyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxyacetamide **(T-7)**

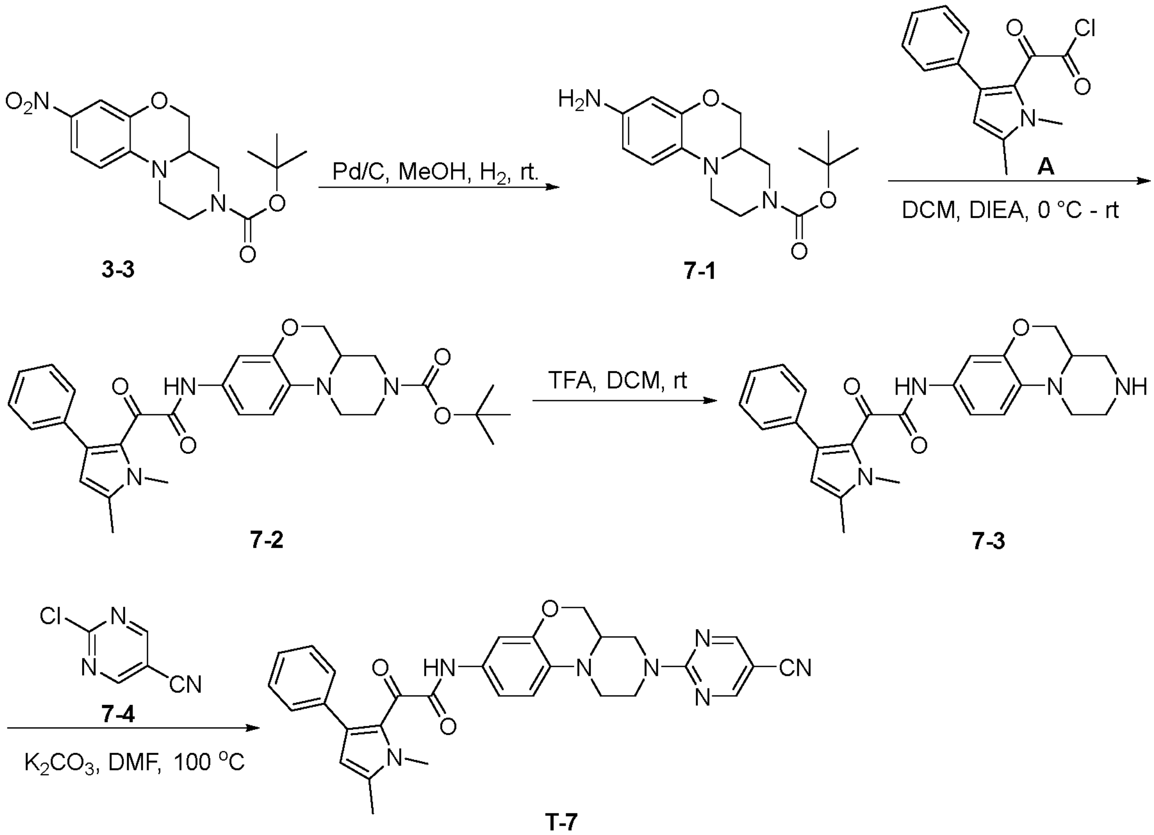

3-3
7-1
7-2
7-3
T-7

Step 1: Preparation of **tert-butyl** 8-amino-1,2,4a,5-tetrahydrobenzo[*b*]pyrazino[1,2-*d*] [1,4] oxazine-3(4*H*)-**carboxylate (7-1)**

Add compound 3-3 (1 g, 2.99 mmol) and methanol (20 mL) to a reaction flask, then add Pd/C (200 mg), and the reaction solution is purged with nitrogen and then hydrogen. The reaction solution is stirred at room temperature in hydrogen atmosphere for 5 h, using TLC to confirm that the reaction of raw materials is complete. Filter, evaporate under vacuum, the crude product is purified through silica gel column to give 850 mg of product 7-1, with the yield of 93%.

Step 2: Preparation of tert-butyl 8-(2-(1,5-dimethyl-3-phenyl-1*H*-pyrrol-2-yl)-2-oxoacetacylamino)-1,2,4a,5-tetrahydrobenzo[*b*]pyrazino[1,2-*d*] [1,4] oxazine-3(4*H*)-carboxylate **(7-2)**

Add compound 7-1 (0.85 g, 2.79 mmol), DIEA (1.08 g, 8.37 mmol) and DCM (5 mL) to a reaction flask, purge the reaction solution with nitrogen, cool to 0 °C in ice bath, then drop in the solution of compound A (0.73 g, 2.79 mmol) in DCM (5 mL). The reaction solution is slowly heated to room temperature and stirred for 4 h, using LC-MS to confirm that the reaction is complete. Add water, and extract twice with dichloromethane. Combine the organic phase, wash with saturated salt water, dry with anhydrous $Na_2SO_4$, and evaporate under vacuum. The crude product is purified through silica gel column to give 1.20 g of product 7-2, with the yield of 81%.

Step 3: Preparation of 2-(1,5-dimethyl-3-phenyl-1*H*-pyrrol-2-yl)-*N*-(1,2,3,4,4a,5-hexahydrobenzo[*b*]pyrazino[1,2-*d*] [1,4] oxazin-8-yl)-2-oxoacetamide **(7-3)**

Add compound 7-2 (1.20 g, 2.26 mmol) and dichloromethane solution (15 mL) to a reaction flask, drop trifluoroacetic acid (5 mL) to the reaction solution, and then the reaction solution is stirred at room temperature for 4 h, using TLC to confirm complete reaction of the raw materials. The reaction solution is adjusted with saturated sodium bicarbonate solution to a pH of about 8, then add an appropriate amount of water, extract three times with dichloromethane. Combine the organic phase, wash with saturated salt water, dry with anhydrous $Na_2SO_4$, evaporate under vacuum, and the crude product is purified through silica gel column to give 900 mg of product 7-3, with the yield of 92%.

Step 4: Preparation of *N*-(3-(5-cyanopyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-7)**

Add compound 7-3 (50 mg, 0.12 mmol) and DMF (1 mL) to a reaction flask, then add 7-4 (26 mg, 0.18 mmol) and potassium carbonate (50 mg, 0.36 mmol), and the reaction solution is purged with nitrogen. Heat to 100 °C and stir overnight, using LC-MS to confirm a complete reaction. Cool, add water, and extract twice with ethyl acetate. Combine the organic phase, wash with water, and saturated salt water respectively, dry with anhydrous $Na_2SO_4$, evaporate under vacuum, and the crude product is purified through thin-layer preparation plates to give 30 mg of product T-7, with the yield of 48%.

LC-MS (ESI) m/z $(M+H)^+$: 534.2
$^1$H NMR (400 MHz, DMSO-$d_6$) $\delta$ 10.18 (s, 1H), 8.81 (s, 2H), 7.24-7.21 (m, 2H), 7.15-7.10 (m, 3H), 6.75 (d, *J*= 8.8 Hz, 1H), 6.70- 6.63 (m, 2H), 6.11 (s, 1H), 4.84-4.70 (m, 2H), 4.36 (dd, *J*= 10.8 Hz, 2.8 Hz, 1H), 3.98-3.91 (m, 1H), 3.89-3.83 (m, 1H), 3.81 (s, 3H) , 3.26-3.16 (m, 1H), 3.09-2.99 (m, 1H), 2.89-2.80 (m, 1H), 2.67-2.58 (m, 1H), 2.33 (s, 3H).

**Examples 8 to 15: Preparation of Compounds of T-8 to T-15**

Compounds **of T-8 to T-15** are prepared by the methods used to prepare compound T-7 and using different raw materials (including replacing compound 7-4 in the fourth step in example 7 with 2-chloro-5-methoxypyrimidine (8-4), 2-chloro-5-(trifluoromethyl) pyrimidine (9-4), 2,4-dichloropyrimidine (10-4), 2-chlorothiazole-5-carbonitrile (12-4), 3,6-difluoropyridazine (13-4), 2,5-difluoropyridine (14-4) and 2-bromo-5-methyl-1,3,4-oxadiazole (15-4)). The structural formulas **of T-8 to T-15,** LC-MS and $^1$H-NMR data are shown in Table 1.

**Table 2: Structures, LC-MS and $^{1}$H-NMR data of examples 8 to 15**

| Examples | Compound structure | LC-MS: (ESI) m/z | $^{1}$H NMR |
|---|---|---|---|
| 8 | 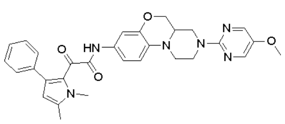 | 539.2 | $^{1}$H NMR (400 MHz, DMSO-$d_6$) δ 10.17 (s, 1H), 8.25 (s, 2H), 7.24-7.20 (m, 2H), 7.16-7.09 (m, 3H), 6.75 (d, *J* = 8.8 Hz, 1H), 6.66 (dd, *J* = 8.8, 2.4 Hz, 1H), 6.63 (d, *J* = 2.4 Hz, 1H), 6.10 (s, 1H), 4.60-4.47 (m, 2H), 4.34 (dd, *J* = 10.8 Hz, 2.8 Hz, 1H), 3.92 (dd, *J* = 10.8 Hz, 8.8 Hz, 1H), 3.80 (s, 3H), 3.78 (s, 3H), 3.82-3.76 (m, 2H), 3.04-2.92 (m, 2H), 2.63-2.51 (m, 2H), 2.32 (s, 3H). |
| 9 | 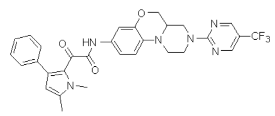 | 577.2 | $^{1}$H NMR (400 MHz, DMSO-$d_6$) δ 10.18 (s, 1H), 8.77 (s, 2H), 7.26-7.21 (m, 2H), 7.17-7.08 (m, 3H), 6.76 (d, *J* = 8.8 Hz, 1H), 6.68 (dd, *J* = 8.8, 2.4 Hz, 1H), 6.65-6.63 (m, 1H), 6.11 (s, 1H), 4.84-4.71 (m, 2H), 4.36 (dd, *J* = 10.8 Hz, 2.4 Hz, 1H), 3.95 (dd, *J* = 10.8 Hz, 8.8 Hz, 1H), 3.89-3.80 (m, 1H), 3.81 (s, 3H), 3.24-3.15 (m, 1H), 3.08-3.00 (m, 1H), 2.87-2.78 (m, 1H), 2.66-2.58 (m, 1H), 2.33 (s, 3H). |
| 10 | 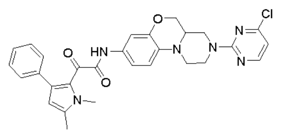 | 543.2 | $^{1}$H NMR (400 MHz, DMSO-$d_6$) δ 10.17 (s, 1H), 8.37 (d, *J* = 0.8 Hz, 1H), 7.25-7.20 (m, 2H), 7.17-7.09 (m, 3H), 6.80 (d, *J* = 5.2 Hz, 1H), 6.78-6.73 (m, 1H), 6.71-6.22 (m, 2H), 6.11 (s, 1H), 4.70-4.52 (m, 2H), 4.36 (dd, *J* = 10.8 Hz, 2.4 Hz, 1H), 3.97-3.89 (m, 1H), 3.87-3.82 (m, 1H), 3.81 (s, 3H), 3.15-2.97 (m, 2H), 2.77-2.55 (m, 2H), 2.33 (s, 3H). |
| 11 | 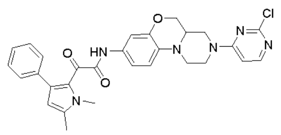 | 543.2 | $^{1}$H NMR (400 MHz, DMSO-$d_6$) δ 10.18 (s, 1H), 8.13 (d, *J* = 6.0 Hz, 1H), 7.22-7.20 (m, 2H), 7.16-7.09 (m, 3H), 6.95 (d, *J* = 6.0 Hz, 1H), 6.78-6.74 (m, 1H), 6.71-6.66 (m, 1H), 6.66-6.63 (m, 1H), 6.11 (s, 1H), 4.52-4.28 (m, 3H), 3.96-3.90 (m, 1H), 3.86-3.82 (m, 1H), 3.81 (s, 3H), 3.15-2.99 (m, 2H), 2.82-2.72 (m, 1H), 2.70-2.58 (m, 1H), 2.33 (s, 3H). |
| 12 | 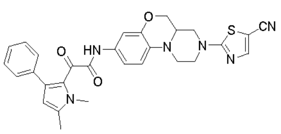 | 539.2 | $^{1}$H NMR (400 MHz, $CDCl_3$) δ 8.18 (s, 1H), 7.73 (s, 1H), 7.34-7.23 (m, 5H), 6.79-6.75 (m, 1H), 6.73-6.68 (m, 2H), 6.15 (s, 1H), 4.28 (dd, *J* = 10.8 Hz, 2.4 Hz, 1H), 4.09-4.01 (m, 2H), 4.01-3.94 (m, 1H), 3.83 (s, 3H), 3.83-3.79 (m, 1H), 3.43 (td, *J* = 12.4, 3.2 Hz, 1H),3.34-3.26 (m, 1H), 3.08-3.00 (m, 1H), 2.99-2.90 (m, 1H), 2.35 (s, 3H). |
| 13 | 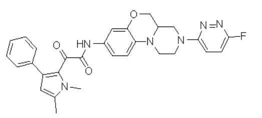 | 527.2 | $^{1}$H NMR (400 MHz, DMSO-$d_6$) δ 10.18 (s, 1H), 7.71-7.63 (m, 1H), 7.48 (d, *J* = 9.6 Hz, 1H), 7.26-7.21 (m, 2H), 7.17-7.09 (m, 3H), 6.11 (s, 1H), 4.41-4.28 (m, 3H), 3.99-3.91 (m, 1H), 3.87-3.82 (m, 1H), 3.82 (s, 3H), 3.12-3.02 (m, 2H), 2.75-2.63 (m, 2H), 2.33 (s, 3H). |
| 14 | 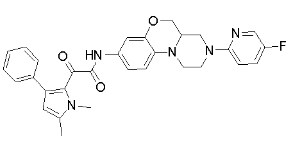 | 526.2 | $^{1}$H NMR (400 MHz, DMSO-$d_6$) δ 10.18 (s, 1H), 8.13 (d, *J* = 3.2 Hz, 1H), 7.59-7.53 (m, 1H), 7.25-7.19 (m, 2H), 7.16-7.08 (m, 3H), 6.98 (dd, *J* = 9.6, 3.6 Hz, 1H), 6.78-6.73 (m, 1H), 6.70-6.61 (m, 2H), 6.10 (s, 1H), 4.31 (dd, *J* = 10.8, 2.4 Hz, 1H), 4.28-4.19 (m, 2H), 3.92 (dd, *J* = 10.4 Hz, 8.8 Hz, 1H), 3.80 (s, 3H), 3.79-3.74 (m, 1H), 3.07-2.98 (m, 1H), 2.96-2.87 (m, 1H), 2.69-2.59 (m, 1H), 2.55-2.50 (m, 1H), 2.32 (s, 3H). |

(continued)

| Examples | Compound structure | LC-MS: (ESI) m/z | $^1H$ NMR |
|---|---|---|---|
| 15 | 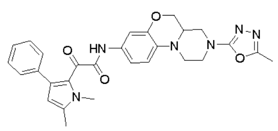 | 513.2 | $^1H$ NMR (400 MHz, $CDCl_3$) δ 8.16 (s, 1H), 7.34-7.23 (m, 5H), 6.78-6.74 (m, 1H), 6.71-6.65 (m, 2H), 6.15 (s, 1H), 4.25 (dd, $J$ = 10.8 Hz, 2.4 Hz, 1H), 4.06-3.97 (m, 2H), 3.94-3.88 (m, 1H), 3.83 (s, 3H), 3.78-3.71 (m, 1H), 3.33-3.22 (m, 2H),2.95-2.84 (m, 2H), 2.44 (s, 3H), 2.35 (s, 3H). |

**Example 16**

Preparation of 2-(1,5-dimethyl-3-phenyl-1*H*-pyrrol-2-yl)-N (3-(5-fluoro-6-oxo-1,6-dihydropyrimidin-2-yl)-1,2,3,4,4a,5-hexahydrobenzo[*b*]pyrazino[1,2-*d*] [1,4] oxazin-8-yl)-2-oxoacetamide **(T-16)**

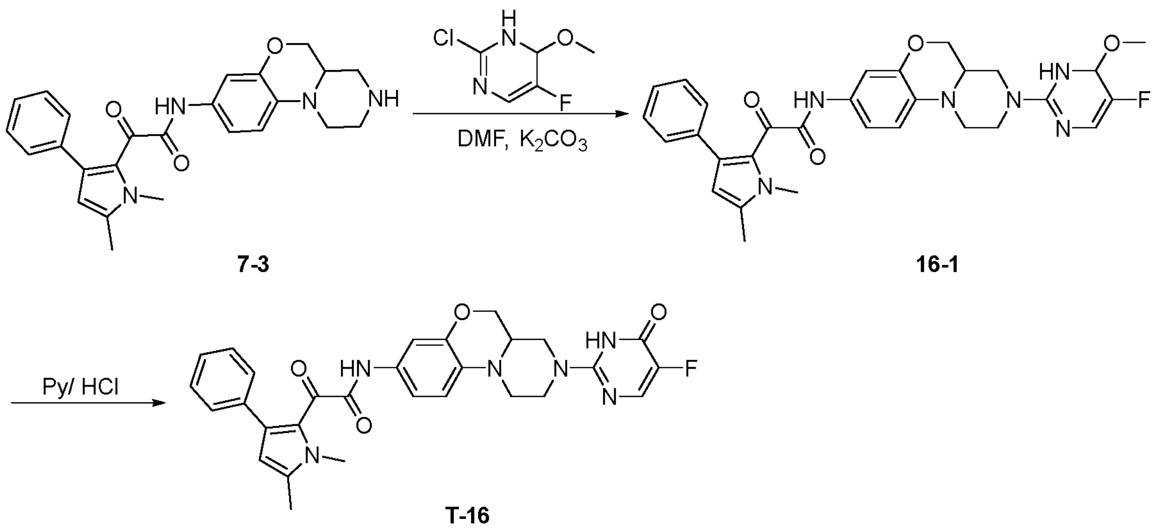


**7-3**

**16-1**

**T-16**

Step 1: Preparation of 2-(1,5-dimethyl-3-phenyl-1*H*-pyrrol-2-yl)-*N*-(3-(5-fluoro-6-methoxy-1,6-dihydropyrimidin-2-yl)-1,2,3,4,4a,5-hexahydrobenzo[*b*]pyrazino[1,2-*d*] [1,4] oxazin-8-yl)-2-oxoacetamide **(16-1)**

Add compound 7-3 (120 mg, 0.28 mmol), 2-chloro-5-fluoro-6-methoxy-1,6-dihydropyrimidine (45 mg, 0.28 mmol), DMF (3 mL) and potassium carbonate (154 mg, 1.11 mmol) sequentially to a reaction flask, and the reaction mixture is heated to 100 °C and stirred overnight, using LC-MS to confirm that the reaction is complete. Cool, dilute with water, and extract with EA. Combine the organic phase, evaporate under vacuum, the crude product is purified through silica gel column to give 50 mg of product 16-1, with the yield of 32%.

Step 2: Preparation of 2-(1,5-dimethyl-3-phenyl-1*H*-pyrrol-2-yl)-*N*-(3-(5-fluoro-6-oxo-1,6-dihydropyrimidin-2-yl)-1,2,3,4,4a,5-hexahydrobenzo[*b*]pyrazino[1,2-*d*] [1,4] oxazin-8-yl)-2-oxoacetamide **(T-16)**

Add compound 16-1 (30 mg, 0.05 mmol) and pyridine hydrochloride (300 mg) to a reaction flask. Purge with nitrogen to protect the reaction mixture, heat to 140 °C, and stir for 30 min, using TLC to confirm that the reaction is complete. Cool to room temperature, and the crude product is purified by reversed-phase HPLC preparation (C-18 column) to give 15 mg of product T-16, with the yield of 50%.

LC-MS (ESI) m/z $(M+H)^+$: 543.2

$^1H$ NMR (400 MHz, DMSO-$d_6$) δ 10.16 (s, 1H),7.66 (s, 1H), 7.24-7.18 (m, 2H), 7.15-7.09 (m, 3H), 6.75-6.71 (m, 1H), 6.67- 6.63 (m, 1H), 6.63-6.60 (m, 1H), 6.09 (s, 1H), 4.41-4.26 (m, 2H), 4.26-4.21 (m, 1H), 3.91-3.84 (m, 1H),

3.79 (s, 3H), 3.75-3.68 (m, 1H), 2.99-2.88 (m, 2H), 2.63-2.52 (m, 2H), 2.31 (s, 3H).

**Example 17**

Preparation of 2-(1,5-dimethyl-3-phenyl-1*H*-pyrrol-2-yl)-*N*-(3-(1-methyl-1H-1,2,4-triazol-3-yl)-1,2,3,4,4a,5-hexahydrobenzo[*b*]pyrazino[1,2-*d*] [1,4] (oxazin-8-yl)-2-oxoacetamide **(T-17)**

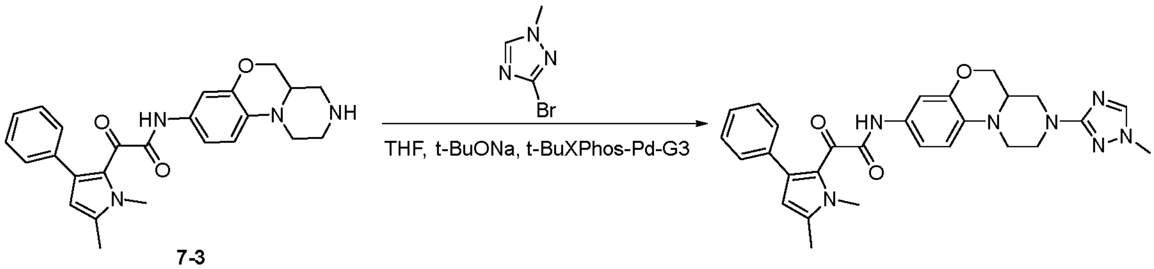


7-3

T-17

Compound 7-3 (110 mg, 0.26 mmol), 3-bromo-1-methyl-1H-1,2,4-triazole (82.5 mg, 0.50 mmol), THF (10 mL) and tert-butanol sodium (99 mg, 1.03 mmol), t-BuXPhos-Pd-G3 (99 mg, 0.12 mmol) sequentially to a reaction flask. The reaction mixture is heated to 90 °C and stirred overnight under nitrogen protection. LC-MS to confirm that about 30% of the raw material 7-3 is not reacted. The reaction system is cool, diluted with water, extracted with EA. Combine the organic phase, spin-dry under vacuum, purify with thin-layer preparation plates to give 20 mg of product T-17, with the yield of 15%.

LC-MS (ESI) m/z $(M+H)^+$: 512.2

$^1$H NMR (400 MHz, DMSO-$d_6$) $\delta$ 10.17 (s, 1H), 8.12 (s, 1H), 7.24-7.20 (m, 2H), 7.15-7.09 (m, 3H), 6.73 (d,*J*= 8.8 Hz, 1H), 6.66 (dd, *J*= 8.8, 2.4 Hz, 1H), 6.62 (d, *J*= 2.4 Hz, 1H), 6.10 (s, 1H), 4.30 (dd, *J*= 10.8 Hz, 2.4 Hz, 1H), 3.98-3.85 (m, 3H), 3.80 (s, 3H), 3.77-3.72 (m, 1H), 3.69 (s, 3H), 3.08-2.99 (m, 1H), 2.92-2.83 (m, 1H), 2.69-2.61 (m, 1H), 2.55-2.51 (m, 1H), 2.32 (s, 3H).

**Example 18**

Preparation of 2-(1,5-dimethyl-3-phenyl-1*H*-pyrrol-2-yl)-*N*-(3-(5-fluoropyrimidin-2-yl)-1,2,3,4,4a,5-hexahydro-7*H*-benzo[*e*]pyrazino[2,1-*c*] [1,4] oxazolin-9-yl)-2-oxoacetamide **(T-18)**

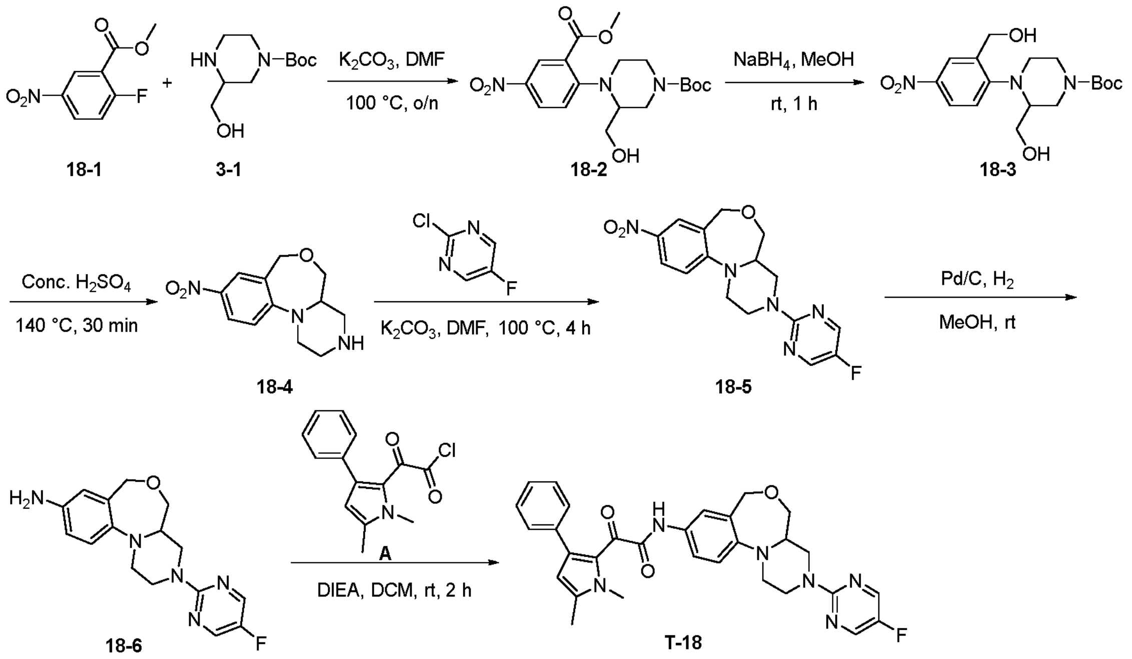


18-1 3-1 18-2 18-3 18-4 18-5 18-6 T-18

Step 1: Preparation of **tert-butyl** 3-(hydroxymethyl)-4-(2-(methoxycarbonyl)-4-nitrophenyl) piperazine-1-**carboxylate (18-2)**

Add compound 18-1 (2.0 g, 10.0 mmol), 3-1 (2.2 g, 10.2 mmol) and N, N-dimethylformamide (12 mL) to a single neck flask, then add potassium carbonate (2.8 g, 20.3 mmol). The reaction mixture is heated to 100 °C and stirred overnight, using TLC to confirm that the reaction is complete. Cool, add water (50 mL), and extract with ethyl acetate (50 mL × 4). Combine the organic phase, wash with saturated saline (50 mL × 2), dry with anhydrous $Na_2SO_4$, evaporate under vacuum, and the crude product is purified by column chromatography to give 2.7 g of product 18-2, with the yield of 68%.

Step 2: Preparation of **tert-butyl** 3-(hydroxymethyl)-4-(2-(hydroxymethyl)-4-nitrophenyl) piperazine-1-**carboxylate (18-3)**

Add compound 18-3 (500 mg, 1.3 mmol) and methanol (10 ml) to a reaction flask, the reaction solution is purged with nitrogen and cool in an ice bath, then add sodium borohydride (239 mg, 6.3 mmol) in portions. The reaction solution is slowly heated to room temperature, stirred for 1 h, using TLC to confirm complete reaction of the raw materials. Add water to stop the reaction, and extract twice with ethyl acetate. Combine the organic phase, wash with saturated salt water, dry with anhydrous $Na_2SO_4$, and evaporate under vacuum. The crude product is purified through silica gel column to give 450 mg of product 18-3, with the yield of 97%.

Step 3: Preparation of 9-nitro-1,2,3,4,4a,5-hexahydro-7*H*-benzo[*e*]pyrazino[2,1-*c*] [1,4] oxazepine **(18-4)**

Add compound 18-3 (400mg, 1.1 mmol) to a reaction flask, then add concentrated sulphuric acid (3 ml). Heat the mixture to 140°C and stir for 30 min, using LC-MS to confirm a complete reaction. Cool to room temperature, add saturated sodium carbonate solution, adjusting pH to 8. Evaporate the mixture under vacuum to dryness, and suspend the residue with DCM/MeOH = 10/1, stir for 10 min, filter. Evaporate the filtrate under vacuum to get 440 mg crude product, and use for next step without purification.

Step 4: Preparation of 3-(5-fluoropyrimidin-2-yl)-9-nitro-1,2,3,4,4a,5-hexahydro-7*H*-benzo[e]pyrazino[2,1-c] [1,4] oxazepine **(18-5)**

Add compound 18-4 (440 mg, crude), DMF (6 ml), 2-chloro-5-fluoropyrimidine (428 mg, 3.24 mmol) and $K_2CO_3$(448 mg, 3.24 mmol) sequentially to a reaction flask. The reaction solution is heated to 100 °C, and stirred for 4 h, using LC-MS to confirm the reaction is complete. Cool the mixture to room temperature, add water, and extract twice with ethyl acetate. Combine the organic phase, wash with saturated salt water, dry with anhydrous $Na_2SO_4$, and evaporate under vacuum. The crude product is purified through silica gel column to give 175 mg of product 18-5, with the two-step

yield of 47%.

Step 5: Preparation of 3-(5-fluoropyrimidin-2-yl)-1,2,3,4,4a,5-hexahydro-7H-benzo[e]pyrazino[2,1-c] [1,4] oxazepin-9-amine **(18-6)**

Add compound 18-5 (70 mg, 0.20 mmol), methanol (6 mL) and ammonia (0.5 mL), and Pd/C (50 mg) sequentially to a reaction flask. The reaction solution is stirred at room temperature in hydrogen atmosphere for 3 h, using TLC to confirm that the reaction of the raw materials is complete. Filter, the filtrate is evaporate under vacuum to give 50 mg of crude product 18-6, which is directly used in the next reaction step.

Step 6: Preparation of 2-(1,5-dimethyl-3-phenyl-1*H*-pyrrol-2-yl)-*N*-(3-(5-fluoropyrimidin-2-yl)-1,2,3,4,4a,5-hexahydro-7*H*-benzo[e]pyrazino[2,1-*c*] [1,4] oxazolin-9-yl)-2-oxoacetamide **(T-18)**

Compound T-18 is synthesized using a similar method to that described in step 11 of example 1.

LC-MS (ESI) m/z $(M+H)^+$: 541.2

$^1H$ NMR (400 MHz, DMSO-$d_6$) δ 10.30 (s, 1H), 8.49 (s, 2H), 7.26-7.21 (m, 2H), 7.16-7.05 (m, 4H), 6.91 (d, *J*= 2.4 Hz, 1H), 6.85 (d, *J*= 8.8 Hz, 1H), 6.11 (s, 1H), 4.63 (d, *J*= 12.0 Hz, 1H), 4.45 (d, *J*= 12.0 Hz, 1H), 4.18-4.08 (m, 2H), 3.84 (s, 3H), 3.72-3.67 (m, 1H), 3.63-3.53 (m, 2H), 3.49-3.42 (m, 1H), 3.34-3.28 (m, 2H), 2.98 -2.91 (m, 1H), 2.33 (s, 3H).

**Example 19**

Preparation of 2-(1,5-dimethyl-3-phenyl-1*H*-pyrrol-2-yl)-*N*-(3-(5-fluoropyrimidin-2-yl)-6,6-dioxido-1,2,3,4,4a,5-hexahydrobenzo[*b*]pyrazino[1,2-*d*] [1,4] thiazine-8-yl)-2-oxoacetamide **(T-19)**

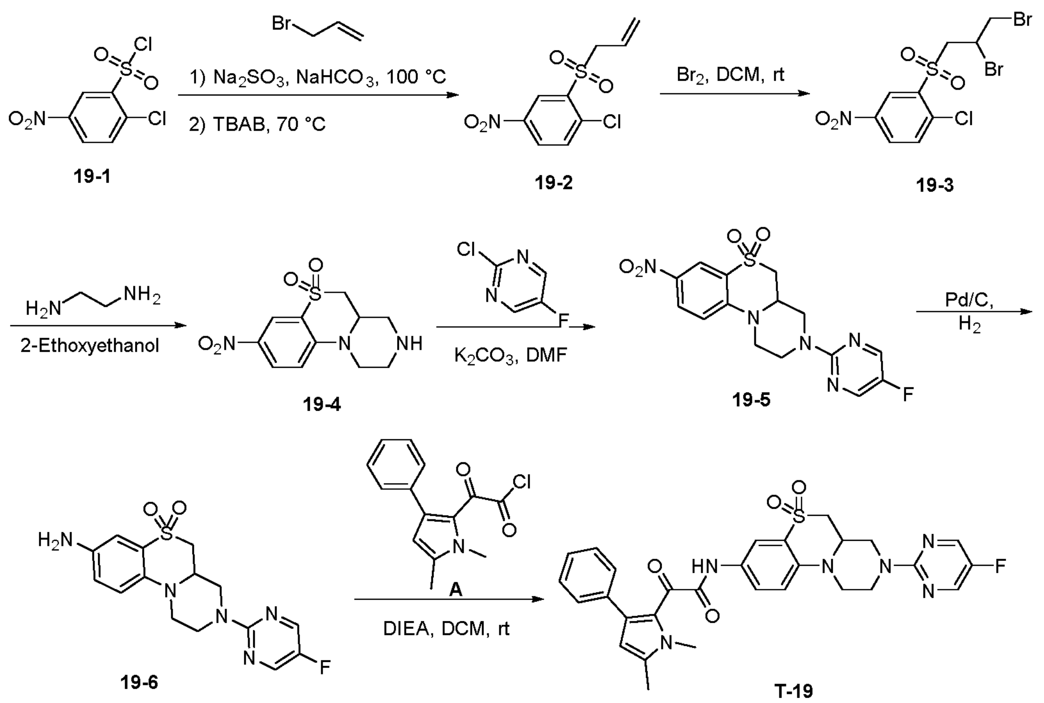


19-1 19-2 19-3 19-4 19-5 19-6 T-19

Step 1: Preparation of 2-(allylsulfonyl)-1-chloro-4-nitrobenzene **(19-2)**

Add compound 19-1 (500 mg, 1.95 mmol), $Na_2SO_3$(492 mg, 3.90 mmol) and water (8 mL) to a reaction flask. The reaction mixture is heated to 100 °C and stirred for 1 h, using TLC to confirm no residual material. Cool to room temperature, add 3-bromopropene (1.2 g, 9.92 mmol) and tetrabutylammonium bromide (39 mg, 0.12 mmol). Heat the reaction mixture to 70 °C and stir overnight. Cool to room temperature, add water, and extract three times with ethyl

acetate. Combine the organic phase, wash with saturated salt water, dry with anhydrous $Na_2SO_4$, evaporate under vacuum, and the crude product obtained is purified by column chromatography to give 260 mg of product 19-2, with the yield of 51%.

Step 2: Preparation of 1-chloro-2-((2,3-dibromopropyl) sulfonyl)-4-nitrobenzene **(19-3)**

Add compound 19-2 (260 mg, 1.0 mmol) and DCM (5 ml) to a reaction flask, the reaction solution is purged with nitrogen, drop in the solution of bromine (319 mg, 2.0 mmol) in DCM (0.1 mL), stir at room temperature overnight, using TLC to confirm the complete reaction of the raw materials. The reaction solution is evaporated under vacuum to give 418 mg of crude 19-3, which is used directly in the next step without further purification.

Step 3: Preparation of 8-nitro-1,2,3,4,4a,5-hexahydrobenzo[*b*]pyrazino[1,2-*d*] [1,4] thiazine 6,6-dioxide (19-4)

Add compound 19-3 (418 mg, crude) and glycol ether (5 ml), then add ethylenediamine, stir the mixture at room temperature for 2h, using TLC to monitor that the starting materials are completed. Heat the mixture to 135 °C and stir for 6 h, using LC-MS to confirm a complete reaction. Cool to room temperature, add saturated sodium bicarbonate solution and stir for 20 min. Evaporate the mixture solution under vacuum to dryness, suspend in DCM/MeOH=10/1, and stir for 10 min. Filter, the filtrate is evaporated under vacuum to obtain the crude product, purify by column chromatography to get 133 mg compound 19-4, with yield of 47% for 2 steps.

Step 4: Preparation of 2-(1,5-dimethyl-3-phenyl-1*H*-pyrrol-2-yl)-*N*-(3-(5-fluoropyrimidin-2-yl)-6,6-dioxido-1,2,3,4,4a,5-hexahydrobenzo[*b*]pyrazino[1,2-*d*] [1, 4] thiazin-8-yl)-2-oxoacetamide **(T-19)**

Compound T-19 is synthesized using a method similar to that described in third to fifth steps of example 3.

LC-MS (ESI) m/z $(M+H)^+$: 575.2
$^1$H NMR (400 MHz, DMSO-$d_6$) δ 10.47 (s, 1H), 8.53 (s, 2H), 7.55 (d, *J*= 2.4 Hz, 1H), 7.27-7.19 (m, 3H), 7.15-7.06 ( m, 4H), 6.13 (s, 1H), 4.70-4.63 (m, 1H), 4.58-4.50 (m, 1H), 4.11-4.04 (m, 1H), 3.87 (dd, *J*= 14.0 Hz, 2.4 Hz, 1H), 3.84 (s, 3H), 3.78-3.69 (m, 1H), 3.47 (dd, *J*= 14.0 Hz, 12.0 Hz, 1H), 3.21 (dt, J = 7.0, 4.1 Hz, 1H), 3.06 (dd, *J*= 12.8, 10.8 Hz, 1H), 2.94 (dt, *J*= 12.0, 3.2 Hz, 1H), 2.34 (s, 3H).

**Example 20**

Preparation of (R)-2-(1,5-dimethyl-3-phenyl-1H-pyrrol-2-yl)-2-oxo-*N*-(3-(5-**sulfamoylpyrimidin**-2-yl)-1,2,3,4,4a,5-hexahydrobenzo[*b*]pyrazino[1,2-*d*] [1,4] oxazin-8-yl) acetamide **(T-20)**

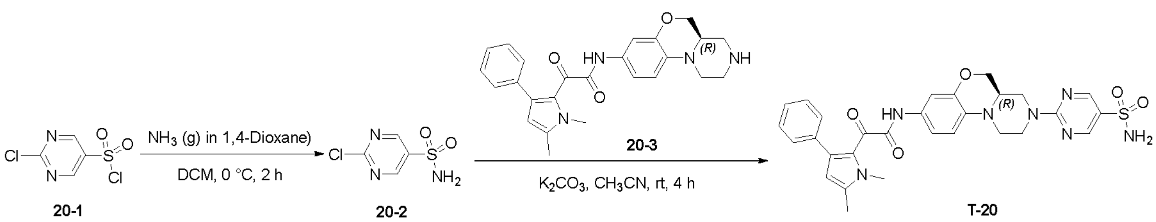


20-1 20-2 20-3 T-20

Step 1: Preparation of 2-chloropyrimidine-5-sulfonamide **(20-2)**

Add compound 20-1 (50 mg, 0.24 mmol) to a single-neck flask, then add dichloromethane (1 mL) to dissolve it. Cool the mixture to 0 °C in an ice bath, add 1,4-dioxane solution of ammonia (1 mL, 0.4M, Adamas), react for 2 h in the ice bath, using TLC to monitor that the reaction is complete. Drop in dilute hydrochloric acid, adjusting pH to 6, and remove the solvent by rotary evaporation. Purify the crude product by column chromatography / silica gel to give 30 mg of the target product 20-2, with the yield of 66%.

Step 2: Preparation of (R)-2-(1,5-dimethyl-3-phenyl-1H-pyrrol-2-yl)-2-oxo-*N*-(3-(5-**sulfamoylpyrimidin**-2-yl)-1,2,3,4,4a,5-hexahydrobenzo[*b*]pyrazino[1,2-*d*] [1,4] oxazin-8-yl) acetamide **(T-20)**

Transfer compound 20-2 (30 mg, 0.15 mmol) to a single-neck flask, then add acetonitrile (3 mL) to dissolve it,

then add compound 20-3 (60 mg, 0.14 mmol) (20-3 is prepared using the synthesis method of 7-3 in example 7) and potassium carbonate (32 mg, 0.23 mmol), stir at room temperature for 4 h, using TLC to confirm that the reaction is complete. Wash the organic phase with saturated salt water, dry with anhydrous $Na_2SO_4$, and evaporate under vacuum. The crude product obtained is purified by column chromatography to give 53 mg of T-20, with the yield of 65%.

LC-MS (ESI) m/z $(M+H)^+$: 588.2
$^1$H NMR (400 MHz, DMSO-$d_6$) δ 10.19 (s, 1H), 8.70 (s, 2H), 7.40 (s, 2H), 7.26-7.20 (m, 2H), 7.17-7.09 (m, 3H), 6.79-6.74 (m, 1H), 6.71-6.63 (m, 2H), 6.11 (s, 1H), 4.84-4.72 (m, 2H), 4.37 (dd, *J*= 10.8, 2.4 Hz, 1H), 3.99-3.90 (m, 1H), 3.89-3.81 (m, 1H), 3.80 (s, 3H), 3.24-3.14 (m, 1H), 3.07-2.99 (m, 1H), 2.82 (t, *J*= 12.0, 1H), 2.67-2.56 (m, 1H), 2.32 (s, 3H).

**Example 21**

Preparation of 2-(1,5-dimethyl-3-phenyl-1*H*-pyrrol-2-yl)-*N*-(3-(5-fluoropyrimidin-2-yl)-5-oxo-1,2,3,4,4a,5,6,7-octahydrobenzo[*f*]pyrazino[1,2-*a*] [1,4] diazepine-9-yl)-2-oxoacetamide **(T-21)**

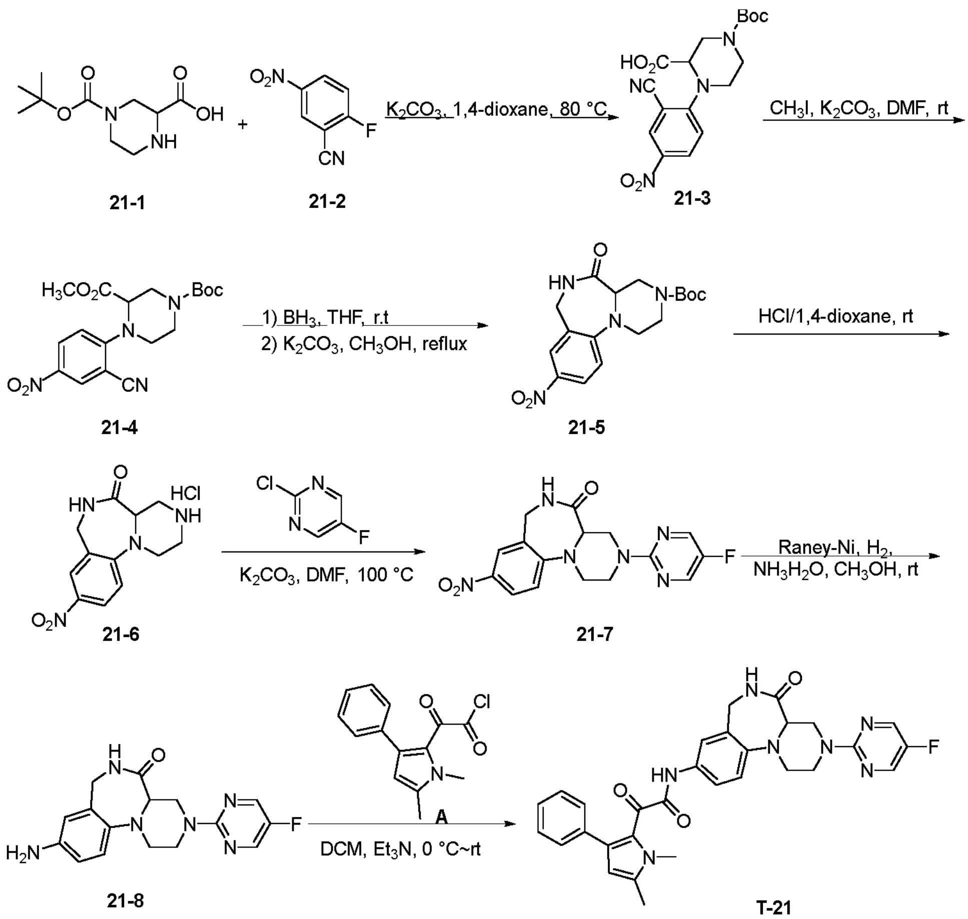

21-1 21-2 21-3 21-4 21-5 21-6 21-7 21-8 T-21

Step 1: Preparation of 4-(tert-butoxycarbonyl)-1-(2-cyano-4-nitrophenyl) piperazine-2-carboxylic acid **(21-3)**

Add compound 21-1 (1.5 g, 6.51 mmol), compound 21-2 (1.6 g, 9.77 mmol), potassium carbonate (2.7 g, 19.53 mmol) and 1, 4-dioxane (6 mL) sequentially to a reaction flask, and then heat the reaction mixture to 80 °C and stir overnight, using LC-MS to confirm a complete reaction. Cool, and pour the mixture into water, extract twice with ethyl acetate. Combine the organic phase, wash with saturated salt water, dry with anhydrous $Na_2SO_4$, and evaporate under vacuum. The crude product obtained is purified through silica gel column to give 2.2 g of product 21-3, with the yield of 90%.

Step 2: Preparation of 1-(tert-butyl)3-methyl 4-(2-cyano-4-nitrophenyl) piperazine-1,3-dicarboxylate (21-4)

Add compound 21-3 (300 mg, 0.80 mmol) and DMF (1.5 mL) to a reaction flask, then add potassium carbonate (275 mg, 1.99 mmol), drop in iodomethane at room temperature. The reaction solution is stirred at room temperature for 4 h, using TLC to confirm the complete reaction of the raw materials. Pour the reaction solution into water, and extract the resulting solution three times with ethyl acetate. Combine the organic phase, wash with water and then saturated salt water, dry with anhydrous $Na_2SO_4$, and evaporate under vacuum. The crude product obtained is purified through silica gel column to give 271 mg of product 21-4, with the yield of 87%.

Step 3: Preparation of tert-butyl 9-nitro-5-oxo-1,2,4a,5,6,7-hexahydrobenzo[*f*]pyrazino[1,2-*a*] [1,4] diazepine-3(4*H*)- carboxylate **(21-5)**

Add compound 21-4 (258 mg, 0.66 mmol) and THF (6.5 mL) to a reaction flask, drop in tetrahydrofuran solution of borane (1 M in THF, 3.2 mL), and stir at room temperature for 6 h, using TLC to confirm the raw material is transformed completely. Drop in methanol to stop the reaction, stir for 30 min, and remov the solvent by rotary evaporation under vacuum. Add potassium carbonate (274 mg, 1.98 mmol) and methanol (6 mL) to the residue, heat and reflux overnight, using LC-MS to confirm a complete reaction. Cool, add water, and extract twice with ethyl acetate. Combine the organic phase, wash with water and then saturated salt water, dry with anhydrous $Na_2SO_4$, and evaporate under vacuum. The crude product obtained is purified through silica gel column to give 106 mg of product 21-5, with the yield of 44%.

Step 4: Preparation of 9-nitro-2,3,4,4a,6,7-hexahydrobenzo[f]pyrazino[1,2-a] [1,4] diazepin-5(1H)-one **hydrogen chloride (21-6)**

Add compound 21-5 (100 mg, 0.28 mmol) and 1,4-dioxane solution of hydrogen chloride (4 M in 1,4-dioxane, 3 mL) to a reaction flask, stir at room temperature for 1 h, using TLC to confirm complete reaction of the raw materials. Evaporate under vacuum to give 85 mg of crude product 21-6, which is directly used in the next step without purification.

Step 5: Preparation of 3-(5-fluoropyrimidin-2-yl)-9-nitro-2,3,4,4a,6,7-hexahydrobenzo[*f*]pyrazino[1,2-*a*] [1,4] diazepin-5(1*H*)-keto **(21-7)**

Add compound 21-6 (85 mg, crude) and DMF (3 mL) to a reaction flask, then add potassium carbonate (116 mg, 0.84 mmol) and 2-chloro-5-fluoropyrimidine (56 mg, 0.42 mmol). Heat the reaction mixture to 100 °C and stir overnight, using LC-MS to confirm a complete reaction. Cool, add water, and extract three times with ethyl acetate. Combine the organic phase, wash with water and saturated salt water sequentially, dry with anhydrous $Na_2SO_4$, and evaporate under vacuum. The crude product obtained is purified through silica gel column to give 63 mg of product 21-7, with the two-step yield of 63%.

Step 6: Preparation of 9-amino-3-(5-fluoropyrimidin-2-yl)-2,3,4,4a,6,7-hexahydrobenzo[*f*]pyrazino[1,2-*a*] [1,4] diazepin-5 (1*H*)-keto **(21-8)**

Add compound 21-7 (60 mg, 0.17 mmol) and methanol (2 mL) to a reaction flask, then add Raney-Ni (15 mg) and ammonia (3 drops), and purge the reaction system three times with hydrogen. Stir at room temperature in hydrogen atmosphere for 1 h, using TLC to confirm complete reaction of the raw materials. Filter, evaporate under vacuum to give 40 mg of crude product 21-8, which is directly used in the next reaction step without purification.

Step 7: Preparation of 2-(1,5-dimethyl-3-phenyl-1*H*-pyrrol-2-yl)-*N*-(3-(5-fluoropyrimidin-2-yl)-5-oxo-1,2,3,4,4a,5,6,7-octahydrobenzo[*f*]pyrazino[1,2-*a*] [1,4] diazepin-9-yl)-2-oxoacetamide **(T-21)**

Add compound 21-8 (40 mg, crude product), DCM (2 mL) and $Et_3N$ (34 mg, 0.34 mmol) to a reaction flask. Purge the reaction system with nitrogen, then cool to 0 °C in an ice water bath, drop in solution of compound A (53 mg, 0.20 mmol) in DCM (2 mL). Let the mixture resumed to room temperature, and then stir for 1 h, using LC-MS to confirm that the reaction is complete. Pour the reaction solution into water, and extract twice with dichloromethane. Combine the organic phase, wash with saturated salt water, dry with anhydrous $Na_2SO_4$, and evaporate under vacuum. The crude product obtained is purified through thin-layer preparation plate to give 16 mg of product T-21, with the two-step yield of 17%.

LC-MS (ESI) m/z $(M+H)^+$: 554.2
$^1H$ NMR (400 MHz, DMSO-$d_6$) δ 10.36 (s, 1H), 8.51 (d, *J*= 0.4 Hz, 2H), 8.33 (dd, *J*= 6.8, 4.4 Hz, 1H), 7.23-7.19 (m,

2H), 7.14-7.04 (m, 4H), 6.97-6.92 (m, 2H), 6.10 (s, 1H), 4.76-4.68 (m, 1H), 4.59-4.49 (m, 2H), 3.83 (s, 3H), 3.60 (dd, *J*= 13.6, 7.2 Hz, 1H), 3.31-3.26 (m, 2H), 3.20-3.10 (m, 2H), 3.09-3.00 (m, 1H), 2.32 (s, 3H).

**Example 22**

Preparation of 2-(1, 5-dimethyl-3-phenyl-1*H*-pyrrol-2-yl)-*N*-(8-(5-fluoropyrimidin-2-yl)-6,6a,7,8,9,10-hexahydropyrazino[1,2-*d*]**pyrido**[3,2-*b*] [1,4] oxazin-3-yl)-2-oxoacetamide **(T-**22)

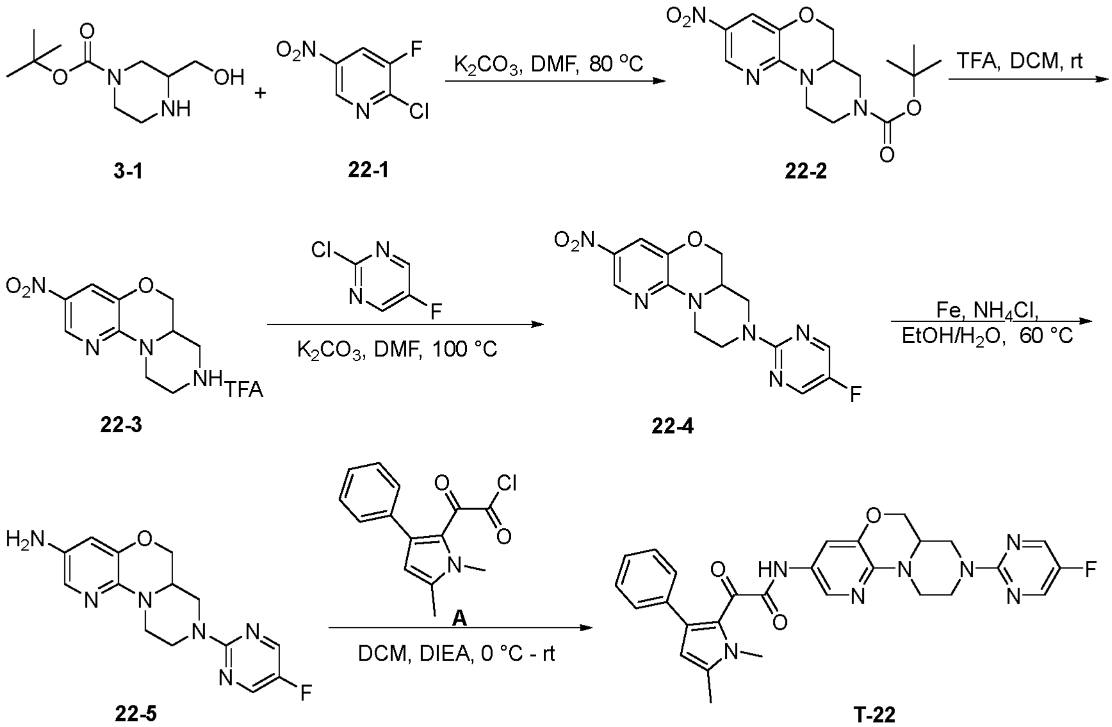


**3-1** + **22-1** → **22-2** → **22-3** → **22-4** → **22-5** + **A** → **T-22**

Step 1: Preparation of **tert-butyl** 3-nitro-6a,7,9,10-tetrahydropyrazino[1,2-*d*]pyrido[3,2-b] [1,4] oxazine-8(6*H*)-**carboxylate (22-2)**

Add compound 3-1 (1.16 g, 5.36 mmol) and DMF (10 mL) to a reaction flask, followed by 22-1 (950 mg, 5.38 mmol) and potassium carbonate (2.23 g, 16.14 mmol). Purge the reaction system with nitrogen, then heat to 80 °C, stir overnight, using LC-MS to confirm complete reaction. Cool, add water, and extract twice with ethyl acetate. Combine the organic phase, wash with water and saturated salt water sequentially, dry with anhydrous $Na_2SO_4$, and evaporate under vacuum. The crude product obtained is purified through silica gel column to give 160 mg of product 22-2, with the yield of 9%.

Step 2: Preparation of 3-nitro-6,6a,7,8,9,10-hexahydropyrazino[1,2-*d*]pyrido[3,2-*b*] [1,4] oxazine trifluoroacetate **(22-3)**

Add compound 22-2 (160 mg, 0.48 mmol) and dichloromethane (4 mL) to a reaction flask, then add trifluoroacetic acid (2 mL), stir at room temperature for 2 h, using TLC to confirm that the reaction of the raw material is complete. Evaporate under vacuum to give 200 mg of crude 22-3, which is directly used in the next reaction step without purification.

Step 3: Preparation of 8-(5-fluoropyrimidin-2-yl)-3-nitro-6,6a,7,8,9,10-hexahydropyrazino[1,2-*d*]pyrido[3,2-*b*] [1,4] oxazine **(22-4)**

Add compound 22-3 (200 mg, crude) and DMF (5 mL) to a reaction flask, followed by potassium carbonate (199 mg, 1.44 mmol) and 2-chloro-5-fluoropyrimidine (96 mg, 0.72 mmol). Purge the reaction solution with nitrogen, then heat to 100 °C and stir overnight, using LC-MS to confirm complete reaction. Cool, add water, and extract twice with ethyl acetate. Combine the organic phase, wash with water and saturated salt water sequentially, dry with anhydrous

$Na_2SO_4$, and evaporate under vacuum. The crude product obtained is purified through silica gel column to give 130 mg of product 22-4, with the two-step yield of 82%.

Step 4: Preparation of 8-(5-fluoropyrimidin-2-yl)-6,6a,7,8,9,10-hexahydropyrazino[1,2-*d*]pyrido[3,2-*b*] [1,4] oxazin-3-amine **(22-5)**

Add compound 22-4 (60 mg, 0.18 mmol), ethanol (2 mL) and water (0.5 mL) to a reaction flask, followed by $NH_4Cl$ (49 mg, 0.92 mmol) and iron powder (51 mg, 0.90 mmol). Heat the reaction mixture to 60 °C and stir for 4 h, using LC-MS to confirm a complete reaction. Cool, and filter, the filtrate is evaporated under vacuum. The crude product obtained is purified through silica gel column to give 40 mg of product 22-5, with the yield of 73%.

Step 5: Preparation of 2-(1,5-dimethyl-3-phenyl-1*H*-pyrrol-2-yl)-*N*-(8-(5-fluoropyrimidin-2-yl)-6,6a,7,8,9,10-hexahydropyrazine[1,2-*d*]pyridyl[3,2-*b*] [1,4] oxazine (-3-yl)-2-oxoacetamide **(T-22)**

Add compound 22-5 (40 mg, 0.13 mmol), DCM (3 mL) and DIEA (50 mg, 0.39 mmol) sequentially to a reaction flask, and purge the reaction system with nitrogen, cool to 0 °C in an ice bath, then drop in solution of compound A (37 mg, 0.14 mmol) in DCM (1 mL). Let the reaction solution resume to room temperature and stir overnight, using LC-MS to confirm a complete reaction. Add water, and extract twice with DCM. Combine the organic phase, wash with saturated saline, dry with anhydrous $Na_2SO_4$, and evaporate under vacuum. The crude product is purified with thin-layer preparation plates to give 20 mg of T-22, with the yield of 29%.

LC-MS (ESI) m/z $(M+H)^+$: 528.2
$^1H$ NMR (400 MHz, DMSO-$d_6$) δ 10.34 (s, 1H), 8.51 (s, 2H), 7.59 (d, *J*= 2.0 Hz, 1H), 7.25-7.20 (m, 2H), 7.19-7.10 ( m, 3H), 6.81 (d, *J*= 2.0 Hz, 1H), 6.12 (s, 1H), 4.69-4.59 (m, 2H), 4.43-4.35 (m, 2H), 3.96 (dd, *J*= 10.8 Hz, 8.8 Hz, 1H), 3.83 (s, 3H), 3.34-3.26 (m, 1H), 3.05-2.95 (m, 1H), 2.75-2.65 (m, 2H), 2.33 (s, 3H).

**Example 23**

Preparation of (*R*)-*N*-(3-(5-(azetidin-1-yl) pyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-23)**

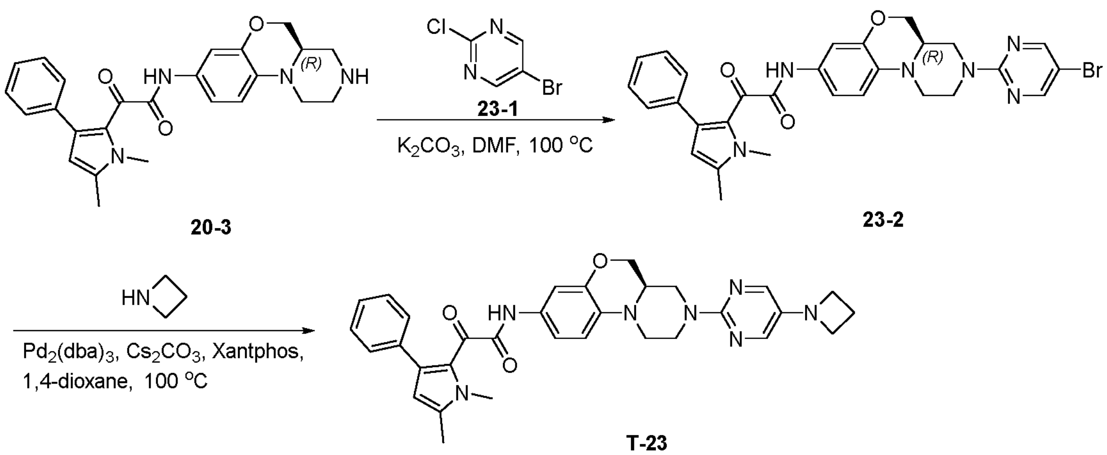


20-3

23-2

T-23

Step 1: Preparation of (*R*)-*N*-(3-(5-bromopyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(23-2)**

Add compound 20-3 (400 mg, 0.93 mmol), 23-1 (215 mg, 1.11 mmol), potassium carbonate (385 mg, 2.79 mmol) and DMF (10 mL) sequentially to a reaction flask. Purge the reaction system with nitrogen, then heat to 100 °C and stir for 4 h, using LC-MS to confirm complete reaction. Cool, add water, and extract twice with ethyl acetate. Combine the organic phase, wash with water and then saturated salt water, dry with anhydrous $Na_2SO_4$, and evaporate under vacuum. The crude product obtained is purified through silica gel column to give 400 mg of product 23-2, with the yield of 73%.

Step 2: Preparation of (*R*)-*N*-(3-(5-(azetidin-1-yl) pyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-23)**

Add compound 23-2 (120 mg, 0.20 mmol), azetidine (35 mg, 0.61 mmol), cesium carbonate (196 mg, 0.60 mmol) and 1,4-dioxane (3 mL) sequentially to a glass tube, and the air in the reaction system is replaced with nitrogen. Then add Xantphos (24 mg, 0.04 mmol) and $Pd_2(dba)_3$(19 mg, 0.02 mmol), purge with nitrogen to replace the air in the system again, heat the reaction mixture to 100 °C and stir for 8 h, using LC-MS to confirm a complete reaction of the raw materials. Cool, add water, and extract twice with ethyl acetate. Combine the organic phase, wash with water and then saturated salt water, dry with anhydrous $Na_2SO_4$, and evaporate under vacuum. The crude product obtained is purified through silica gel column to give 20 mg of crude product, followed by purification through thin-layer preparation plates to give 7 mg of product T-23, with the yield of 6%.

LC-MS (ESI) m/z $(M+H)^+$: 564.5
$^1$H NMR (400 MHz, DMSO-$d_6$) δ 10.18 (s, 1H),7.83 (s, 2H), 7.25-7.20 (m, 2H), 7.16-7.09 (m, 3H), 6.77-6.72 (m, 1H), 6.69- 6.62 (m, 2H), 6.11 (s, 1H), 4.55-4.42 (m, 2H), 4.33 (dd, *J*= 10.8, 2.4 Hz, 1H), 3.96-3.89 (m, 1H), 3.81 (s, 3H), 3.80-3.74 (m, 4H), 3.34-3.31 (m, 1H), 3.01-2.88 (m, 3H), 2.61-2.55 (m, 1H), 2.32 (s, 3H), 2.35-2.27 (m, 2H).

**Example 24**

Preparation of (*R*)-2-(1,5-dimethyl-3-phenyl-1*H*-**pyrrol**-2-yl)-*N*-(3-(5-morpholinopyrimidin-2-yl)-1,2,3,4,4a,5-hexahydrobenzo[*b*]pyrazino[1,2-*d*] [1,4] oxazin-8-yl)-2-oxoacetamide **(T-24)**

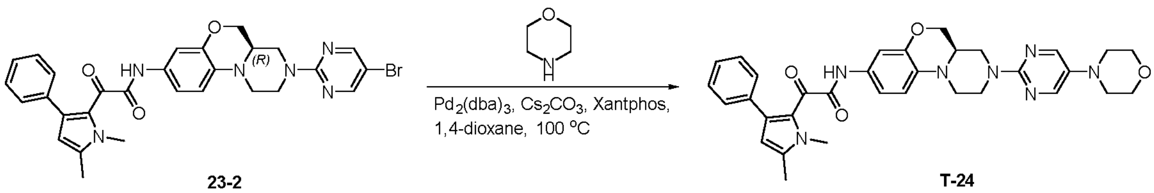


23-2

T-24

Compound T-24 is synthesized using a similar method to that described in Example 23. LC-MS (ESI) m/z $(M+H)^+$: 594.3

$^1$H NMR (400 MHz, DMSO-$d_6$) δ 10.18 (s, 1H), 8.24 (s, 2H), 7.25-7.20 (m, 2H), 7.16-7.09 (m, 3H), 6.78-6.73 (m, 1H), 6.70- 6.62 (m, 2H), 6.11 (s, 1H), 4.61-4.49 (m, 2H), 4.34 (dd, *J*= 10.8, 2.4 Hz, 1H), 3.96-3.89 (m, 1H), 3.81 (s, 3H), 3.76-3.71 (m, 3H), 3.38-3.30 (m, 4H), 3.04-2.99 (m, 4H), 2.99-2.93 (m, 1H), 2.62-2.55 (m, 1H), 2.33 (s, 3H).

**Example 25**

Preparation of (*R*)-2-(1,5-dimethyl-3-phenyl-1*H*-pyrrol-2-yl)-*N*-(10-fluoro-3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-25)**

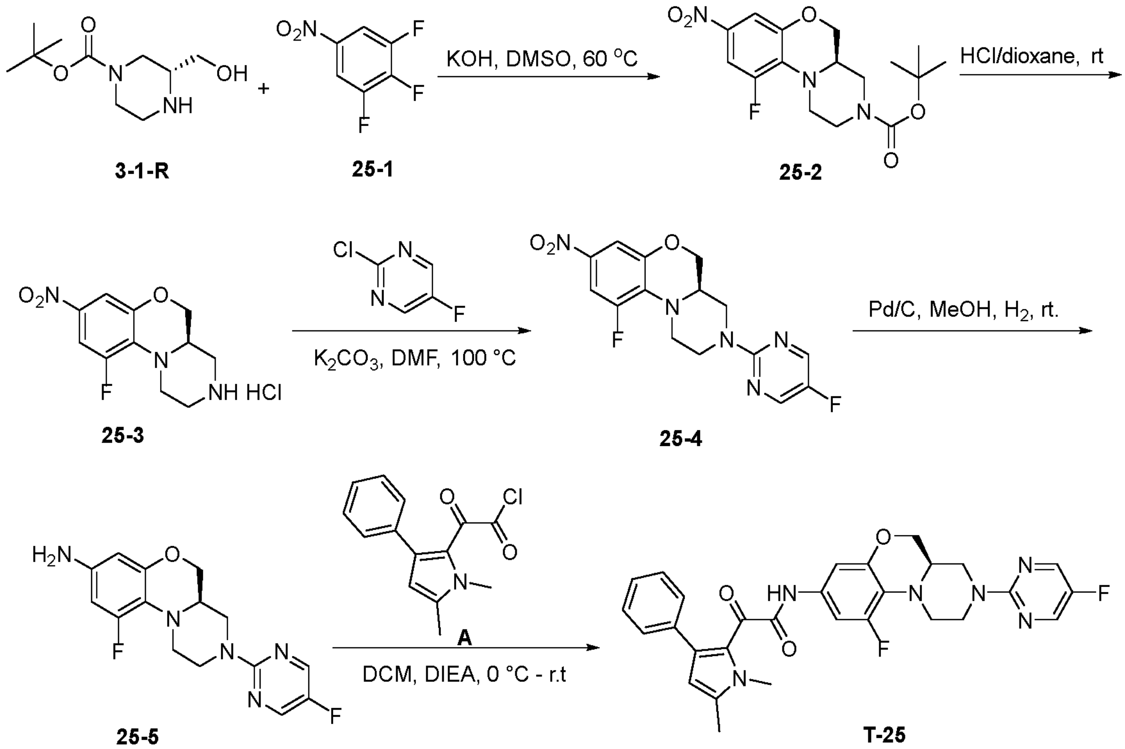


3-1-R 25-1 25-2 25-3 25-4 25-5 A T-25

Compound T-25 is synthesized using a similar method to that described in Example 3. LC-MS (ESI) m/z $(M+H)^+$: 545.2

$^1H$ NMR (400 MHz, DMSO-$d_6$) δ 10.38 (s, 1H), 8.50 (s, 2H), 7.22-7.17 (m, 2H), 7.16-7.09 (m, 3H), 6.62 (dd, *J*= 15.2, 2.4 Hz, 1H), 6.55-6.52 (m, 1H), 6.12 (s, 1H), 4.26 (dd, *J*= 10.8 Hz, 2.4 Hz, 1H), 4.16 (dd, *J*= 13.2 Hz, 3.2 Hz, 1H), 4.08-4.01 (m , 1H), 3.99-3.93 (m, 1H), 3.83 (s, 3H), 3.69-3.61 (m, 1H), 3.61-3.52 (m, 1H), 3.40 (dd, *J*= 13.2 Hz, 8.0 Hz, 1H), 3.22-3.14 (m, 1H), 2.98 (ddd, J = 11.2, 8.4, 2.8 Hz, 1H), 2.33 (s, 3H).

**Examples 26 to 27: Preparation of compounds T-26 to T-27**

Compounds **T-26 to T-27** were prepared by the same method used for the preparation of compound T-18 and using different raw materials (replacing the raw material (3-(hydroxymethyl) piperazine-1-carboxylate (3-1)) in the first step in example 18 with (R)-3-(hydroxymethyl) piperazine-1-carboxylic acid tert-butyl ester and (S)-3-(hydroxymethyl) piperazine-1-carboxylic acid tert-butyl ester, respectively), and their structural formulas, LC-MS and $^1H$-NMR data are shown in Table 1.

**Table 3: Structures, LC-MS and $^1H$-NMR data of examples 26 to 27**

| Examples | Chemical constructions | LC-MS: (ESI) m/z | $^1H$ NMR |
|---|---|---|---|
| 26 | 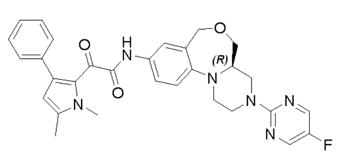 | 541.2 | $^1H$ NMR (400 MHz, DMSO-$d_6$) δ 10.31 (s, 1H), 8.49 (s, 2H), 7.26-7.21 (m, 2H), 7.16-7.05 (m, 4H), 6.91 (d, *J* = 2.4 Hz, 1H), 6.85 (d, *J* = 8.4 Hz, 1H), 6.11 (s, 1H), 4.63 (d, *J* = 12.0 Hz, 1H), 4.45 (d, *J* = 12.0 Hz, 1H), 4.18-4.08 (m, 2H), 3.84 (s, 3H), 3.69 (dd, *J* = 12.4, 2.8 Hz, 1H), 3.64-3.52 (m, 2H), 3.50-3.41 (m, 1H), 3.33-3.28 (m, 2H), 2.98 -2.91 (m, 1H), 2.33 (s, 3H). |

(continued)

| Examples | Chemical constructions | LC-MS: (ESI) m/z | $^1$H NMR |
|---|---|---|---|
| 27 | 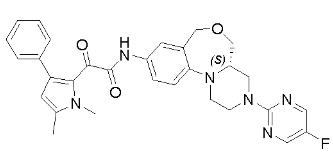 | 541.2 | $^1$H NMR (400 MHz, DMSO-$d_6$) δ 10.31 (s, 1H), 8.48 (s, 2H), 7.25-7.20 (m, 2H), 7.15-7.04 (m, 4H), 6.90 (d, *J* = 2.4 Hz, 1H), 6.84 (d, *J* = 8.8 Hz, 1H), 6.11 (s, 1H), 4.62 (d, *J* = 12.0 Hz, 1H), 4.44 (d, *J* = 12.0 Hz, 1H), 4.17-4.07 (m, 2H), 3.83 (s, 3H), 3.68 (dd, *J* = 12.4, 2.8 Hz, 1H), 3.64-3.52 (m, 2H), 3.49-3.40 (m, 1H), 3.32-3.26 (m, 2H), 2.97 -2.90 (m, 1H), 2.32 (s, 3H).3.60-3.51 (m, 1H), 3.39 (dd, *J* = 13.2, 7.6 Hz, 1H), 3.21-3.14 (m, 1H), 2.97 (ddd, *J* = 11.6, 8.4, 2.8 Hz, 1H), 2.32 (s, 3H). |

**Example 28**

Preparation of (*R*)-*N*-(3-(1,3,5-triazin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-28)**

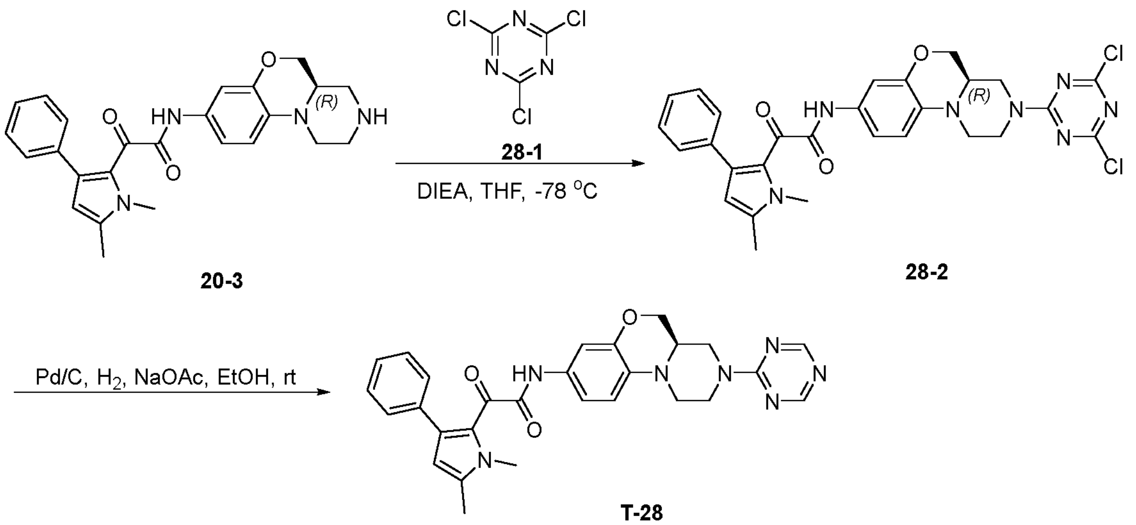


**20-3** **28-2** **T-28**

Step 1: Preparation of (*R*)-*N*-(3-(4,6-dichloro-1,3,5-triazin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(28-2)**

Compound 20-3 (100 mg, 0.232 mmol) and THF (10 mL) were added to a reaction flask, the reaction system is processed with nitrogen replacement, and the mixed solution is cooled to -78 °C. Then a solution of compound 28-1 (85 mg, 0.461 mmol) and DIEA (90 mg, 0.696 mmol) in DMSO (2 mL) is add dropwise to the system. The reaction system is stirred at - 78 °C for 4 h. LC-MS to confirm a complete reaction. The reaction system is quenched by adding hydrogen chloride in 1,4-dioxane solution (4 M in 1,4-dioxane), and then the reaction system is slowly heated to room temperature.

Step 2: Preparation of (*R*)-*N*-(3-(1,3,5-triazin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-28)**

Compound 28-2 (80 mg, 0.086 mmol), ethanol (6 mL), anhydrous sodium acetate (14 mg, 0.171 mmol) and Pd/C (20 mg) were sequentially added to a reaction flask, then processed with hydrogen replacement, and the reaction solution is stirred at room temperature in hydrogen overnight. TLC used to confirm a complete reaction of the raw

materials. The reaction solution is filtered, and the filtrate is evaporate under vacuum. The crude product obtained is purified by thin-layer preparation plates to give 20 mg of product T-28, with the yield of 45%.

LC-MS (ESI) m/z $(M+H)^+$: 510.5
$^1$H NMR (400 MHz, DMSO-$d_6$) δ 10.20 (s, 1H), 8.63 (s, 2H), 7.25-7.20 (m, 2H), 7.17-7.09 (m, 3H), 6.79-6.74 (m, 1H), 6.71- 6.63 (m, 2H), 6.11 (s, 1H), 4.76-4.65 (m, 2H), 4.35 (dd, *J*= 10.8, 2.8 Hz, 1H), 3.94 (dd, *J*= 10.8, 8.8 Hz, 1H), 3.89-3.82 (m, 1H), 3.81 ( s, 3H), 3.19-3.10 (m, 1H), 3.06-2.98 (m, 1H), 2.83-2.74 (m, 1H), 2.65-2.56 (m, 1H), 2.33 (s, 3H).

**Example 29**

Preparation of *N*-((*R*)-3-(5-((*R*)-1,2-dihydroxyethyl) pyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-29)**

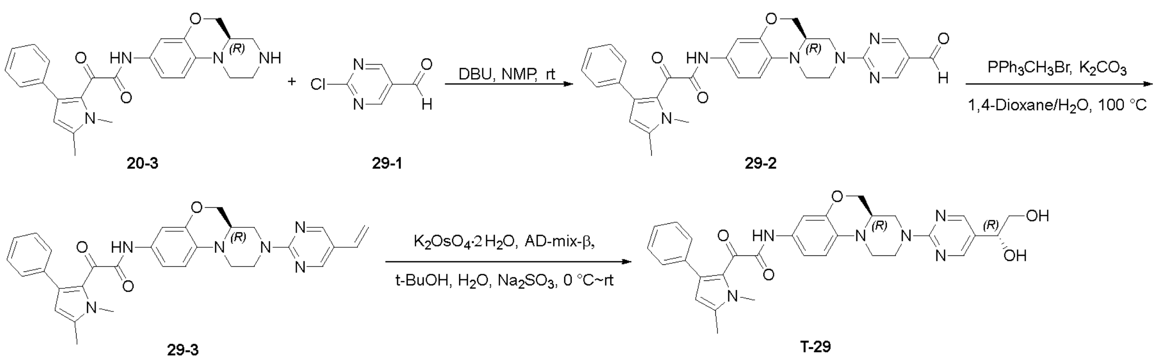


20-3 29-1 29-2

29-3 T-29

Step 1: Preparation of (*R*)-2-(1,5-dimethyl-3-phenyl-1*H*-pyrrol-2-yl)-*N*-(3-(5-formylpyrimidin-2-yl)-1,2,3,4,4a,5-hexahydrobenzo[*b*]pyrazino[1,2-*d*] [1,4] oxazin-8-yl)-2-oxoacetamide **(29-2)**

Add compound 20-3 (150 mg, 0.35 mmol) and DMF (4 mL) to a reaction flask, followed by compound 29-1 (53 mg, 0.37 mmol) and 1,8-diazabicycloundec-7-ene (61 mg, 0.40 mmol), stir at room temperature for 4 h, using TLC monitoring to confirm a complete reaction of the raw materials. Add saturated sodium bicarbonate solution to the reaction mixture, and extract three times with ethyl acetate. Combine the organic phase, wash with saturated salt solution, dry with anhydrous sodium sulfate, and evaporate under vacuum. The crude product obtained is purified through silica gel column to give 155 mg of the product 29-2, with the yield of 83%.

Step 2: preparation of (R*)*-2-(1,5-dimethyl-3-phenyl-1*H*-pyrrol-2-yl)-2-oxo-*N*-(3-(5-vinylpyrimidin-2-yl)-1,2,3,4,4a,5-hexahydrobenzo[*b*]pyrazino[1,2-*d*] [1,4] oxazin-8-yl) acetamide **(29-3)**

Add compound 29-2 (150 mg, 0.28 mmol), 1,4-dioxane (5 mL) and water (1 mL) to a reaction flask, followed by phosphorus triphenyl methyl bromide (118 mg, 0.33 mmol) and potassium carbonate (46 mg, 0.33 mmol), heat and reflux at 100 °C overnight, using TLC monitoring to confirm the reaction is complete. Remove the solvent by rotary evaporation, add water, and extract three times with ethyl acetate. The organic phase is wash with saturated salt water, dry with anhydrous sodium sulfate, and evaporate under vacuum. The crude product obtained is purified through silica gel column to give 75 mg of the product 29-3, with the yield of 50%.

Step 3: Preparation of *N*-((*R*)-3-(5-((*R*)-1,2-dihydroxyethyl) pyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetamide **(T-29)**

Add tert-butanol (2 mL) and water (2 mL) to a reaction flask, then add AD-mix-β (218 mg, 0.28 mmol). Cool to 0 °C in an ice-water bath, drop in tert-butanol solution (1 mL) of compound 29-3 (75 mg, 0.14 mmol) and then add potassium osmate dihydrate (77 mg, 0.21 mmol), stir for 2 h at 0 °C, the stir at room temperature overnight, using TLC monitoring to confirm that the reaction is complete. Add aqueous sodium sulfite to quench the reaction, stir for 10 min, and extract three times with ethyl acetate. Combine the organic phase, wash with saturated salt water, dry with anhydrous sodium sulfate, and evaporate under vacuum. The crude product obtained is purified with thin-layer preparation plate

to give 26 mg of the product T-29, with the yield of 33%.

LC-MS (ESI) m/z $(M+H)^+$: 569.3

$^1H$ NMR (400 MHz, DMSO-$d_6$) δ 10.17 (s, 1H), 8.35 (s, 2H), 7.25-7.21 (m, 2H), 7.17-7.10 (m, 3H), 6.78-6.74 (m, 1H), 6.68 (dd J*J*= 8.8, 2.4 Hz, 1H), 6.65-6.62 (m, 1H), 6.11 (s, 1H), 5.28 (d, *J*= 4.4, 1H), 4.76 (t, *J*= 5.6 Hz, 1H), 4.74-4.62 (m, 2H), 4.48-4.42 (m, 1H), 4.36 (dd, *J*= 10.8, 2.4 Hz, 1H), 3.97-3.89 (m, 1H), 3.85-3.77 (m, 1H), 3.81 (s, 3H), 3.54-3.48 (m, 1H), 3.44-3.37 (m, 1H), 3.09-2.93 (m, 2H), 2.70-2.64 (m, 1H), 2.61-2.53 (m, 1H), 2.33 (s, 3H).

**Example 30**

Preparation of *N*-((*R*)-3-(5-((*R*)-2,3-dihydroxypropoxy) pyrimidin-2-yl)-1,2,3,4,4a,5-hexahydrobenzo[*b*]pyrazino[1,2-*d*] [1,4] oxazin-8-yl)-2-(1,5-dimethyl-3-phenyl-1*H*-pyrrol-2-yl)-2-oxoacetoacetamide **(T-30)**

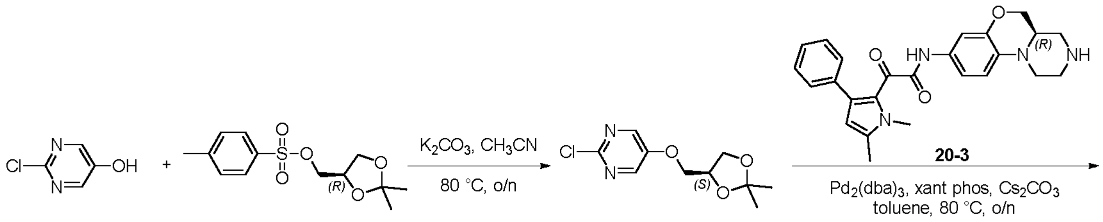


**30-1** **30-2** **30-3**

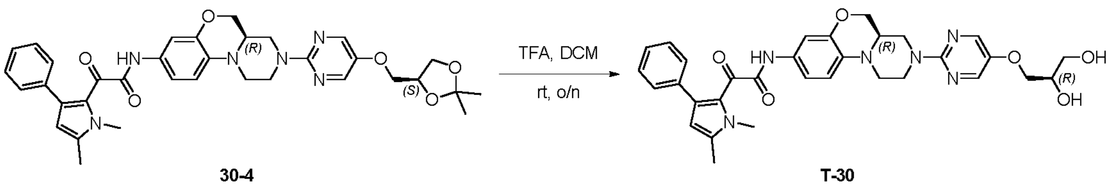


**30-4** **T-30**

Step 1: Preparation of (*S*)-2-chloro-5-((2,2-dimethyl-1,3-dioxan-4-yl) methoxy) pyrimidine **(30-3)**

Add compound 30-1 (100 mg, 0.77 mmol), 30-2 (260 mg, 0.91 mmol), potassium carbonate (157 mg, 1.14 mmol) and acetonitrile (5 mL) sequentially to a reaction flask and heat to 80 °C and react overnight, using TLC monitoring to confirm the reaction is complete. Remove the solvent by rotary evaporation, then add water, and extract three times with ethyl acetate. Combine the organic phase, dry with anhydrous sodium sulfate, and evaporate under vacuum. The crude product obtained is purified through silica gel column to give 119 mg of the product 30-3, with the yield of 63%.

Step 2: Preparation of *N*-((*R*)-3-(5-(((*S*)-2,2-dimethyl-1,3-dioxan-4-yl) methoxy) pyrimidin-2-yl)-1,2,3,4,4a,5-hexahydrobenzo[*b*]pyrazino[1,2-*d*] [1,4] oxazin-8-yl)-2-(1, 5-dimethyl-3-phenyl-1*H*-pyrrol-2-yl)-2-oxoacetamide **(30-4)**

Add compound 30-3 (90 mg, 0.37 mmol), 20-3 (155 mg, 0.36 mmol), cesium carbonate (352 mg, 1.08 mmol) and toluene (5 mL) sequentially to a reaction flask and the air in the flask is replaced with nitrogen. Then add tri (dibenzylacetone) dipalladium (37 mg, 0.04 mmol) and 4, 5-bisdiphenylphosphine, 9, 9-dimethylxanthrene (46 mg, 0.08 mmol), processed twice with nitrogen displacement. Heat to 80 °C and react overnigh, using TLC monitoring to confirma complete reaction. Add water, and extract three times with ethyl acetate. Combine the organic phase, dry with anhydrous sodium sulfate, and evaporate under vacuum. The crude product obtained is purified through silica gel column to give 15 mg of the product 30-4, with the yield of 6%.

Step 3: Preparation of *N*-((*R*)-3-(5-((*R*)-2,3-dihydroxypropoxy) pyrimidin-2-yl)-1,2,3,4,4a,5-hexahydrobenzo[*b*]pyrazine[1,2-*d*] [1,4] oxazin-8-(1,5-dimethyl-3-phenyl-1*H*-pyrrol-2-yl)-2-oxoacetoacetamide **(T-30)**

Add compound 30-4 (15 mg, 0.023 mmol) to a reaction flask, followed by dichloromethane (1 mL) and then drop in trifluoroacetic acid (1 mL). Stir overnight at room temperature, using TLC monitoring to confirm a complete reaction. Remove the solvent by rotary evaporation, then add water, adjusting pH to 8 with saturated sodium bicarbonate solution, extract three times with ethyl acetate. Combine the organic phase, dry with anhydrous sodium sulfate, and

evaporate under vacuum. The crude product obtained is purified with thin-layer preparation plates to give 5 mg of the target product T-30, with the yield of 36%.

LC-MS (ESI) m/z $(M+H)^+$: 599.5
$^1H$ NMR (400 MHz, DMSO-$d_6$) δ 10.17 (s, 1H), 8.25 (s, 2H), 7.25-7.20 (m, 2H), 7.16-7.10 (m, 3H), 6.77-6.74 (m, 1H), 6.67 (dd JJ= 8.8, 2.4 Hz, 1H), 6.64-6.62 (m, 1H), 6.11 (s, 1H), 5.00 (d, *J* = 4.8, 1H), 4.70 (t, *J* = 5.6 Hz, 1H), 4.61-4.48 (m, 2H), 4.34 (dd, *J* = 10.8, 2.4 Hz, 1H), 4.05-4.00 (m, 1H), 3.96-3.87 (m, 2H), 3.81 (s, 3H), 3.79-3.72 (m, 2H), 3.03-2.94 (m, 2H), 2.64-2.56 (m, 2H), 2.32 (s, 3H).

**Example 31**

Preparation of 2-(1,5-dimethyl-3-phenyl-1*H*-pyrrol-2-yl)-*N*-(3-(5-fluoropyrimidin-2-yl)-6-methyl-1,2,3,4,4a,5,6,7-octahydrobenzo[*f*]pyrazino[1,2-*a*] [1,4] (diazepin-9-yl)-2-oxoacetamide **(T-31)**

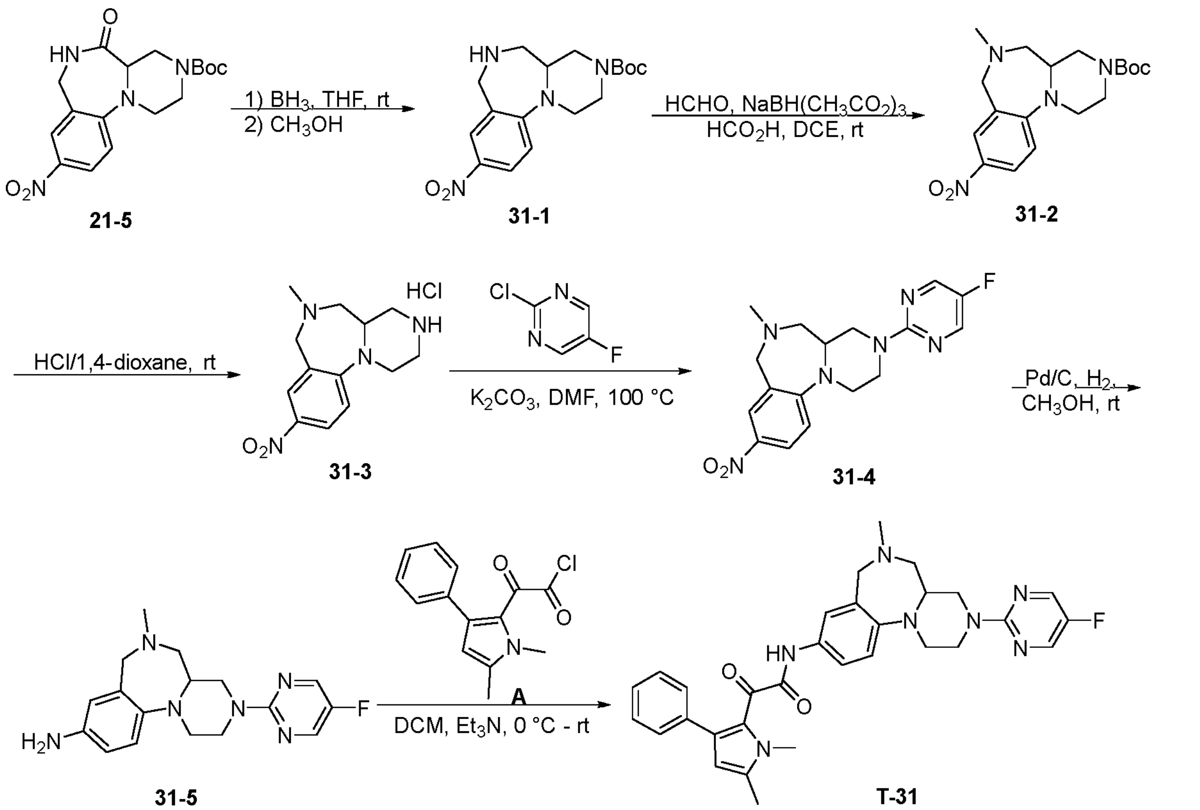


21-5 31-1 31-2 31-3 31-4 31-5 T-31

Step 1: Preparation of **tert-butyl 9-nitro-1,2,4a,5,6,7-hexahydrobenzo[f]pyrazino[1,2-a][1,4]diazepine-3(4H)-carboxylate (31-1)**

Add compound 21-5 (120 mg, 0.33 mmol) and THF (5 mL) to a reaction flask, then drop in borane tetrahydrofuran solution (5 mL). The reaction is carried out overnight at room temperature, using TLC to confirm the complete reaction of the raw materials. Add methanol (10 mL) slowly, and heat and reflux overnight. Concentrate the reaction solution directly to dryness to give 105 mg of crude product 31-1, with the yield of 91%.

Step 2: Preparation of **tert-butyl 6-methyl-9-nitro-1,2,4a,5,6,7-hexahydrobenzo[f)pyrazino[1,2-a][1,4]diazepine-3(4H)-carboxylate (31-2)**

Add compound 31-1 (105 mg, 0.30 mmol) and 1,2-dichloroethane (5 mL) to a reaction flask, followed by aqueous formaldehyde (37%-40% in water, 245 mg, 3.02 mmol) and formic acid (10 mg, 0.22 mmol), stir at room temperature for 1 h, then add sodium triacetyloxyl borohydride (318 mg, 1.50 mmol) and stir overnight at room temperature, using LC-MS to confirm that the reaction of raw materials is complete. Add appropriate amount of water to quench the reaction,

neutralized with saturated $Na_2CO_3$ aqueous solution, and extract three times with dichloromethane. Combine the organic phase, wash with saturated aqueous salt solution, dry with anhydrous $Na_2SO_4$, and evaporate under vacuum. 100 mg of crude product 31-2 is obtained, with the yield of 92%.

Step 3: Preparation of 6-methyl-9-nitro-1,2,3,4,4a,5,6,7-octahydrobenzo[*f*]pyrazino[1,2-*a*] [1,4] diazepine hydrochloride **(31-3)**

Add compound 31-2 (100 mg, 0.28 mmol) and 1, 4-dioxane (2 mL) to a reaction flask, then add 1, 4-dioxane solution of hydrogen chloride (4 M in 1,4-dioxane, 2 mL). Stir at room temperature for 1 h, using TLC to confirm complete reaction of the raw materials. Evaporate under vacuum to give 70 mg of crude product 31-3, which is directly used in the next step without purification.

Step 4: Preparation of 3-(5-fluoropyrimidin-2-yl)-6-methyl-9-nitro-1,2,3,4,4a,5,6,7-octahydrobenzo[*f*]pyrazino[1,2-*a*] [1,4] diazepine **(31-4)**

Add compound 31-3 (70 mg) and DMF (3 mL) to a reaction flask, followed by 2-chloro-5-fluoropyrimidine (53 mg, 0.40 mmol) and potassium carbonate (110 mg, 0.80 mmol), heat to 100 °C and stir overnight, using LC-MS to confirm a complete reaction. Cool, add water, and extract three times with ethyl acetate. Combine the organic phase, wash with water and saturated salt water, dry with anhydrous $Na_2SO_4$, and evaporate under vacuum. The crude product is purified through silica gel column to give 67 mg of product 31-4, with the two-step yield of 68%.

Step 5: Preparation of 3-(5-fluoropyrimidin-2-yl)-6-methyl-1,2,3,4,4a,5,6,7-octahydrobenzo[*f*]pyrazino[1,2-a] [1,4] diazepin-9-amine **(31-5)**

Add compound 31-4 (67 mg, 0.19 mmol) and methanol (2 mL) to a reaction flask, followed by Pd/C (7 mg), processed with hydrogen replacement three times. Stir overnight at room temperature under hydrogen atmosphere, using TLC monitoring to confirm a complete reaction. Filter, and the filtrate is evaporated under vacuum. The crude product obtained is purified through thin-layer preparation plates to give 37 mg of product 31-5, with the yield of 60%.

Step 6: Preparation of 2-(1,5-dimethyl-3-phenyl-1*H*-pyrrol-2-yl)-*N*-(3-(5-fluoropyrimidin-2-yl)-6-methyl-1,2,3,4,4a,5,6,7-octahydrobenzo[*f*]pyrazino[1,2-*a*] [1,4] diazepin-9-yl)-2-oxoacetamide **(T-31)**

Add compound 31-5 (37 mg, 0.11 mmol), DCM (2 mL) and $Et_3N$ (22 mg, 0.22 mmol) to a reaction flask, cool to 0 °C in an ice-water bath, drop in a solution of compound A (35 mg, 0.13 mmol) in DCM (2 mL), let the temperature slowly resume to room temperature and stir for 1 h, using LC-MS to confirm that the reaction is complete. Pour the reaction solution into water, and extract twice with dichloromethane. Combine the organic phase, wash with saturated salt water, dry with anhydrous $Na_2SO_4$, and evaporate under vacuum. The crude product is purified with thin-layer preparation plates to give 20 mg of product T-31, with the yield of 32%.

LC-MS (ESI) m/z $(M+H)^+$: 554.3
$^1H$ NMR (400 MHz, DMSO-$d_6$) δ 10.25 (s, 1H), 8.47 (s, 2H), 7.25-7.21 (m, 2H), 7.15-7.06 (m, 3H), 7.01 (dd, *J*= 8.8 , 2.4 Hz, 1H), 6.91-6.88 (m, 1H), 6.81-6.77 (m, 1H), 6.10 (s, 1H), 4.18-4.10 (m, 2H), 3.82 (s, 3H), 3.82-3.75 (m, 1H), 3.52-3.35 (m, 3H), 3.35-3.25 (m, 2H), 3.13-3.05 (m, 1H), 2.65-2.55 (m, 2H), 2.32 (s, 3H), 2.26 (s, 3H).

**Example 32**

Preparation of 2-(1,5-dimethyl-3-phenyl-1*H*-pyrrol-2-yl)-*N*-(3-(5-fluoropyrimidin-2-yl)-2,3,4,4a,5,6-hexahydro-1*H*-pyrazino[1,2-*a*]quinolin-8-yl)-2-oxoacetamide **(T-32)**

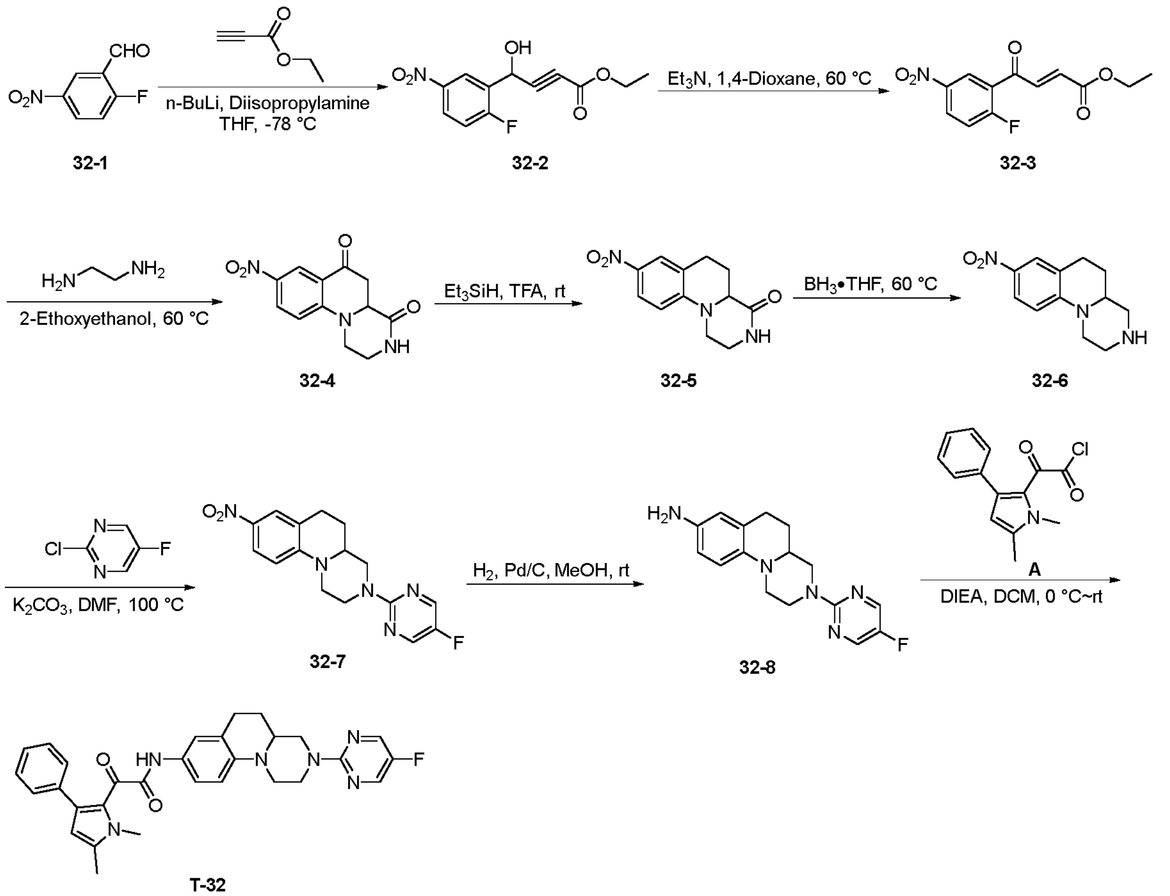


T-32

Step 1: Preparation of 4-(2-fluoro-5-nitrophenyl)-4-hydroxybutan-2-acid ethyl ester **(32-2)**

Add diisopropylamine (6.6 g, 65.2 mmol) to a dry three-necked flask, processed with nitrogen replacement, add anhydrous THF (80 mL), cool to about -10 °C, then drop in n-butyllithium (2.5 M in hexane, 26 mL, 65.0 mmol), and stir at this temperature for 0.5 h. Cool the reaction solution to -78 °C, drop in a solution of ethyl propiolate (6.4 g, 65.2 mmol) in anhydrous THF (300 mL) during which the system temperature is controlled below -70 °C. Stir the reaction mixture at -78 °C for 0.5 h, using TLC to confirm that the reaction is complete. Add dilute hydrochloride acid solution to the mixture, adjusting the pH to be acidic. Let the temperature resume to room temperature, add appropriate amount of water, and extract three times with ethyl acetate. Combine the organic phase, wash with saturated saline, dry with anhydrous sodium sulfate, and evaporate under vacuum. The crude product is purified through silica gel column to give 6.3 g of product 32-2, with the yield of 40%.

Step 2: Preparation of (*E*)-4-(2-fluoro-5-nitrophenyl)-4-oxobut-2-enoic acid ethyl ester (32-3)

Add compound 32-2 (2.0 g, 7.5 mmol) and 1, 4-dioxane (20 mL) to a reaction flask, and then drop in $Et_3N$ (1.9 g, 18.8 mmol). Heat to 60 °C and stir for 2 h, using TLC to confirm a complete reaction. The reaction solution is evaporate under vacuum, and the crude product obtained is purified through silica gel column to give 1.8 g of product 32-3, with the yield of 90%.

Step 3: Preparation of 8-nitro-2,3,4a,5-tetrahydro-1*H*-pyrazino [1,2-*a*] quinoline-4,6-dione (32-4)

Add ethylenediamine (225 mg, 3.7 mmol) and ethylene glycol ether (5 mL) to a reaction flask, cool to 0 °C. Drop in a solution of compound 32-3 (1.0 g, 3.74 mol) in ethylene glycol ether (10 mL). Stir at this temperature for 10 min, then heat to 60 °C, and stir for 2 h when solid product is produced and TLC shows the reaction is complete. Filter, wash the filter cake several times with petroleum ether, dry under vacuum to give 440 mg of product 32-4, with the yield of 45%.

Step 4: Preparation of 8-nitro-2,3,5,6-tetrahydro-1*H*-pyrazino [1,2-*a*] quinolin-4(4a*H*)-keto (32-5)

Add 32-4 (200 mg, 0.77 mmol) and trifluoroacetic acid (5 mL) to a reaction flask, followed by triethylsilane (1.2 mL), stir at room temperature overnight till TLC confirms a complete reaction. Add saturated aqueous sodium bicarbonate solution, and then extract three times with ethyl acetate. Combine the organic phase, wash with saturated salt water, dry with anhydrous sodium sulfate, and evaporate under vacuum to give 187 mg of crude product 32-5, which is directly used in the next reaction step.

Step 5: Preparation of 8-nitro-2,3,4,4a,5,6-hexahydro-1*H*-pyrazino [1,2-*a*]quinoline **(32-6)**

Add compound 32-5 (187 mg) and anhydrous THF (5 mL) to a dry three-necked flask, followed by $BF_2$•THF (1 M in THF, 2.3 mL, 2.3 mmol), heat to 66 °C and stir overnight till TLC confirms a complete reaction. Cool to room temperature, add methanol to stop the reaction, stir for 0.5 h, then add an appropriate amount of water, and extract three times with ethyl acetate. Combine the organic phase, wash with saturated salt water, dry with anhydrous sodium sulfate, and evaporate under vacuum. The crude product is purified through silica gel column to give 158 mg of product 32-6, with the two-step yield of 88%.

Step 6: Preparation of 3-(5-fluoropyrimidin-2-yl)-8-nitro-2,3,4,4a,5,6-hexahydro-1*H*-pyrazino [1,2-*a*] quinoline **(32-7)**

Add compound 32-6 (100 mg, 0.43 mmol) and DMF (3 mL) to a reaction flask, followed by 2-chloro-5-fluoropyrimidine (171 mg, 1.29 mmol) and potassium carbonate (178 mg, 1.29 mmol), heat to 100 °C and stir for 5 h till TLC confirms that the reaction is complete. Add an appropriate amount of water to stop the reaction, and extract three times with ethyl acetate. Combine the organic phase, wash with saturated salt water, dry with anhydrous sodium sulfate, and evaporate under vacuum. The crude product is purified through silica gel column to give 50 mg of product 32-7, with the yield of 35%.

Step 7: Preparation of 3-(5-fluoropyrimidin-2-yl)-2,3,4,4a,5,6-hexahydro-1*H*-pyrazino [1,2-*a*] \quinolin-8-amine **(32-8)**

Add compound 32-7 (50 mg, 0.15 mmol) and methanol (3 mL) to a reaction flask, followed by the addition of Pd/C (10 mg), then processed with hydrogen replacement. Stir under hydrogen atmosphere for 3 h till TLC confirms a complete reaction. Filter, the filtrate is evaporate under vacuum, and the resulting crude product is purified by thin-layer preparation plates to give 35 mg of product 32-8, with the yield of 77%.

Step 8: Preparation of 2-(1,5-dimethyl-3-phenyl-1*H*-pyrrol-2-yl)-*N*-(3-(5-fluoropyrimidin-2-yl)-2,3,4,4a,5,6-hexahydro-1*H*-pyrazino [1,2-*a*] quinolin-8-yl)-2-oxo acetamide **(T-32)**

Add compound 32-8 (35 mg, 0.12 mmol), DCM (2 mL) and DIEA (47 mg, 0.36 mmol) to a reaction flask, cool to 0 °C in an ice water bath, add DCM (1 mL) solution of compound A (31 mg, 0.12 mmol). Let the temperature of reaction solution resume slowly to room temperature and stir for 3 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 38 mg of product T-32, with the yield of 62%.

LC-MS (ESI) m/z $(M+H)^+$: 525.2
$^1$H NMR (400 MHz, DMSO-$d_6$) δ 10.11 (s, 1H), 8.49 (s, 2H), 7.26-7.21 (m, 2H), 7.18-7.11 (m, 3H), 6.86-6.81 (m, 1H), 6.75- 6.69 (m, 2H), 6.10 (s, 1H), 4.60-4.48 (m, 2H), 3.90-3.83 (m, 1H), 3.81 (s, 3H), 3.12-3.03 (m, 1H), 2.93-2.85 (m, 1H), 2.80-2.58 (m, 4H), 2.32 (s, 3H), 2.00- 1.90 (m, 1H), 1.72-1.60 (m, 1H).

**Example 33**

Preparation of (*S*)-2-(1,5-dimethyl-3-phenyl-1*H*-pyrrol-2-yl)-*N*-(2-(5-fluoropyrimidin-2-yl)-1,2,3,4,10,10a-hexahydropyrazino[1,2-*a*]indol-8-yl)-2-oxoacetamide **(T-33)**

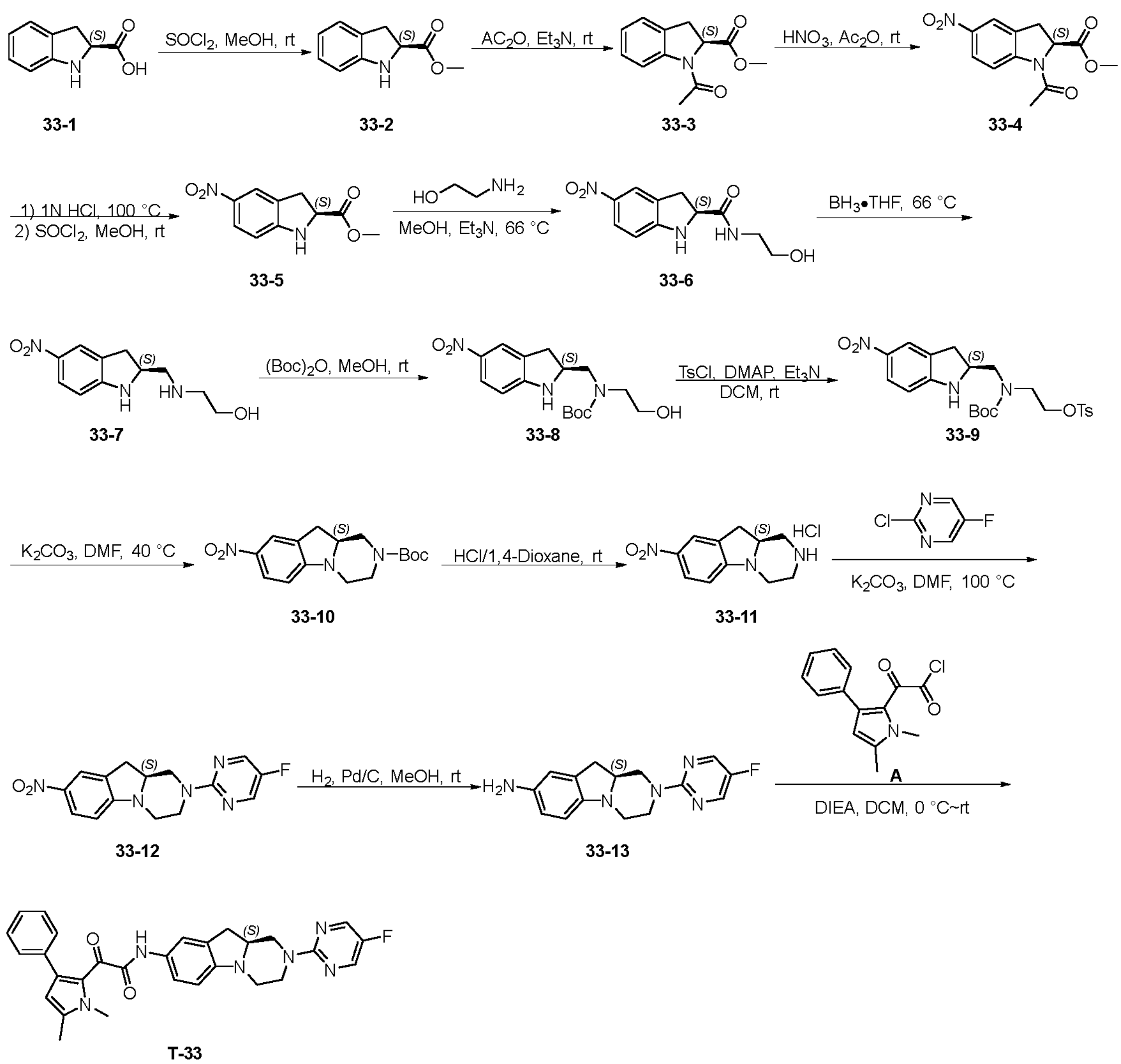


33-1 33-2 33-3 33-4 33-5 33-6 33-7 33-8 33-9 33-10 33-11 33-12 33-13 T-33

Step 1: Preparation of (S)-dihydroindole-2-carboxylic acid methyl ester **(33-2)**

Add 33-1 (2.0 g, 12.3 mmol) and methanol (20 mL) to a dry three-necked flask, cool to 0 °C in an ice bath, then drop in thionyl chloride (2.9 g, 24.4 mmol). Let the temperature of reaction solution return to room temperature, and stir for 1 h till TLC confirms the reaction is complete. The reaction solution is concentrated under reduced pressure to remove the solvent to give 2.1 g of crude product 33-2, which is directly used in the next reaction step.

Step 2: Preparation of (*S*)-1-acetyldihydroindole-2-carboxylic acid methyl ester **(33-3)**

Add compound 33-2 (2.1 g) and acetic anhydride (20 mL) to a reaction flask, followed by $Et_3N$ (2.4 g, 23.7 mmol). Stir at room temperature for 2 h till TLC confirms a complete reaction, add ethyl acetate, then wash with saturated sodium bicarbonate solution and then saturated citric acid solution. The organic phase is dried with anhydrous sodium sulfate, and the evaporated under vacuum. The crude product is purified through silica gel column to give 2.2 g of product 33-3, with the two-step yield of 82%.

Step 3: Preparation of (*S*)-1-acetyl-5-nitroindole-2-carboxylic acid methyl ester **(33-4)**

Add compound 33-3 (2.0 g, 9.1 mmol) and acetic anhydride (20 mL) to a dry three-necked flask, cool to 0 °C in an ice bath, and then slowly drop in a solution of fuming nitric acid (0.8 mL, 17.9 mmol) in acetic anhydride (20 mL) during which the temperature of the system is kept below 5 °C, then stir at room temperature for 2 h till TLC confirms that the reaction is complete. Add ice water to stop the reaction, and extract three times with ethyl acetate. Combine the organic phase, wash with saturated salt water, dry with anhydrous sodium sulfate, and evaporate under vacuum. The crude product obtained is purified through silica gel column to give 892 mg of product 33-4, with the yield of 37%.

Step 4: Preparation of (S)-5-nitrodihydroindole-2-carboxylic acid methyl ester **(33-5)**

Add compound 33-4 (890 mg, 3.4 mmol) and 1 M dilute hydrochloric acid solution (25 mL) to a reaction flask. Heat to 100 °C and reflux for 2 h, then concentrate under reduced pressure to remove the solvent therein, dissolve the residue in methanol (25 mL), and cool to 0 °C in an ice bath, and then drop in thionyl chloride (3 mL). Let the temperature of reaction solution return to room temperature and stir for 1 h till TLC confirms that the reaction is complete. The solvent is removed by concentration under reduced pressure, and the crude product obtained is purified through silica gel column to give 711 mg of product 33-5, with the yield of 95%.

Step 5: Preparation of (*S*)-*N*-(2-hydroxyethyl)-5-nitroindole-2-carboxyamide **(33-6)**

Add compound 33-5 (700 mg, 3.2 mmol) and methanol (10 mL) to a reaction flask, followed by ethanolamine (770 mg, 12.6 mmol) and $Et_3N$ (1.3 g, 12.8 mmol). Heat to 66 °C and stir for 5 h till TLC confirms that the reaction is complete. Add appropriate amount of water to the mixture, and extract three times with ethyl acetate. Combine the organic phase, dry with anhydrous sodium sulfate, and evaporate under vacuum. The crude product obtained is purified through silica gel column to give 632 mg of product 33-6, with the yield of 80%.

Step 6: Preparation of (*S*)-2-(((5-**nitroindolin**-2-yl) methyl) amino) ethan-1-ol **(33-7)**

Add compound 33-6 (400 mg, 1.6 mmol) and anhydrous THF (40 mL) to a dry three-necked flask, followed by $BH_3$•THF (1 M in THF, 4.0 mL, 4.0 mmol). Heat to 66 °C and stir for 0.5 h till TLC confirms a complete reaction. Cool to room temperature, add methanol to stop the reaction, and stir for 10 min. The reaction solution is concentrated to dryness under reduced pressure, and the resulting crude product is purified through silica gel column to give 337 mg of product 33-7, with the yield of 89%.

Step 7: Preparation of (*S*)-(2-hydroxyethyl)((5-**nitroindolin**-2-yl) methyl) carbamic acid tert-butyl ester **(33-8)**

Add compound 33-7 (300 mg, 1.3 mmol) and methanol (5 mL) to a reaction flask, followed by di-tert-butyl dicarbonate (330 mg, 1.5 mmol). Stir at room temperature for 1 h till TLC confirms a complete reaction. The solvent is removed by concentration under reduced pressure, and the resulting crude product is purified through silica gel column to give 404 mg of product 33-8, with the yield of 95%.

Step 8: Preparation of (*S*)-2-((tert-butoxycarbonyl) ((5-**nitroindolin**-2-yl) methyl) amino) ethyl 4-methylbenzenesulfonate **(33-9)**

Add compound 33-8 (400 mg, 1.2 mmol), DCM (5 mL) and $Et_3N$ (180 mg, 1.8 mmol) to a reaction flask, followed by p-toluenesulfonyl chloride (339 mg, 1.8 mmol) and 4-dimethylaminopyridine (12 mg, 0.1 mmol). Stir at room temperature for 5 h till TLC confirms that the reaction is complete. Add an appropriate amount water, and extract 3 times with DCM. Combine the organic phase, wash with saturated salt water, dry with anhydrous sodium sulfate, and evaporate under vacuum to give 580 mg of crude product 33-9, which is directly used in the next reaction step.

Step 9: Preparation of (*S*)-8-nitro-3,4,10,10a-tetrahydropyrazino [1,2-*a*] indole-2(1*H*)-carboxylic acid tert-butyl ester **(33-10)**

Add compound 33-9 (580 mg, 1.2 mmol) and DMF (5 mL) to a reaction flask, followed by potassium carbonate (329 mg, 2.4 mmol). Heat to 40 °C and stir for 0.5 h till TLC confirms a complete reaction. Add water to stop the reaction, and extract three times with ethyl acetate. Combine the organic phase, wash with saturated salt water, dry with anhydrous sodium sulfate, and evaporate under vacuum. The crude product is purified through silica gel column to give 140 mg of product 33-10, with the two-step yield of 37%.

Step 10: Preparation of (*S*)-8-nitro-1,2,3,4,10,10a-hexahydropyrazino[1,2-*a*] indole hydrochloride **(33-11)**

Add compound 33-10 (140 mg, 0.44 mmol) and 1, 4-dioxane (2 mL) to a reaction flask, followed by a solution of hydrogen chloride in 1,4-dioxane (4 M in 1,4-dioxane, 3 mL), stir at room temperature for 2 h till TLC confirms that the reaction is complete. The reaction solution is evaporated under vacuum to give 110 mg of crude product 33-11, which is used directly in the next reaction step without purification.

Step 11: Preparation of (*S*)-2-(5-fluoropyrimidin-2-yl)-8-nitro-1,2,3,4,10,10a-hexahydropyrazino [1,2-*a*] indole **(33-12)**

Add compound 33-11 (110 mg, 0.43 mmol), DMF (5 mL) and potassium carbonate (182 mg, 1.3 mmol) to a reaction flask, followed by 2-chloro-5-fluoropyrimidine (175 mg, 1.3 mmol). Heat to 100 °C and stir for 5 h till TLC confirms that the reaction is complete. Add water to quench the reaction, and extract three times with ethyl acetate. Combine the organic phase, wash with saturated salt water, dried with anhydrous sodium sulfate, and evaporate under vacuum. The crude product is purified through silica gel column to give 117 mg of product 33-12, with the two-step yield of 85%.

Step 12: Preparation of (*S*)-2-(5-fluoropyrimidin-2-yl)-1,2,3,4,10,10a-hexahydropyrazino [1,2-*a*] indol-8-amine **(33-13)**

Add compound 33-12 (60 mg, 0.19 mmol) and methanol (3 mL) to a reaction flask, followed by the addition of Pd/C (15 mg), and processed with hydrogen replacement. Stir under hydrogen atmosphere for 1 h till TLC confirms complete reaction. Filter, the resulting filtrate is evaporated under vacuum, and the resulting crude product is purified by thin-layer preparation plates to give 50 mg of product 33-13, with the yield of 92%.

Step 13: Preparation of (*S*)-2-(1,5-dimethyl-3-phenyl-1*H*-pyrrol-2-yl)-*N*-(2-(5-fluoropyrimidin-2-yl)-1,2,3,4,10,10a-hexahydropyrazino [1, 2-*a*] indol-8-yl)-2-oxyacetamide **(T-33)**

Add compound 33-13 (50 mg, 0.18 mmol), DCM (2 mL) and DIEA (70 mg, 0.54 mmol) to a reaction flask, cool to 0 °C in an ice water bath, drop in a solution of compound A (52 mg, 0.20 mmol) in DCM (1 mL). Let the reaction solution slowly return to room temperature and stir for 1 h till TLC confirms a complete reaction. Pour the resulting solution into water, and extract twice with dichloromethane. Combine the organic phase, wash with saturated salt water, dry with anhydrous $Na_2SO_4$, and evaporate under vacuum. The crude product is purified with thin-layer preparation plates to give 53 mg of product T-33, with the yield of 59%.

LC-MS (ESI) m/z $(M+H)^+$: 511.2

$^1$H NMR (400 MHz, DMSO-$d_6$) δ 10.10 (s, 1H), 8.48 (s, 2H), 7.26-7.22 (m, 2H), 7.17-7.09 (m, 3H), 6.87-6.84 (m, 1H), 6.84- 6.80 (m, 1H), 6.40 (d, *J*= 8.4 Hz, 1H), 6.10 (s, 1H), 4.68-4.62 (m, 1H), 4.60-4.53 (m, 1H), 3.82 (s, 3H), 3.67-3.61 (m, 1H), 3.39-3.30 (m, 1H), 3.01 (td, *J*= 12.8, 3.2 Hz, 1H), 2.95-2.85 (m, 2H), 2.78 (td, *J*= 12.0, 3.2 Hz, 1H), 2.56-2.52 (m, 1H), 2.32 (s, 3H).

**Example 34**

Preparation of (*R*)-2-(1,5-dimethyl-3-phenyl-1*H*-pyrrol-2-yl)-*N*-(2-(5-fluoropyrimidin-2-yl)-1,2,3,4,10, 10a-hexahydropyrazino [1,2-*a*] indol-8-yl)-2-oxoacetamide **(T-34)**

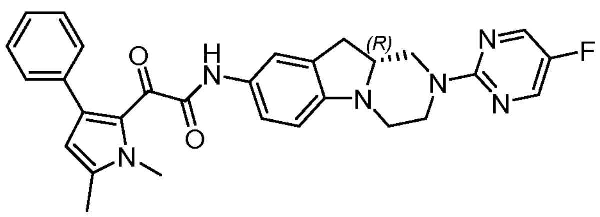

Compound **T-34** is prepared by the same method used for the preparation of compound T-33 and using different raw materials ((*R*)-dihydroindole-2-carboxylic acid ((34-1) replacing the raw material (*S*)-dihydroindole-2-carboxylic acid (33-1) in the first step in example 33))

LC-MS (ESI) m/z $(M+H)^+$: 511.2

$^1$H NMR (400 MHz, DMSO-$d_6$) δ 10.11 (s, 1H), 8.48 (s, 2H), 7.26-7.22 (m, 2H), 7.17-7.09 (m, 3H), 6.87-6.85 (m, 1H), 6.84- 6.80 (m, 1H), 6.40 (d, *J*= 8.4 Hz, 1H), 6.10 (s, 1H), 4.68-4.61 (m, 1H), 4.60-4.53 (m, 1H), 3.82 (s, 3H), 3.67-3.61 (m, 1H), 3.39-3.30 (m, 1H), 3.05 -2.96 (m, 1H), 2.94-2.85 (m, 2H), 2.83-2.74 (m, 1H), 2.56-2.53 (m, 1H), 2.32 (s, 3H).

**Example 35**

Preparation of (*R*)-2-(3-(2-fluorophenyl)-1, 5-dimethyl-1*H*-pyrrol-2-yl)-*N*-(3-(5-fluoropyrimidin-2-yl)-1, 2, 3, 4, 4a, 5-hexahydrobenzo [b] pyrazino[1,2-d] [1,4] oxazin-8-yl)-2-oxoacetamide **(T-35)**

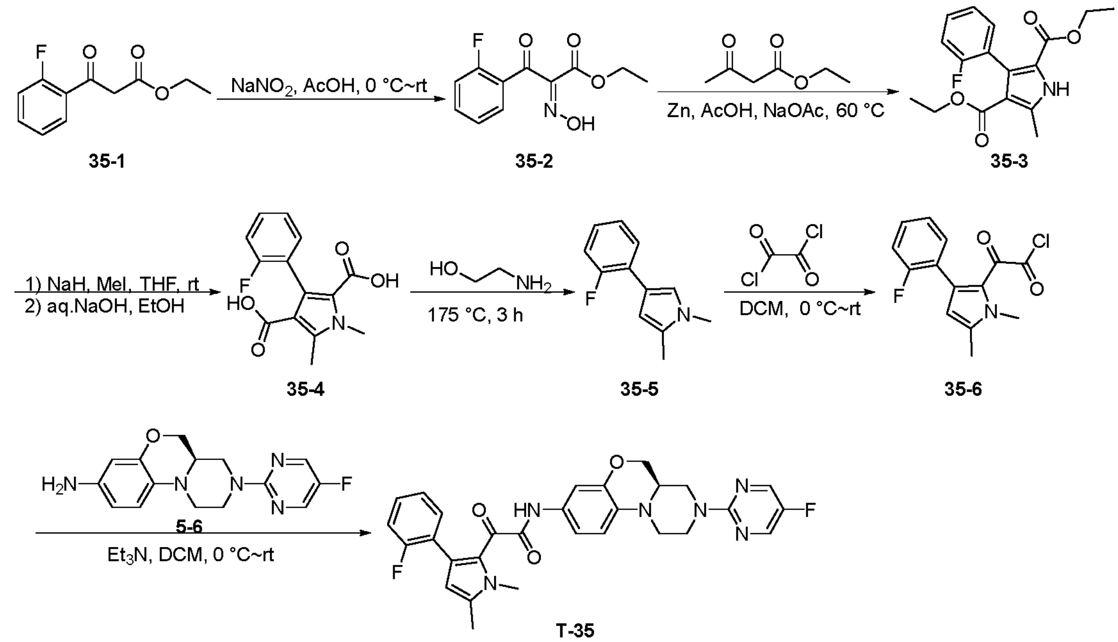


Step 1: Preparation of (*Z*)-3-(2-fluorophenyl)-2-(hydroxyimino)-3-oxopropanoic acid ethyl ester **(35-2)**

Add compound 35-1 (1.0 g, 4.8 mmol) and glacial acetic acid (2 mL) to a reaction flask, and drop in a solution of sodium nitrite (493 mg, 7.1 mmol) and water (3 mL) at a temperature between 0 °C and 10 °C. Stir at room temperature for 1 h till LC-MS confirms that the reaction is complete. Add 2 ml of water, and stir for 1 h, filter. The filter cake is washed with 2 mL of water, then dissolve in dichloromethane (2 mL). Wash the solution with water and saturated salt water successively, dry with anhydrous $Na_2SO_4$, and evaporate under vacuum to give 1.0 g of product 35-2, with the yield of 88%.

Step 2: Preparation of diethyl 3-(2-fluorophenyl)-5-methyl-1*H*-pyrrole-2,4-dicarboxylate **(35-3)**

Add zinc powder (863 mg, 13.2 mmol), anhydrous sodium acetate (902 mg, 11.0 mmol), ethyl acetoacetate (640 mg, 4.9 mmol) and glacial acetic acid (4 mL) to a reaction flask, heat in an oil bath to 60 °C. Add the solution of compound 35-2 (1.0 g, 4.2 mmol) in glacial acetic acid (6 mL) in three portions, heat to 70 °C, and stir for 3 h. Then add zinc powder (431 mg, 6.6 mmol), the reaction is carried out for 1 h till TLC confirms that the reaction of the raw materials is complete. Cool to room temperature, and filter. Evaporate the filtrate under vacuum, and pour the residual liquid into water, and extract with ethyl acetate. The organic phase is wash with saturated salt water, dry with anhydrous $Na_2SO_4$, and evaporate under vacuum. The crude product is purified through silica gel column to give 1.2 g of product 35-3, with the yield of 90%.

Step 3: Preparation of 3-(2-fluorophenyl)-1, 5-dimethyl-1*H*-pyrrole-2,4-dicarboxylic acid **(35-4)**

Add sodium hydride (60% in mineral oil, 150 mg, 3.8 mmol) and THF (10 mL) to a reaction flask, and drop in a solution of compound 35-3 (600 mg, 1.9 mmol) in THF (10 mL) at 0 °C. Stir at room temperature for 1 h, then place the reaction solution in an ice-water bath, and add iodomethane (667 mg, 4.7 mmol) slowly. The reaction is carried out overnight at room temperature, using LC-MS to confirm that the reaction is complete. Add water to quench the reaction, and evaporate under vacuum to remove the solvent therein. Then add ethanol (3 mL) and aqueous solution (2.5 mL) of sodium hydroxide (752 mg, 18.8 mmol), heat to 100 °C and reflux for 18 h till LC-MS confirms that the reaction is complete. Evaporate under vacuum to remove the ethanol, add an appropriate amount of water, and cool in an ice water bath. Adjust to a pH of 2 with concentrated hydrochloric acid, and stir below 10 °C for 1 h. Filter, wash the filter cake

with water and then petroleum ether, and the resulting solid is dried under vacuum to give 480 mg of product 35-4, with the yield of 92%.

Step 4: Preparation of 4-(2-fluorophenyl)-1,2-dimethyl-1*H*-pyrrole **(35-5)**

Add compound 35-4 (300 mg, 1.1 mmol) and ethanolamine (2 mL) to a reaction flask, purge with nitrogen, then heat to 175 °C and stir for 3 h till LC-MS confirms a complete reaction. Cool to room temperature, add an appropriate amount of water, and extract twice with ethyl acetate. Combine the organic phase, wash with water and then saturated salt water, dry with anhydrous $Na_2SO_4$, and evaporate under vacuum below 40 °C. The crude product is purified through a neutral alumina column to give 109 mg of product 35-5, with the yield of 53%.

Step 5: Preparation of 2-(3-(2-fluorophenyl)-1,5-dimethyl-1*H*-pyrrol-2-yl)-2-oxoacetyl chloride **(35-6)**

Add compound 35-5 (26 mg, 0.14 mmol) and DCM (2 mL) to a reaction flask, then add oxalyl chloride (20 mg, 0.15 mmol) slowly at 0 °C. Then the reaction is carried out at room temperature for 1 h till LC-MS confirms a complete reaction. The solution is directly concentrated under reduced pressure to give 38 mg of product 35-6, which is directly used in the next step without further treatment.

Step 6: Preparation of (*R*)-2-(3-(2-fluorophenyl)-1,5-dimethyl-1*H*-pyrrol-2-yl)-*N*-(3-(5-fluoropyrimidin-2-yl)-1,2,3,4,4a,5-hexahydrobenzo[*b*]pyrazino[1,2-*d*] [1, 4] oxazin-8-yl)-2-oxoacetamide **(T-35)**

Add compound 5-6 (35 mg, 0.12 mmol), DCM (1 mL) and $Et_3N$ (24 mg, 0.24 mmol) to a reaction flask, cool to 0 °C in an ice water bath, and drop in a solution of compound 35-6 (38 mg, 0.14 mmol) in DCM (1 mL). Let the temperature of reaction solution slowly recover to room temperature and stir for 1 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 24 mg of product T-35, with the yield of 38%.

LC-MS (ESI) m/z $(M+H)^+$: 545.2
$^1H$ NMR (400 MHz, DMSO-$d_6$) δ 10.16 (s, 1H), 8.50 (s, 2H), 7.21-7.11 (m, 2H), 7.03-6.96 (m, 1H), 6.93 (td, *J*= 12.8, 3.2 Hz, 1H), 6.77-6.73 (m, 1H), 6.70-6.65 (m, 1H), 6.65-6.62 (m, 1H), 6.11 (s, 1H), 4.64-4.52 (m, 2H), 4.34 (dd, *J*= 10.8, 2.4 Hz, 1H), 3.92 (dd, *J*= 10.8, 8.8 Hz, 1H), 3.82 (s, 3H), 3.82-3.77 (m, 1H), 3.10-2.95 (m, 2H), 2.72-2.52 (m, 2H), 2.32 (s, 3H).

**Example 36**

Preparation of (*R*)-2-(3-(3-fluorophenyl)-1,5-dimethyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-36)**

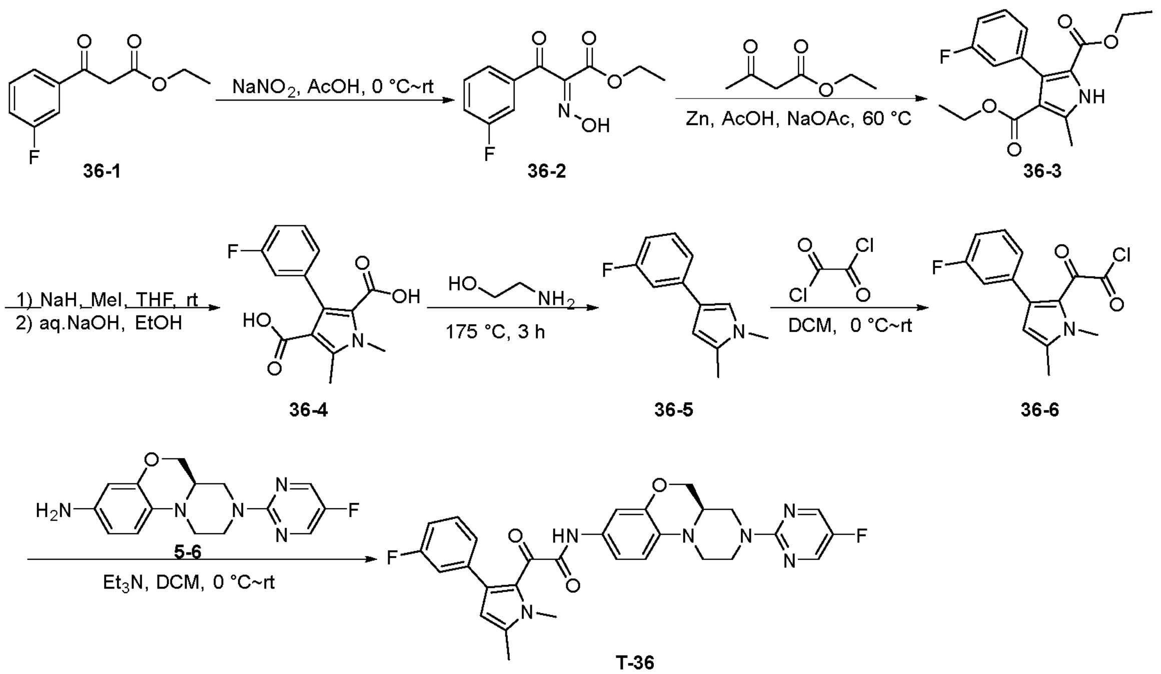


36-1 36-2 36-3 36-4 36-5 36-6 5-6 T-36

Step 1: Preparation of (*Z*)-3-(3-fluorophenyl)-2-(hydroxyimino)-3-oxopropanoic acid ethyl ester **(36-2)**

Add compound 36-1 (1.0 g, 4.8 mmol) and glacial acetic acid (2 mL) to a reaction flask, then drop in a solution of sodium nitrite (493 mg, 7.1 mmol) and water (3 mL) at a temperature between 0 °C and 10 °C, stir at room temperature for 1 h till LC-MS confirms that the reaction is complete. Add 2 mL of water and stir for 1 h. Filter, wash the filter cake with 2 mL of water, then dissolve in dichloromethane (2 mL). Wash the solution with water and then saturated salt water, dry with anhydrous $Na_2SO_4$, and evaporate under vacuum to give 1.1 g of product 36-2, with the yield of 97%.

Step 2: Preparation of diethyl 3-(3-fluorophenyl)-5-methyl-1*H*-pyrrole-2, 4-dicarboxylate **(36-3)**

Add zinc powder (896 mg, 13.7 mmol), anhydrous sodium acetate (935 mg, 11.4 mmol), ethyl acetoacetate (664 mg, 5.1 mmol) and glacial acetic acid (4 mL) to a reaction flask, heat in an oil bath to 60 °C, add a solution of compound 36-2 (1.1 g, 4.6 mmol) and glacial acetic acid (6 mL) in three portions. Heat to 70 °C and stir for 3 h, add zinc powder (447 mg, 6.8 mmol), and the reaction is continued for 1 h till TLC confirms that the reaction of the raw materials is complete. Cool to room temperature, and filter, evaporate the filtrate under vacuum, pour the residual liquid into water, and extract with ethyl acetate. Wash the organic phase with saturated salt water, dry with anhydrous $Na_2SO_4$, and evaporate under vacuum. The crude product is purified through silica gel column to give 1.2 g of product 36-3, with the yield of 82%.

Step 3: Preparation of 3-(3-fluorophenyl)-1,5-dimethyl-1*H*-pyrrole-2,4-dicarboxylic acid **(36-4)**

Add sodium hydride (60% in mineral oil, 150 mg, 3.8 mmol) and THF (10 mL) to a reaction flask, then drop in a solution of compound 36-3 (600 mg, 1.9 mmol) in THF (10 mL) at 0 °C, stir at room temperature for 1 h, then place the reaction solution in an ice-water bath, add iodomethane (667 mg, 4.7 mmol) slowly, and react overnight at room temperature till LC-MS confirms that the reaction is complete. Add water to quench the reaction, evaporate under vacuum to remove the solvent therein, then add ethanol (3 mL) and aqueous solution (2.5 mL) of sodium hydroxide (752 mg, 18.8 mmol), heat to 100 °C, and reflux for 18 h till LC-MS confirms that the reaction is complete, evaporate under vacuum to remove the ethanol, add an appropriate amount of water, cool in an ice-water bath, adjusted to pH 2 with concentrated hydrochloric acid, stir below 10 °C for 1 h. Filter, wash the filter cake with water and petroleum ether successively, and the solid obtained is dry under vacuum to get 500 mg of product 36-4, with the yield of 96%.

Step 4: Preparation of 4-(3-fluorophenyl)-1,2-dimethyl-1*H*-pyrrole **(36-5)**

Add compound 36-4 (300 mg, 1.1 mmol) and ethanolamine (2 mL) to a reaction flask, purge with nitrogen, heat to 175 °C with stirring for 3 h till LC-MS confirms a complete reaction. Cool to room temperature, add an appropriate

amount of water, and extract twice with ethyl acetate. Combine the organic phase, wash sequentially with water and saturated salt water, dry with anhydrous $Na_2SO_4$, and evaporate under vacuum below 40 °C. The crude product is purified through neutral alumina column to give 133 mg of product 36-5, with the yield of 65%.

Step 5: Preparation of 2-(3-(3-fluorophenyl)-1, 5-dimethyl-1*H*-pyrrol-2-yl)-2-oxoacetyl chloride **(36-6)**

Add compound 36-5 (25 mg, 0.13 mmol) and DCM (2 mL) to a reaction flask, add oxalyl chloride (20 mg, 0.15 mmol) slowly at 0 °C. The reaction is carried out at room temperature for 1 h till LC-MS confirms a complete reaction. The reaction solution is directly concentrated to dryness under reduced pressure to give 36 mg of crude product 36-6, which is used directly in the next reaction step without further treatment.

Step 6: Preparation of (*R*)-2-(3-(3-fluorophenyl)-1,5-dimethyl-1*H*-pyrrol-2-yl)-*N*-(3-(5-fluoropyrimidin-2-yl)-1,2,3,4,4a,5-hexahydrobenzo[*b*]pyrazino[1,2-*d*] [1, 4] oxazin-8-yl)-2-oxoacetamide **(T-36)**

Add compound 5-6 (33 mg, 0.11 mmol), DCM (1 mL) and $Et_3N$ (22 mg, 0.22 mmol) to a reaction flask, cool to 0 °C in an ice water bath, drop in a solution of compound 36-6 (36 mg, 0.13 mmol) in DCM (1 mL). Let the temperature of reaction solution slowly recover to room temperature and stir for 1 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 18 mg of product T-36, with the yield of 30%.

LC-MS (ESI) m/z $(M+H)^+$: 545.2

$^1$H NMR (400 MHz, DMSO-$d_6$) δ 10.22 (s, 1H), 8.49 (s, 2H), 7.18-7.11 (m, 1H), 7.06-6.97 (m, 2H), 6.96-6.89 (m, 1H), 6.79- 6.74 (m, 1H), 6.73-6.65 (m, 2H), 6.14 (s, 1H), 4.65-4.52 (m, 2H), 4.34 (dd, *J*= 10.8, 2.4 Hz, 1H), 3.97-3.89 (m, 1H), 3.85-3.81 (m, 1H), 3.81 (s, 3H), 3.11-2.96 (m, 2H), 2.72-2.53 (m, 2H), 2.31 (s, 3H).

**Example 37**

Preparation of (R)-2-(3-(4-fluorophenyl)-1, 5-dimethyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-37)**

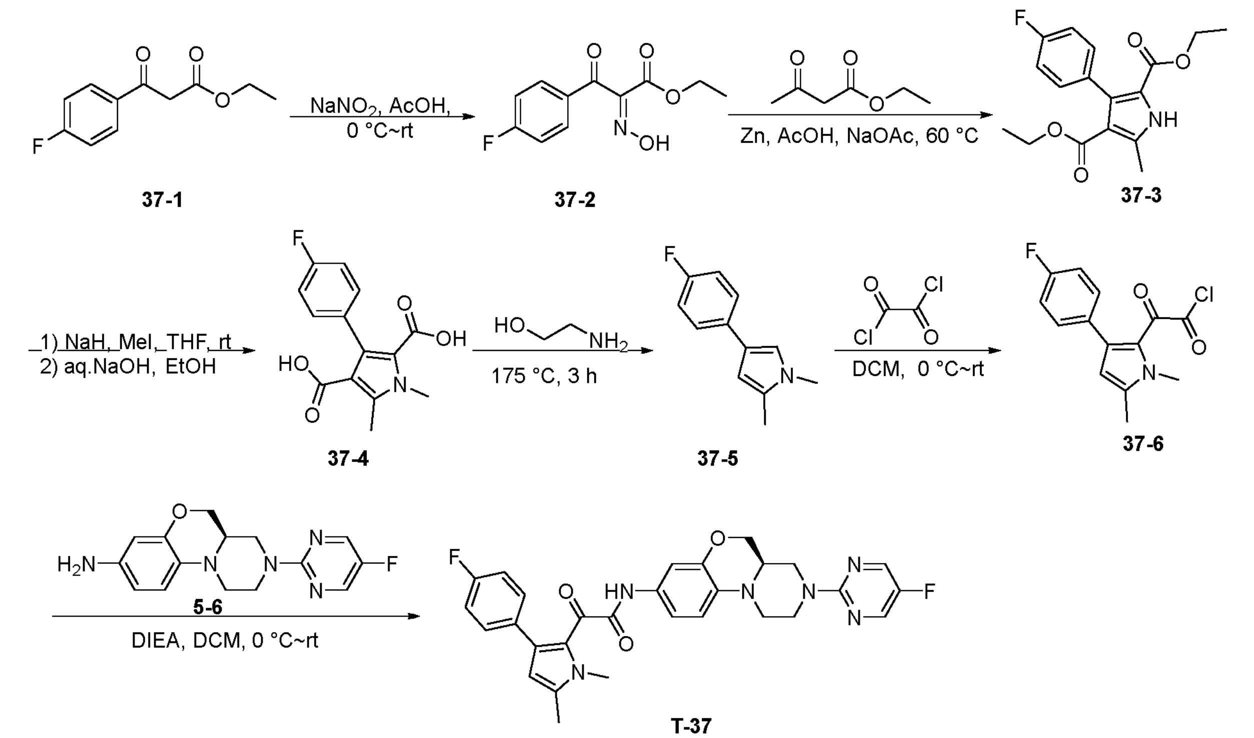


37-1 37-2 37-3 37-4 37-5 37-6 5-6 T-37

Step 1: Preparation of (*Z*)-3-(4-fluorophenyl)-2-(hydroxyimino)-3-oxopropanoic acid ethyl ester **(37-2)**

Add compound 37-1 (1.4 g, 6.7 mmol) and glacial acetic acid (4 mL) to a reaction flask, drop in a solution of sodium nitrite (1.9 g, 27.5 mmol) in water (6 mL) during which the temperature is maintained between 0 °C and 10 °C, using LC-MS to confirm that the reaction is complete. Add 4 mL of water and stir for 1 h, filter. Wash the filter cake with 5 mL of water, then dissolve in dichloromethane (5 mL), wash the solution with water and then saturated salt water, dry with anhydrous $Na_2SO_4$, and evaporate under vacuum to give 1.6 g of the crude product 37-2.

Step 2: Preparation of diethyl 3-(4-fluorophenyl)-5-methyl-1*H*-pyrrole-2,4-dicarboxylate **(37-3)**

Add zinc powder (1.3 g, 19.9 mmol), anhydrous sodium acetate (1.4 g, 17.1 mmol), ethyl acetoacetate (1.1 g, 8.5 mmol) and glacial acetic acid (5 mL) to a reaction flask, heat in an oil bath to 60 °C, add a solution of compound 37-2 (1.6 g, 6.7 mmol) and glacial acetic acid (5 mL) in three portions. Heat to 70 °C, stir for 3 h, then add zinc powder (661 mg, 10.1 mmol), and reacted for 1 h till TLC confirms that the reaction of the raw materials is complete. Cool to room temperature, filter. Evaporate the filtrate under vacuum, pour the residual liquid into water, and extract with ethyl acetate. Wash the organic phase with saturated salt water, dry with anhydrous $Na_2SO_4$, and evaporate under vacuum. The crude product is purified through silica gel column to give 1.2 g of product 37-3, with the two-step yield of 56%.

Step 3: Preparation of 3-(4-fluorophenyl)-1,5-dimethyl-1*H*-pyrrole-2,4-dicarboxylic acid **(37-4)**

Add sodium hydride (60% in mineral oil, 602 mg, 15.0 mmol) and THF (10 mL) to a reaction flask, drop in a solution of compound 37-3 (1.2 g, 3.8 mmol) in THF (10 mL) at 0 °C, then stir at room temperature for 1 h, after which place the reaction solution in an ice water bath, add iodomethane (2.7 g, 19.0 mmol) slowly, react overnight at room temperature till LC-MS confirms that the reaction is complete. Add water to quench the reaction, evaporate under vacuum to remove the solvent therein, and then add ethanol (10 mL) and aqueous solution (8 mL) of sodium hydroxide (1.8 g, 45.0 mmol). Heat to 100 °C and reflux for 18 h till LC-MS confirms that the reaction is complete, evaporate under vacuum to remove the ethanol, add an appropriate amount of water, cool in an ice-water bath, adjust to a pH of 2 with concentrated hydrochloric acid, and stir below 10 °C for 1 h. Filter, wash the filter cake obtained with water and petroleum ether successively, and the solid obtained is dry under vacuum to get 500 mg of product 37-4, with the yield of 67%.

Step 4: Preparation of 4-(4-fluorophenyl)-1,2-dimethyl-1*H*-pyrrole **(37-5)**

Add compound 37-4 (400 mg, 1.4 mmol) and ethanolamine (4 mL) to a reaction flask, purge with nitrogen, heat to 175 °C with stirring for 3 h till LC-MS confirms a complete reaction. Cool to room temperature, add an appropriate amount of water, and extract twice with ethyl acetate. Combine the organic phase, wash sequentially with water and saturated salt water, dry with anhydrous $Na_2SO_4$, and evaporate under vacuum. The crude product is purified by neutral alumina column to give 130 mg of product 37-5, with the yield of 48%.

Step 5: Preparation of 2-(4-(2-fluorophenyl)-1,5-dimethyl-1*H*-pyrrol-2-yl)-2-oxoacetyl chloride **(37-6)**

Add compound 37-5 (60 mg, 0.32 mmol) and DCM (2 mL) to a reaction flask, add oxalyl chloride (45 mg, 0.35 mmol) slowly at 0 °C. The reaction is carried out at room temperature for 1 h till LC-MS confirms a complete reaction. The reaction solution is directly concentrated to dryness under reduced pressure to give 88 mg of product 37-6, which is used directly in the next reaction step without further treatment.

Step 6: Preparation of (*R*)-2-(4-(2-fluorophenyl)-1,5-dimethyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-37)**

Add compound 5-6 (87 mg, 0.29 mmol), DCM (3 mL) and DIEA (112 mg, 0.87 mmol) to a reaction flask, cool to 0 °C in an ice water bath, drop in a solution of compound 35-6 (88 mg, 0.31 mmol) in DCM (2 mL). Let the temperature of reaction solution slowly recover to room temperature and stir for 1 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 80 mg of product T-37, with the yield of 51%.

LC-MS (ESI) m/z $(M+H)^+$: 545.2
$^1$H NMR (400 MHz, DMSO-$d_6$) δ 10.17 (s, 1H), 8.50 (s, 2H), 7.24-7.18 (m, 2H), 6.94-6.87 (m, 2H), 6.79-6.75 (m, 1H), 6.72- 6.64 (m, 2H), 6..09 (s, 1H), 4.65-4.53 (m, 2H), 4.34 (dd, *J*= 10.8, 2.8 Hz, 1H), 3.93 (dd, *J*= 10.8, 8.8 Hz,

1H), 3.85-3.82 (m, 1H), 3.81 (s, 3H), 3.11-2.95 (m, 2H), 2.73-2.65 (m, 1H), 2.59 (dd, *J*= 12.0, 3.2 Hz, 1H), 2.31 (s, 3H).

**Example 38**

Preparation of (*S*)-2-(3-(2-fluorophenyl)-1,5-dimethyl-1*H*-pyrrol-2-yl)-*N*-(2-(5-fluoropyrimidin-2-yl)-1,2,3,4,10,10a-hexahydropyrazino[1,2-*a*] indol-8-yl)-2-oxyacetamide **(T-38)**

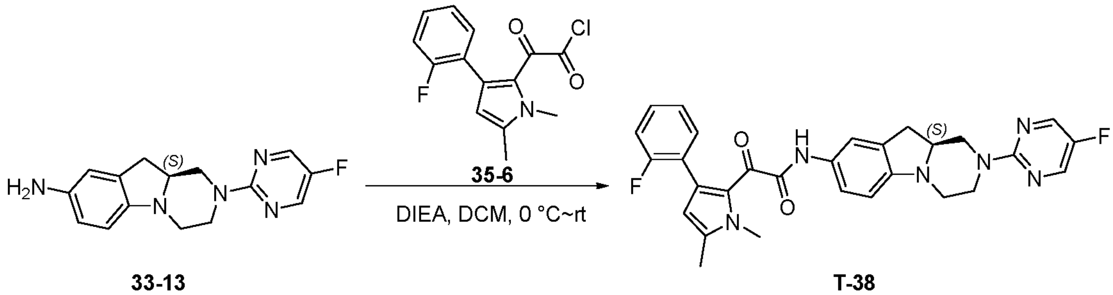


**33-13** **T-38**

Step 1: Preparation of (*S*)-2-(3-(2-fluorophenyl)-1,5-dimethyl-1*H*-pyrrol-2-yl)-*N*-(2-(5-fluoropyrimidin-2-yl)-1,2,3,4,10,10a-hexahydropyrazino[1,2-*a*] indol-8-yl)-2-oxoacetamide **(T-38)**

Add compound 33-13 (57 mg, 0.20 mmol), DCM (2 mL) and DIEA (78 mg, 0.60 mmol) to a reaction flask, cool to 0 °C in an ice water bath, drop in a solution of compound 35-6 (62 mg, 0.22 mmol) in DCM (1 mL). Let the temperature of reaction solution slowly recover to room temperature and stir for 1 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 80 mg of product T-38, with the yield of 76%.

LC-MS (ESI) m/z $(M+H)^+$: 529.2
$^1H$ NMR (400 MHz, DMSO-$d_6$) δ 10.08 (s, 1H), 8.48 (s, 2H), 7.23-7.14 (m, 2H), 7.05-6.99 (m, 1H), 6.98-6.92 (m, 1H), 6.88- 6.81 (m, 2H), 6.40 (d, *J*= 8.4 Hz, 1H), 6.11 (s, 1H), 4.68-4.62 (m, 1H), 4.60-4.53 (m, 1H), 3.84 (s, 3H), 3.67-3.61 (m, 1H), 3.40-3.33 (m, 1H), 3.06 -2.97 (m, 1H), 2.95-2.85 (m, 2H), 2.84-2.75 (m, 1H), 2.57-2.52 (m, 1H), 2.33 (s, 3H).

**Example 39**

Preparation of 2-(3-(2-fluorophenyl)-1,5-dimethyl-1*H*-pyrrol-2-yl)-*N*-(3-(5-fluoropyrimidin-2-yl)-1,2,3,4,4a,5-hexahydro-7*H*-benzo[e]pyrazine[2,1-*c*] [1,4] oxazolin-9-yl)-2-oxoacetamide **(T-39)**

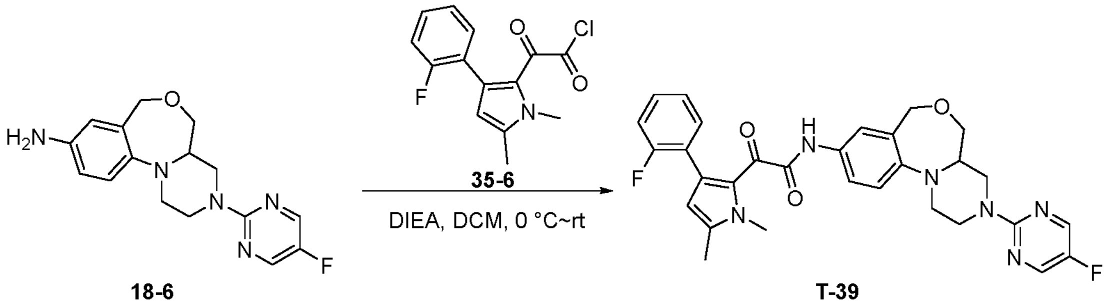


**18-6** **T-39**

Step 1: Preparation of 2-(3-(2-fluorophenyl)-1,5-dimethyl-1*H*-pyrrol-2-yl)-*N*-(3-(5-fluoropyrimidin-2-yl)-1,2,3,4,4a,5-hexahydro-7*H*-benzo[e]pyrazine[2,1-*c*] [1,4] oxazolin-9-yl)-2-oxoacetamide **(T-39)**

Add compound 18-6 (50 mg, 0.16 mmol), DCM (2 mL) and DIEA (62 mg, 0.48 mmol) to a reaction flask, cool

to 0 °C in an ice water bath, drop in a solution of compound 35-6 (50 mg, 0.18 mmol) in DCM (1 mL). Let the temperature of reaction solution slowly recover to room temperature and stir for 1 h till TLC confirms a complete reaction. Pour the reaction solution into water, and extract twice with dichloromethane. Combine the organic phase, wash with saturated salt water, dry with anhydrous $Na_2SO_4$, and evaporate under vacuum. The crude product is purified through thin-layer preparation plates to give 65 mg of product T-39, with the yield of 73%.

LC-MS (ESI) m/z $(M+H)^+$: 559.2

$^1$H NMR (400 MHz, DMSO-$d_6$) δ 10.31 (s, 1H), 8.49 (s, 2H), 7.22-7.16 (m, 1H), 7.16-7.11 (m, 1H), 7.08 (dd, *J*= 8.8, 2.4 Hz, 1H), 7.03-6.97 (m, 1H), 6.96-6.90 (m, 2H), 6.87-6.83 (m, 1H), 6.12 (s, 1H), 4.66-4.61 (m, 1H), 4.47-4.42 (m, 1H), 4.18-4.09 (m, 2H), 3.85 (s, 3H), 3.73-3.67 (m, 1H), 3.64-3.53 (m, 2H), 3.40-3.41 (m, 1H), 3.33-3.28 (m, 2H), 2.99-2.92 (m, 1H), 2.32 (s, 3H).

**Example 40**

Preparation of 2-(3-(2-fluorophenyl)-1,5-dimethyl-1*H*-pyrrol-2-yl)-*N*-(8-(5-fluoropyrimidin-2-yl)-6,6a,7,8,9,10-hexahydropyrazino[1,2-*d*] pyrido [3,2-*b*] [1,4] (oxazin-3-yl)-2-oxoacetamide **(T-40)**

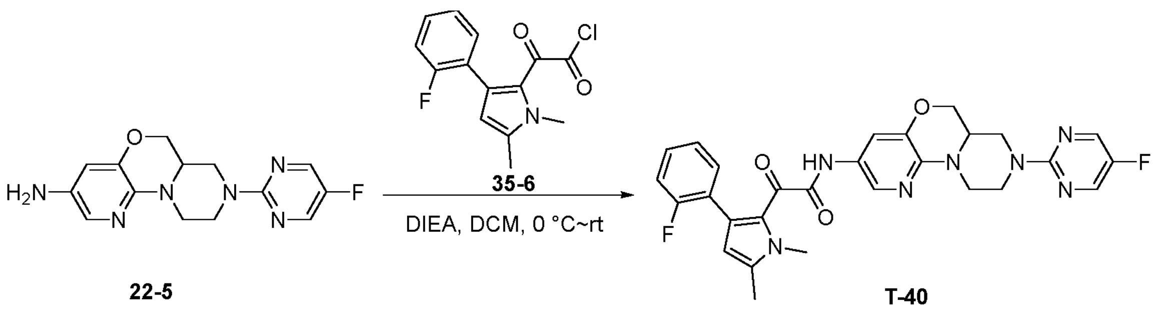


22-5 T-40

Step 1: Preparation of 2-(3-(2-fluorophenyl)-1,5-dimethyl-1*H*-pyrrol-2-yl)-*N*-(8-(5-fluoropyrimidin-2-yl)-6,6a,7,8,9,10-hexahydropyrazino[1,2-d] pyrido [3,2-*b*] [1,4] oxazin-3-yl)-2-oxoacetamide **(T-40)**

Add compound 22-5 (50 mg, 0.17 mmol), DCM (4 mL) and DIEA (64 mg, 0.50 mmol) to a reaction flask, cool to 0 °C in an ice water bath, drop in a solution of compound 35-6 (51 mg, 0.18 mmol) in DCM (1 mL). Let the temperature of reaction solution slowly recover to room temperature and stir overnight, using TLC to confirm a complete reaction. Pour the reaction solution into water, and extract 3 times with dichloromethane. Combine the organic phase, wash with saturated salt water, dry with anhydrous $Na_2SO_4$, and evaporate under vacuum. The crude product is purified through thin-layer preparation plates to give 30 mg of product T-40, with the yield of 33%.

LC-MS (ESI) m/z $(M+H)^+$: 546.2

$^1$H NMR (400 MHz, DMSO-$d_6$) δ 10.34 (s, 1H), 8.51 (s, 2H), 7.62 (d, *J*= 2.0 Hz, 1H), 7.22-7.15 (m, 2H), 7.06-6.94 ( m, 2H), 6.84 (d, *J*= 2.0 Hz, 1H), 6.13 (s, 1H), 4.70-4.59 (m, 2H), 4.43-4.36 (m, 2H), 4.01-3.93 (m, 1H), 3.85 (s, 3H), 3.33-3.26 (m, 1H), 3.05- 2.95 (m, 1H), 2.76-2.65 (m, 2H), 2.34 (s, 3H).

**Example 41**

Preparation of (*R*)-2-(5-cyano-1-methyl-3-phenyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-41)**

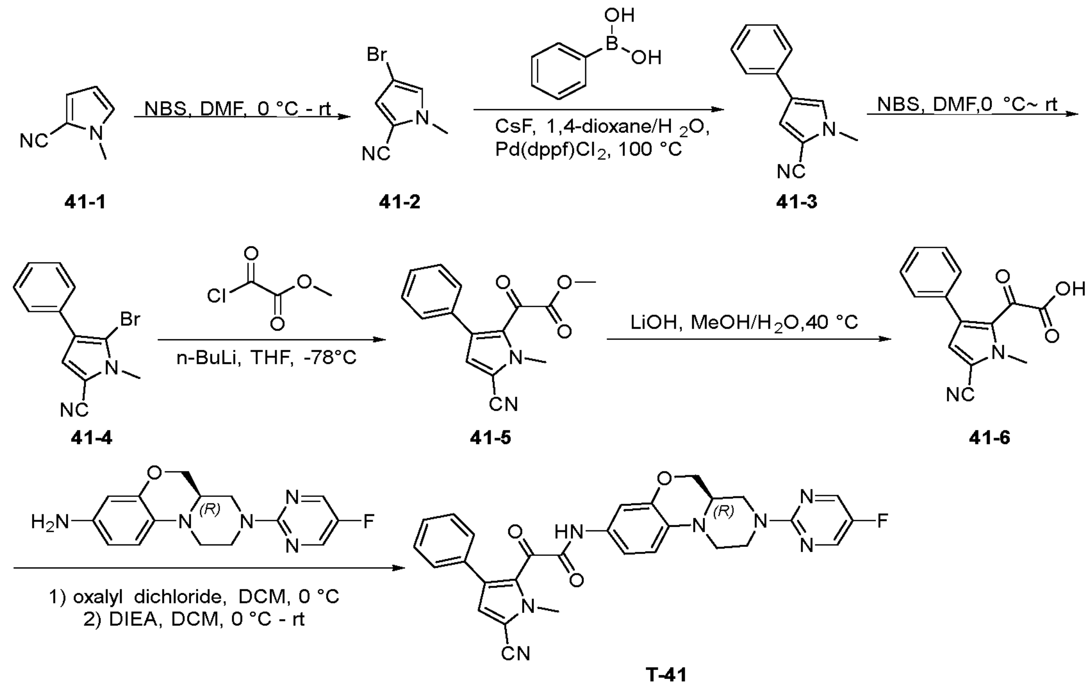


41-1 41-2 41-3 41-4 41-5 41-6 T-41

Step 1: Preparation of 4-bromo-1-methyl-1*H*-pyrrole-2-**carbonitrile (41-2)**

Add compound 41-1 (500 mg, 4.7 mmol) and DMF (5 mL) to a reaction flask, cool to 0 °C in an ice bath, drop in a solution of NBS (839 mg, 4.7 mmol) in DMF (5 mL). Let the temperature of reaction solution slowly recover to room temperature and stir overnight, using LC-MS to confirm a complete reaction. Add an appropriate amount of water, and extract 3 times with ethyl acetate. Combine the organic phase, wash with water and then saturated salt water, dry with anhydrous $Na_2SO_4$. Filter, and the filtrate is evaporated under vacuum to give 700 mg of crude product 41-2, which is directly used in the next reaction step.

Step 2: Preparation of 1-methyl-4-phenyl-1*H*-pyrrolid-2-**carbonitrile (41-3)**

Add compound 41-2 (700 mg, 3.8 mmol), phenylboronic acid (923 mg, 7.6 mmol), cesium fluoride (1.7 g, 11.2 mmol) and 1,4-dioxane/water (20 mL/1 mL) to a reaction flask, processed with nitrogen replacement, and then add Pd(dppf)$Cl_2$(139 mg, 0.19 mmol). Heat to 100°C and stir overnight till LC-MS confirms a complete reaction. Add an appropriate amount of water, and extract 3 times with ethyl acetate. Combine the organic phase, wash sequentially with water and saturated salt water, dry with anhydrous $Na_2SO_4$. Filter, the filtrate is evaporated under vacuum. The crude product is purified through silica gel column to give 470 mg of product 41-3, with the two-step yield of 55%.

Step 3: Preparation of 5-bromo-1-methyl-4-phenyl-1*H*-pyrrole-2-carbonitrile **(41-4)**

Add compound 41-3 (440 mg, 2.4 mmol) and DMF (5 mL) to a reaction flask, cool to 0 °C with an ice bath, drop in a solution of NBS (429 mg, 2.4 mmol) and DMF (5 mL). Let reaction solution resumes to room temperature and stir overnight till LC-MS confirms a complete reaction. Add an appropriate amount of water, and extract three times with ethyl acetate. Combine the organic phase, wash sequentially with water and saturated salt water, dry with anhydrous $Na_2SO_4$. Filter, the filtrate is evaporated under vacuum. The crude product obtained is purified through silica gel column to give 570 mg of product 41-4, with the yield of 90%.

Step 4: Preparation of methyl 2-(5-cyano-1-methyl-3-phenyl-1*H*-pyrrol-2-yl)-2-oxoacetate **(41-5)**

Add compound 41-4 (500 mg, 1.9 mmol) and anhydrous THF (25 mL) to a three-necked flask, processed with nitrogen replacement, Cool the reaction solution to -78 °C, drop in n-BuLi solution (2.5 M in hexane, 0.8 mL, 2.0 mmol), then stir at -78 °C for 1 h, add a solution of methyl valeryl chloride (350 mg, 2.86 mmol) and anhydrous THF (5 mL) slowly, and then stir at -78 °C for 4 h. Let the temperature of reaction solution slowly recover to room temperature and stir overnight till TLC confirms that the reaction is complete. Add saturated $NH_4Cl$ aqueous solution (5 mL) to quench the reaction, and extract three times with ethyl acetate. Combine the organic phase, wash with saturated saline, and

dry with anhydrous $Na_2SO_4$. Filter, the filtrate is evaporated under vacuum. The crude product is purified through silica gel column to give 250 mg of the product 41-5, with the yield of 49%.

Step 5: Preparation of 2-(5-cyano-1-methyl-3-phenyl-1*H*-pyrrol-2-yl)-2-oxoacetic acid **(41-6)**

Add compound 41-5 (230 mg, 0.86 mmol) and methanol (3 mL) to a reaction flask, and add a solution of lithium hydroxide (103 mg, 4.3 mmol) and water (3 mL). The reaction solution is slowly heated to 40 °C and stir overnight till LC-MS confirms a complete reaction. Drop in 2 M dilute hydrochloric acid, adjusting the pH to 4. Concentrate the reaction solution to dryness under reduced pressure and the resulting crude product is purified through silica gel column to give 120 mg of product 41-6, with the yield of 55%.

Step 6: Preparation of (*R*)-2-(5-cyano-1-methyl-3-phenyl-1*H*-pyrrol-2-yl)-*N*-(3-(5-fluoropyrimidin-2-yl)-1,2,3,4,4a,5-hexahydrobenzo[*b*]pyrazino[1,2-*d*] [1,4] oxazin-8-yl)-2-oxoacetamide **(T-41)**

Add compound 41-6 (70 mg, 0.28 mmol), DCM (5 mL) and DMF (4 mg, 0.055 mmol) to a reaction flask and the reaction solution is cooled to 0 °C. Drop in a solution of oxalyl chloride (39 mg, 0.31 mmol) in DCM (1 mL), stir at 0 °C for 1 hour. Drop the above prepared solution to a reaction flask containing compound 5-6 (75 mg, 0.25 mmol) and DIEA (97 mg, 0.75 mmol) in DCM (2 mL) solution in ice bath, stir overnight after its temperature slowly recovers to room temperature, using TLC to confirm a complete reaction. Add an appropriate amount of water to stop the reaction, and extract with dichloromethane. Wash the organic phase with saturated salt water, and dry with anhydrous $Na_2SO_4$, then evaporate under vacuum. The crude product is purified through silica gel column to give 70 mg of product T-41, with the yield of 52%.

LC-MS (ESI) m/z $(M+H)^+$: 538.2
$^1H$ NMR (400 MHz, DMSO-$d_6$) δ 10.50 (s, 1H), 8.51 (s, 2H), 7.30-7.20 (m, 6H), 6.82-6.78 (m, 1H), 6.76-6.72 (m, 1H), 670- 6.68 (m, 1H), 4.65-4.53 (m, 2H), 4.34 (dd, *J*= 10.8, 2.8 Hz, 1H), 3.97 (s, 3H), 3.95-3.90 (m, 1H), 3.86-3.79 (m, 1H), 3.11-2.97 (m, 2H), 2.72-2.55 (m, 2H).

**Test case 1**: **Measurement of minimum inhibitory concentration (MIC)**

1.1 Preparation of spore

New streaks are made with glycerol stock solution of the strains of Aspergillus fumigatus (ATCC MYA-4609/AF 293/CBS 101355) on Sabouraud's dextrose agar (SDA), incubate at 35°C and a humidity of 40-70% for 72 hours. Other strains are processed as the same as AF293.

1.2 Culture medium preparation

RPMI1640: one packet of RPMI1640 powder is dissolved in 1L purified water and mixed well, add 0.165M MOPS, adjust the pH to 7.0, sterilize by filtration with 0.22um membrane, and store at 4°C for no more than 3 months.
Physiological saline: weigh 9 g of sodium chloride powder and dissolve in 1 L of purified water, and mixed well. Sterilize at 121°C for 30 min, store at room temperature (RT) for no more than 1 week.

1.3 Preparation of test plates

1.3.1 Preparation of stock solution

Dissolve the test compound and stock solution of one control compound with DMSO and obtain solutions at a concentration of 12.8 mg/mL

1.3.2 Preparation of 100× working solution

The maximum concentration of each compound is 0.8 mg/mL (e.g., 150 μL of dimethyl sulfoxide + 10 uL of 12.8 mg/mL compound/control). Column 1 is filled with 40 μL of compound and columns 2 to 11 with 20 μL of dimethyl sulfoxide. Transfer 20 μL of compound from column 1 to column 2 to dilute the concentration. Repeat the above steps up to column 11 to give 2× serial dilutions.

1.3.3 Test plate preparation

2 μL of the above 2× serial dilutions are injected into each hole of the corresponding test plate using a multichannel pipette.

1.4 Preparation of inoculum

Drop 5 ml of saline on the Aspergillus fumigatus SDA plate. The spores were carefully wiped off from the surface of the plate with an L-shaped coating rod. Then the suspension is transferred into a sterile tube. After shaking, let the tube stand for 5 to 10 minutes. Count the number of spores in the upper suspension with a hemocytometer and check the purity. Adjust the inoculum concentration to 0.2-2.5 × $10^4$ spores/mL.

1.5 Addition of fungi

Inject 198 μL of diluted inoculum into each holes of the corresponding test plate using a multichannel pipette.

1.6 Incubation

Incubate at 35°C for 48 hours.

1.7 Measurement of MIC

The lowest concentration of each sample where they completely/significantly (e.g., 80% inhibition) inhibite the visible growth of the fungus after incubation is recorded as the corresponding MIC. To facilitate scoring of the presence or absence of growth on the wall, a magnifying glass is used. The tested 96-well microplates were photographed and the optical density at 530 nm is read with an enzyme marker (Tecan Spark).

The structure of positive control compound F901318 is:

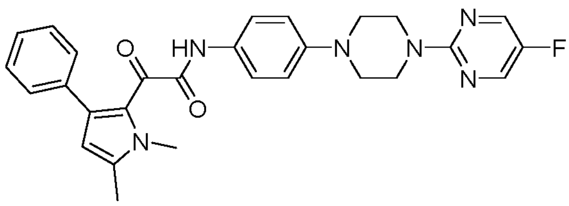

.

The MICs of the compounds of the present invention are shown in Table 4.

**Table 4: MICs of the compounds**

| Examples | AF293: MIC (μg/mL) | ATCC 204305: MIC (μg/mL) | ATCC 204304: MIC (μg/mL) | ATCC MYA-1004: MIC (μg/mL) |
|---|---|---|---|---|
| 1 | 0.03 | | | |
| 2 (T-2-a) | 0.06 | | | |
| 2 (T-2-b) | 0.06 | | | |
| 3 | 0.125 | 0.016 | 0.008 | 0.008 |
| 4 | 0.03 | 0.016 | 0.016 | 0.008 |
| 5 | 0.016 | 0.016 | 0.004 | 0.004 |
| 6 | 0.03 | | | |
| 7 | 0.06 | | | |
| 8 | 0.03 | | | |
| 9 | 0.06 | | | |
| 10 | 0.06 | | | |

(continued)

| Examples | AF293: MIC (μg/mL) | ATCC 204305: MIC (μg/mL) | ATCC 204304: MIC (μg/mL) | ATCC MYA-1004: MIC (μg/mL) |
|---|---|---|---|---|
| 11 | 0.5 | | | |
| 12 | 0.13 | | | |
| 13 | 0.25 | | | |
| 14 | 0.06 | | | |
| 17 | 1 | | | |
| 18 | 0.03 | | | |
| 22 | 0.13 | | | |
| 23 | 0.13 | | | |
| 24 | 0.13 | | | |
| 25 | 0.06 | | | |
| 26 | 0.03 | | | |
| 27 | 0.03 | | | |
| 28 | 0.25 | | | |
| 31 | 1 | | | |
| 32 | 0.008 | 0.03 | 0.016 | 0.016 |
| 33 | 0.004 | 0.016 | 0.008 | 0.008 |
| 34 | 0.016 | 0.03 | 0.016 | 0.008 |
| 35 | 0.008 | 0.016 | | |
| 36 | 0.016 | | | |
| 37 | 0.016 | | | |
| 38 | | 0.008 | | |
| 39 | | 0.03 | | |
| 40 | | 0.06 | | |
| F901318 | 0.03 | 0.016 | 0.004 | 0.004 |

The compounds of the present invention shows strong inhibitory activity against Aspergillus fumigatus (ATCC MYA-4609 / AF293 / CBS 101355, ATCC-204305) and Aspergillus flavus (ATCC 204304, ATCC MYA-1004) in the measurement test of minimum inhibitory concentration (MIC).

**Test case 2: pharmacokinetic test**

Pharmacokinetic study is performed on SD rats receiving single oral administration of each test compound mixed with F901318 (10 mg/kg, 3 rats per group). Test compounds and F901318 are dissolved with 5% DMSO+10% solutol+85% saline, vortexed for 1-2 mins and ultra-sonicated for 5-10 mins to form a colorless clear drug solution. The animals are fasted overnight before oral administration, and resumed feeding 4 hours after administration. After oral administration, pharmacokinetic samples are collected from orbital blood of SD rats at 0.083 h, 0.25 h, 0.5 h, 1 h, 2 h, 4 h, 6 h, 8 h, and 24 h, and three whole blood samples are collected at each time point in a volume of 0.2-0.3 mL. Blood samples are placed on ice immediately after collected, and the plasma is separated by centrifugation within 15 min (centrifugation conditions: 8000 rpm, 1 min, and at room temperature).

The collected plasma is stored at -20°C prior to analysis. Transfer 20 μL of plasma sample into an 0.5mL EP tube, add 200 μL of working internal standard solution (blank group is without internal standard, using the same volume of solvent instead), vortex and mix for 3 min, centrifuge at 13500 rpm for 10 min, take 100 μL of supernatant and analyze with LC-MS/MS.

The results of pharmacokinetic tests of some compounds of the present invention are shown in Table 5 below:

**Table 5: Pharmacokinetic test results of some compounds of the present invention**

| Experiment number | Compounds | Cmax (ng/ml) | AUC (ng/ml*h) | T 1/2, (h) | Tmax, (h) |
|---|---|---|---|---|---|
| 1 | T-1 | 3862.514 | 48343.076 | 6.477 | 2.333 |
| | F901318 | 1499.015 | 9641.325 | 2.848 | 1.333 |
| 2 | T-3 | 1421.250 | 18720.938 | 5.352 | 0.917 |
| | F901318 | 561.393 | 7541.724 | 9.523 | 0.500 |
| 3 | T-5 | 1932.917 | 15986.428 | 4.291 | 1.000 |
| | F901318 | 1380.419 | 9170.038 | 5.409 | 1.000 |
| 4 | T-33 | 3494.113 | 36077.441 | 7.522 | 1.000 |
| | F901318 | 1281.168 | 7652.703 | 5.617 | 1.000 |
| 5 | T-35 | 1524.750 | 12849.761 | 4.956 | 0.833 |
| | F901318 | 1380.419 | 9170.038 | 5.409 | 1.000 |
| 6 | T-38 | 3053.085 | 41566.714 | 11.202 | 1.000 |
| | F901318 | 1281.168 | 7652.703 | 5.617 | 1.000 |
| Note: Cmax: maximum concentration of compound; AUC: exposure; T1/2: half-life; Tmax: time to reach Cmax. | | | | | |

The compounds of the present invention have better exposure and maximum concentration of compound in the pharmacokinetic tests compared to F901318.

**Test case 3: Liver microsomal stability test**

1: Add 10 μL of working solutions of test product or reference product and 80 μL of microsomal working solution (liver microsomal protein concentration of 0.5 mg/mL) to wells of T0, T5, T10, T20, T30, T60, and NCF60, and only microsomal working solution is added to the Blank60 wells. Then the samples of Blank60, T10, T20, T30 and T60, except for T0 and NCF60, are placed in a water bath set at 37°C and incubate for approximately 10 mins;
2: Add 300 μL of termination solution (acetonitrile solution containing 200 ng/mL tolbutamide and 200 ng/mL labetalol) to Sample T0, followed by 10 ul of NADPH regeneration system working solution;
3: After incubation of Blank60, T5, T10, T20, T30 and T60 on incubation plates, add 10 μL of NADPH regeneration system working solution to each sample well to start the reaction, and 10 uL of 100 mM potassium phosphate buffer is add to the NCF60 sample wells;
4: After incubation for appropriate times (e.g., 5, 10, 20, 30 and 60 mins), 300 μL of termination solution is add to each sample well of test product and reference product on Blank60, T5, T10, T20, T30, T60 and NCF60 plates, respectively, to terminate the reaction.
5: All sample plates are shaken well and centrifuged at 4000 rpm for 20 min, take 100 μL of the supernatant of the test product or reference product, dilute in 300 μL of pure water for LC-MS/MS analysis, respectively.
6: Data analysis, T1/2 and $CL_{int(mic)}$ (μL/min/mg) values are calculated based on the first-order elimination kinetics, and the equations of first-order elimination kinetics are:

$$C_t = C_0 \bullet e^{-k_e \bullet t}$$

*when*

$$C_t = \frac{1}{2}C_0 ,$$

$$T_{1/2} = \frac{Ln2}{k_e} = \frac{0.693}{k_e}$$

$$CL_{int(mic)} = \frac{0.693}{\text{(in vitro)}\ T_{1/2}} \cdot \frac{1}{mg/mL\ \text{(the concentration of liver microsomal protein in reaction system)}}$$

$$CL_{int\ (liver)} = CL_{int(mic)} \cdot \frac{mg\ \text{(the weight of liver microsome)}}{g\ \text{(the weight of liver)}} \cdot \frac{g\ \text{(the weight of liver)}}{kg\ \text{(body weight)}}$$

The results of liver microsomal stability test for some compounds of the present invention are shown in Table 6 below:

**Table 6. Results of liver microsomal stability testing for some compounds of the present invention**

| Species / Compounds | Human | | | Mouse | | |
|---|---|---|---|---|---|---|
| | $T_{1/2}$ (min) | $CL_{int}$ (mL/min/kg) | Remaining, (T=60min) | $T_{1/2}$ (min) | $CL_{int}$ (mL/min/kg) | Remaining, (T=60min) |
| F901318 | 33.0 | 37.8 | 27.7% | 25.2 | 98.9 | 18.7% |
| T-1 | 38.3 | 32.6 | 35.7% | 38.3 | 65.2 | 34.4% |
| T-4 | 18.8 | 66.4 | 10.5% | 21.8 | 114.3 | 14.3% |
| T-5 | 25.6 | 48.8 | 20.4% | 21.8 | 114.6 | 15.7% |
| T-9 | 23.6 | 52.9 | 16.5% | 30.7 | 81.1 | 24.0% |
| T-32 | 22.1 | 56.4 | 26.6% | 27.8 | 89.8 | 26.2% |
| T-33 | 48.2 | 25.9 | 43.4% | 49.6 | 50.2 | 44.8% |
| T-34 | 39.9 | 31.3 | 34.5% | 54.4 | 45.9 | 48.5% |
| T-38 | 30.7 | 40.7 | 25.3% | 29.3 | 85.1 | 24.5% |

Compared to F901318, some of the compounds of the present invention have better hepatic microsomal stability for species of human or/and rat.

**Test case 5: Survival rate experiment I of mouse bloodstream infection model with Aspergillus fumigatus**

Experimental animals: male CD-1 mouse, 6-8 weeks old, 10 animals per group.

Induction of immunosuppression: the day of infection is defined as day 0, and mouses are injected intraperitoneally with corresponding doses of cyclophosphamide on day -4 (150 mg/kg), day -1 (100 mg/kg) and day 2 (100 mg/kg).

Microbial pathogen: Aspergillus fumigatus ATCCMYA-4690 (AF293).

Collection conditions of inoculum: culture on SDA culture plates, incubate at 25 °C for at least 7 days, collect with PBS + 0.1% Tween 20, and count microscopically;

Inoculation level, route and volume: 3.60E+05 CFU/mouse, tail vein injection, injection volume of 100 μL/mouse;

Treatment: Treatment is started 24 hours after infection, and test compounds are dissolved in PEG400 (0.3 mg/mL), and used for administration orally to mouses at 3 mg/kg, twice daily for 6 days. The administrated mouses are observed for 8 hours.

The experimental results of survival rate of mouse bloodstream infection model with Aspergillus fumigatus are shown in Figure 1.

According to the experimental results, it can be seen that T-5, T-33 and F901318 all prolong the survival time of mouses. At the same dose, the day 7 survival rate with T-33 is 40%, while the survival rates of both the reference compound F901318 and T-5 are 0%. The *in vivo* efficacy of T-33 is significantly better than that of F901318 and T-5 in this model.

### Test case 6: Survival rate experiment II of mouse bloodstream infection model with Aspergillus fumigatus

Animal for experiment: male CD-1 mouse, 6-8 weeks old, 10 animals per group. Induction of immunosuppression: the day of infection is defined as day 0, and mouses are injected intraperitoneally with corresponding doses of cyclophosphamide on day -4 (150 mg/kg), day -1 (100 mg/kg), and day 2 (100 mg/kg), respectively.

Microbial pathogen: Aspergillus fumigatus ATCCMYA-4690 (AF293).

Collection conditions of inoculum: culture on SDA culture plates, incubate at 25 °C for at least 7 days, collect with PBS + 0.1% Tween 20, and count microscopically;

Inoculation level, route and volume: 3.90E+05 CFU/mouse, tail vein injection, injection volume of 100 μL/mouse;

Treatment: Treatment is started 24 hours after infection, and test compounds are dissolved in PEG400 (0.3 mg/mL), and used for administration orally to mouses at 3 mg/kg twice daily for 6 days. The administrated mouses are observed for 8 hours.

The experimental results of survival rate of the mouse bloodstream infection model with Aspergillus fumigatus are shown in Figure 2.

It can be seen from the experimental results that T-33, T-38 and F901318 all prolong the survival time of mouses. At a dose of 3 mg/kg (bid), the day 7 survival rate with T-33 is 30%, with T-38 50%, and with the reference compound F901318 0%. A dose-dependent relationship is observed in T-33 at three doses of 1 mg/kg, 3 mg/kg and 10 mg/kg, and the survival rate of mouses with T-33 reached 80% at a dose of 10 mg/kg (bid). The *in vivo* efficacy of T-33 and T-38 is significantly better than that of F901318 in this model.

Although embodiments of the present invention have been shown and described, it should be understood to those of ordinary skill in the field that a variety of changes, modifications, substitutions and variants of these embodiments can be made without departing from the principles, spirit and scope of the present invention as limited by the appended claims and their equivalents.

## Claims

1. A compound **characterized by** having the structure shown in formula I or its tautomer, mesomer, racemate, enantiomer, diastereoisomer or its mixture thereof, and pharmaceutically acceptable hydrate, solvate or salt:

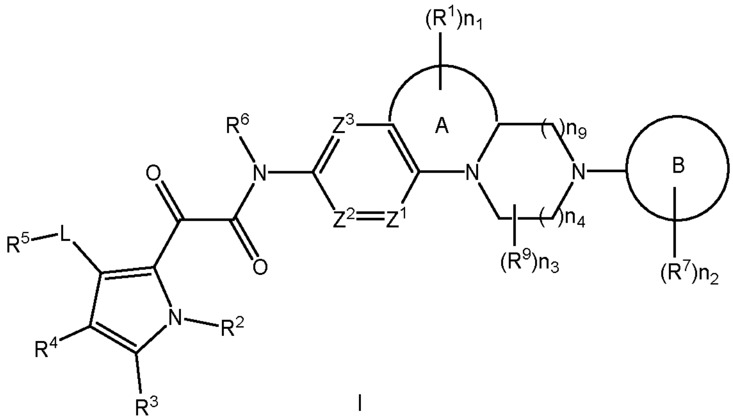

I

Ring A is a 5- to 10-membered rings, and

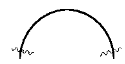

in ring A is selected from alkyl, heteroalkyl, acyl, ester, acylamino, sulfonyl, sulfonamido, sulfinyl, and sulfinamido group;

Ring B is selected from substituted or non-substituted aryl, substituted or non-substituted heteroaryl, substituted or non-substituted cycloalkyl, substituted or non-substituted heterocycloalkyl;

Each occurrence of $R^1$ is independently selected from hydrogen, halogen, cyano group, =O, hydroxyl, $-NH_2$, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted aryl, substituted or non-substituted heteroaryl, ester, acyl, acylamino, sulfinyl, sulfonyl, sulfonamido, and sulfinamido group, or any two $R^1$s and their connected atoms form substituted or non-substituted 3- to 12-membered hydrocarbon rings, substituted or non-substituted 3- to 12-membered heterohydrocarbon rings;

$R^2$, $R^3$, and $R^4$ are independently selected from hydrogen, halogen, nitro, cyano group, hydroxyl, $-NH_2$, substituted or non-substituted C1 to C10 alkyl, substituted or non-substituted 2- to 10-membered heteroalkyl, substituted or non-substituted 3- to 12-membered cycloalkyl, substituted or non-substituted 3- to 12- membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester, acylamino, sulfonyl, sulfonamido, boronic acid group, boronic acid ester group, phosphoryl, or $R^2$ and $R^3$ together form substituted or non-substituted substituted C1 to C6 alkyl, substituted or non-substituted 3- to 10-membered heteroalkyl, or $R^2$, $R^3$ and their connected atoms form substituted or non-substituted heteroaryl;

$R^5$ is selected from substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted aryl, substituted or non-substituted heteroaryl;

$R^6$ is selected from hydrogen, substituted or non-substituted C1 to C10 alkyl, substituted or non-substituted 2- to 10-membered heteroalkyl, substituted or non-substituted 3- to 12-membered cycloalkyl, substituted or non-substituted 3- to 12- membered heterocycloalkyl, substituted or non-substituted alkenyl;

Each occurrence of $R^7$ is independently selected from hydrogen, halogen, cyano group, =O, hydroxyl, $-NH_2$, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted aryl, substituted or non-substituted heteroaryl, acyl, ester, acylamino, sulfonyl, sulfonamido, boronic acid group, boronic acid ester group, or any two $R^7$s and their connected atoms form substituted or non-substituted 3- to 12-membered hydrocarbon rings, substituted or non-substituted 3- to 12-membered heterohydrocarbon rings;

Each occurrence of $R^9$ is independently selected from hydrogen, halogen, nitro, cyano group, hydroxyl, $-NH_2$, =O, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester,

acylamino, sulfonyl, sulfonamido, boronic acid group, boronic acid ester group, phosphoryl, or any two $R^9$s and their connected atoms form substituted or non-substituted hydrocarbon ring and substituted or non-substituted heterohydrocarbon ring;

$Z^1$, $Z^2$ and $Z^3$ are independently selected from N or $CR^8$;

Each occurrence of $R^8$ is independently selected from hydrogen, halogen, nitro, cyano group, hydroxyl, $-NH_2$, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted aryl, substituted or non-substituted heteroaryl, acyl, ester, acylamino, sulfonyl, sulfonamido, boronic acid group, boronic acid ester group, phosphoryl, or any two $R^8$s and their connected atoms form substituted or non-substituted 5- to 9-membered alkenyl rings, substituted or non-substituted 5- to 9-membered heteroalkenyl rings, substituted or non-substituted benzene ring, substituted or non-substituted 5- to 9-membered heteroaromatic rings;

Or $R^8$ and the adjacent $R^9$ together form substituted or non-substituted C1 to C6 alkyl group, substituted or non-substituted 2- to 6-membered heteroalkyl, substituted or non-substituted acyl group, substituted or non-substituted ester, substituted or non-substituted acylamino, substituted or non-substituted sulfonyl, substituted or non-substituted sulfonamido, substituted or non-substituted sulfinyl, substituted or non-substituted sulfinamido group;

$n_1$, $n_2$ and $n_3$ are independently selected from 0, 1, 2, 3, 4, 5 and 6; $n_4$ and $n_9$ are independently selected from 1, 2 and 3;

L is selected from one bond, O, S, S(=O), S(=O)2, C(=O), $NR^{10}$, $-(CR^{11}R^{12})n_5-$, - $S(=O)n_6NR^{13}-$, $-NR^{14}S(=O)n_7NR^{15}-$, $-C(=O)NR^{16}-$, $-NR^{17}C(=O)NR^{18}-$, C2 to C4 alkenylidene, C2 to C4 alkynylidene;

$n_5$ is selected from 1, 2, 3 and 4; $n_6$ and $n_7$ are independently selected from 0, 1 and 2;

$R^{10}$, $R^{13}$, $R^{14}$, $R^{15}$, $R^{16}$, $R^{17}$ and $R^{18}$ are independently selected from hydrogen, acyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted alkenyl;

Or each occurrence of $R^{11}$ and $R^{12}$, are independently selected from hydrogen, halogen, hydroxyl, and C1 to C10 alkyl;

Wherein, the substituents in $R^1$, $R^2$, $R^3$, $R^4$, $R^5$, $R^6$, $R^7$, $R^8$, $R^9$, $R^{10}$, $R^{13}$, $R^{14}$, $R^{15}$, $R^{16}$, $R^{17}$ and $R^{18}$ are independently selected from halogen, alkyl, cycloalkyl, heteroalkyl, heterocycloalkyl, alkenyl, alkynyl, hydroxyl, cyano group, $-NH_2$, nitro, carboxyl, acyl, ester, acylamino, aryl, heteroaryl, sulfinyl, sulfonyl, sulfonamido and sulfinamido group.

2. The compound stated in claim 1 is **characterized by** that $Z^2$ is $CR^8$, $Z^1$ and $Z^3$ are independently selected from N or $CR^8$; further, $Z^2$ and $Z^3$ are both $CR^8$, $Z^1$ is selected from N or $CR^8$.

3. The compound stated in claim 1 or 2 are **characterized by** that ring A is substituted or non-substituted 5- to 7-membered rings, and the heteroatoms in ring A are selected from O, N, S.

4. The compound stated in any of claims 1 to 3 are **characterized by** that, each occurrence of $R^1$ is independently selected from hydrogen, halogen, cyano group, =O, hydroxyl, $-NH_2$, carboxyl, substituted or non-substituted C1 to C6 alkyl, substituted or non-substituted 2- to 7-membered heteroalkyl, substituted or non-substituted 3- to 9-membered cycloalkyl, substituted or non-substituted substituted or non-substituted 3- to 9-membered heterocycloalkyl, substituted or non-substituted C2 to C6 alkenyl, substituted or non-substituted phenyl, substituted or non-substituted 5- to 10-membered heteroaryl, ester, acyl, acylamino, sulfinyl, sulfonyl, sulfonamido and sulfinamido group, or any two $R^1$s and their connected atoms form substituted or non-substituted 3- to 6-membered hydrocarbon rings, wherein, the substituent of $R^1$ is selected from halo, alkyl, cycloalkyl, heteroalkyl, heterocycloalkyl, alkenyl, alkynyl, hydroxy, cyano group, amino, carboxyl, acyl, ester, acylamino, aryl and heteroaryl;

Further, each occurrence of $R^1$ is independently selected from hydrogen, halogen, cyano group, =O, substituted or non-substituted C1 to C6 alkyl, substituted or non-substituted 2- to 7-membered heteroalkyl, substituted or non-substituted 3- to 9-membered cycloalkyl, substituted or non-substituted 3- to 9-membered heterocycloalkyl, or two $R^1$s in the same substitution site and their connected atoms form substituted or non-substituted 3- to 6-membered hydrocarbon rings, wherein the substituent of $R^1$ is selected from halogen, alkyl, cycloalkyl, heteroalkyl, heterocycloalkyl, hydroxyl, cyano and amino group.

5. The compound stated in claim 4 is **characterized by** that, each occurrence of $R^1$ is independently selected from hydrogen, halogen, =O, substituted or non-substituted C1 to C4 alkyl, substituted or non-substituted 2- to 5-membered alkoxies, substituted or non-substituted 3- to 6-membered cycloalkyl, substituted or non-substituted 3- to 6-membered heterocycloalkyl, or two $R^1$s at the same substitution site and their connected atoms form substituted or non-substituted 3- to 6-membered cycloalkyl, wherein, the substituent of $R^1$ is selected from halogen, alkyl, cycloalkyl, heteroalkyl, heterocycloalkyl, hydroxyl, cyano and amino group;

Further, each occurrence of $R^1$ is independently selected from hydrogen, halogen, =O, C1 to C4 alkyl substituted or non-substituted by halogen, 3- to 6-membered cycloalkyl substituted or non-substituted by halogen, or two $R^1$s at the same substitution site and their connected atoms form substituted or non-substituted 3- to 6-membered cycloalkyl;
Further, each occurrence of $R^1$ is independently selected from hydrogen, F, Cl, =O, C1 to C4 alkyl substituted or non-substituted by F, cyclopropyl substituted or non-substituted by halogen, or two $R^1$s at the same substitution site with atoms connected thereto form substituted or non-substituted cyclopropyl.

6. The compound according to any of claims 1 to 5 are **characterized by** that $n_1$ is selected from 0, 1, 2, 3 and 4; further selected from 0, 1 and 2.

7. The compound stated in any of claims 1 to 6 are **characterized by** having the structure shown in formula II or its tautomer, endo-, racemate, enantiomer, diastereoisomer or its mixture, pharmaceutically acceptable hydrate, solvate or salt;

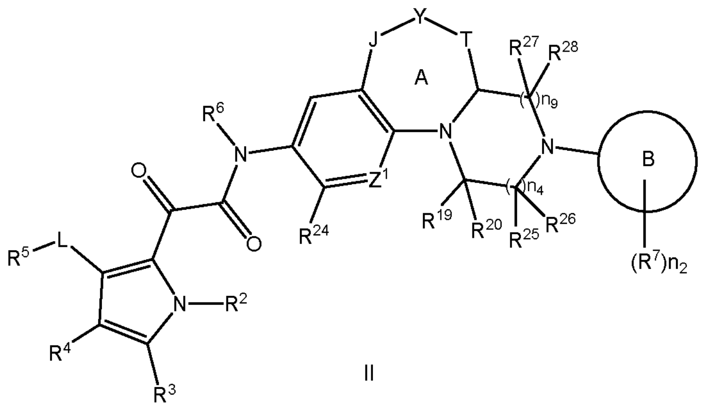

II

J, Y and T are independently selected from one bond, O, S, S(=O), $S(=O)_2$, $CR^{21}R^{22}$, C=O and $NR^{23}$;
$R^{21}$, $R^{22}$, $R^{23}$ and $R^{24}$ are independently selected from hydrogen, halogen, hydroxyl, substituted or non-substituted C1 to C4 alkyl, substituted or non-substituted C1 to C4 alkenyl, or $R^{21}$ and $R^{22}$ and their connected atoms form substituted or non-substituted 3- to 6-membered cycloalkyl; the substituents in $R^{21}$, $R^{22}$, $R^{23}$, $R^{24}$ are independently selected from halogen, hydroxyl, $-NH_2$, ester, acyl, C1- to C4 alkyl, and 3- to 6-membered cycloalkyl;
$Z^1$ is selected from N or $CR^8$;
$R^8$ is selected from hydrogen, halogen, nitro, cyano group, hydroxyl, $-NH_2$, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, substituted or non-substituted C2 to C10 alkynyl, substituted or non-substituted phenyl, substituted or non-substituted 5- to 9-membered heteroaryl, acyl, ester, acylamino, sulfonyl, sulfonamido, boronic acid group, boronic acid ester group, phosphoryl, or $R^8$, $R^{24}$ and their connected atoms form substituted or non-substituted 5- to 7-membered alkenyl rings, substituted or non-substituted 5- to 7-membered heteroalkene rings, substituted or non-substituted benzene ring, substituted or non-substituted 5- to 7-membered heteroaromatic rings;
$R^{19}$ and $R^{20}$ are independently selected from hydrogen, halogen, cyano group, carboxyl, substituted or non-substituted C1 to C10 alkyl, substituted or non-substituted 2- to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, acyl, ester, acylamino, sulfonyl, sulfonamido, or $R^{19}$ and $R^{20}$ together form = O, or $R^{19}$, $R^{20}$ and the atoms connected thereto form substituted or non-substituted 3- to 6-membered cycloalkyl;
Or $R^8$ and $R^{19}$ together form substituted or non-substituted C1 to C6 alkyl group, substituted or non-substituted 2- to 6-membered heteroalkyl, substituted or non-substituted acyl, substituted or non-substituted ester, substituted or non-substituted acylamino, substituted or non-substituted sulfonyl, substituted or non-substituted sulfonamido, substituted or non-substituted sulfinyl group, substituted or non-substituted sulfinamido;

Each occurrence of $R^{25}$, $R^{26}$, $R^{27}$ and $R^{28}$ are independently selected from hydrogen, halogen, cyano group, hydroxyl, $-NH_2$, carboxyl, substituted or non-substituted C1 to C10 alkyl, substituted or non-substituted 2 to 10-membered heteroalkyl, substituted or non-substituted 3- to 12-membered cycloalkyl, substituted or non-substituted 3- to 12-membered heterocycloalkyl, substituted or non-substituted C2 to C10 alkenyl, acyl, ester, acylamino, sulfonyl, and sulfonamido group, or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;

The substituents in $R^8$, $R^{19}$, $R^{20}$, $R^{25}$, $R^{26}$, $R^{27}$ and $R^{28}$ are independently selected from halogen, C1 to C10 alkyl, 3- to 12-membered cycloalkyl, 2- to 10-membered heteroalkyl, 3-to 9-membered heterocycloalkyl, C2- to C10 alkenyl, C2- to C10 alkynyl, hydroxyl, cyano group, $-NH_2$, carboxyl, acyl, ester, acylamino, phenyl, 5- to 9-membered heteroaryl, sulfinyl, sulfonyl, sulfonamido, and sulfinamido group;

Further, $R^{21}$ and $R^{22}$ are independently selected from hydrogen, halogen, hydroxyl, C1 to C4 alkyl substituted or non-substituted by halogen, or $R^{21}$, $R^{22}$ and their connected atoms form substituted or non-substituted cyclopropyl; $R^{23}$ is selected from hydrogen, and C1 to C4 alkyl.

8. The compound stated in claim 7 is **characterized by** that J is selected from O, S, S(=O), $S(=O)_2$, $CH_2$, CHF, $CF_2$, CHOH, C=O, $NR^{23}$, and

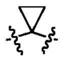;

Y is selected from one bond, O, S, S(=O), $S(=O)_2$, $CH_2$, CHF, $CF_2$, C=O, and $NR^{23}$; T is selected from one-bond, S(=O), $S(=O)_2$, $CH_2$, CHF, $CF_2$, C=O, and

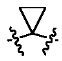;

Preferably, J is selected from O, $CH_2$, $CF_2$, CHF, CHOH, C=O, NH, and

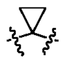;

Y is selected from one bond, O, $CH_2$, C=O, and NH; and T is selected from one bond, $CH_2$, C=O, and

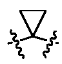.

9. The compound stated in claim 7 is **characterized by** that the ring A is selected from substituted or non-substituted groups as follows:

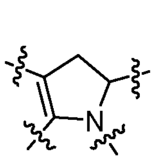、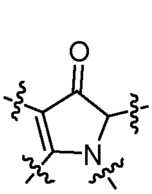、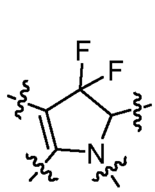、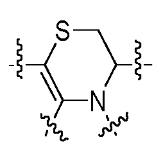、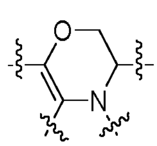、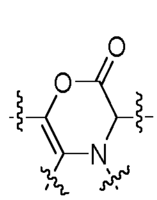、

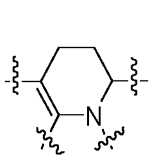、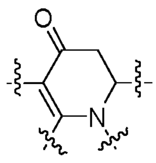、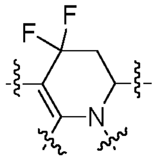、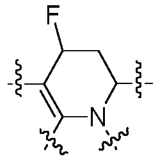、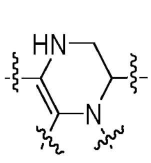、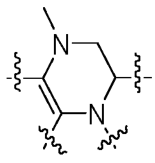、

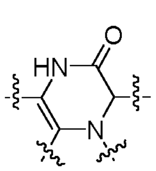、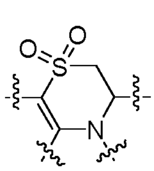、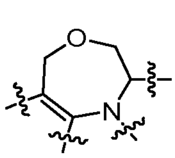、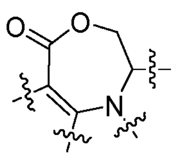、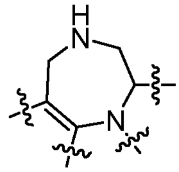、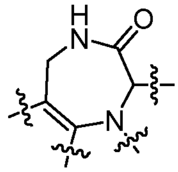、

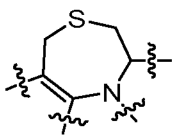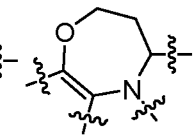、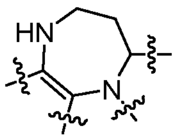、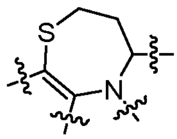、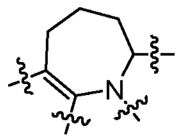;

Further, ring A is selected from substituted or non-substituted groups as follows:

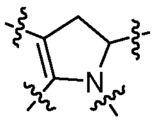、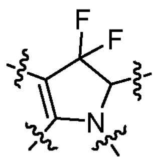、、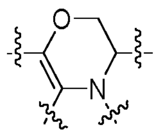、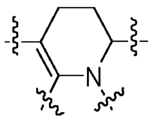、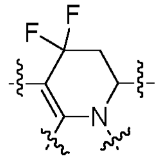、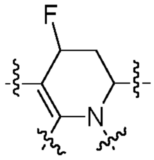、

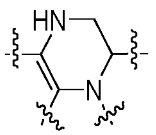、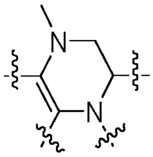、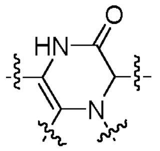、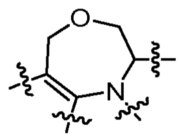、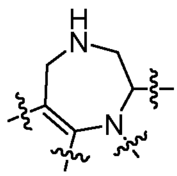、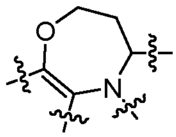、

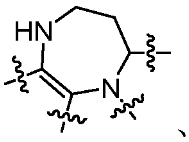、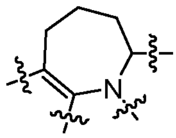;

Further, the ring A is selected from the substituted or non-substituted groups as follows:

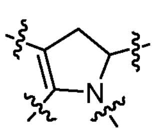、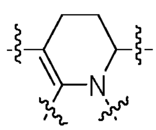、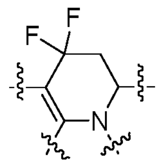、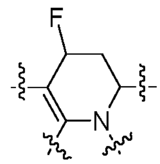、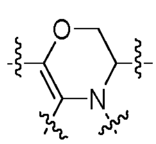、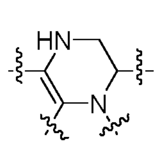、

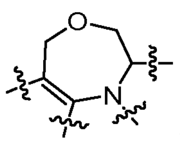、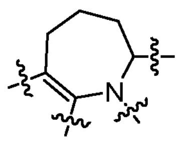.

**10.** The compound stated in claim 9 is **characterized by** that the ring A is selected from the following substituted or

non-substituted groups:

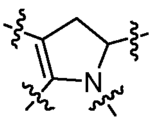 , 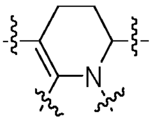 , 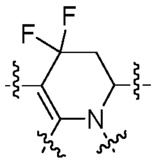 , 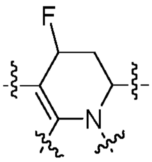 , 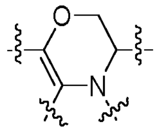 , 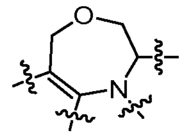 .

**11.** The compound stated in any of claims 1 to 10 are **characterized by** that L is selected from one bond, S, S(=O), $S(=O)_2$, C(=O), $-(CR^{11}R^{12})n_5-$, $-C(=O)NR^{16}-$, C2 to C4 alkynylene;
Further, L is selected from the one-bond, C2 to C4 alkynylenes; and even further, L is selected from the one-bond.

**12.** The compound stated in any of claims 1 to 11 are **characterized by** having the structures shown in formula III or formula IV or their tautomers, mesomers, racemates, enantiomers, diastereoisomers or their mixtures, pharmaceutically acceptable hydrates, solvates or salts:

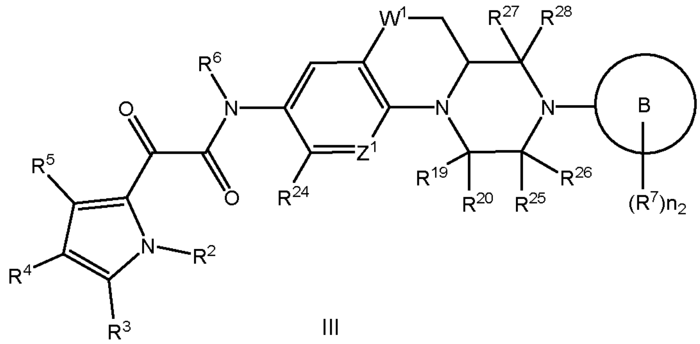

III

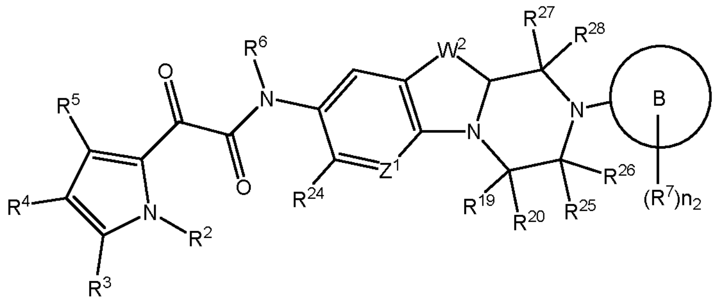

IV

$W^1$ and $W^2$ are independently selected from O, $CH_2$, CHF, $CF_2$, NH, and $NCH_3$, preferably O, $CF_2$ and $CH_2$;
$Z^1$ is selected from N or $CR^8$;
$R^8$ is selected from hydrogen, halogen, cyano group, hydroxyl, $-NH_2$, substituted or non-substituted C1 to C4 alkyl, substituted or non-substituted 2- to 5-membered alkoxies, substituted or non-substituted 2- to 5-membered N-heteroalkyl, substituted or non-substituted 3- to 6-membered cycloalkyl, substituted or non-substituted C2 to C4 alkenyl, acyl, ester, acylamino, boronic acid group, or boronic acid ester group, or $R^8$ and $R^{19}$ together form substituted or non-substituted C1 to C2 alkyl, substituted or non-substituted 2-membered heteroalkyl, and substituted or non-substituted ester group;
Further, $R^8$ is selected from H, F, Cl, cyano group, -OH, $-N(CH_3)_2$, $-N(CH_2CH_3)_2$, $-C(=O)CH_3$, $-C(=O)CH_2CH_3$, $-C(=O)OCH_3$, $-C(=O)OCH_2CH_3$, $-B(OH)_2$,

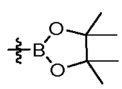 ,

$-CH_3$ , $-CH_2CH_3$, $-CH(CH_3)_2$,

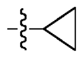,

$-CH_2OH$, $-OCH_3$, $-OCH_2CH_3$, $-CF_3$, $-CHF_2$, and $-CF_2CF_3$, or $R^8$ and $R^{19}$ together form substituted or non-substituted group as follows:

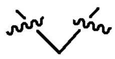, 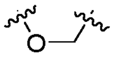,

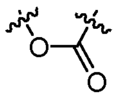.

**13.** The compound stated in claim 12 is **characterized by** that $R^8$ is selected from H, F, - OH, $-B(OH)_2$, or $R^8$ and $R^{19}$ and their connected atoms form the following groups:

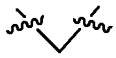,

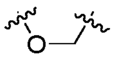, 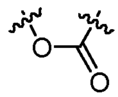;

Further, $R^8$ is selected from Hand F.

**14.** The compound stated in any of claims 7 to 13 are **characterized by** that $R^{24}$ is selected from hydrogen and halogen, preferably H, F, and Cl; more preferably H.

**15.** The compound stated in any of claims 7 to 14 are **characterized by** that, each occurrence of $R^{25}$, $R^{26}$, $R^{27}$ and $R^{28}$ are independently selected from hydrogen, halogen, cyano group, hydroxyl, $-NH_2$, carboxyl, substituted or non-substituted C1 to C6 alkyl, substituted or non-substituted 2- to 6-membered heteroalkyl, substituted or non-substituted 3- to 6-membetred cycloalkyl, substituted or non-substituted 3- to 6-membetred heterocycloalkyl, substituted or non-substituted C2 to C6 alkenyl, acyl, ester, acylamino, sulfonyl, sulfonamido, or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;

Further, each occurrence of $R^{25}$, $R^{26}$, $R^{27}$, and $R^{28}$, are independently selected from hydrogen, halogen, substituted or non-substituted C1 to C6 alkyl, substituted or non-substituted 2- to 6-membered heteroalkyl, substituted or non-substituted 3- to 6-membered cycloalkyl, 3- to 6-membered heterocycloalkyl, acyl, ester group, or $R^{25}$ and $R^{26}$ together form = O, or $R^{27}$ and $R^{28}$ together form = O, or $R^{25}$, $R^{26}$ and their connected atoms form substituted or non-substituted of 3- to 6-membered cycloalkyl, or $R^{27}$, $R^{28}$ and their connected atoms form substituted or non-substituted 3- to 6-membered cycloalkyl;
The substituents in $R^{25}$, $R^{26}$, $R^{27}$ and $R^{28}$ are independently selected from halogen, C1 to C6 alkyl, 3- to 6-membered cycloalkyl, 2- to 6-membered heteroalkyl, 3- to 6-membered heterocycloalkyl, hydroxyl, cyano group, $-NH_2$, carboxyl, acyl, ester group, acylamino, sulfinyl, sulfonyl, sulfonamido, and sulfinamido group;
Further, each occurrence of $R^{25}$ and $R^{27}$ are independently selected from hydrogen, halogen, substituted or non-substituted C1 to C6 alkyl, substituted or non-substituted 2- to 6-membered heteroalkyl, substituted or non-substituted 3- to 6-membered cycloalkyl, acyl, and ester, $R^{26}$ and $R^{28}$ are both H;
Or $R^{25}$ and $R^{26}$ together form = O, or $R^{27}$ and $R^{28}$ together form = O, or $R^{25}$, $R^{26}$ and their connected atoms form substituted or non-substituted 3- to 6-membered cycloalkyl, or $R^{27}$, $R^{28}$ and their connected atoms form substituted or non-substituted 3- to 6-membered cycloalkyl;
Further, $R^{25}$, $R^{26}$, $R^{27}$, and $R^{28}$ are all H.

**16.** The compounds stated in any of claims 7 to 15 are **characterized by** that $R^{19}$ is selected from hydrogen, halogen, cyano group, substituted or non-substituted C1 to C6 alkyl, substituted or non-substituted 2- to 7-membered heteroalkyl, acyl, and ester; $R^{20}$ is H, or $R^{19}$ and $R^{20}$ together form =O, or $R^{19}$, $R^{20}$ and their connected atoms form substituted or non-substituted 3- to 6-membered cycloalkyl;

Or $R^{19}$ and $R^8$ together form the groups defined in the claims cited therein;
Further, $R^{19}$, $R^{20}$ are both H, or $R^{19}$ and $R^{20}$ together form =O, or $R^{19}$, $R^{20}$ and their connected atoms form substituted or non-substituted cyclopropyl;
Or $R^{19}$and $R^8$ together form groups defined in the claims cited therein;
Further, $R^{19}$ and $R^{20}$ are both H.

**17.** The compounds stated in any of claims 1 to 16 are **characterized by** that ring B is selected from phenyl, naphthyl, substituted or non-substituted 5- to 10-membered heteroaryl, substituted or non-substituted 3- to 9-membered cycloalkyl, substituted or non-substituted 3-to 9-membered heterocycloalkyl;

Further, ring B is selected from substituted or non-substituted 5- to 6-membered heteroaryl;
Further, ring B is selected from substituted or non-substituted groups as follows: phenyl, pyrimidinyl, pyridyl, pyrazinyl, pyridazinyl, thienyl, pyrazolyl, imidazolyl, oxazolyl, thiazolyl, triazolyl, tetrazolyl, thiadiazolyl, and oxadiazolyl.

**18.** The compound stated in claim 17 is **characterized by** that ring B is selected from pyrimidinyl, pyridyl, pyridazinyl, thiazolyl, triazolyl, oxadiazolyl; preferably pyrimidinyl, pyridyl, pyridazinyl, thiazolyl; more preferably pyrimidinyl.

**19.** The compound stated in any of claims 1 to 18 are **characterized by** that,

Each occurrence of $R^7$ is selected from hydrogen, halogen, cyano group, hydroxyl, substituted or non-substituted C1 to C6 alkyl, substituted or non-substituted 2- to 7-membered heteroalkyl, substituted or non-substituted 3- to 6-membered cycloalkyl, substituted or non-substituted 3- to 6-membered heterocycloalkyl, acyl, ester, acylamino, sulfonyl, sulfonamido, or two adjacent $R^7$s and their connected atoms form substituted or non-substituted 3- to 6-membered hydrocarbon rings, substituted or non-substituted 3- to 6-membered heterocyclic rings; the heteroatoms of heteroalkyl, heterocycloalkyl, heteroaryl, heterocyclic ring stated in $R^7$ are selected from one or more of N, O and S;
Further, each occurrence of $R^7$ is independently selected from hydrogen, halogen, cyano group, substituted or non-substituted C1 to C6 alkyl, substituted or non-substituted 2- to 7-membered oxyalkyl, substituted or non-substituted 2- to 7-membered N-heteroalkyl, substituted or non-substituted 3- to 6-membered cycloalkyl, substituted or non-substituted 3-to 6-membered heterocycloalkyl, and sulfonamido group, or two adjacent $R^7$s and their connected atoms form substituted or non-substituted 3 to 6-membered heterocyclic rings, and the substituents in $R^7$ are selected from halogen, C1 to C6 alkyl, 3- to 6-membered cycloalkyl, 2- to 7-membered heteroalkyl, 3- to 6-membered heterocycloalkyl, hydroxyl, cyano group, - $NH_2$, acyl, ester, acylamino, sulfinyl, sulfonyl, sulfonamido, sulfinamido group; $n_2$ is selected from 0, 1, 2, 3, 4, and 5;
Further, each occurrence of $R^7$ is independently selected from hydrogen, halogen, cyano group, -OH, $-N(CH_3)_2$, $-N(CH_2CH_3)_2$, $-S(=O)_2NH_2$, $-CH_3$, $-CH_2CH_3$, $-CH(CH_3)_2$,

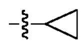,

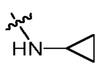 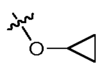,

morpholinyl, piperidinyl, piperazinyl, azetidinyl, pyrrolidinyl, $-(CH_2)n_8-OH$, - $CH(OH)-CH_2OH$, $-C(CH_3)_2OH$, $-OCH_2CH(OH)-CH_2OH$, $-OCH_2CH_2OCH_3$, - $OCH_2CH_2OCH_2CH_3$, $-OCH_3$, $-OCH_2CH_3$, $-OCH(CH_3)_2$, $-CF_3$, $-CHF_2$, and $-CF_2CF_3$, or two adjacent $R^7$s and their connected atoms form substituted or non-substituted 3- to 6-membered azaheterocyclic rings, 3- to 6-membered oxyheterocyclic rings, $n_8$ is selected from 1, 2, 3, and 4; $n_2$ is selected from 0, 1, 2, and 3.

**20.** The compound stated in claim 19 is **characterized by** that each occurrence of $R^7$ is independently selected from F, Cl, cyano group, -OH, $-N(CH_3)_2$, $-S(=O)_2NH_2$, $-CH_3$,

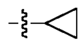,

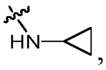, 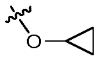,

morpholinyl, $-CH(OH)-CH_2OH$, $-C(CH_3)_2OH$, $-OCH_3$, and $-CF_3$, or two adjacent $R^7$s and their connected atoms form substituted or non-substituted

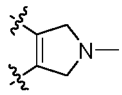;

the substituents in $R^7$ are selected from halogen, C1 to C6 alkyl, 3- to 6-membered cycloalkyl, 2-to 6-membered heteroalkyl, 3- to 6-membered heterocycloalkyl, hydroxyl, cyano group, - $NH_2$, acyl, and ester;
Further, each occurrence of $R^7$ is independently selected from F, $-OCH_3$, $-CF_3$, $-CH_3$, - OH, cyano group; further, $n_2$ is 1.

**21.** The compound stated in any of claims 1 to 20 are **characterized by** that $R^5$ is selected from substituted or non-substituted C1 to C4 alkyl, substituted or non-substituted 2- to 6-membered heteroalkyl, substituted or non-substituted 3- to 6-membered cycloalkyl, substituted or non-substituted 3- to 6-membered heterocycloalkyl, substituted or non-substituted phenyl, substituted or non-substituted 5- to 6-membered heteroaryl; the substituents in $R^5$ are selected from halogen, C1 to C6 alkyl, 3- to 6-membered cycloalkyl, 2-to 6-membered heteroalkyl, 3- to 6-membered heterocycloalkyl, hydroxyl, cyano group, amino, acyl, ester, acylamino, sulfinyl, sulfonyl, sulfonamido, sulfinamido group; Further, $R^5$ is selected from substituted or non-substituted tetrahydropyranyl, substituted or non-substituted C1 to C4 alkyl, substituted or non-substituted phenyl, substituted or non-substituted thienyl, substituted or non-substituted pyridinyl, substituted or non-substituted pyrimidinyl, preferably substituted or non-substituted phenyl, the substituents in $R^5$ are selected from halogen or C1-C6 alkyl; further, the substituents in $R^5$ are selected from F, Cl, and C1 to C4 alkyl.

**22.** The compound stated in any of claims 1 to 21 are **characterized by** that $R^2$ and $R^3$ are independently selected from hydrogen, halogen, cyano group, hydroxyl, $-NH_2$, substituted or non-substituted C1 to C6 alkyl, substituted or non-substituted 2- to 6-membered heteroalkyl, substituted or non-substituted 3- to 6-membered cycloalkyl, substituted or non-substituted 3-to 6-membered heterocycloalkyl, substituted or non-substituted C2 to C6 alkenyl, acyl, ester, acylamino, sulfonyl, and sulfonamido, or $R^2$ and $R^3$ together form substituted or non-substituted C3 to C4 alkyl, substituted or non-substituted 3- to 4-membered heteroalkyl; substituents in $R^2$ and $R^3$ are independently selected from halogen, C1 to C6 alkyl, 3- to 6-membered cycloalkyl, 2- to 6-membered heteroalkyl, 3- to 6-membered heterocycloalkyl, C2 to C4 alkenyl, C2 to C4 alkynyl, hydroxyl, cyano group, $-NH_2$, nitro, carboxyl, acyl, ester, acylamino, sulfinyl, sulfonyl, sulfonamido, sulfinamido group;

$R^4$ is selected from hydrogen, C1 to C4 alkyl, C1 to C4 haloalkyl, C1 to C4 alkoxy, and C2 to C4 alkenyl;
Further, $R^2$ and $R^3$are independently selected from hydrogen, cyano group, substituted or non-substituted C1 to C4 alkyl, and substituted or non-substituted 2- to 5-membetred heteroalkyl, or $R^2$ and $R^3$ together form substituted or non-substituted C3 to C4 alkyl, substituted or non-substituted 3- to 4-membered heteroalkyl; the substituents in $R^2$ and $R^3$ are independently selected from halogen, hydroxyl, C1 to C4 alkyl, 3- to 6-membered cycloalkyl, 2- to 4-membered heteroalkyl, 3- to 6-membered heterocycloalkyl, and C2 to C4 alkenyl;
$R^4$ is selected from hydrogen and methyl;
Further, $R^2$ and $R^3$ are independently selected from hydrogen, cyano group, substituted or non-substituted methyl, substituted or non-substituted ethyl, substituted or non-substituted

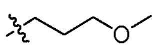,

substituted or non-substituted

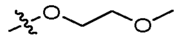,

or $R^2$ and $R^3$ together form substituted or non-substituted

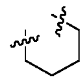,

substituted or non-substituted

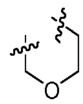;

preferably, $R^2$ and $R^3$ are independently selected from hydrogen, cyano group, substituted or non-substituted methyl, substituted or non-substituted

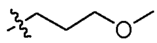,

or $R^2$ and $R^3$ together form substituted or non-substituted

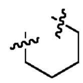,

substituted or non-substituted

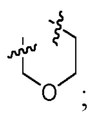;

more preferably, $R^2$ and $R^3$ are independently selected from hydrogen, cyano group, and substituted or non-substituted methyl;
$R^4$ is selected from hydrogen;
Further, the substituents in $R^2$ and $R^3$ are independently selected from F, Cl, and hydroxyl.

**23.** The compound stated in any of claims 1 to 22 are **characterized by** that $R^6$ is selected from hydrogen, halogen-substituted or non-substituted C1 to C6 alkyl, and substituted or non-substituted 3- to 6-membered cycloalkyl; Further, $R^6$ is selected from hydrogen, fluorine-substituted or non-substituted C1 to C4 alkyl, preferably H.

**24.** The compound stated in claim 1 is **characterized by** that the stated compound is selected from the following structures, or their R or S configuration:

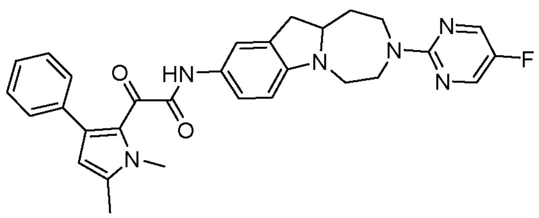,

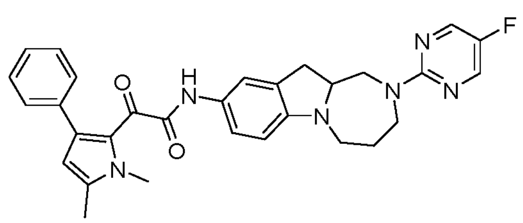,

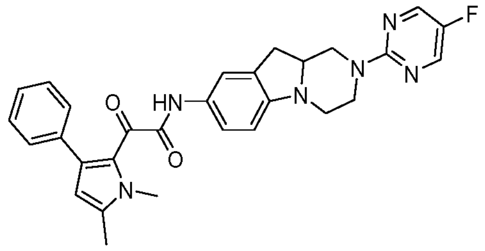
,

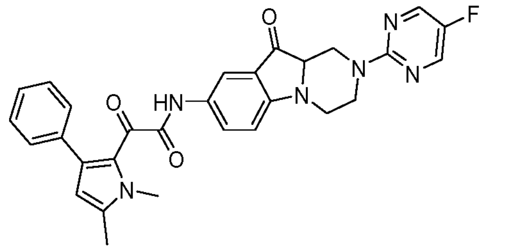
,

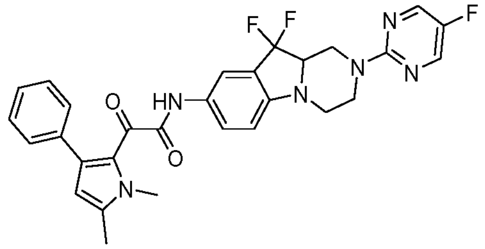
,

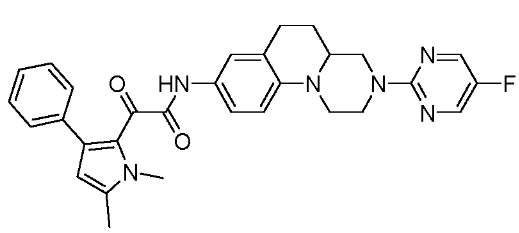
,

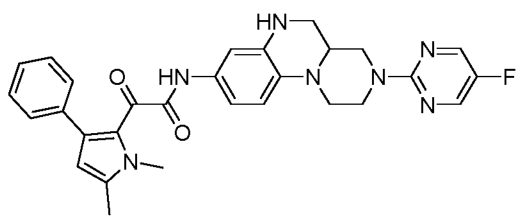
,

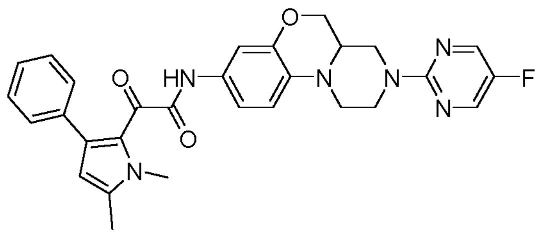
,

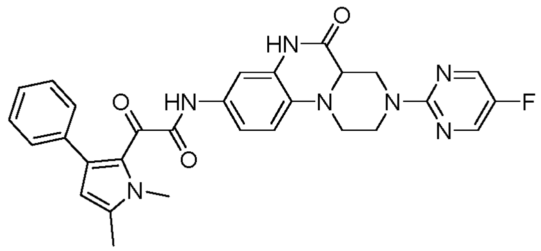
,

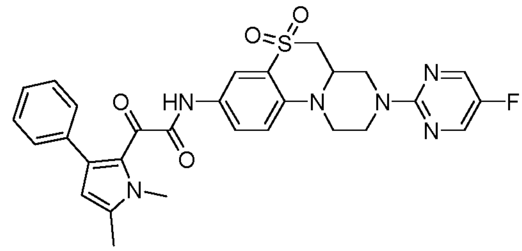
,

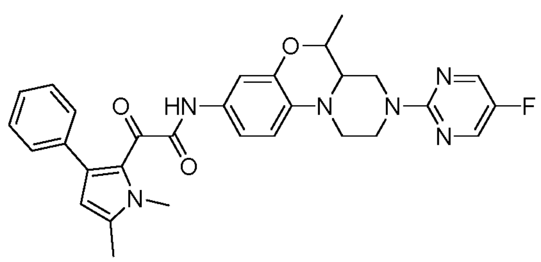
,

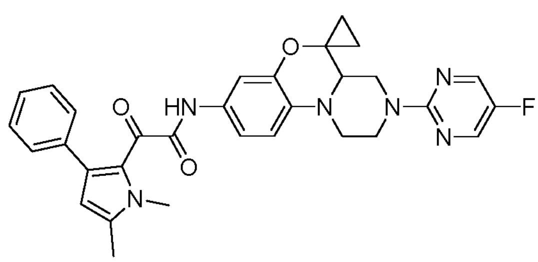
,

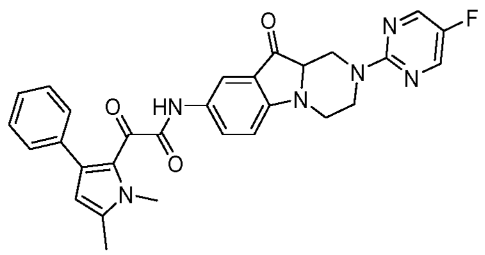
,

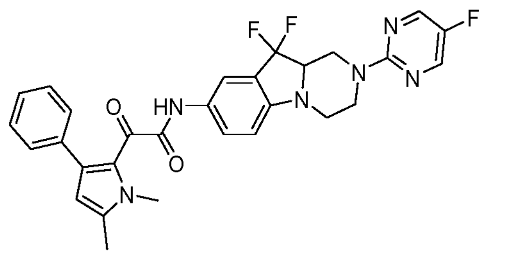
,

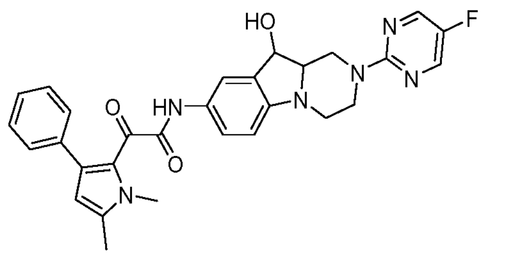,

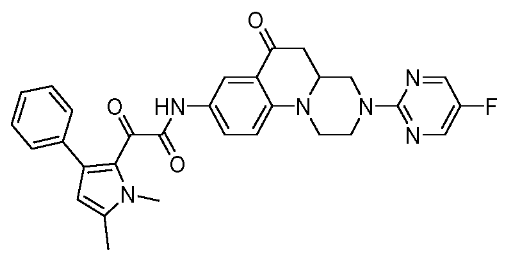,

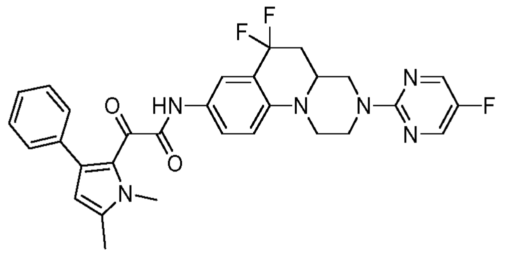,

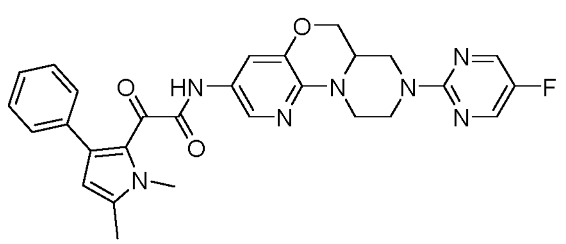,

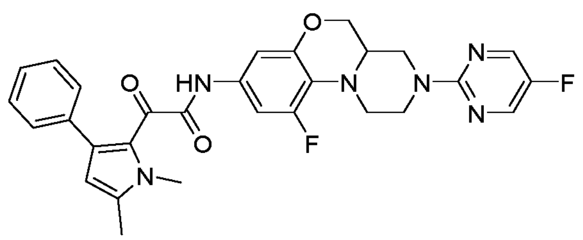,

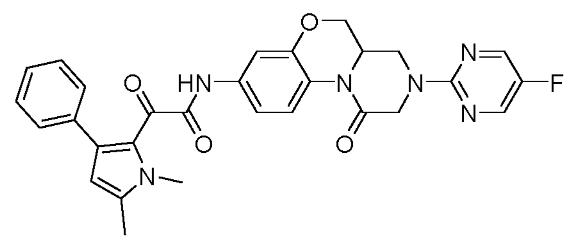,

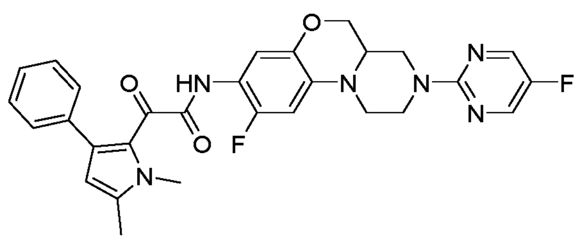,

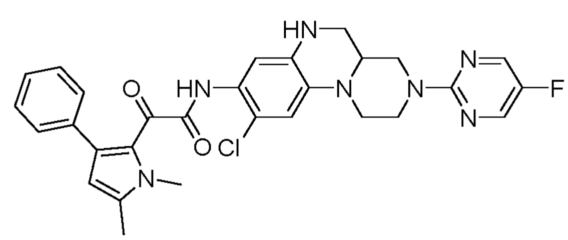,

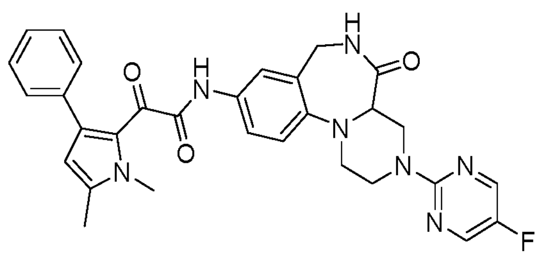,

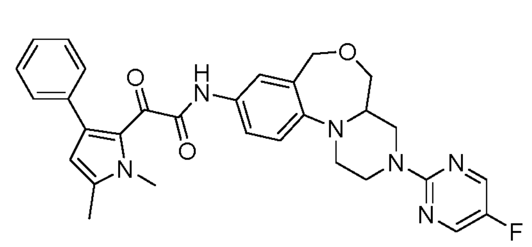,

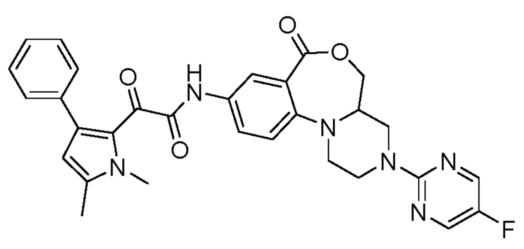,

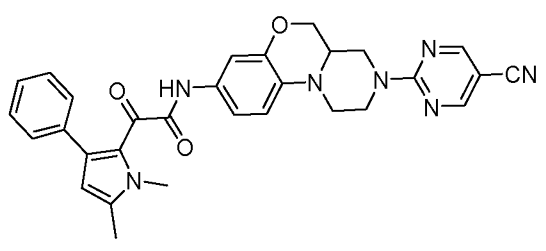,

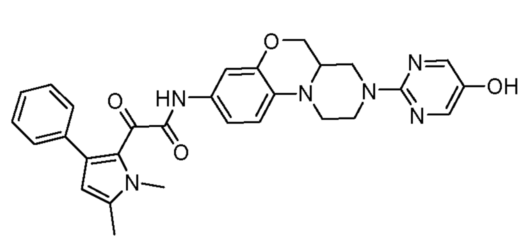,

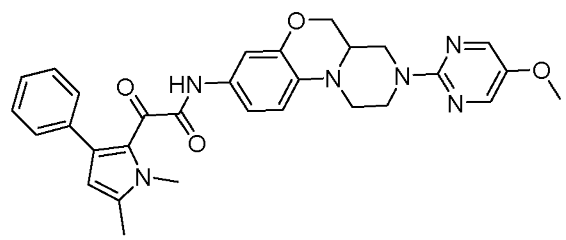,

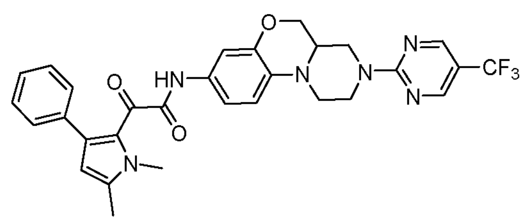,

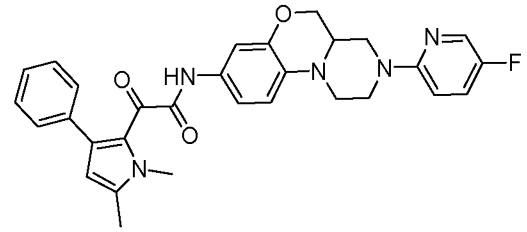,

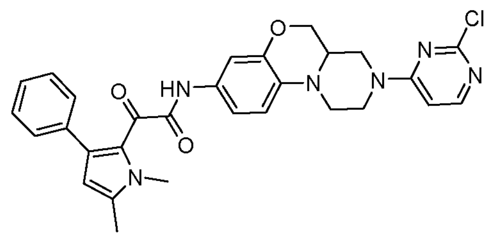,

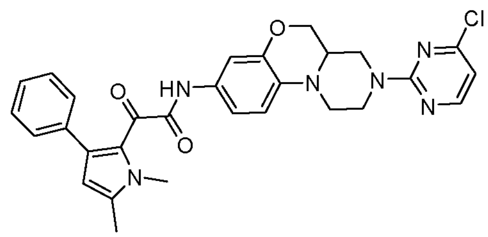,

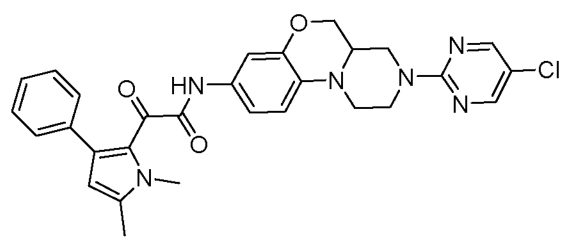,

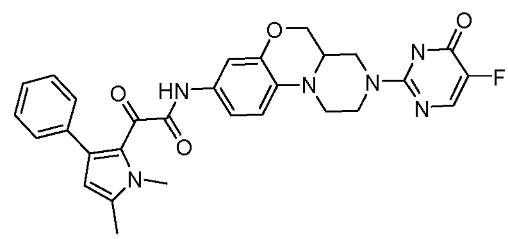,

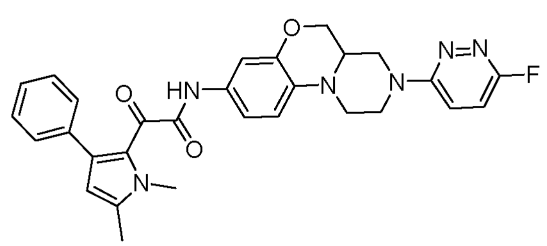,

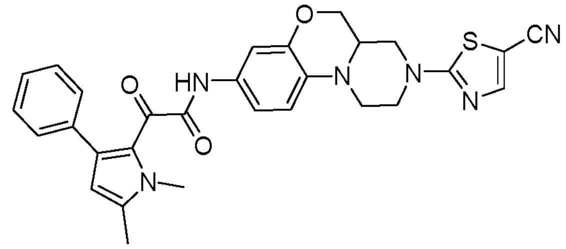,

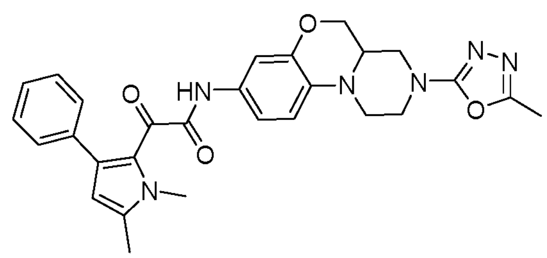,

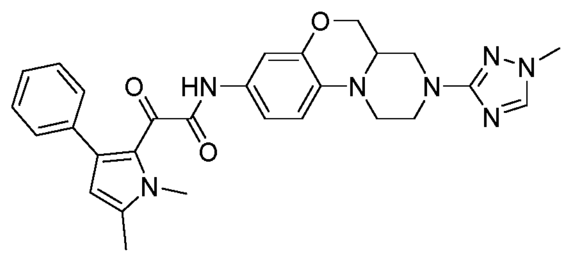

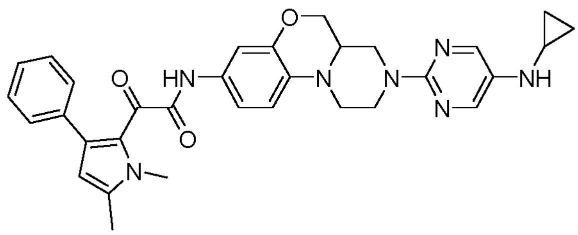

,

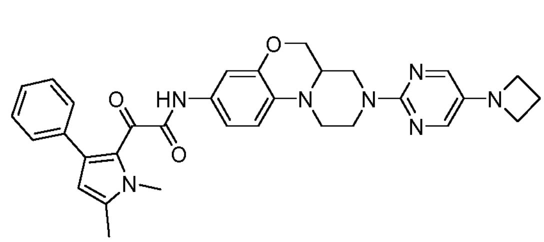

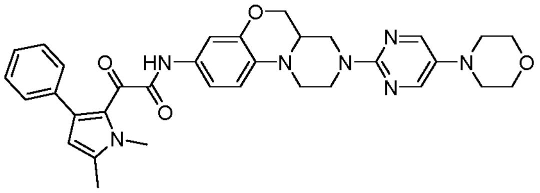

,

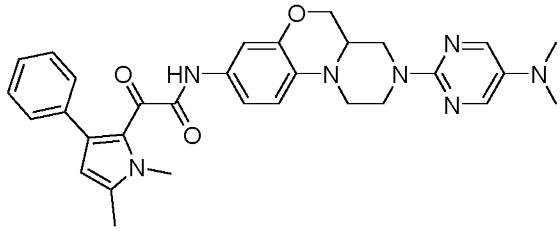

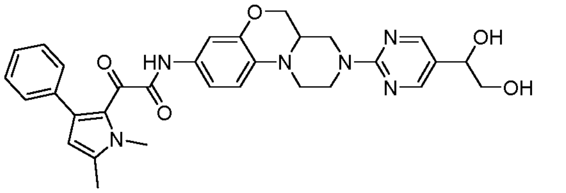

,

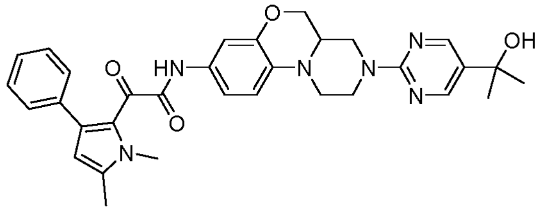

,

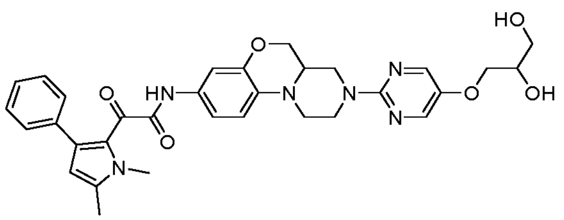

,

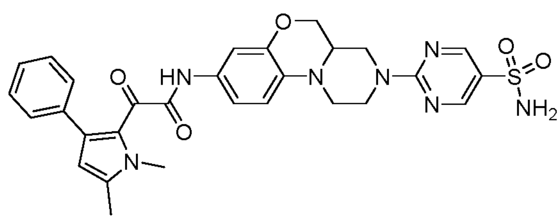

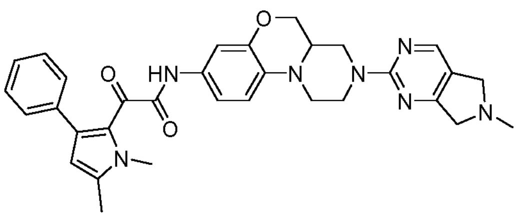

,

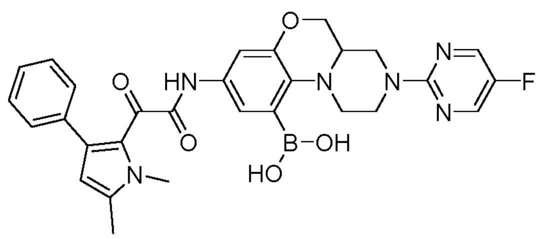

,

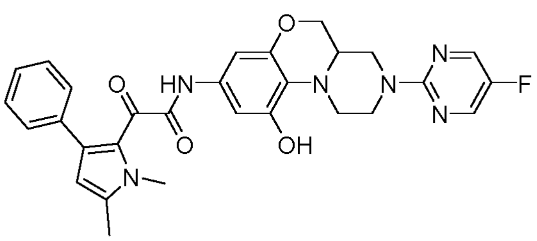

,

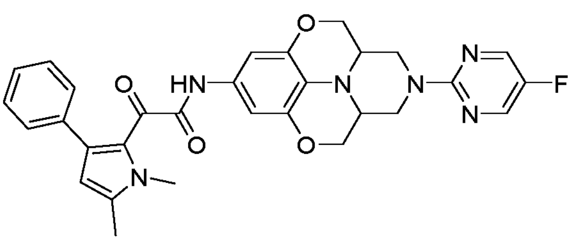

,

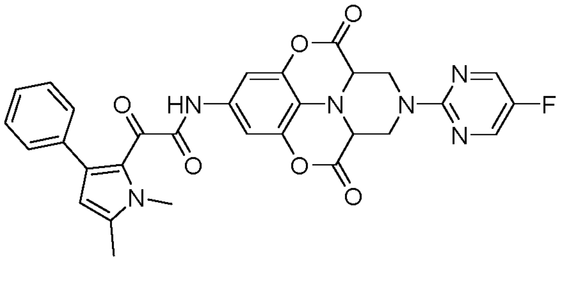

,

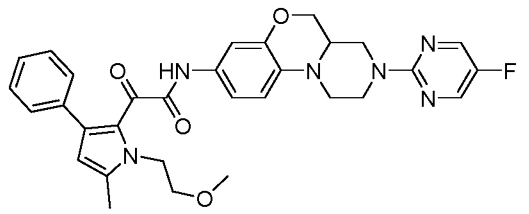

,

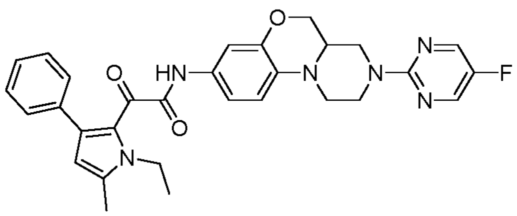

,

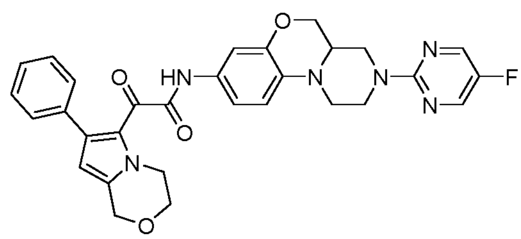

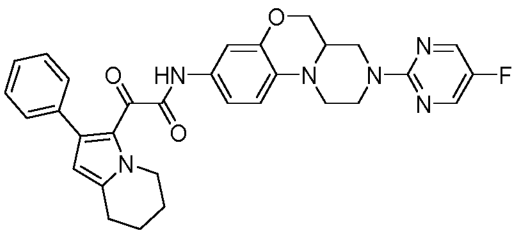

,

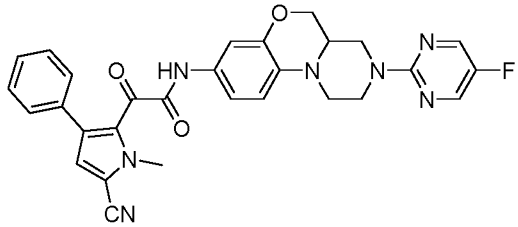

,

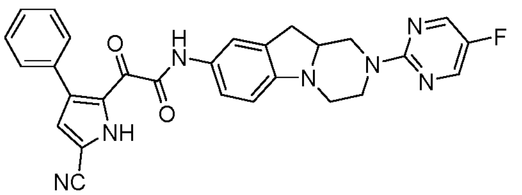

,

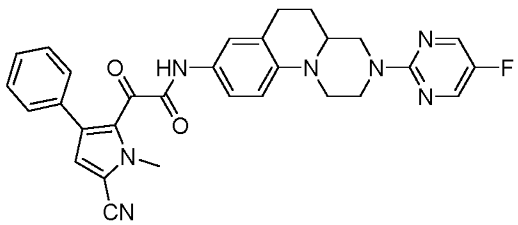

,

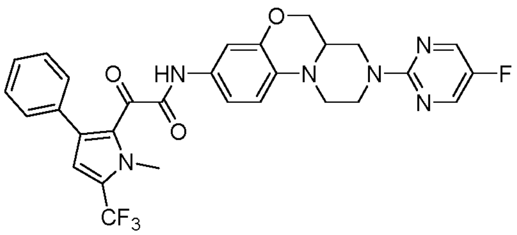

,

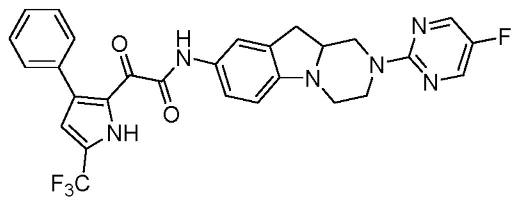

,

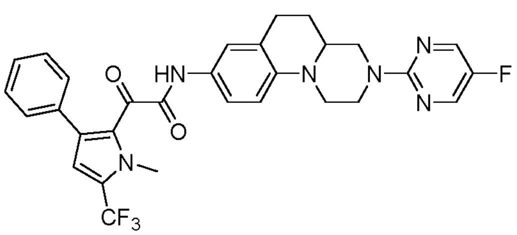

,

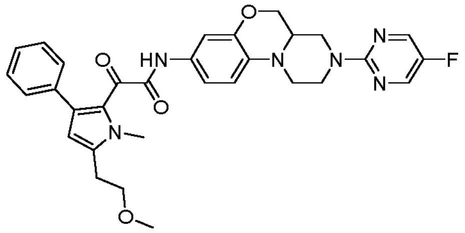

,

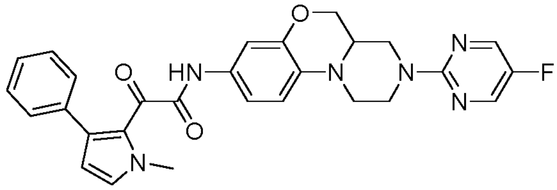

,

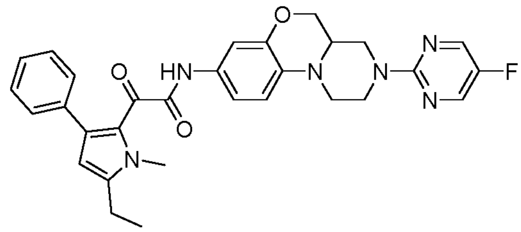

,

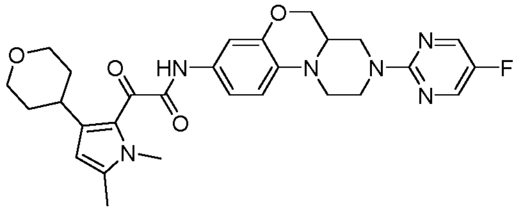

,

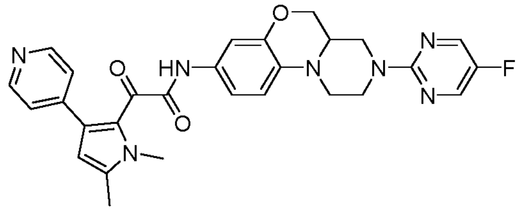

,

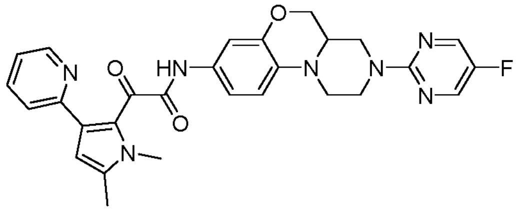

,

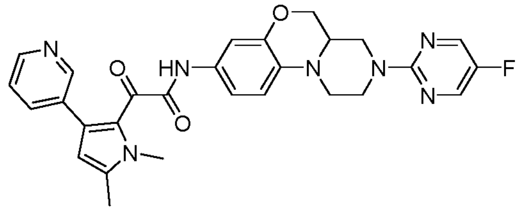

,

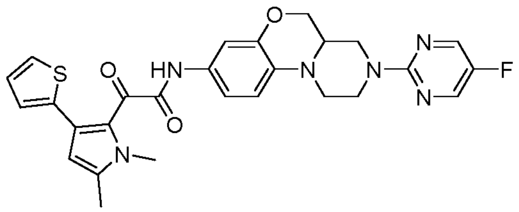

,

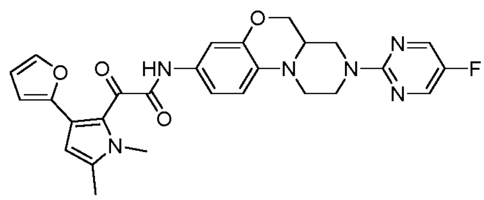

,

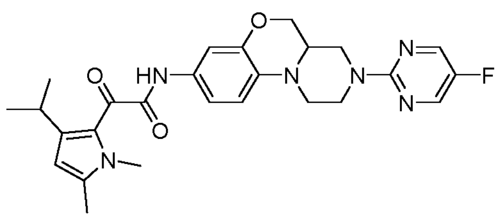

,

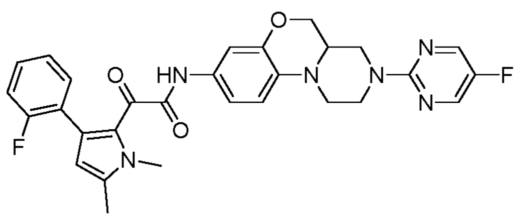

,

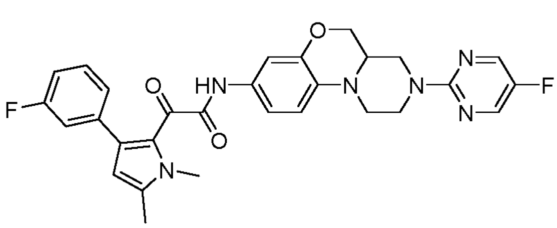

,

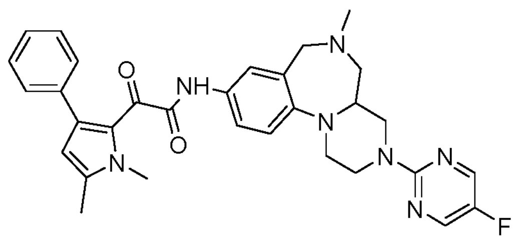

,

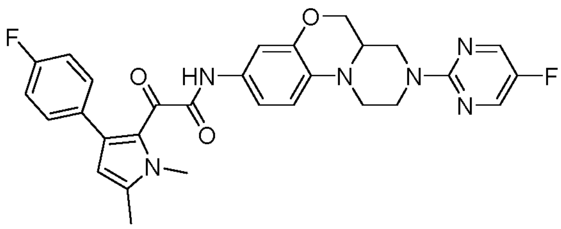

,

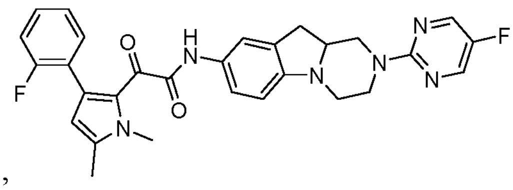

,

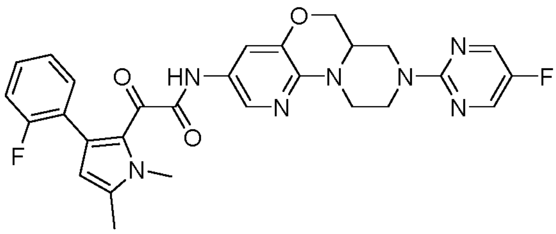

,

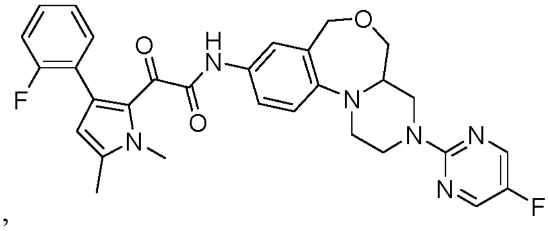

.

**25.** The compound stated in claim 24 is **characterized by** that the stated compound is selected from the following structures:

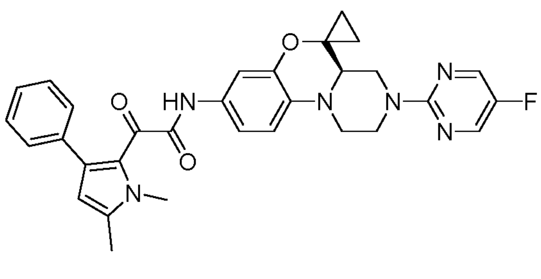

,

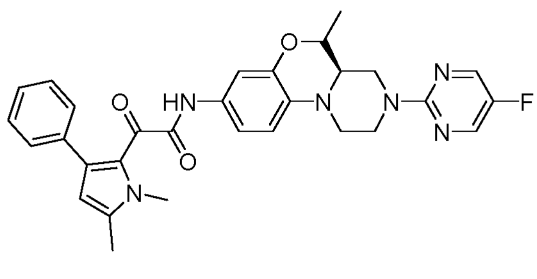

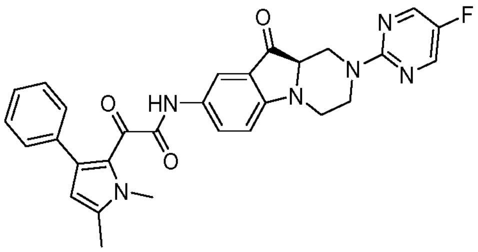

,

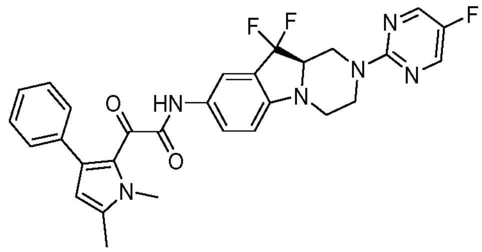

,

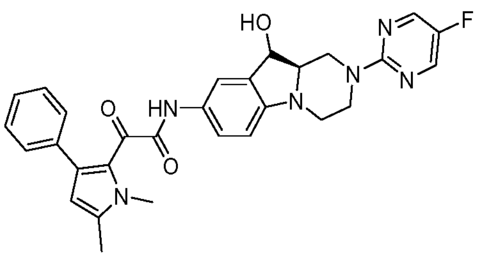

,

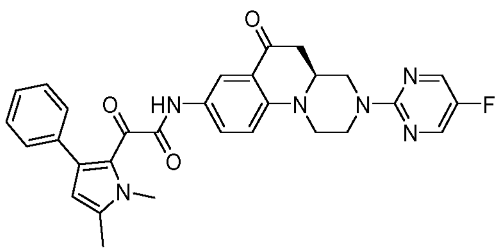

,

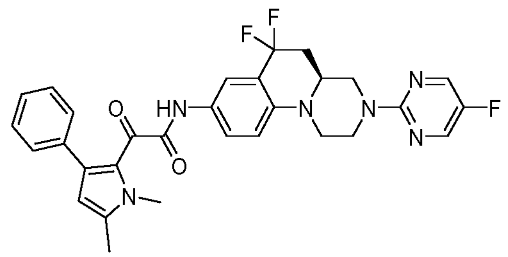

,

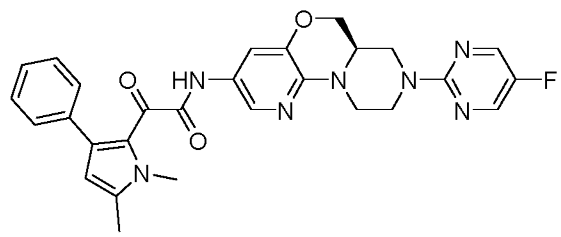

,

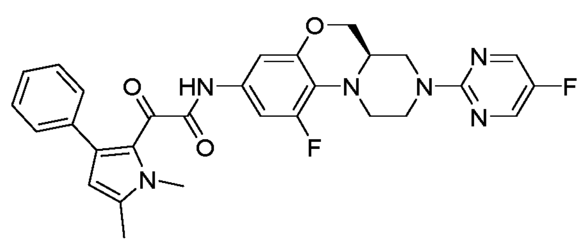

,

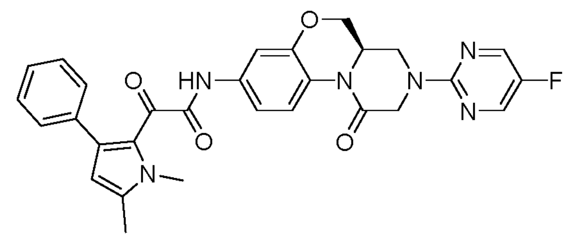

,

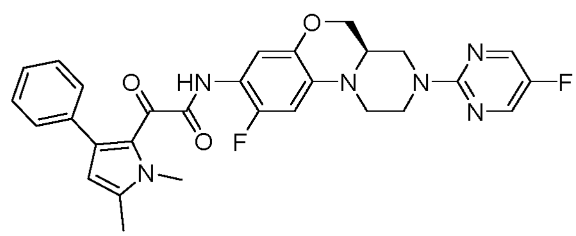

,

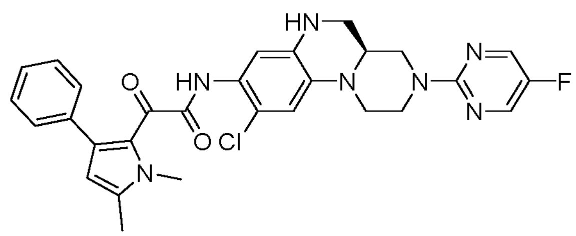

,

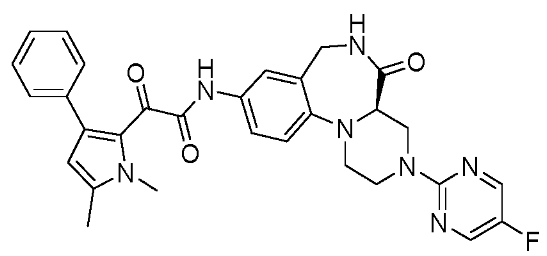

,

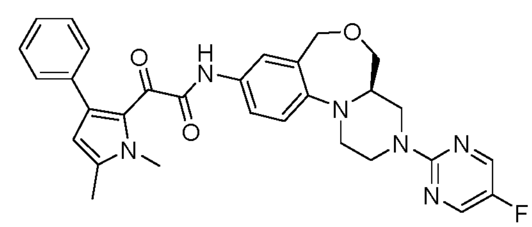

,

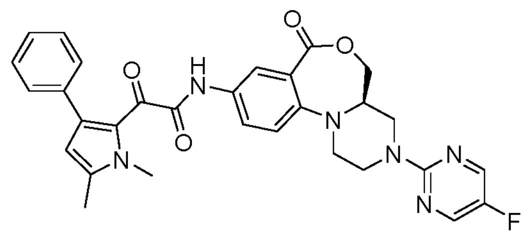

,

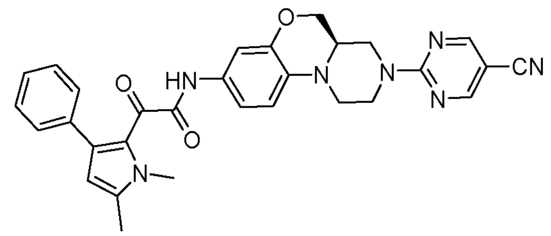

,

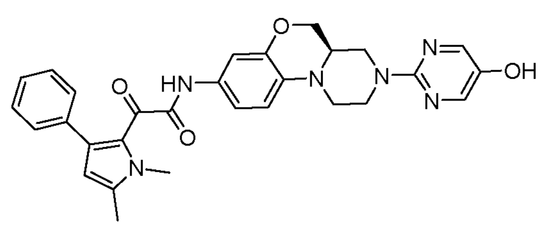

,

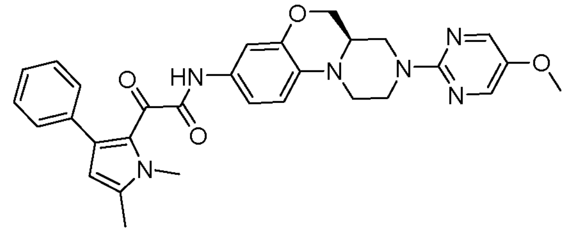

,

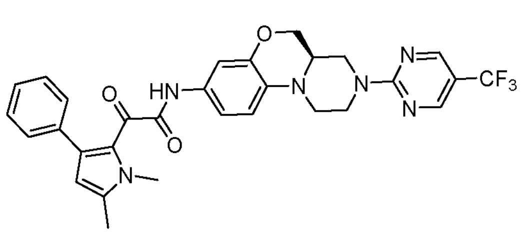
,
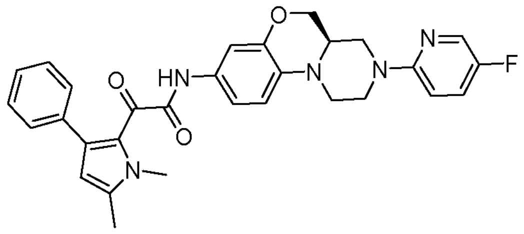
,

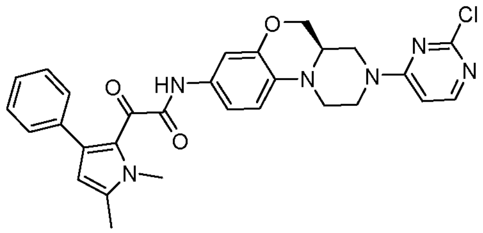
,
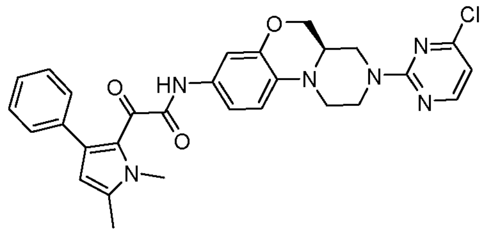
,

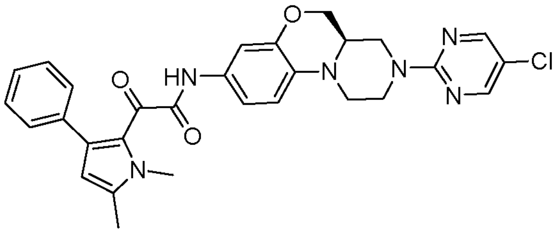
,
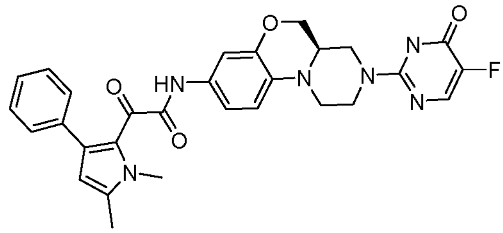
,

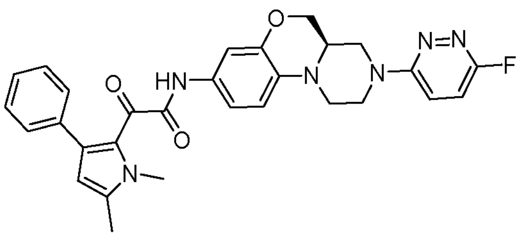
,
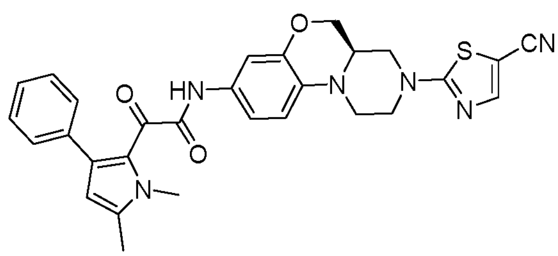
,

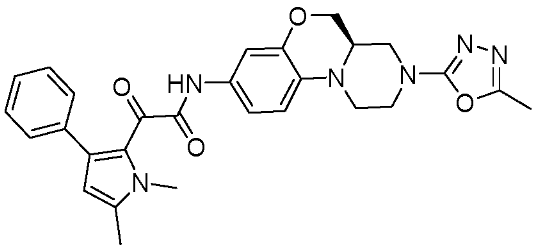
,
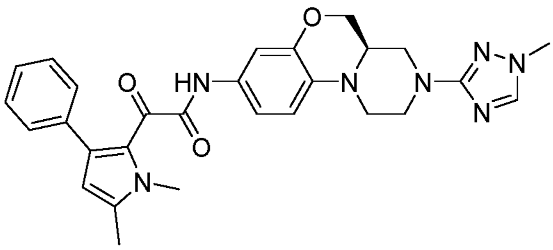
,

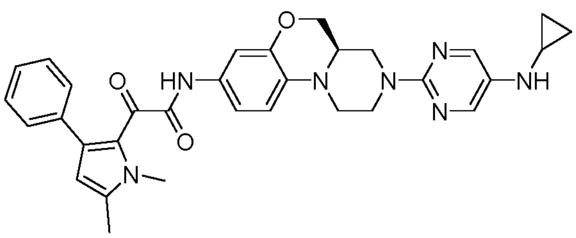
,
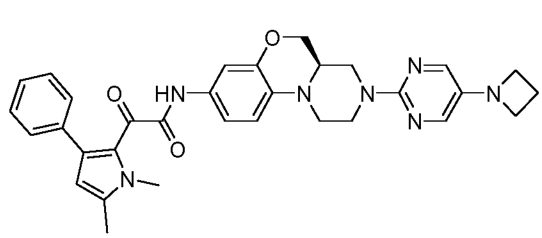
,

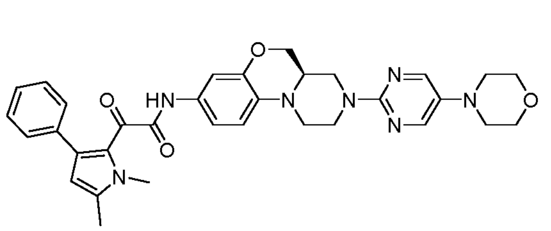

,

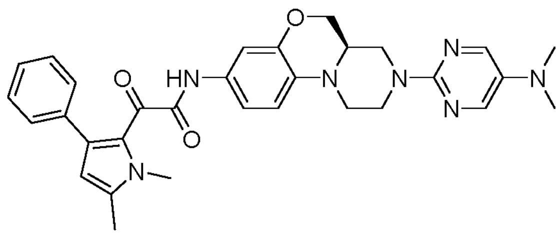

,

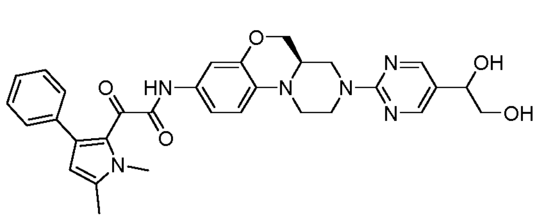

,

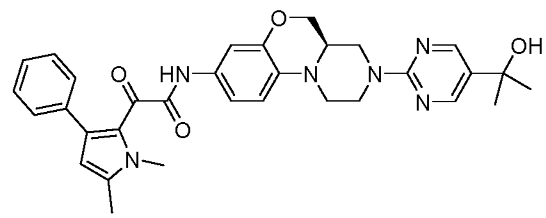

,

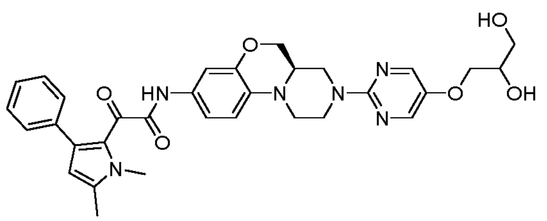

,

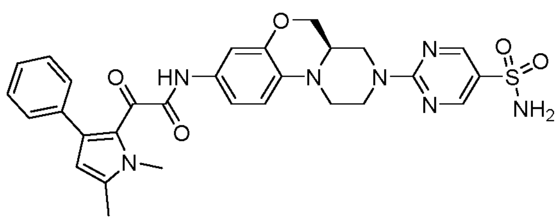

,

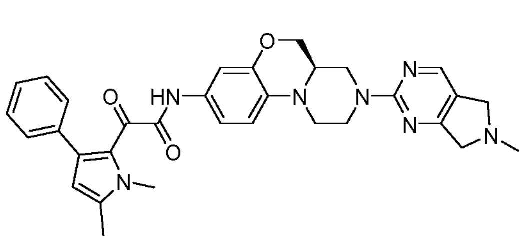

,

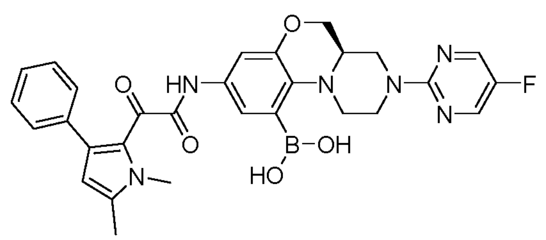

,

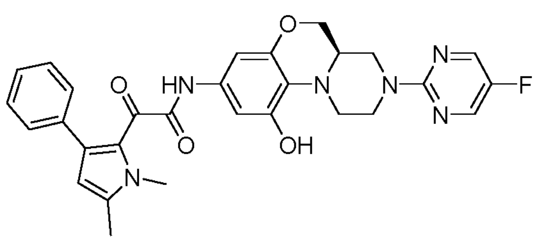

,

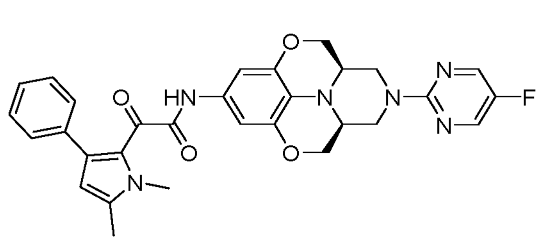

,

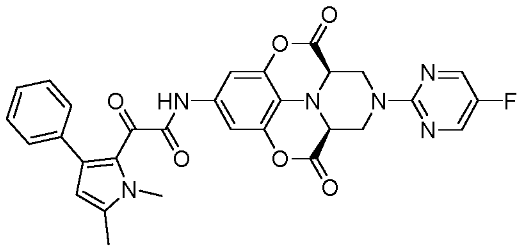

,

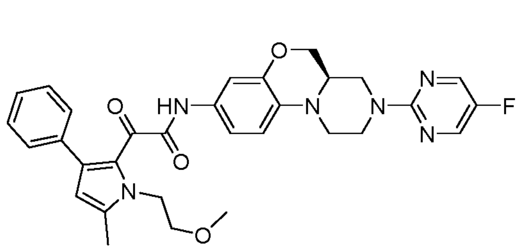

,

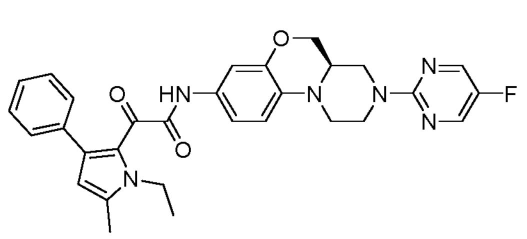

,

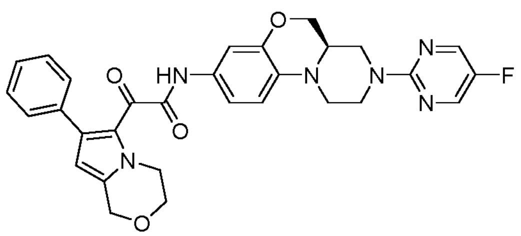

,

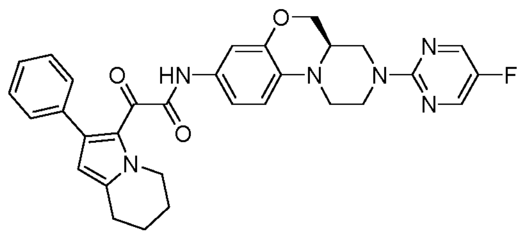

,

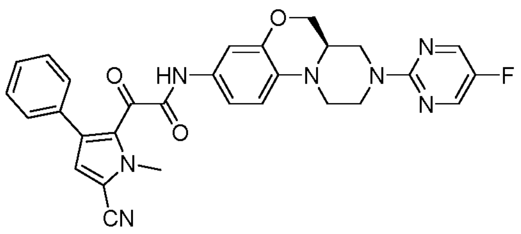

,

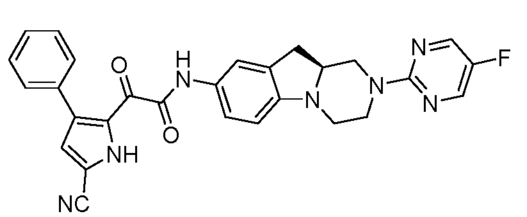

,

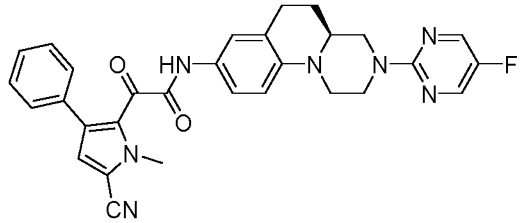

,

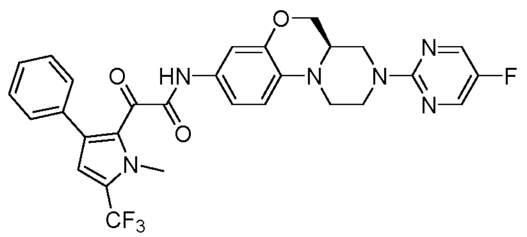

,

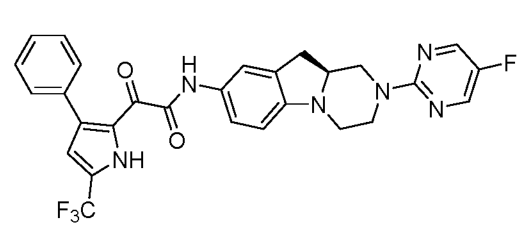

,

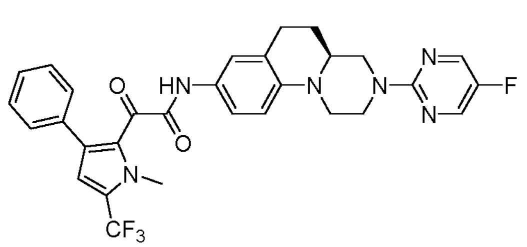

,

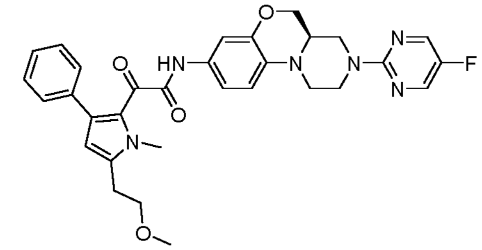

,

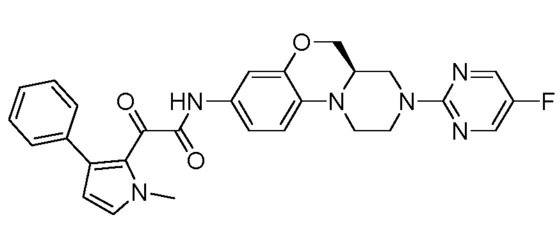

,

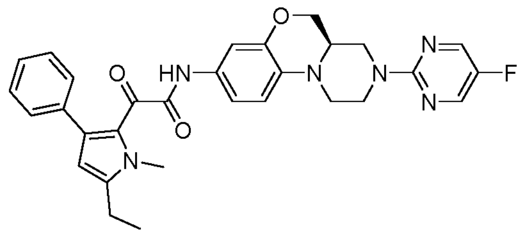

,

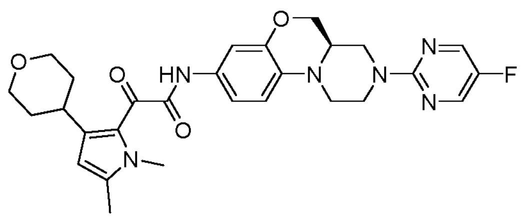,
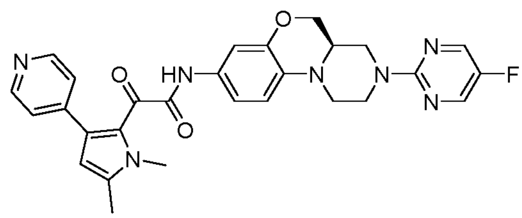,

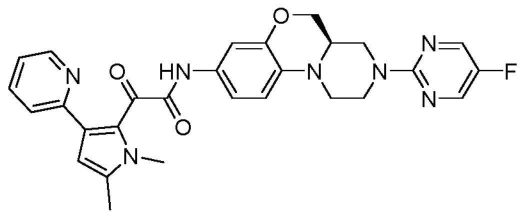,
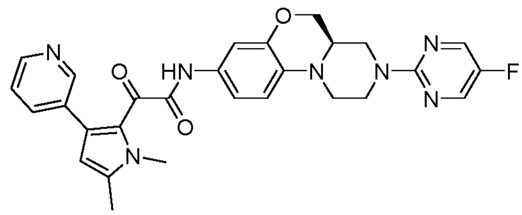,

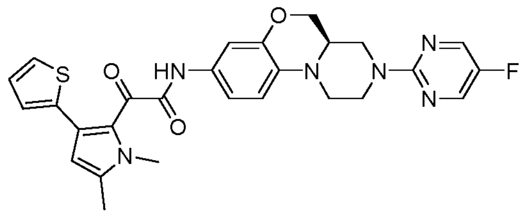,
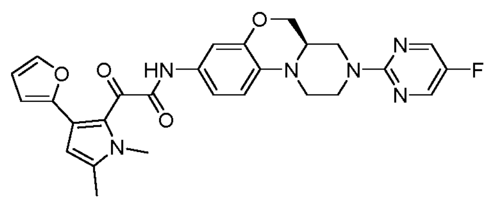,

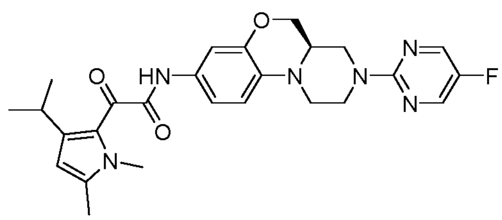,
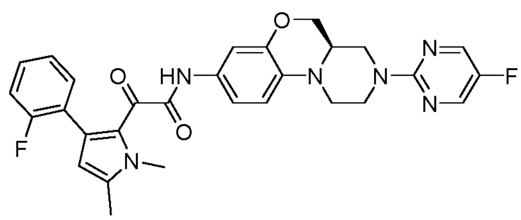,

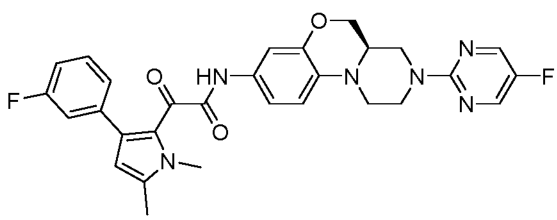,
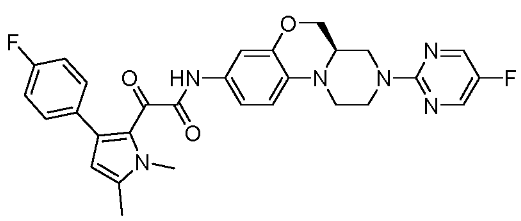

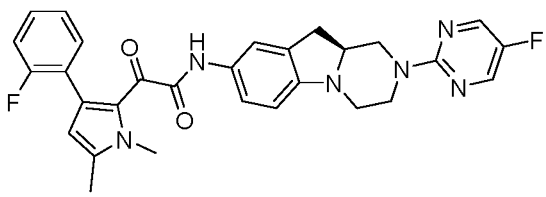.

**26.** The compound stated in claim 24 is **characterized by** that the stated compound is selected from the following structures:

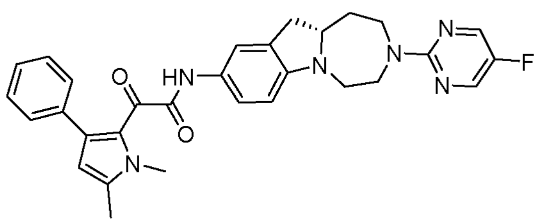,

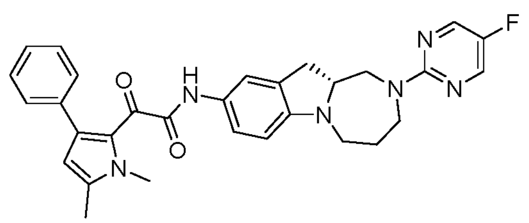,

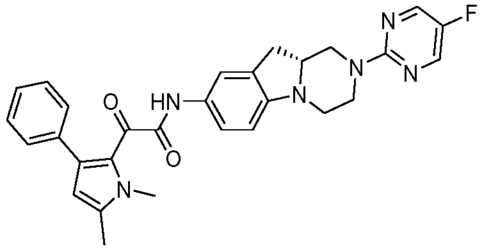,

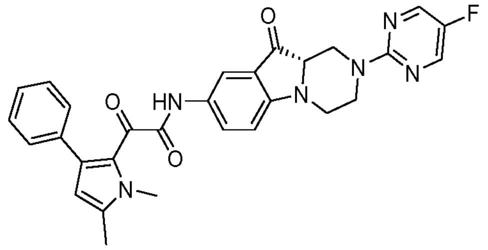,

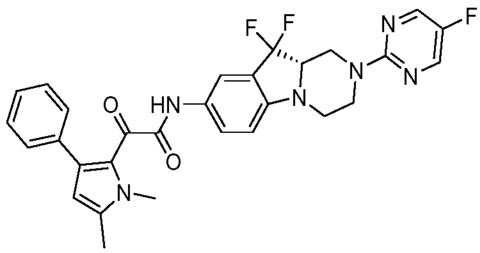,

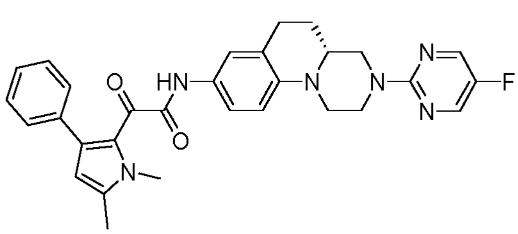,

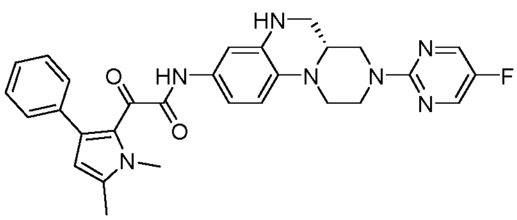,

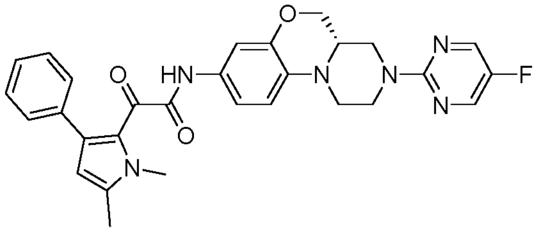,

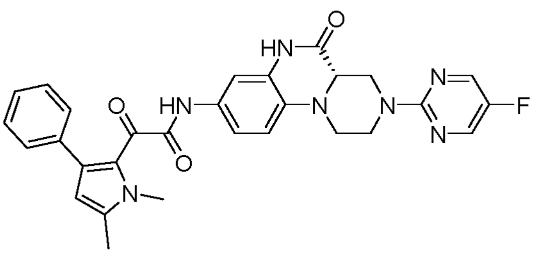,

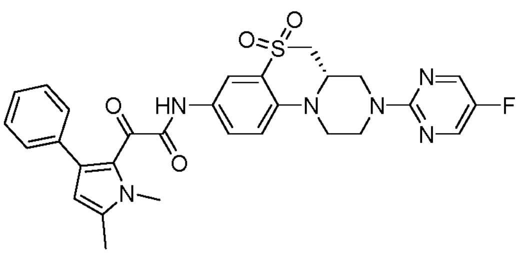],

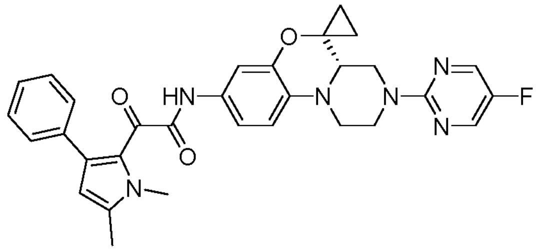,

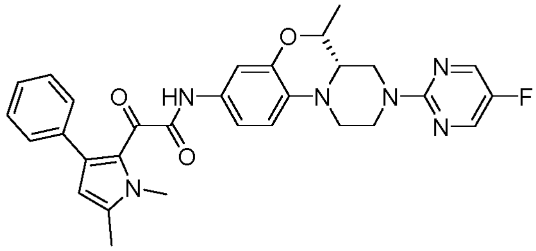

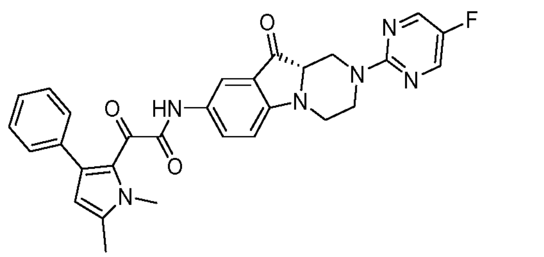
,
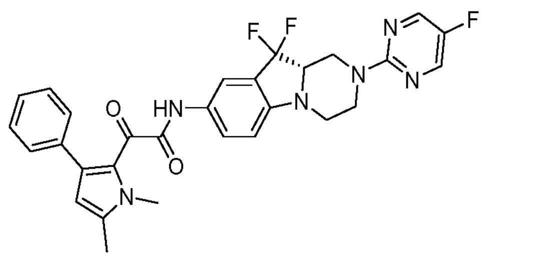
,
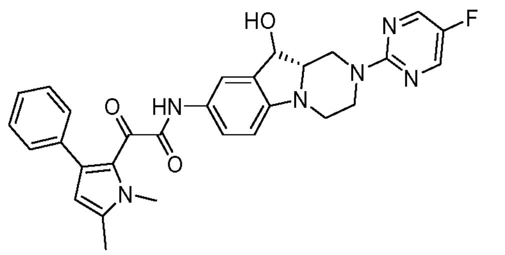
,
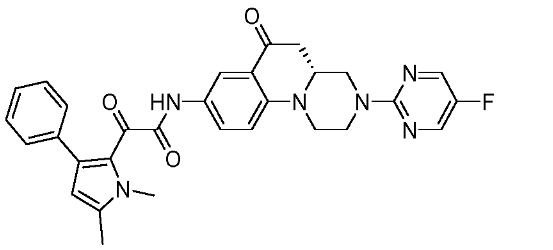
,
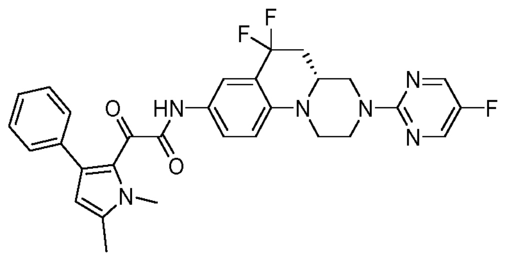
,
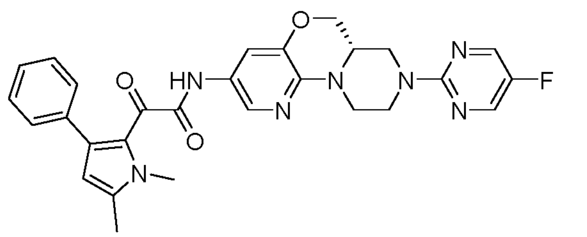
,
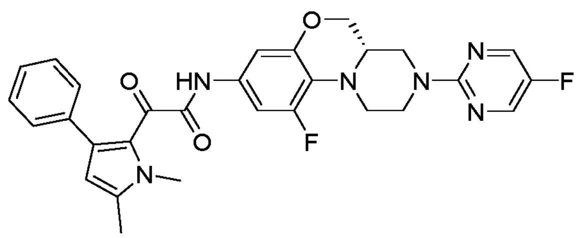
,
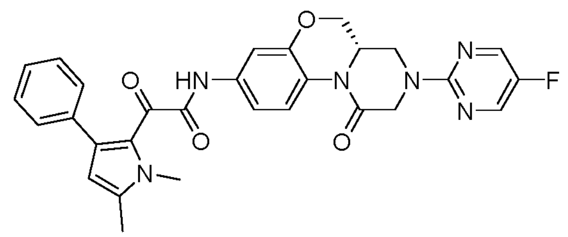
,
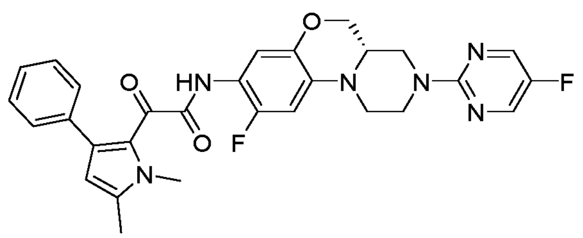
,
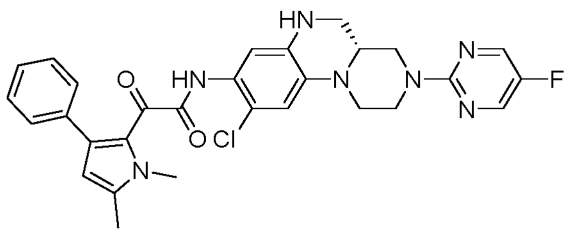
,
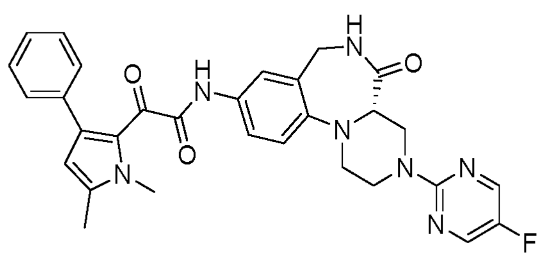
,
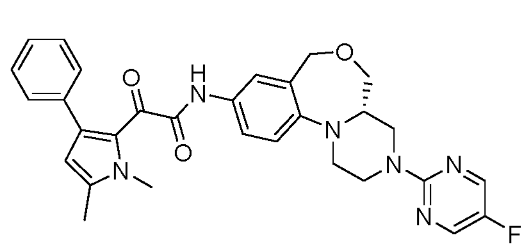
,

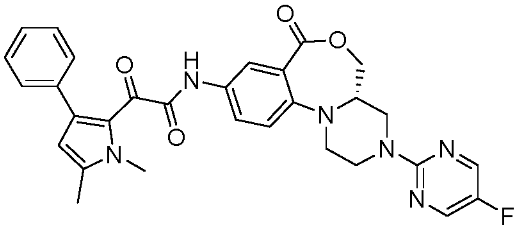
,
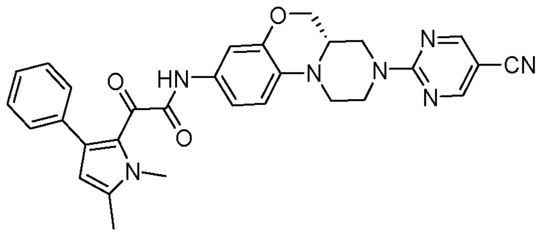
,

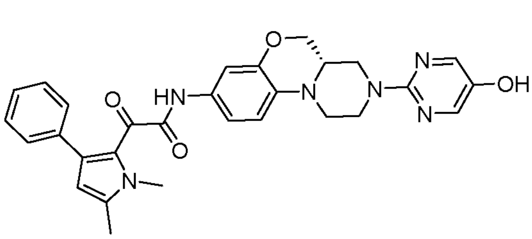
,
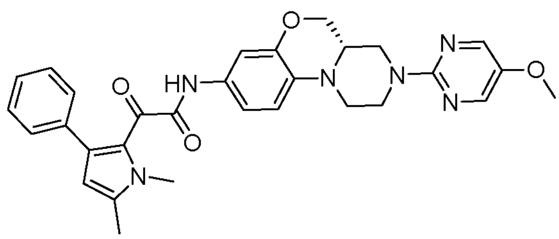
,

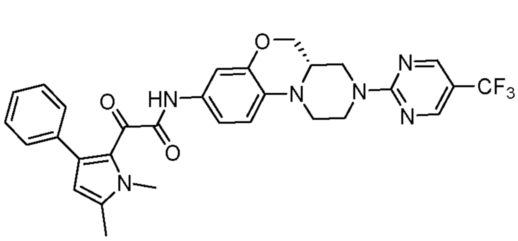
,
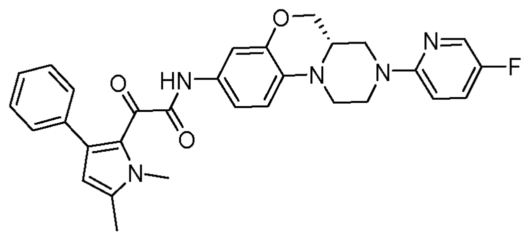
,

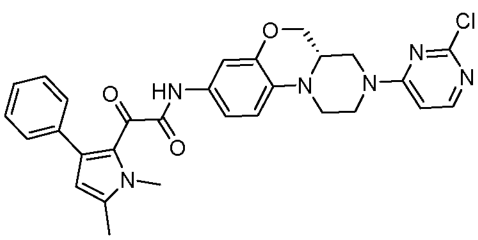
,
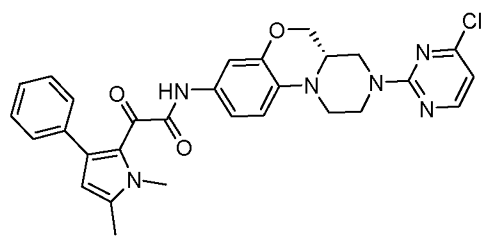
,

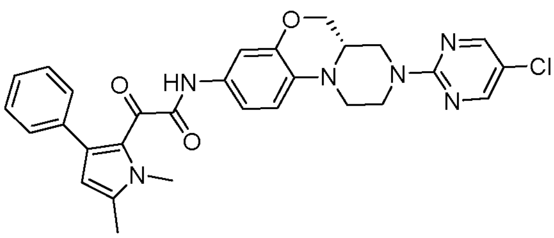
,
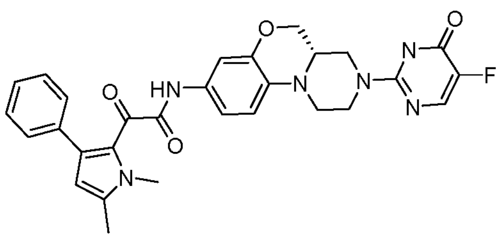
,

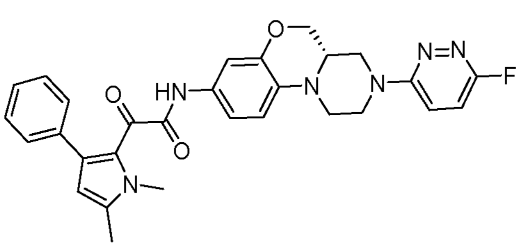
,
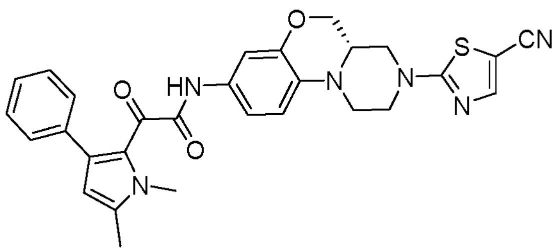
,

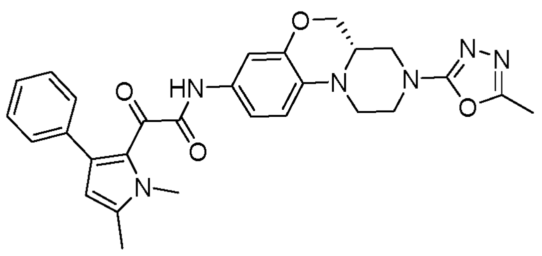
,
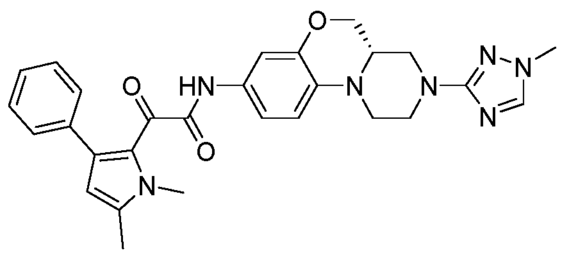
,

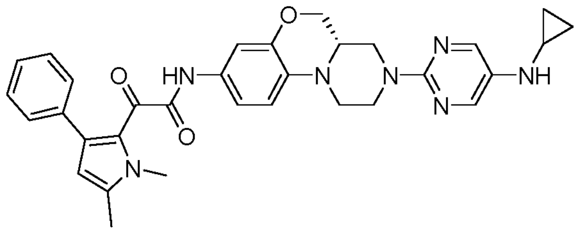
,
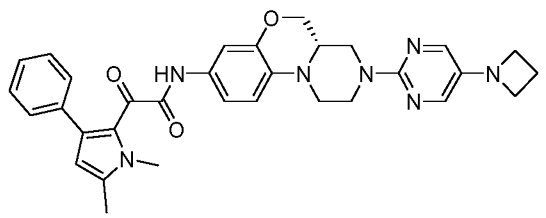
,

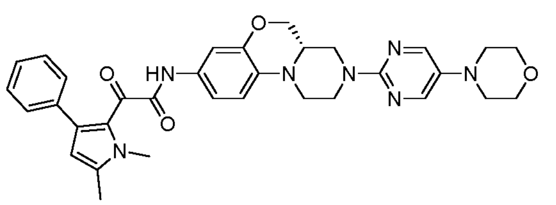
,
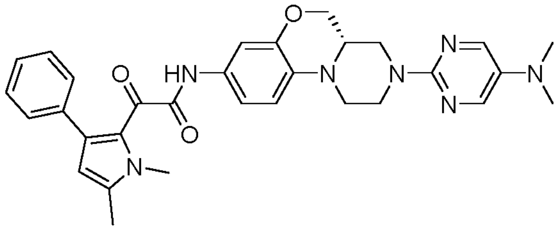
,

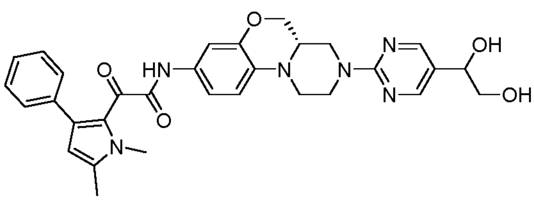
,
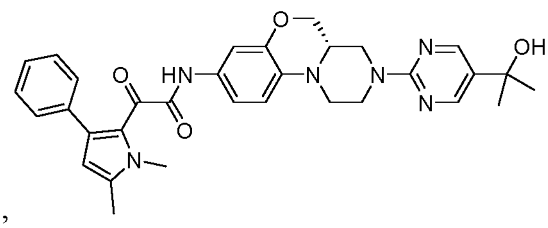
,

and

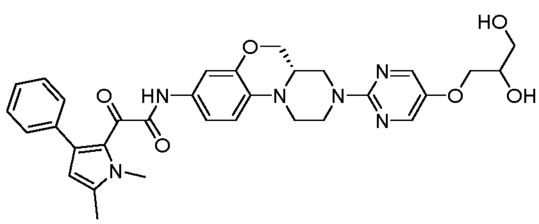
,
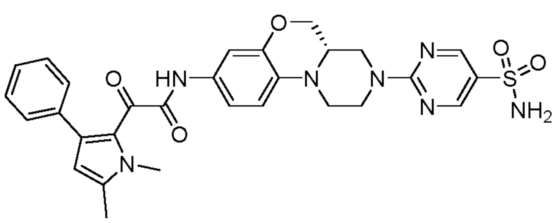
,

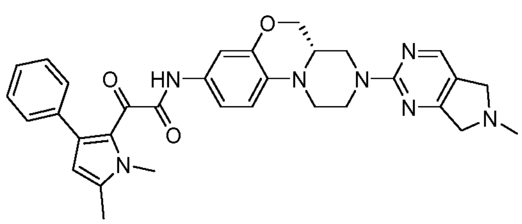
,
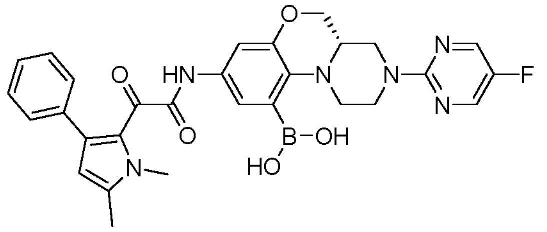
,

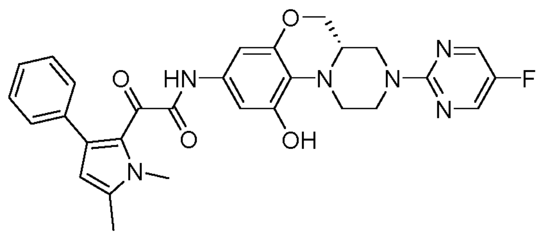
,
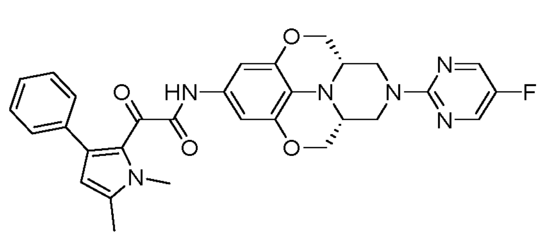
,
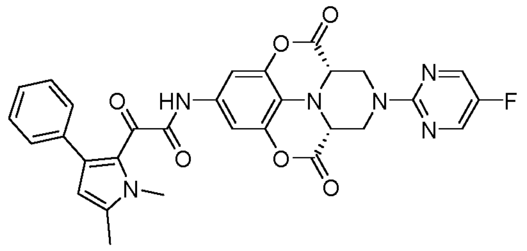
,
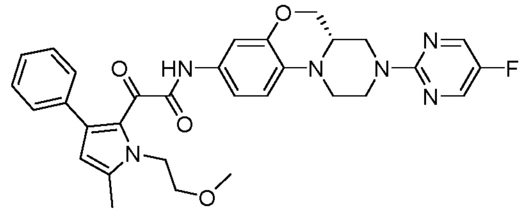
,
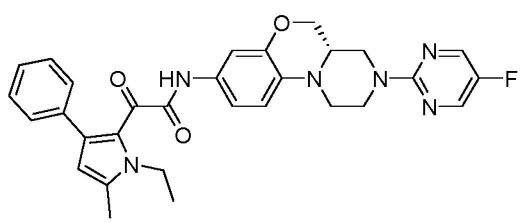
,
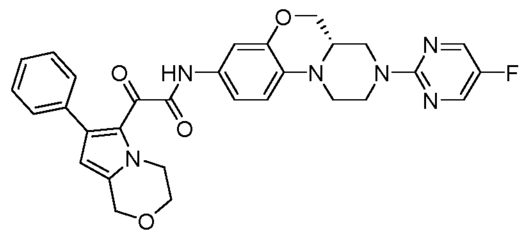
,
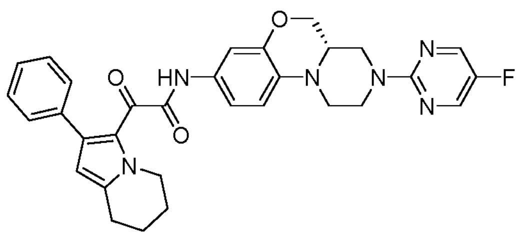
,
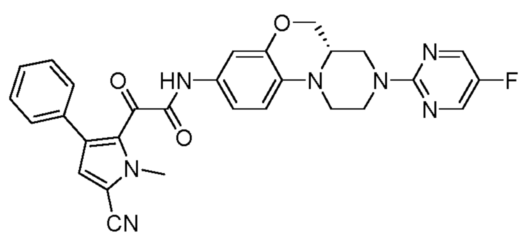
,
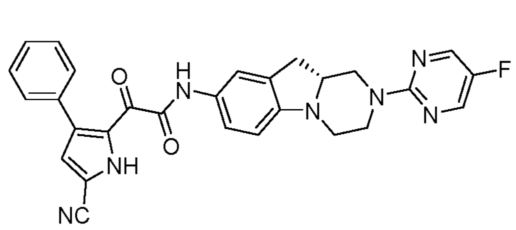
,
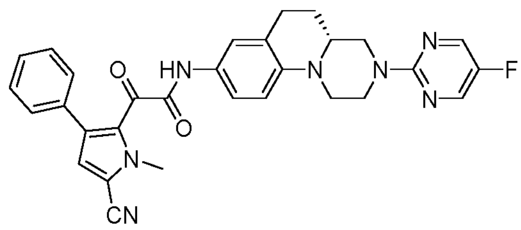
,
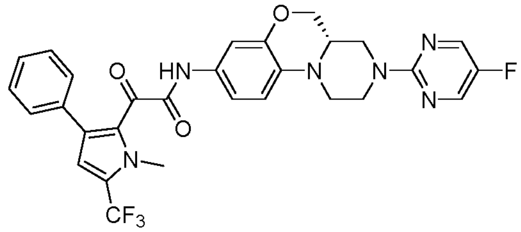
,
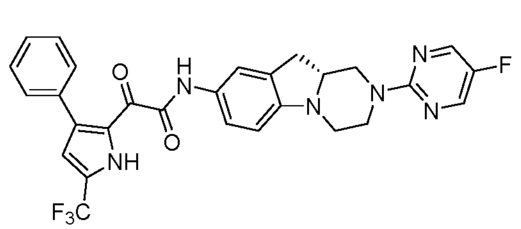
,

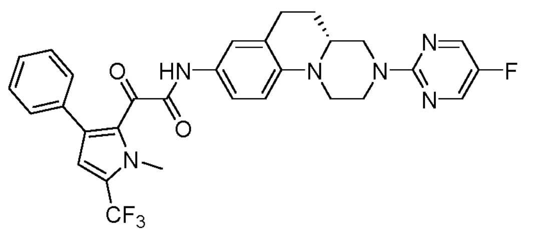

,

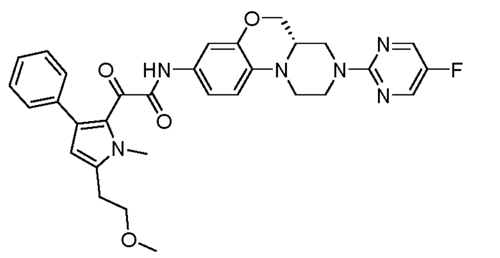

,

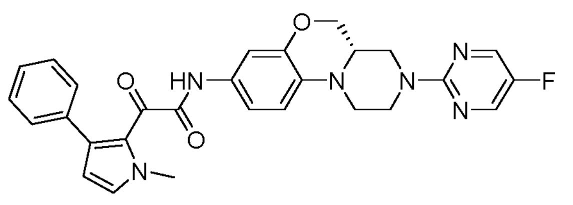

,

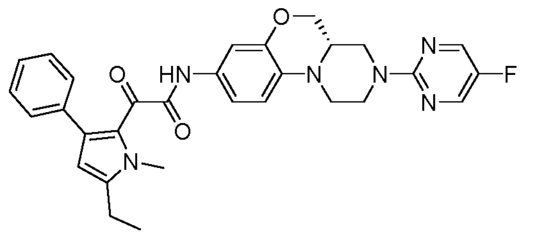

,

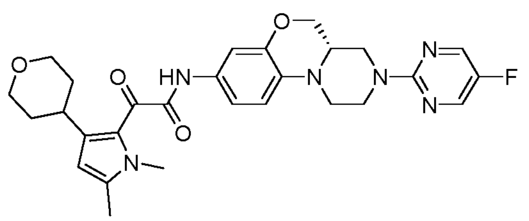

,

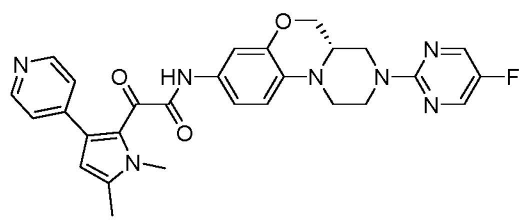

,

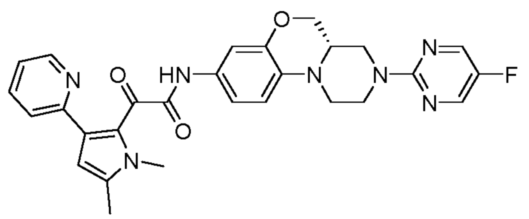

,

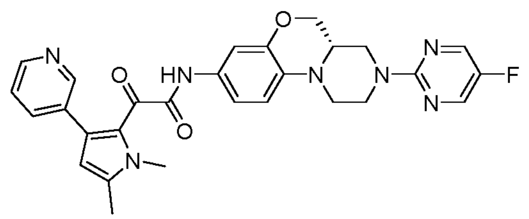

,

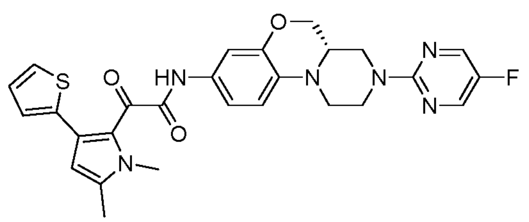

,

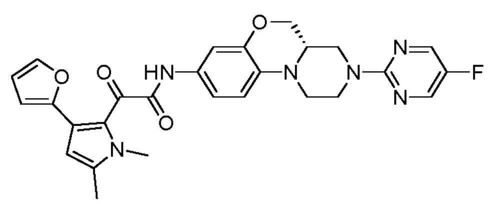

,

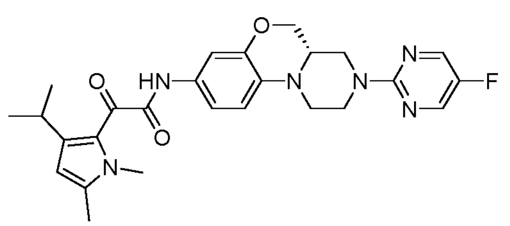

,

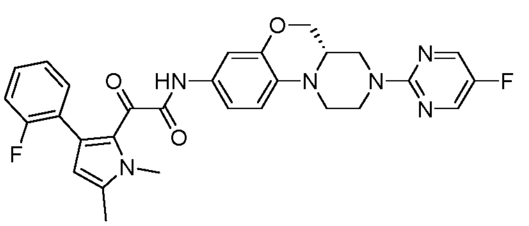

,

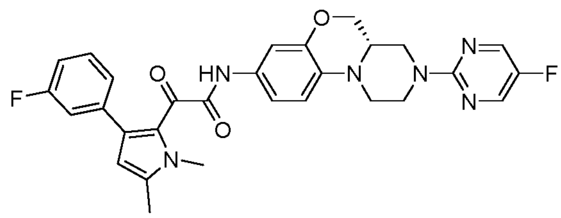

.

**27.** A pharmaceutical composition is **characterized by** that the active ingredients of the composition are selected from one or more combination from the followings: the compounds of stated in any of claims 1 to 26 or their stereoisomers, solvates, hydrates, pharmaceutically acceptable salts, esters, co-crystals, N-oxides, isotopically labeled compounds, metabolites, and pre-drugs.

**28.** The uses of the followings in the preparation of DHODH-inhibitor: the compounds stated in any of claims 1 to 26, their stereoisomers, solvates, hydrates, pharmaceutically acceptable salts or co-crystals.

**29.** The uses of the followings in the preparation of drugs for the treatment or prevention of fungal infections or diseases caused by fungal infections: the compounds stated in any one of claims 1 to 26 or their stereoisomers, solvates, hydrates, pharmaceutically acceptable salts, or co-crystals.

**30.** The uses of the followings in the preparation of fungicide: the compounds stated in any one of claims 1 to 26 or their stereoisomers, solvates, hydrates, pharmaceutically acceptable salts, or co-crystals.

**31.** The uses stated in claim 29 or 30 are **characterized by** that the stated fungus is selected from one or more organisms of the following: Absidia, Acremonium, Alternaria, Aspergillus, Bipolaris, Blastomyces, Blumeria, Cladosporium, Coccidioides, Colletotrichium, Curvularia, Encephalitozoon, Epicoccum, Epidermophyton, Exophiala, Exserohilum, Fusarium, Histoplasma Leptosphaeria, Microsporum, Mycosphaerella, Neurospora, Paecilomyces, Penicillium, Phytophthora, Plasmopara, Pneumocystis, Pyricularia Pythium, Puccinia, Rhizoctonia, Rhizomucor, Scedosporium, Scopula riopsis, Trichophyton, Trichosporon, and Ustilago;
Further, the stated fungus is selected from one or more organisms of the following: Aspergillus, Scedosporium, Fusarium, preferably Aspergillus and/or Scedosporium, more preferably Aspergillus.

**32.** The use stated in claim 31 is **characterized by** that the stated fungus is selected from one or more of the followings: A. fumigatus, A. flavus, A. terreus, A. niger, A. lentulus, S. apiospermum, S. prolificans, and S. species;
Further, the stated fungus is selected from one or more of the followings: A. fumigatus, A. flavus, A. terreus, A. niger, and S. prolificans.

**33.** The use stated in claim 29 is **characterized by** that the fungal-induced diseases stated are selected from diseases of systemic infection with fungi, diseases of superficial infection with fungi.

Further, the stated diseases of systemic infection with fungi are selected from pulmonary aspergillosis, allergic bronchopulmonary aspergillosis, systemic aspergillosis, asthma, coccidioidomycosis, paracoccidioidomycosis, sporotrichosis, chromoblastomycosis, rhinocerebral mucormycosis, blastomycosis, histoplasmosis, lobomycosis, phaeohyphomycosis, disseminated sporotrichosis, fungal colonization of cystic fibrosis, and sinusitis;
The stated diseases of superficial infection with fungi are selected from ringworm, onychomycosis, and tinea pedis.

**34.** The methods of preparing the compounds stated in any one of claims 1 to 26 are **characterized by** that the preparation of intermediate IM-2 or IM-3 are also included;

The synthetic route of IM-2 is as follows:

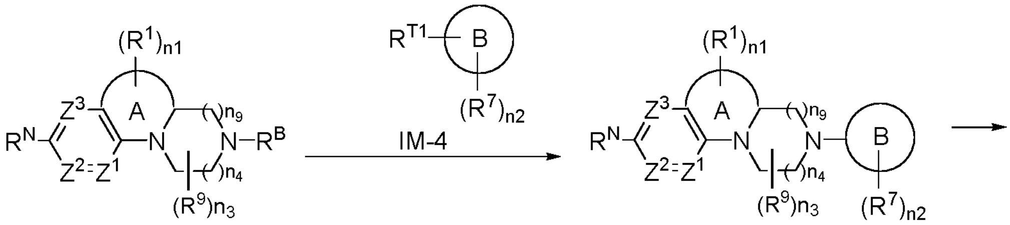


M-9 M-10

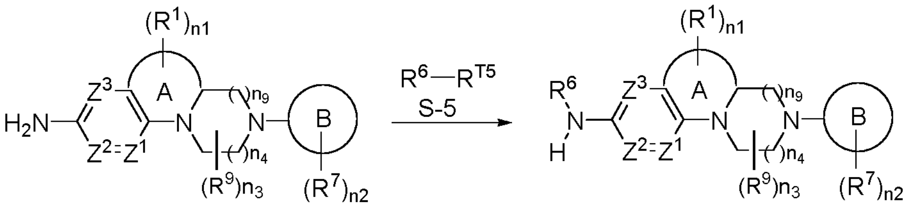


M-11 IM-2

(1) Reaction of compounds M-9 and IM-4 results in M-10 with the presence of a base or transition metal catalyst;

(2) $R^N$ in compound M-10 is transformed to $-NH_2$;

(3) Reaction of M-11 and S-5 generates IM-2 in the presence of a base or reducing agent;

$R^{T1}$, and $R^{T5}$ are independently selected from halogen, aldehyde group, and C1 to C6 alkyl sulfonate group substituted with halogen;

Further, the alkali in step (1) is an inorganic base, preferably potassium carbonate and/or sodium carbonate; the transition metal catalyst in step (1) is palladium catalyst, further selected from one or more of the followings: tetrakis (triphenylphosphine) palladium, palladium acetate, tris (dibenzylideneacetone) dichloropalladium, [1,1'-bis (diphenylphosphino) ferrocene] palladium dichloride, bis (triphenylphosphine) palladium dichloride, and t-BuXPhos-Pd-G3;

Further, IM-2 and IM-1, in the presence of a base or a condensation agent, generate the compounds stated in any one of claims 1 to 26, compound IM-1 having the following structure:

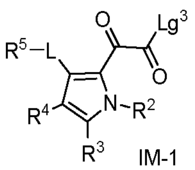

IM-1 ,

wherein $Lg^3$ is selected from leaving group, preferably halogen, C1 to C6 alkoxy, C1 to C6 alkyl sulfonate group substituted by halogen;

The synthetic route of IM-3 is as follows:

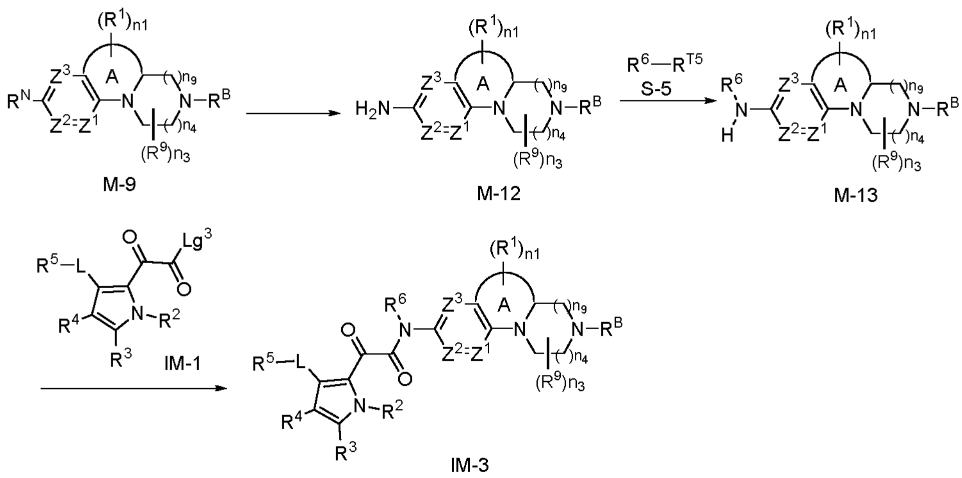


M-9 M-12 M-13

IM-1

IM-3

(a) The $R^N$ in M-9 is transformed to $-NH_2$;

(b) M-12 and S-5, in the presence of a base or reducing agent, generate M-13;

(c) M-13 and IM-1, in the presence of a base or reducing agent, generate IM-3;

$Lg^3$ is selected from leaving groups, preferably halogen, C1 to C6 alkoxy, and C1 to C6 alkyl sulfonate groups substituted with halogen;

Further, the base in step (c) is N, N-diisopropylethylamine; the condensation agent in step (c) is selected from 1-(3-dimethylaminopropyl)-3-ethylcarbodiimide and/or O-(7-azabenzotriazol-1-yl)-N, N, N', N'-tetramethyluronium hexafluorophosphate;

Further, compound IM-3 and compound IM-4, in the presence of a base or transition metal catalyst, generate the compounds stated in any one of claims 1 to 26;

Wherein,

$R^N$ is selected from amino, or groups capable of being transformed to amino group, preferably amino, nitro, carboxyl, ester, halogen, more preferably amino, nitro;

$R^B$ is selected from hydrogen or the protecting groups of amino, further selected from hydrogen, tert-butoxycarbonyl, benzyloxycarbonyl, 9-fluorenylmethoxycarbonyl, benzyl, p-methoxybenzyl, and 2, 4-dimethoxy-benzyl;

$R^1$, $R^2$, $R^3$, $R^4$, $R^5$, $R^6$, $R^7$, $R^9$, L, $Z^1$, $Z^2$, $Z^3$, ring A, ring B, $n_1$, $n_2$, $n_3$, $n_4$, and $n_9$ are as defined in the compounds stated in any one of claims 1 to 26.

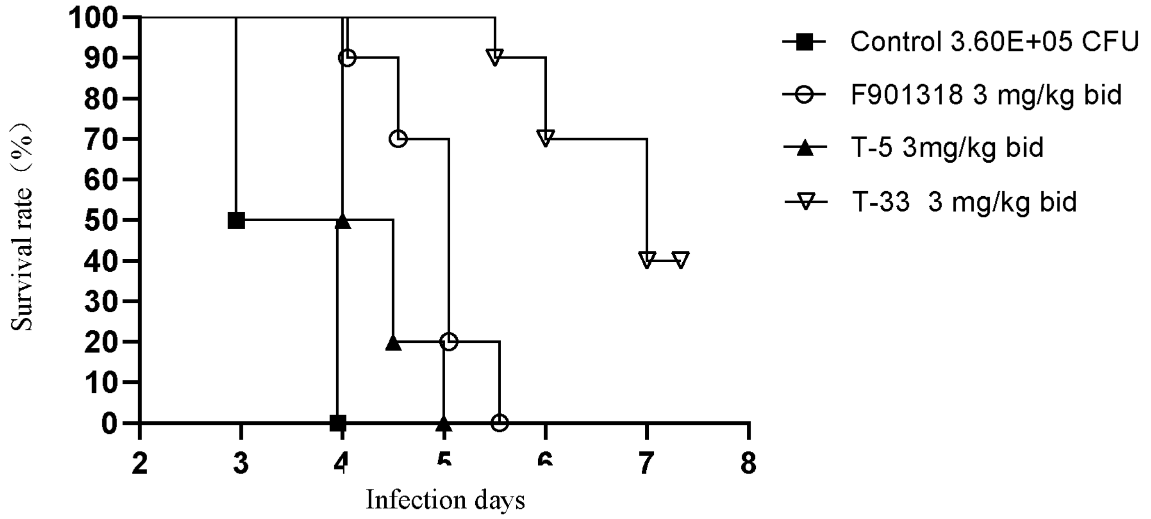


Figure 1

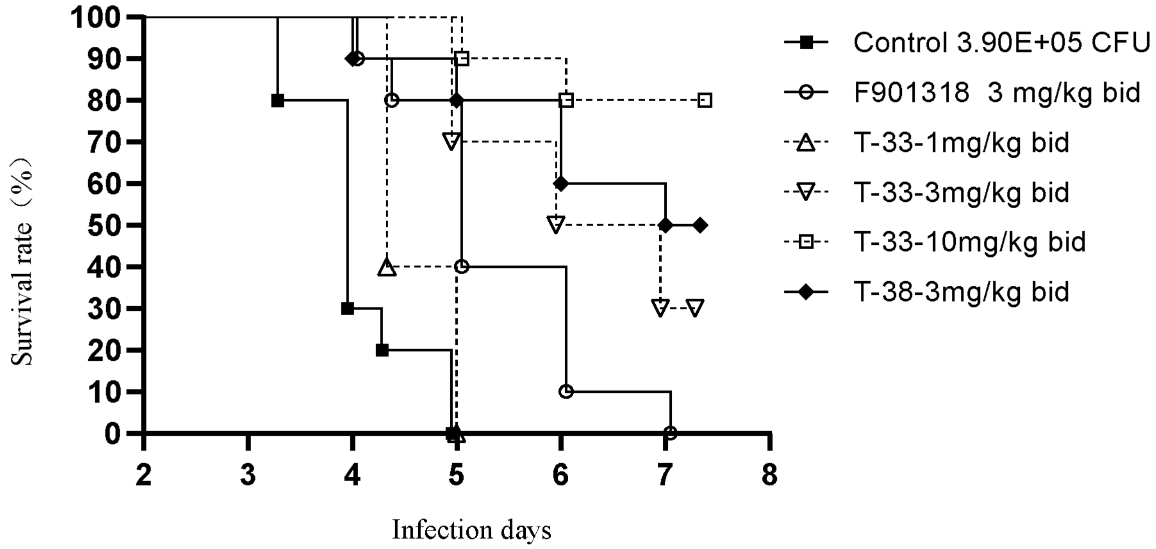


Figure 2

**INTERNATIONAL SEARCH REPORT**

International application No.
**PCT/CN2021/126634**

**A. CLASSIFICATION OF SUBJECT MATTER**

C07D 498/04(2006.01)i; C07D 487/04(2006.01)i; A61K 31/5383(2006.01)i; A61K 31/4985(2006.01)i; A61K 31/506(2006.01)i; A61P 31/10(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

WPI, EPODOC, CNPAT, CAPLUS(STN), REGISTRY(STN): 哌嗪, 吡咯, 嘧啶, 真菌, piperazin? , pyrrol? , pymidin? , DHODH, fung?, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 112898316 A (WIGEN BIOMEDICINE TECHNOLOGY (SHANGHAI) CO., LTD.) 04 June 2021 (2021-06-04)<br>see claims 1-11 | 1-34 |
| PX | WO 2021110010 A1 (WIGEN BIOMEDICINE TECHNOLOGY (SHANGHAI) CO., LTD.) 10 June 2021 (2021-06-10)<br>see claims 1-11 | 1-34 |
| A | WO 2009130481 A1 (F2G LTD et al.) 29 October 2009 (2009-10-29)<br>see description pages 2-4, 37-50, claim 10 and abstract | 1-34 |
| A | CN 101743246 A (F2G LTD.) 16 June 2010 (2010-06-16)<br>see claims 1, 8-19 | 1-34 |
| A | CN 101679410 A (F2G LTD.) 24 March 2010 (2010-03-24)<br>see claims 1, 19-29 | 1-34 |
| A | WO 2006123145 A1 (F2G LTD et al.) 23 November 2006 (2006-11-23)<br>see abstract | 1-34 |
| A | CN 107207470 A (F2G LTD.) 26 September 2017 (2017-09-26)<br>see claims 1, 9, 27-30 | 1-34 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 December 2021** | **26 January 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/126634**

**C. DOCUMENTS CONSIDERED TO BE RELEVANT**

| Category* | Citation of document, with indication, where appropriate, of the relevant passages | Relevant to claim No. |
|---|---|---|
| A | CN 103384470 A (E. I. DU PONT DE NEMOURS AND COMPANY) 06 November 2013 (2013-11-06) see abstract | 1-34 |
| A | CN 108430980 A (SYNGENTA PARTICIPATIONS AG) 21 August 2018 (2018-08-21) see abstract | 1-34 |

Form PCT/ISA/210 (second sheet) (January 2015)

**INTERNATIONAL SEARCH REPORT**
**Information on patent family members**

International application No.
**PCT/CN2021/126634**

| Patent document cited in search report | Publication date (day/month/year) | Patent family member(s) | Publication date (day/month/year) |
|---|---|---|---|
| CN 112898316 A | 04 June 2021 | None | |
| WO 2021110010 A1 | 10 June 2021 | None | |
| WO 2009130481 A1 | 29 October 2009 | DK 2283006 T3 | 26 May 2015 |
| | | PL 2283006 T3 | 31 August 2015 |
| | | US 2015238500 A1 | 27 August 2015 |
| | | US 9452168 B2 | 27 September 2016 |
| | | US 2011152285 A1 | 23 June 2011 |
| | | US 8993574 B2 | 31 March 2015 |
| | | NZ 589010 A | 30 November 2012 |
| | | EP 2283006 A1 | 16 February 2011 |
| | | EP 2283006 B1 | 25 February 2015 |
| | | JP 2015051984 A | 19 March 2015 |
| | | JP 5903475 B2 | 13 April 2016 |
| | | EP 2626361 A1 | 14 August 2013 |
| | | EP 2626361 B1 | 15 October 2014 |
| | | AU 2009239778 A1 | 29 October 2009 |
| | | AU 2009239778 B2 | 13 March 2014 |
| | | JP 2011518818 A | 30 June 2011 |
| | | JP 5657524 B2 | 21 January 2015 |
| | | PT 2283006 E | 29 June 2015 |
| | | ES 2526083 T3 | 05 January 2015 |
| | | ES 2535516 T3 | 12 May 2015 |
| | | DK 2626361 T3 | 19 January 2015 |
| | | CA 2721944 A1 | 29 October 2009 |
| | | CA 2721944 C | 07 June 2016 |
| CN 101743246 A | 16 June 2010 | JP 2010527979 A | 19 August 2010 |
| | | JP 5409610 B2 | 05 February 2014 |
| | | ES 2359187 T3 | 19 May 2011 |
| | | EP 2173749 A1 | 14 April 2010 |
| | | EP 2173749 B1 | 12 January 2011 |
| | | IN 7413DELNP2009 A | 09 July 2010 |
| | | WO 2008145963 A1 | 04 December 2008 |
| | | CA 2687790 A1 | 04 December 2008 |
| | | CA 2687790 C | 18 November 2014 |
| | | CN 101743246 B | 17 July 2013 |
| | | US 2011009390 A1 | 13 January 2011 |
| | | US 8524705 B2 | 03 September 2013 |
| | | AU 2008257196 A1 | 04 December 2008 |
| | | AU 2008257196 B2 | 20 June 2013 |
| | | DK 2173749 T3 | 02 May 2011 |
| | | NZ 581143 A | 29 July 2011 |
| | | KR 20100033383 A | 29 March 2010 |
| | | KR 101595579 B1 | 18 February 2016 |
| | | GB 0710121 D0 | 04 July 2007 |
| | | DE 602008004540 D1 | 24 February 2011 |
| | | AT 495174 T | 15 January 2011 |
| CN 101679410 A | 24 March 2010 | JP 2014074025 A | 24 April 2014 |
| | | JP 5712263 B2 | 07 May 2015 |
| | | JP 2010515666 A | 13 May 2010 |
| | | JP 5372767 B2 | 18 December 2013 |

Form PCT/ISA/210 (patent family annex) (January 2015)

**INTERNATIONAL SEARCH REPORT**
**Information on patent family members**

International application No.
**PCT/CN2021/126634**

| Patent document cited in search report | Publication date (day/month/year) | Patent family member(s) | Publication date (day/month/year) |
|---|---|---|---|
| | | CN 101679410 B | 27 November 2013 |
| | | AU 2007324385 A1 | 29 May 2008 |
| | | AU 2007324385 B2 | 21 March 2013 |
| | | US 2010056511 A1 | 04 March 2010 |
| | | US 8604029 B2 | 10 December 2013 |
| | | EP 2097413 A1 | 09 September 2009 |
| | | EP 2097413 B1 | 25 December 2013 |
| | | DK 2097413 T3 | 24 March 2014 |
| | | KR 20090093998 A | 02 September 2009 |
| | | KR 101527615 B1 | 09 June 2015 |
| | | CA 2670165 A1 | 29 May 2008 |
| | | CA 2670165 C | 18 November 2014 |
| | | WO 2008062182 A1 | 29 May 2008 |
| | | ES 2451659 T3 | 28 March 2014 |
| | | GB 0623209 D0 | 03 January 2007 |
| | | NZ 577072 A | 28 October 2011 |
| | | IN 1013MUMNP2009 A | 12 November 2010 |
| | | IN 263734 A1 | 21 November 2014 |
| WO 2006123145 A1 | 23 November 2006 | AT 420639 T | 15 January 2009 |
| | | ES 2321230 T3 | 03 June 2009 |
| | | EP 1888063 A1 | 20 February 2008 |
| | | EP 1888063 B1 | 14 January 2009 |
| | | DE 602006004865 D1 | 05 March 2009 |
| | | DK 1888063 T3 | 11 May 2009 |
| | | JP 2008545647 A | 18 December 2008 |
| | | JP 5172661 B2 | 27 March 2013 |
| | | US 2008161302 A1 | 03 July 2008 |
| | | US 8486962 B2 | 16 July 2013 |
| | | NZ 563397 A | 30 April 2010 |
| | | CA 2608977 A1 | 23 November 2006 |
| | | CA 2608977 C | 23 September 2014 |
| | | AU 2006248812 A1 | 23 November 2006 |
| | | AU 2006248812 B2 | 16 February 2012 |
| CN 107207470 A | 26 September 2017 | EP 3415511 A1 | 19 December 2018 |
| | | US 2017340607 A1 | 30 November 2017 |
| | | US 10201524 B2 | 12 February 2019 |
| | | EP 3221308 A1 | 27 September 2017 |
| | | EP 3221308 B1 | 19 September 2018 |
| | | ES 2694637 T3 | 26 December 2018 |
| | | IL 252368 D0 | 31 July 2017 |
| | | IL 252368 A | 30 April 2020 |
| | | BR 112017010439 A2 | 26 December 2017 |
| | | US 2019314333 A1 | 17 October 2019 |
| | | US 10596150 B2 | 24 March 2020 |
| | | SI 3221308 T1 | 31 December 2018 |
| | | LT 3221308 T | 27 December 2018 |
| | | MX 2017006447 A | 30 January 2018 |
| | | RU 2017121370 A | 21 December 2018 |
| | | RU 2017121370 A3 | 28 March 2019 |
| | | RU 2727520 C2 | 22 July 2020 |

Form PCT/ISA/210 (patent family annex) (January 2015)

**INTERNATIONAL SEARCH REPORT**
**Information on patent family members**

International application No.
**PCT/CN2021/126634**

| Patent document cited in search report | Publication date (day/month/year) | Patent family member(s) | Publication date (day/month/year) |
|---|---|---|---|
| | | US 2020179340 A1 | 11 June 2020 |
| | | US 11065228 B2 | 20 July 2021 |
| | | CA 2982660 A1 | 26 May 2016 |
| | | HR P20182139 T1 | 03 May 2019 |
| | | PT 3221308 T | 17 December 2018 |
| | | AU 2015348043 A1 | 08 June 2017 |
| | | AU 2015348043 B2 | 22 August 2019 |
| | | CN 111096967 A | 05 May 2020 |
| | | CY 1120907 T1 | 11 December 2019 |
| | | JP 2017536371 A | 07 December 2017 |
| | | JP 6681894 B2 | 15 April 2020 |
| | | WO 2016079536 A1 | 26 May 2016 |
| | | DK 3221308 T3 | 10 December 2018 |
| | | KR 20170102241 A | 08 September 2017 |
| | | PL 3221308 T3 | 29 March 2019 |
| CN 103384470 A | 06 November 2013 | None | |
| CN 108430980 A | 21 August 2018 | US 2018370927 A1 | 27 December 2018 |
| | | WO 2017109044 A1 | 29 June 2017 |
| | | EP 3394041 A1 | 31 October 2018 |
| | | BR 112018012825 A2 | 04 December 2018 |
| | | JP 2019504828 A | 21 February 2019 |

Form PCT/ISA/210 (patent family annex) (January 2015)